# EUROPEAN PATENT SPECIFICATION

(11) **EP 0 837 852 B1**
(45) Date of publication and mention of the grant of the patent: **24.01.2001**
(21) Application number: 96917483.8
(22) Date of filing: 31.05.1996
(51) Int. Cl.: C07D 231/44, A01N 43/56

(54) **PESTICIDAL SULFUR COMPOUNDS**
PESTIZIDE SCHWEFELVERBINDUNGEN.
COMPOSES DE SOUFRE PESTICIDES

(30) Priority: 05.06.1995 US 464372
(43) Date of publication of application: 29.04.1998
(73) Proprietor: AVENTIS CROPSCIENCE S.A., 69009 Lyon (FR)
(72) Inventor: LOWDER, Patrick, Doyle, Raleigh, NC 27607 (US); MANNING, David, Treadway, Cary, NC 27511 (US); PHILLIPS, Jennifer, Lantz, Apex, NC 27502 (US); PILATO, Michael, Thomas, Cary, NC 27513 (US); WU, Tai-Teh, Chapel Hill, NC 27514 (US)
(86) International application number: EP9602363
(87) International publication number: WO9639389

(56) References cited:
- EP-A- 0 201 852
- GB-A- 1 603 122

## Description

The present invention relates to new sulfur compounds, including sulfilimines and sulfoximines intermediates thereto, and processes to prepare the compounds. The invention further pertains to compositions of said compounds and methods, using said compounds, for the control of arthropod, nematode, helminth or protozoan pests. In particular, it pertains to the application of compounds or compositions thereof in agricultural methods of use, particularly as pesticides.

In the instant specification, the word sulfilimine is used to name compounds comprising the group 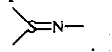 In the instant specification, the word sulfoximine is used to name compounds comprising

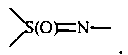

Various pesticidal pyrazoles, pyrroles, and imidazoles have been disclosed; European Patent Publication No 418016; European Patent Publication No 403309; U.S. Patent 5,104,994; European Patent Publication No 352944; U.S. Patent 5,079,370; U.S. Patent 5,047,550; U.S. Patent 5,232,940; U.S. Patent 4,810,720; U.S. Patent 4,804,675; U.S. Patent 5,306,694; U.S. Patent 4,614,533; WPO Publication No. WO 93/06089; and WPO Publication No. WO 94/21606 describe pesticidal (4-pyrazolyl) sulfides, sulfoxides and sulfones. U.S. Patent 5,187,185 describes 4-pyrrolyl sulfides, sulfoxides and sulfones. U.S. Patent 5,223,525 describes pesticidal 4-imidazolyl sulfides, sulfoxides and sulfones.

Due to the many existing pests and crops and conditions of attacks of crops by pests, there is a need for further novel pesticidal compounds.

In one aspect, the present invention provides compounds for use in controlling arthropod, nematode, helminth and protozoan pests, said compounds having the general formula: 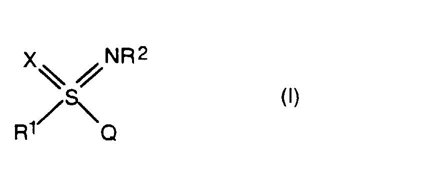 or 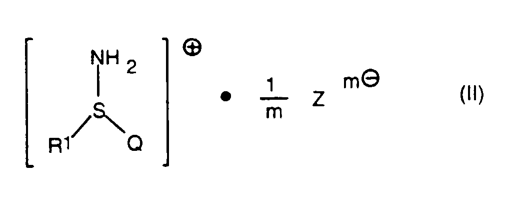 wherein:
=X is =NR³, =O or an electron pair (it being understood that, in this situation, another way to represent Formula (I) is to delete =X);
R¹ is C₁-C₆ alkyl, C₃-C₆ cycloalkyl, or C₄-C₈ (cycloalkyl)alkyl, each of which is optionally substituted by one or more halogen;
R² and R³ are independently selected from H; C₁-C₆ alkyl; C₁-C₆ haloalkyl; COR⁴; S(O)ₚR⁴; CN; NO₂; COOR⁴; CONR⁴R⁵; C(O)SR⁴; C(S)OR⁴; SO₂NR⁴R⁵; P(O)_{q}(R⁴)(R⁵); P(O₎q(OR⁴)(R⁵); P(O)_{q}(OR⁴)(OR⁵); C=(NR⁴)NR⁵R⁶; CH=NR⁴; C=(NR⁴)(OR⁵); C(S)N(R⁴)(R⁵); C(O)C(O)R⁴; C(O)C(O)OR⁴; C(O)C(O)NR⁴R⁵; and CONR⁴SO₂R⁵;
m is 1 or 2;
p is 0, 1 or 2;
q is 0 or 1;
R⁴, R⁵ and R⁶ are independently selected from H; NO₂; CN; CHO; R¹⁴; phenyl optionally substituted by one or more of R¹⁴, halogen, CN, NO₂, OR¹⁴, SR¹⁴, COR¹⁴, COOR¹⁴, or OR¹⁴; halogen; COR¹⁴; COOR¹⁴; CHO; and OH;
Q is Q-1, Q-2 or Q-3, as designated below: 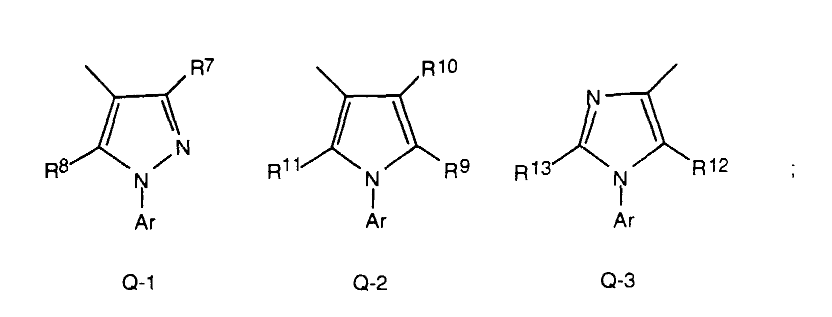
R⁷ is CN, NO₂, H, C₁-C₄ alkyl, C₁-C₄ haloalkyl, halogen, CHO, or COR²⁰;
R⁸ is H, halogen, CN, S(O)ₚR¹⁴, OR⁴, NR¹⁶R¹⁷, N(R¹⁶)CON(R¹⁷)(R¹⁸), N₃ or NH(C₁-C₅ alkyl) substituted by one or more OH, OR¹⁴, S(O)ₚR¹⁴, CN, NO₂, COOR¹⁴ or CON(R¹⁶)(R¹⁷);
R⁹ and R¹¹ are independently selected from H, halogen, OR¹⁴, SR¹⁴ and R¹⁴;
R¹⁰ is CN;
R¹² is H, halogen, R¹⁴, OR¹⁴, S(O)ₚR¹⁴ or 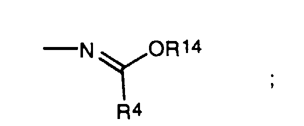
R¹³ is H, halogen or R¹⁴;
Ar is phenyl, optionally bearing one or more substituents selected from the group consisting of halogen, R¹⁵, OR¹⁵, SF₅ and S(O)ₚR¹⁵; or Ar is 2-pyridyl, optionally bearing one or more substituents selected from the group consisting of halogen, R¹⁵, OR¹⁵, SF₅ and S(O)ₚR¹⁴;
R¹⁴ and R¹⁹ are independently selected from C₁-C₆ alkyl, C₃-C₆ cycloalkyl, and C₄-C₈ (cycloalkyl)alkyl, each of which is optionally substituted by one or more halogen;
R¹⁵ is C₁-C₆ alkyl, optionally substituted by one or more halogen;
R¹⁶, R¹⁷ and R¹⁸ are independently selected from H, NO₂, CN, CHO, R¹⁹, COR¹⁹, and COOR¹⁹;
R²⁰ is selected from C₁-C₆ alkyl, optionally substituted by one or more halogen; and
Z is an anionic counter ion, such as Cl⁻, Br⁻, I⁻, F⁻, OSO₂R⁴⁻, ClO₄⁻, OCOR⁴⁻, BF₄⁻, SbF₆⁻, SO₃⁻⁻ or HSO₄⁻, a phosphate anion or a hydrogenophosphate anion or other agriculturally acceptable anion.

The stereoisomers, *e.g*. diastereomers and optical isomers, having the Formula (I) or (II) are included in the invention as well.

Preferred compounds of the present invention include one or more of the following features:
(1) R¹ is optionally halogenated C₁-C₆ alkyl, preferably methyl or ethyl; and/or
(2) R² is H, COOR⁴, S(O)ₚR⁴, CONR⁴R⁵, CN, COR⁴, P(O)_{q}(OR⁴)(OR⁵) or P(O)_{q}(R⁴)(R⁵); more preferably R² is H; or CN; or COOR⁴ (in which R⁴ is C₁-C₆ alkyl); or R² is S(O)ₚR⁴ (in which p is two and R⁴ is C₁-C₆ alkyl or optionally substituted phenyl); or R² is CONR⁴R⁵ (in which R⁴ is H or C₁-C₆ alkyl and R⁵ is C₁-C₆ alkyl, or in which R⁴ is H and R⁵ is COR¹⁴ in which R¹⁴ is optionally halogenated C₁-C₆ alkyl); or R² is COR⁴ (in which R⁴ is H or C₁-C₆ alkyl); or R² is P(O)_{q}(OR⁴)(OR⁵) or P(O)_{q}(R⁴)(R⁵) (in which q is one and R⁴ and R⁵ are each C₁-C₆ alkyl);and/or
(3) Q is Q-1,preferably wherein R⁷ is CN or H, or C₁-C₄ alkyl and/or when R⁸ is N(R¹⁶)(R¹⁷), most preferably when R⁸ is NH₂;and/or
(4) Ar is phenyl substituted in the 2 and 6 positions by halogen and in the 4 position by halogenated C₁-C₆ alkyl or SF₅ or OR¹⁵, preferably when Ar is 2,6-dichloro-4-trifluoromethylphenyl; or Ar is 2-pyridyl substituted in the 3 position by halogen and in the 5 position by halogenated C₁-C₆ alkyl or SF₅ or OR¹⁵, preferably when Ar is 3-chloro-5-trifluoromethylpyrid-2-yl.

In another aspect, the present invention provides a pesticidal composition *(i.e.* an arthropodicidal, nematocidal, anti-helminth or anti-protozoal composition) comprising a pesticidally effective amount *(i.e.* an arthropodicidally effective amount, a nematocidally effective amount, an effective anti-helminth amount or an effective anti-protozoal amount) of a compound of formula (I) or (II) and an agriculturally acceptable inert carrier therefor. The expression "a compound of formula (I) or (II)" used here and throughout this application includes within its ambit the various stereoisomeric forms of the compounds of formulas (I) and (II) and salts thereof.

In yet another aspect, the invention provides a method for controlling arthropod, nematode, helminths or protozoan pests at a locus, said method comprising applying to said locus a pesticidically effective amount *(i.e.* an arthropodicidally or nematocidally effective amount or an effective anti-helminth or anti-protozoal amount) of a compound of formula (I) or (II) or of a pesticidal composition as defined above.

Compounds of Formula (I) wherein X is O and R² is not H are prepared from compounds of Formula (I) wherein X is an electron pair and R² is not H said compounds of Formula (I) also being part of the invention] by oxidation of a sulfur atom into a sulfoximine group according to any method known *per se* by those skilled in the art. Such transformations can be made by various oxidizing reagents, including, but not limited to, potassium permanganate, sodium periodate and ruthenium tetroxide. This transformation can take place in common solvents such as water, ethers *e.g.* dioxane, nitriles *e.g.* acetonitrile, or halohydrocarbons such as dichloromethane, chloroform, carbontetrachloride and the like, at temperatures between about -100°C and about 100°C, preferably between about 0°C and about 50°C. Such chemistry can be similar to that found in *Tetrahedron, 1975,* **31**, 505.

Compounds of Formula (I) in which X is an electron pair and R² is other than H can be prepared from compounds of Formula (II) by reaction of an appropriate electrophile R²-L in the presence of a base. R² has the hereinabove-given meaning except that it is not H, and L is a leaving group. Examples of leaving groups L are halides, acetate, phenyl sulfonates, alkyl sulfonates and phenoxy groups. Examples of bases which can be used in the reaction are sodium hydride, triethylamine and potassium tert-butoxide.

Such reactions can take place in a variety of common solvents, such as, but not limited to, ethers such as tetrahydrofuran or dioxane; water; halohydrocarbons such as dichloromethane or chloroform; nitriles such as acetonitrile; and the like. Such reactions can take place at temperatures between about -100°C and about 100°C, but preferably between about 0°C and the boiling temperature of the solvent.

Compounds of Formula (I) in which X is an electron pair and in which R² is H can be prepared from the corresponding compounds of Formula (I) in which R² is an ester grouping COOR¹⁴, by conventional hydrolysis.

Compounds of Formula (II) can be prepared from compounds of Formula (III) by amination according to a reaction known *per se* to those skilled in the art.

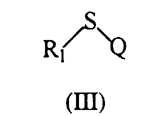

Such chemistry can occur upon mixing a sulfide of Formula (III) with an aminating reagent such as, but not limited to, an O-benzoylhydroxylamine, *e.g.* O-(2,4,6-trimethylbenzoyl)hydroxylamine or O-(3-chlorobenzoyl)hydroxylamine; an O-sulfonylhydroxylamines such as, but not limited to, hydroxylamine sulfonic acid or O-(2,4,6-triisopropylbenzenesulfonyl)hydroxylamine; or an O-phosphinic hydroxylamine, such as O-(diphenylphosphinyl)hydroxylamine. This can take place in a variety of solvents, including, but not limited to, water, halogenated hydrocarbons such as dichloromethane, chloroform, dichloroethane and the like, and aromatic solvents like benzene, toluene, xylenes and the like, and can take place at a temperature between about -100°C and about 100°C, 0°C to the boiling point of the solvent being preferred. Such chemistry may be similar to that found in *Aldrichimica Acta,* 1980, **13**(1)3.

Compounds of Formula (I) wherein X is NH and R² is H can be prepared from compounds of Formula (III) by reaction with chloramine [*Tetrahedron, 1970,* **51**, 4449]. Such a reaction can take place in ammonia at a temperature between about -100°C and -33°C.

Compounds of Formula (I) wherein =X is =NR³ (wherein R³ is as defined above except that it is not hydrogen) and wherein R² is H, can be derived from compounds of Formula (I) wherein =X is NH and R² is H, by reaction of an appropriate electrophile R³-L in the presence of a base. R³ is as defined above except that it is not H, and L is a leaving group. Examples of leaving groups L are halides, acetate, phenyl sulfonates, alkyl sulfonates and phenoxy groups. Examples of bases which can be used in the reaction are sodium hydride, triethylamine and potassium tert-butoxide.

Such reactions can take place in a variety of common solvents, such as, but not limited to, ethers such as tetrahydrofuran or dioxane; water; halohydrocarbons such as dichloromethane or chloroform; nitriles such as acetonitrile; and the like. Such reactions can take place at temperatures between about -100°C and about 100°C, but preferably between about 0°C and the boiling temperature of the solvent. Similar reactions are found in *Chem. Ber., 1984,* **117**, 2779.

Compounds of Formula (I) wherein =X is =NR³ wherein R² and R³ are the same and neither is hydrogen can be derived from compounds of Formula (I) wherein R² is H and X is NH by reaction with at least two equivalents of an appropriate electrophile R³-L in the presence of a base. R³ has the hereinabove-given meaning except that it is not H, and L is a leaving group. Examples of leaving groups L are halides, acetate, phenyl sulfonates, alkyl sulfonates and phenoxy groups. Examples of bases which can be used in the reaction are sodium hydride, triethylamine and potassium tert-butoxide.

Such reactions can take place in a variety of common solvents, such as, but not limited to, ethers such as tetrahydrofuran or dioxane; water; halohydrocarbons such as dichloromethane or chloroform; nitriles such as acetonitrile; and the like. Such reactions can take place at temperatures between about -100°C and about 100°C, but preferably between about 0°C and the boiling temperature of the solvent. Similar reactions are found in *Lieb. Ann. Chem., 1972*, **759**, 107.

Compounds of Formula (III) are known in the art. For example, those wherein Q is Q-1 are described in European Patent Publication No 418016; European Patent Publication No 403309; U.S. Patent 5,104,994; European Patent Publication No 352944; U.S. Patent 5,079,370; U.S. Patent 5,047,550; U.S. Patent 5,232,940; U.S. Patent 4,810,720; U.S. Patent 4,804,675; U.S 5,306,694; U.S. Patent 4,614,533; WPO Publication No. 93/06089 and WPO Publication No. 94/21606. The compounds of Formula (III) wherein Q is Q-2 are described in U.S. Patent 5,187,185. Compounds of Formula (III) wherein Q is Q-3 are described in U.S. Patent 5,223,525.

In the many transformations hereinabove described, it is apparent that selected substituents may occasionally interfere in the contemplated reactions. Such undesired effects can be avoided by using appropriate protecting groups to prevent the unwanted side reactions. It is also possible to use reagents that do not affect functional groups other than those desired to be changed. The particular choice of the appropriate protecting groups and reagents will be easily understood by those skilled in the art. The use of *Chemical Abstracts* is considered and suggested as part of the knowledge of the person skilled in the art.

In order to further illustrate the present invention and the advantages thereof, the following specific examples are given, it being understood that these examples are for the purpose of illustration only and in no way limit the scope of the invention.

### EXAMPLE 1

### 2,4,6-Trimethylbenzenesulfonic acid, compound with S-4-[5-amino-3-cyano-1-(2,6-dichloro-4-trifluoromethyl)phenyl]pyrazolyl-S-ethylsulfilimine:

Ethyl O-mesitylene sulfonylacetohydroxamate (10 g, 0.035 mol) was dissolved in dioxane (10 mL) and cooled to 0°C. 70% Perchloric acid (3.3 mL, 0.038 mol) was added and the mixture was stirred for 10 minutes. Water (200 mL) was added and the remaining solid was filtered, washed with water, then dissolved in methylene chloride (50 mL) and separated from the aqueous phase.

5-Amino-3-cyano-1-(2,6-dichloro-4-trifluoromethyl)phenyl-4-ethylthiopyrazole (10.7 g, 0.028 mol) was dissolved in methylene chloride (50 mL) and the resulting solution was cooled to 0°C. The solution from above was added. After stirring for 1 hr at 0°C and then at room temperature, methylene chloride was removed and diethyl ether was added (200 mL). The resulting solid was filtered to provide the title compound as a white solid (12.45 g, 75%), m.p. about 193°C.

In a similar manner, the following were prepared: 2,4,6-Trimethylbenzene-sulfonic acid, compound with S-4-[5-amino-3-cyano-1-(2,6-dichloro-4-trifluoromethyl)phenyl]pyrazolyl-S-methylsulfilimine, m.p. about 180°C hereinafter know as EXAMPLE la; 2,4,6-Trimethylbenzenesulfonic acid, compound with S-4-[5-amino-1-(2,6-dichloro-4-trifluoromethyl)phenyl]pyrazolyl-S-methylsulfilimine, m.p. about 187°C C hereinafter know as EXAMPLE 1b.

The following two compounds were also prepared by the above method, but were not isolated, but used as in EXAMPLE 2: 2,4,6-Trimethylbenzenesulfonic acid, compound with S-4-[5-amino-1-(2,6-dichloro-4-trifluoromethyl)phenyl-3-methyl]pyrazolyl-S-ethylsulfilimine and 2,4,6-Trimethylbenzenesulfonic acid, compound with S-4-[5-amino-1-(2,6-dichloro-4-trifluoromethyl)phenyl-3-ethyl]pyrazolyl-S-ethylsulfilimine.

### EXAMPLE 2

### S-{[5-Amino-3-cyano-1-(2,6-dichloro-4-trifluoromethyl)phenyl]-4-(1H-pyrazolyl)}-S-ethyl-N-(tert-butoxycarbonyl)sulfilimine:

The product of EXAMPLE 1 (1.0 g, 1.7 mmol) was stirred in methylene chloride (10 mL), cooled to 0°C, and triethylamine (1.2 mL, 8.5 mmol) was added. Di-tert-butyldicarbonate (0.6 mL, 2.6 mmol) was added and the reaction was stirred 50 minutes. The mixture was diluted with diethyl ether and washed with water (3 x 50 mL) and saturated aqueous sodium chloride solution (75 mL). The organic layer was dried, filtered and concentrated. The resulting oil was mixed with cold pentane and the precipitate was filtered. The solid was recombined with the filtrate and solvent was removed and the oil filtered through silica gel using 2:3 ethyl acetate:hexane as eluent. After removal of solvent, precipitation from diethyl ether/pentane provided the title compound as a white solid (0.36 g, 43%), m.p. about 191°C.

In a similar manner, the following compounds were prepared (**TABLE 1**).

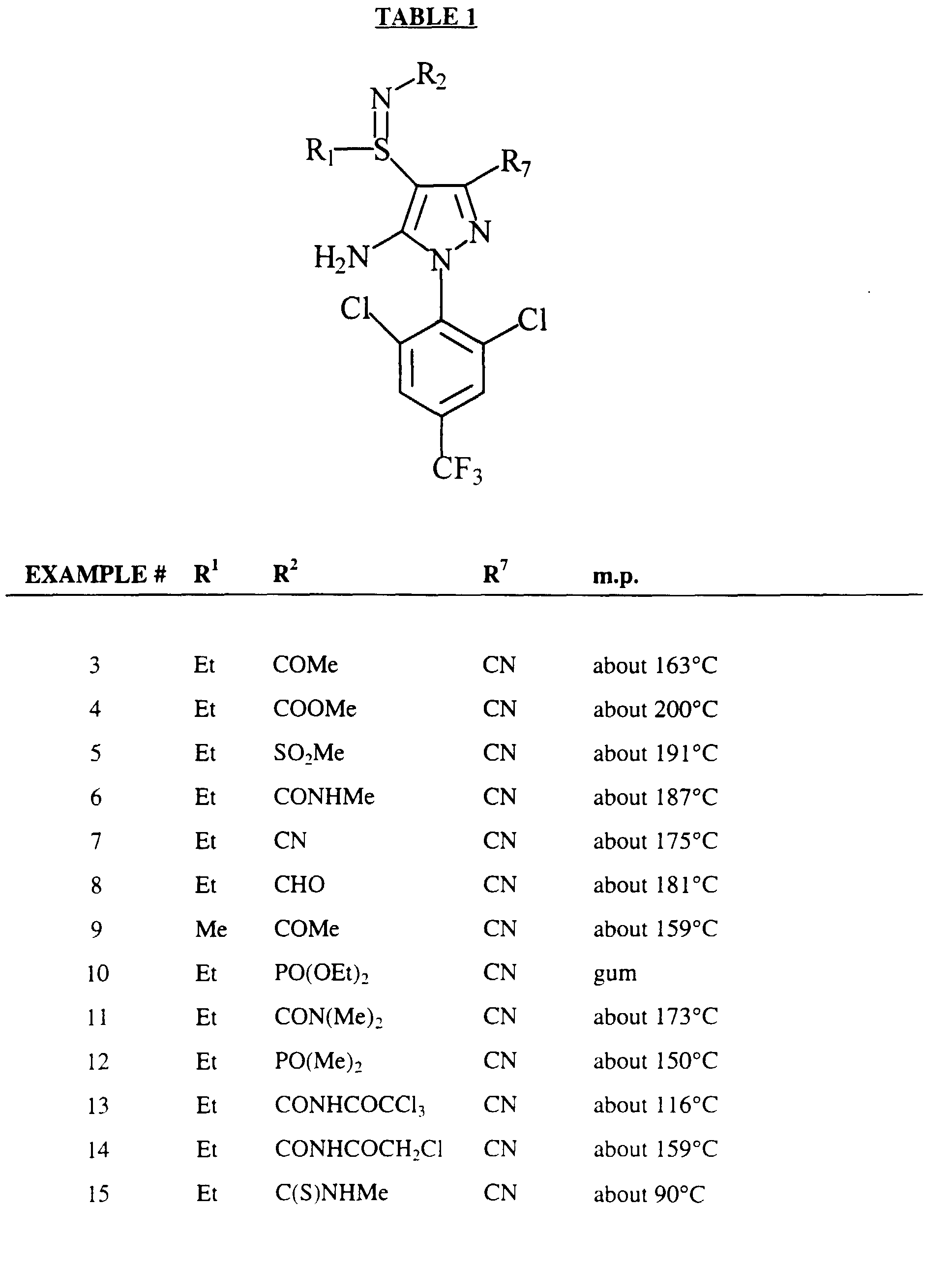

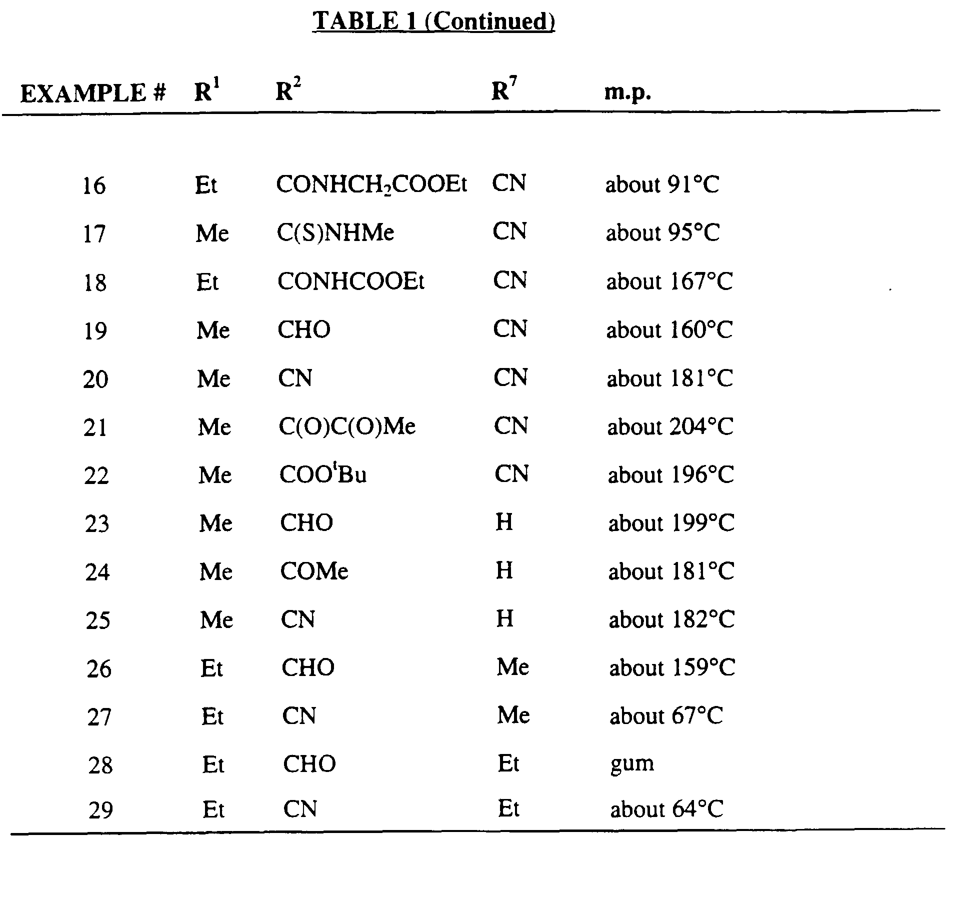

### EXAMPLE 30

### N-(tert-Butoxycarbonyl)-S-4-[5-amino-3-cyano-1-(2,6-dichloro-4-trifluoromethyl)phenylpyrazolyl]-S-ethylsulfoximine:

The product of EXAMPLE 2 (1.55 g, 31 mmol) was dissolved in a 1:1 mixture of acetonitrile:carbon tetrachloride (19 mL). Sodium metaperiodate (2.7 g, 12.4 mmol) was added as a solution in water (19 mL). Ruthenium (III) chloride hydrate (20 mg; used to make the tetroxide *in situ*) and acetonitrile (25 mL) were added. After 2 hours, more sodium periodate (1.8 g, 8.2 mmol) and ruthenium (III) chloride hydrate (15 mg; forming the oxide *in situ)* were added and the reaction was stirred for 30 minutes. Then water (100 mL) and diethyl ether (100 mL) and isopropanol (2 mL) were successively added and mixed. Separation of the organic phase was followed by washes with water (100 mL) and saturated aqueous sodium chloride solution (100 mL). After drying over magnesium sulfate, the mixture was filtered and the solvent was removed to provide an oily residue which was triturated with cold diethyl ether and methylene chloride to provide the title compound as a brown, gummy solid (0.94 g, 60%). The proton nuclear magnetic resonance spectrum made in CDCl₃ has the following chemical shifts: 7.81 (m, 2H); 5.57 (brs, 2H); 3.7 - 3.5 (m, 2H); 1.44 (s, 9H); 1.39 (t, 3H).

In a similar manner, N-Acetyl-S-4-[5-amino-3-cyano-1-(2,6-dichloro-4-trifluoromethyl)phenylpyrazolyl]-S-ethylsulfoximine (m.p. about 217°C) was prepared, hereinafter known as EXAMPLE 30a.

### EXAMPLE 31

### S-4-[5-Amino-3-cyano-1-(2,6-dichloro-4-trifluoromethyl)phenylpyrazolyl]-S-ethylsulfoximine:

The product of EXAMPLE 30 (0.1 g, 0.2 mmol) was dissolved in 2 mL of acetonitrile. Powdered molecular sieves (0.1 g), sodium iodide (0.07 g, 0.46 mmol) and chlorotrimethylsilane (0.06 mL, 0.46 mmol) were combined in 2 mL acetonitrile and the above solution added to it. After 25 minutes, the mixture was diluted with diethyl ether (7 mL) and sequentially washed with water, 10% aqueous hydrochloric acid solution and saturated aqueous sodium chloride solution. The organic phase was dried over sodium sulfate, filtered and concentrated under reduced pressure. The residual oil was triturated with cold pentane and dichloromethane to leave the desired product as a yellow solid (46 mg) having a melting point of about 86°C.

### EXAMPLE 32

### S-[5-Amino-3-cyano-1-(2,6-dichloro-4-trifluoromethyl)phenyl-4-(1H-pyrazolyl)]-S-methyl-N-[(4-methylphenyl)sulfonyl]sulfilimine:

To a stirred solution of 5-amino-1-(2,6-dichloro-4-trifluoromethyl)phenyl-4-methylthio-1H-pyrazole-3-carbonitrile (0.99 g, 2.7 mmol) in acetonitrile (25 mL) was added an aqueous solution of N-chloro-p-toluenesulfonamide, sodium salt, hydrate (Chloramine-T hydrate) (0.61 g, 2.7 mmol) in water (5 mL), over a 2-minute interval. Stirring under ambient conditions was continued for approximately 2 hours and the mixture then heated to boiling on a steam bath for 1 hour. Volatiles were removed under reduced pressure and the residue chromatographed on silica gel, eluting with 3:1 ethyl acetate/hexane, to give 0.81 g of the title compound as a solid having a melting point of about 197°C.

### EXAMPLE 33

### S-4-[5-Amino-3-cyano-1-(2-(3-chloro-5-trifluoromethyl)pyridyl)pyrazolyl]-S-ethylsulfoxide

Ethyl O-mesitylene sulfonylacetohydroxamate (0.47 g, 1.64 mmol) was dissolved in dioxane (1 mL) and cooled to 0°C. 70% Perchloric acid (0.5 mL) was added and the mixture was stirred for 10 minutes. Water was added and the remaining solid was filtered, washed with water, then dissolved in methylene chloride and separated from the aqueous phase.

5-Amino-3-cyano-1-(2-(3-chloro-5-trifluoromethyl)pyridyl)-4-ethylsulfinylpyrazole (0.5 g, 1.37 mmol) was dissolved in methylene chloride (25 mL) and the resulting solution was cooled to 0°C. The solution from above was added. After one month, the mixture was concentrated to a solid and washed repeatedly until no starting pyrazole remained. This provided the title compound as a white solid, m.p. around 193°C.

Further illustrative specific compounds of the invention are set forth below in **TABLES 2-5** below. Throughout these tables, the notation "^{..}" is used to indicate a lone electron pair.

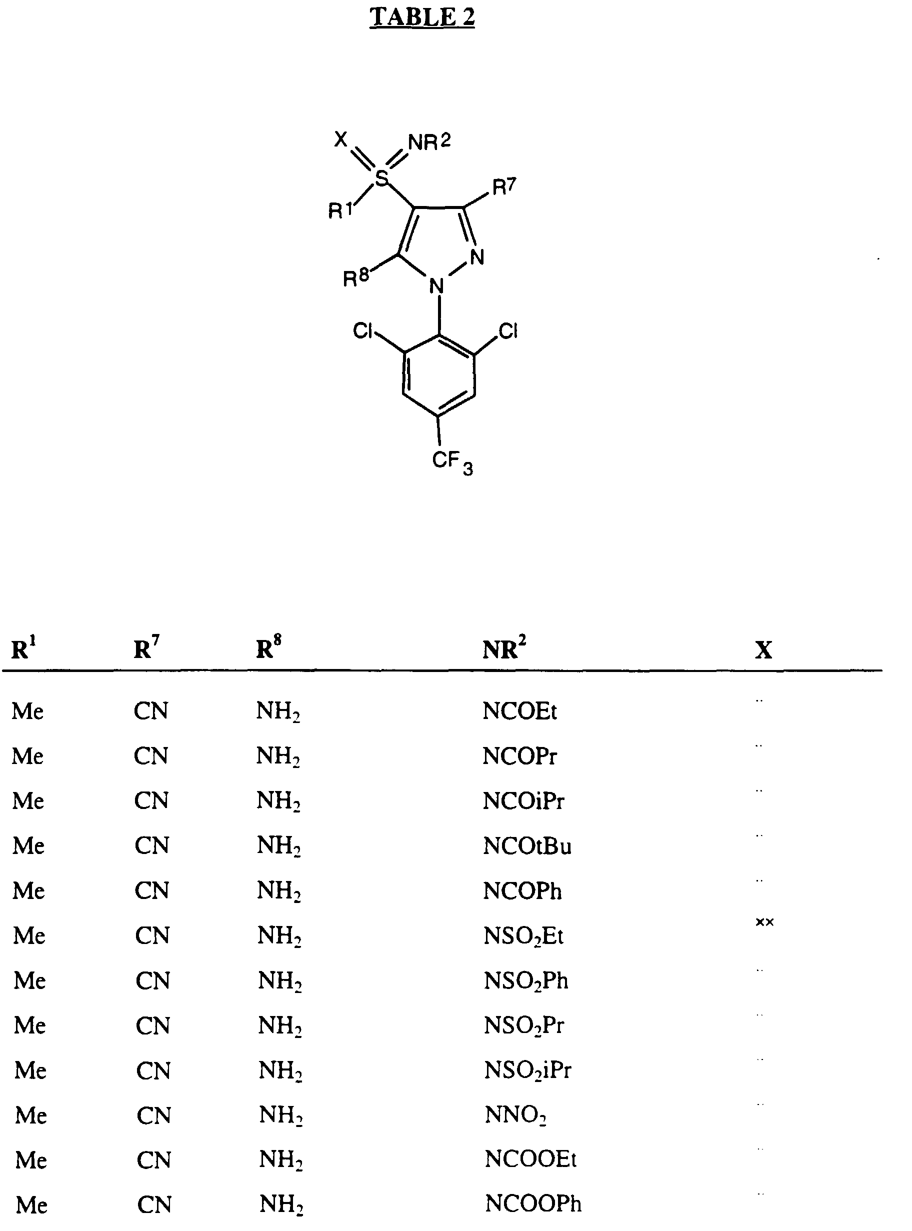

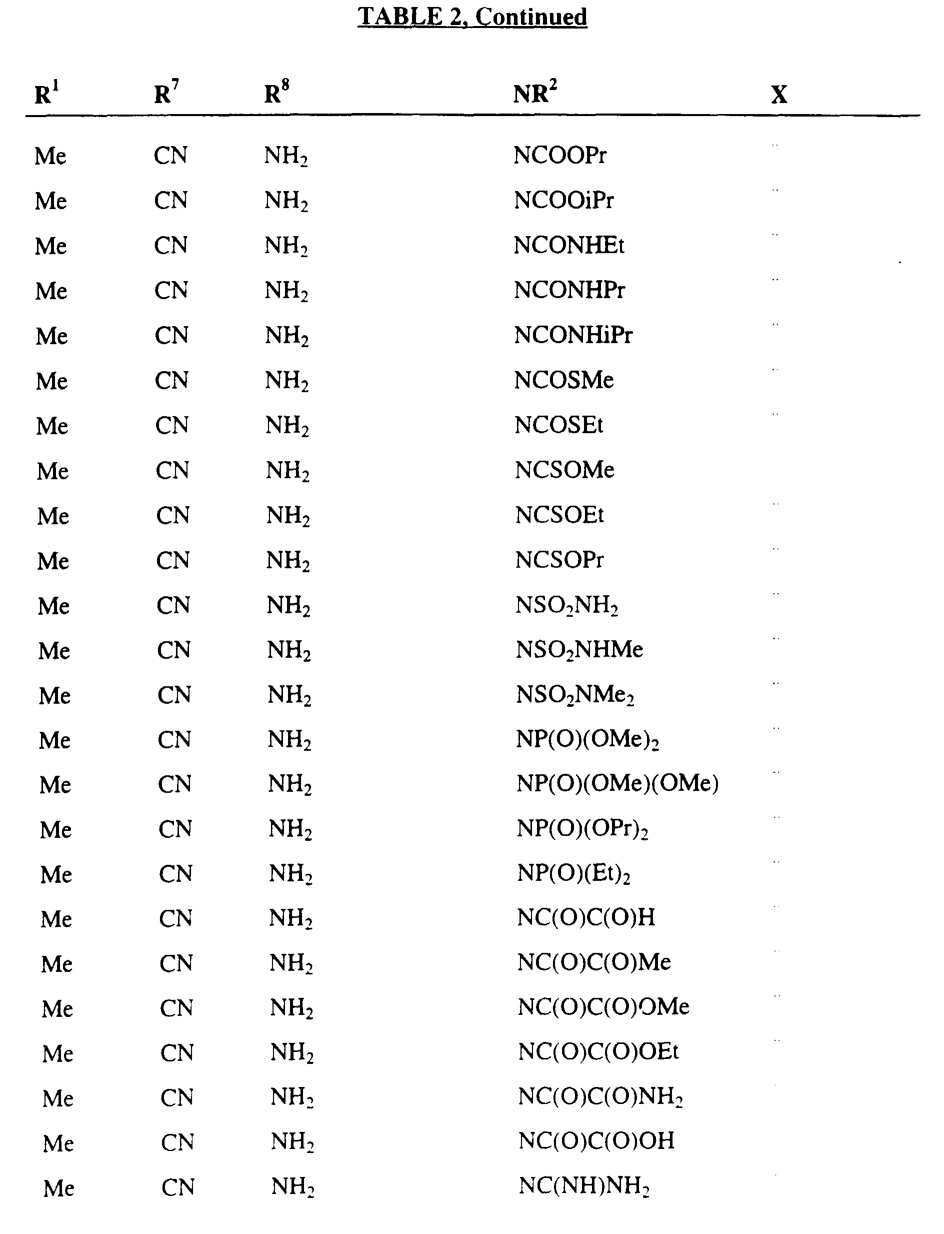

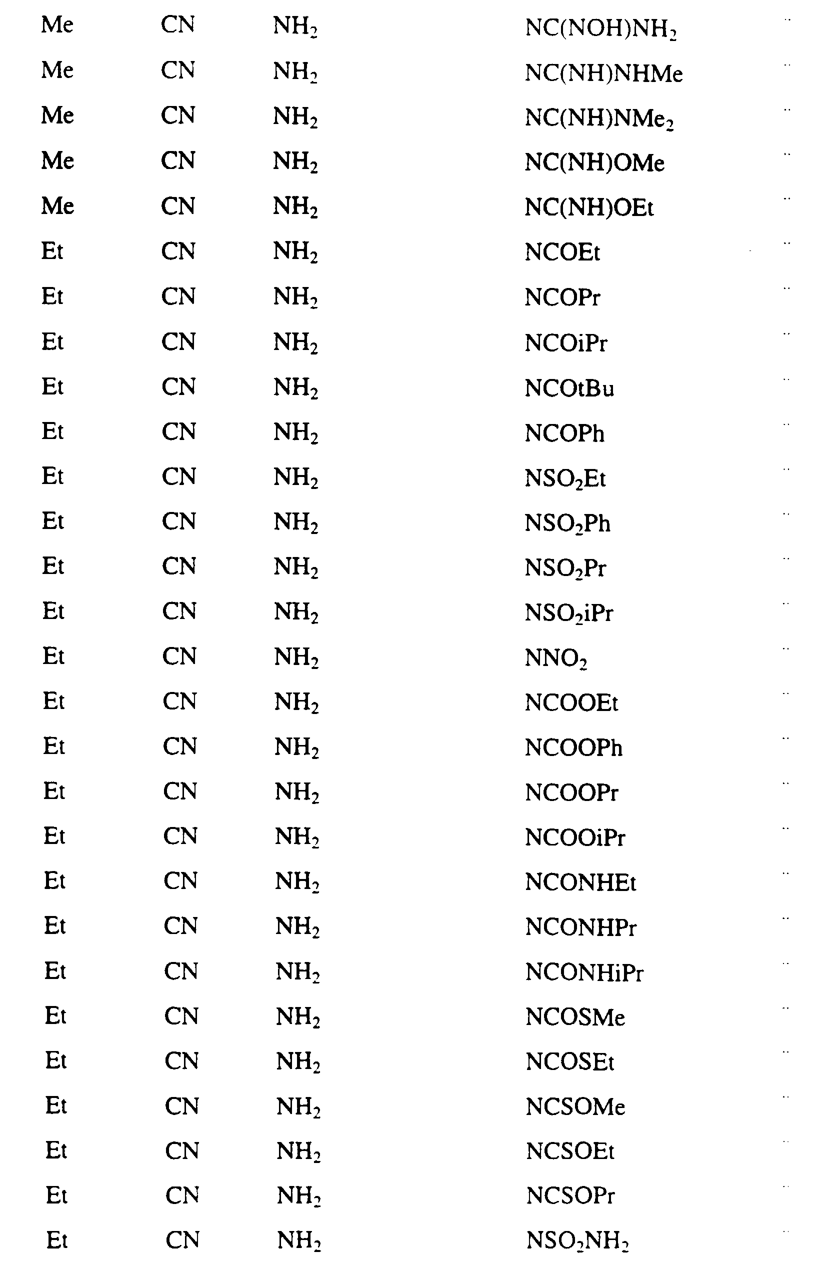

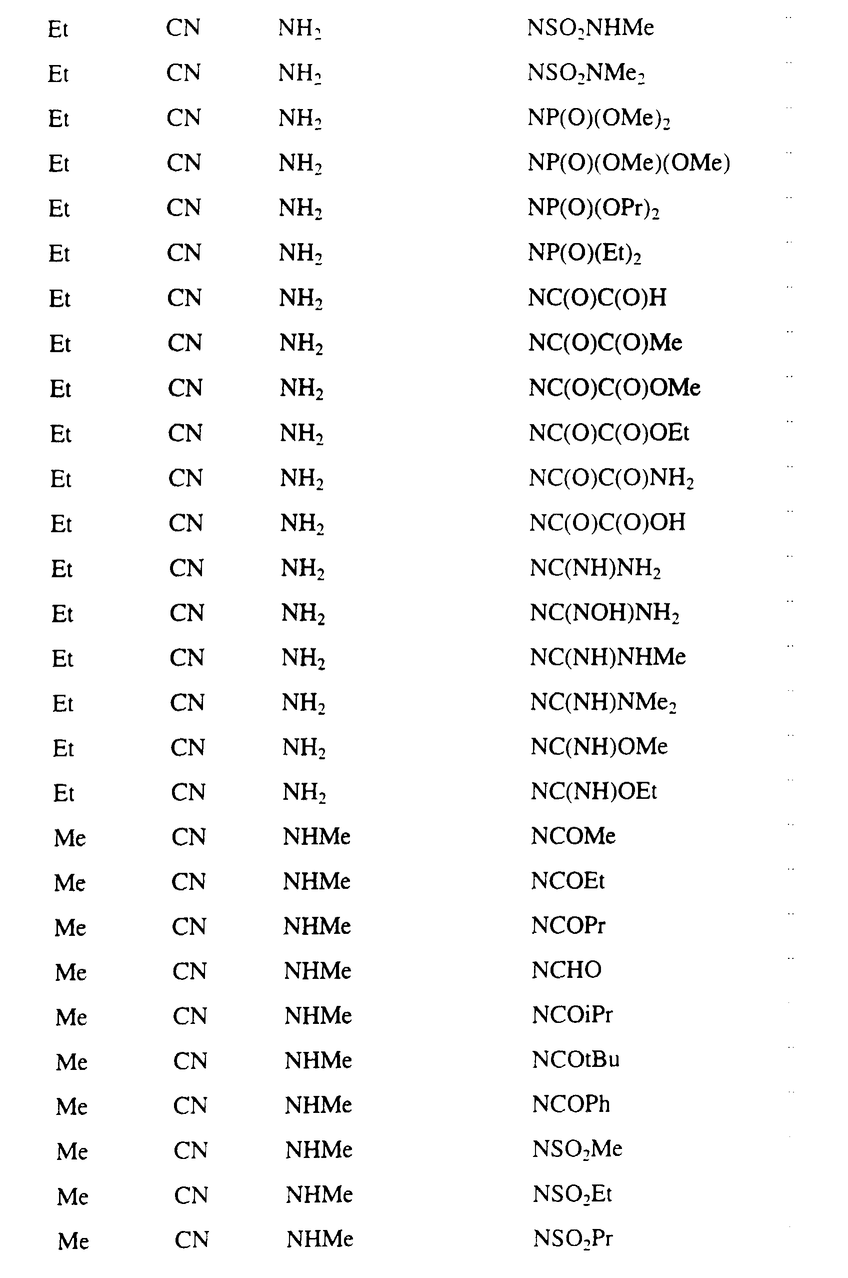

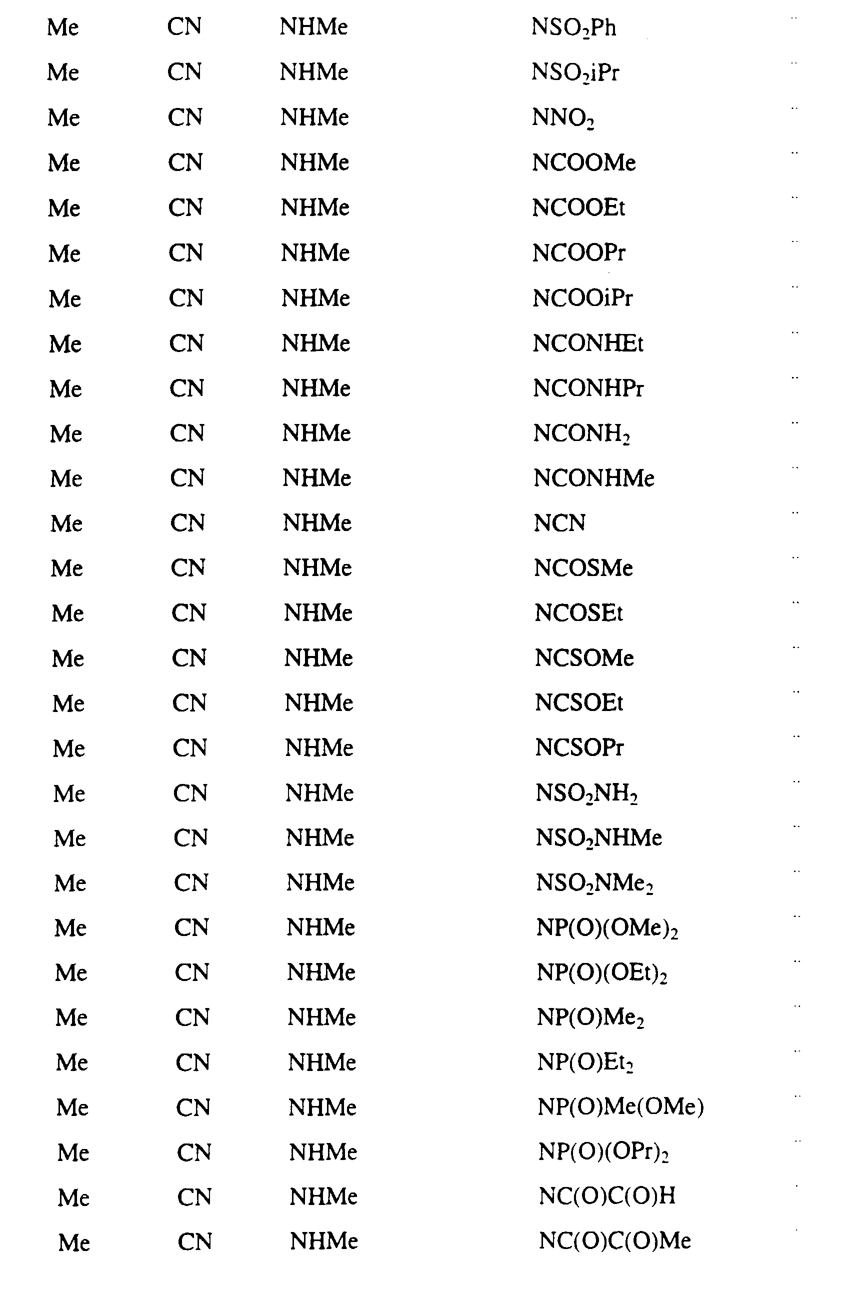

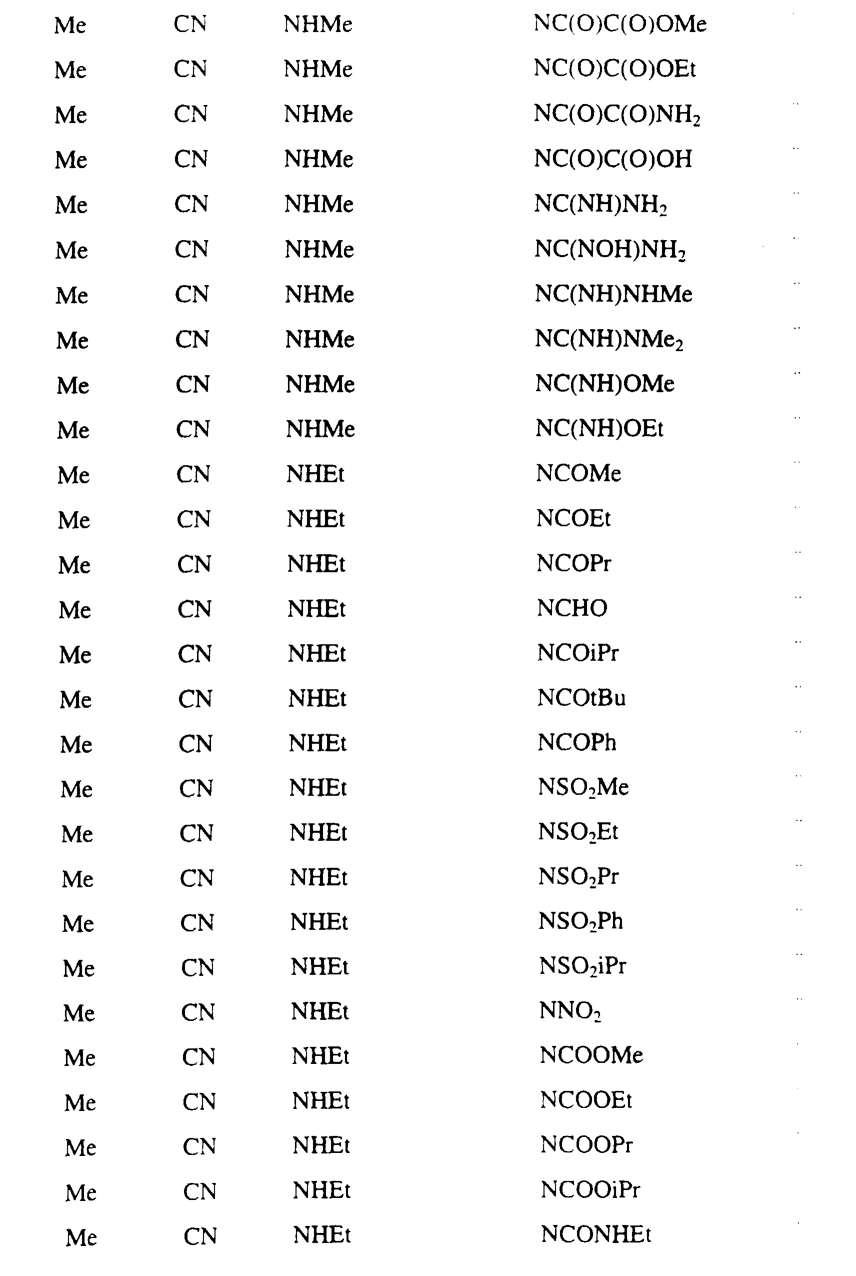

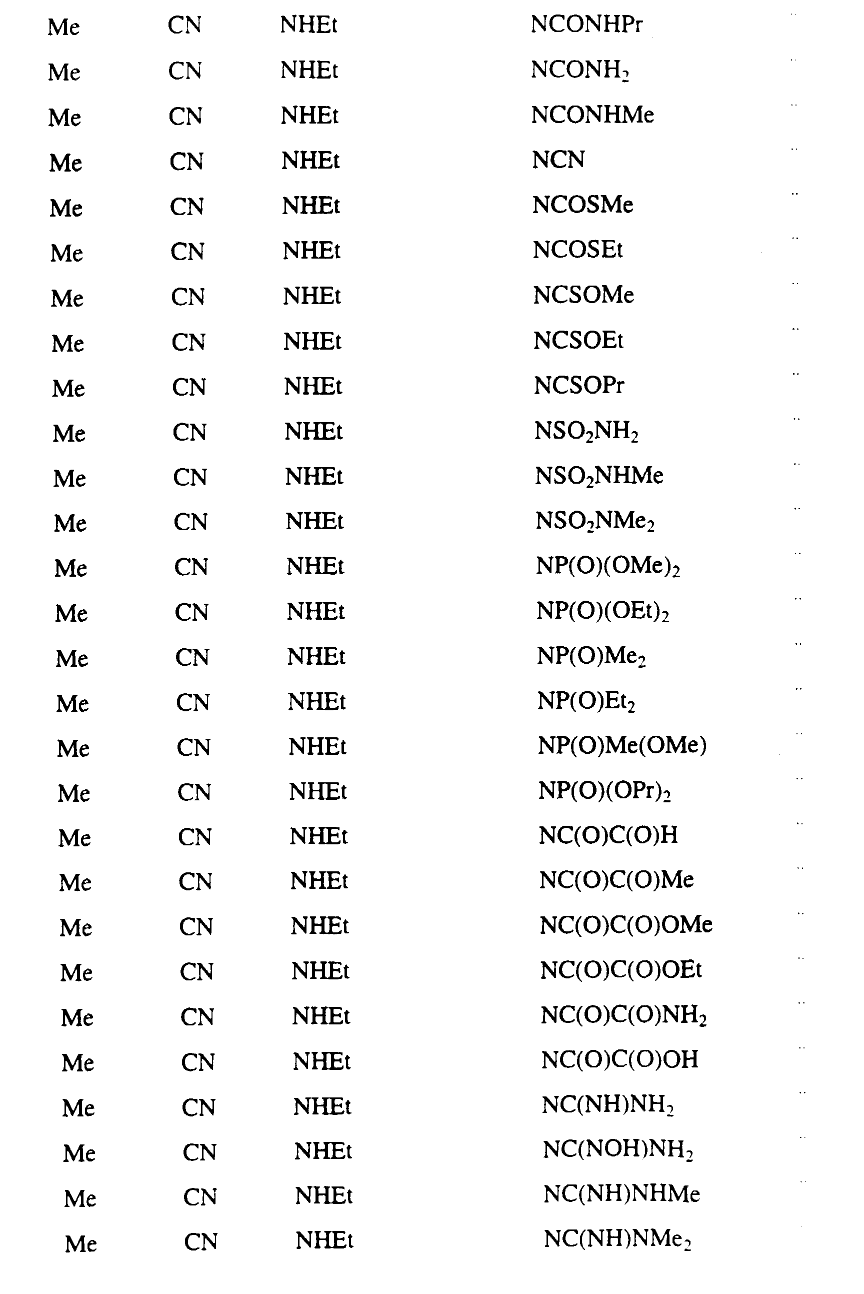

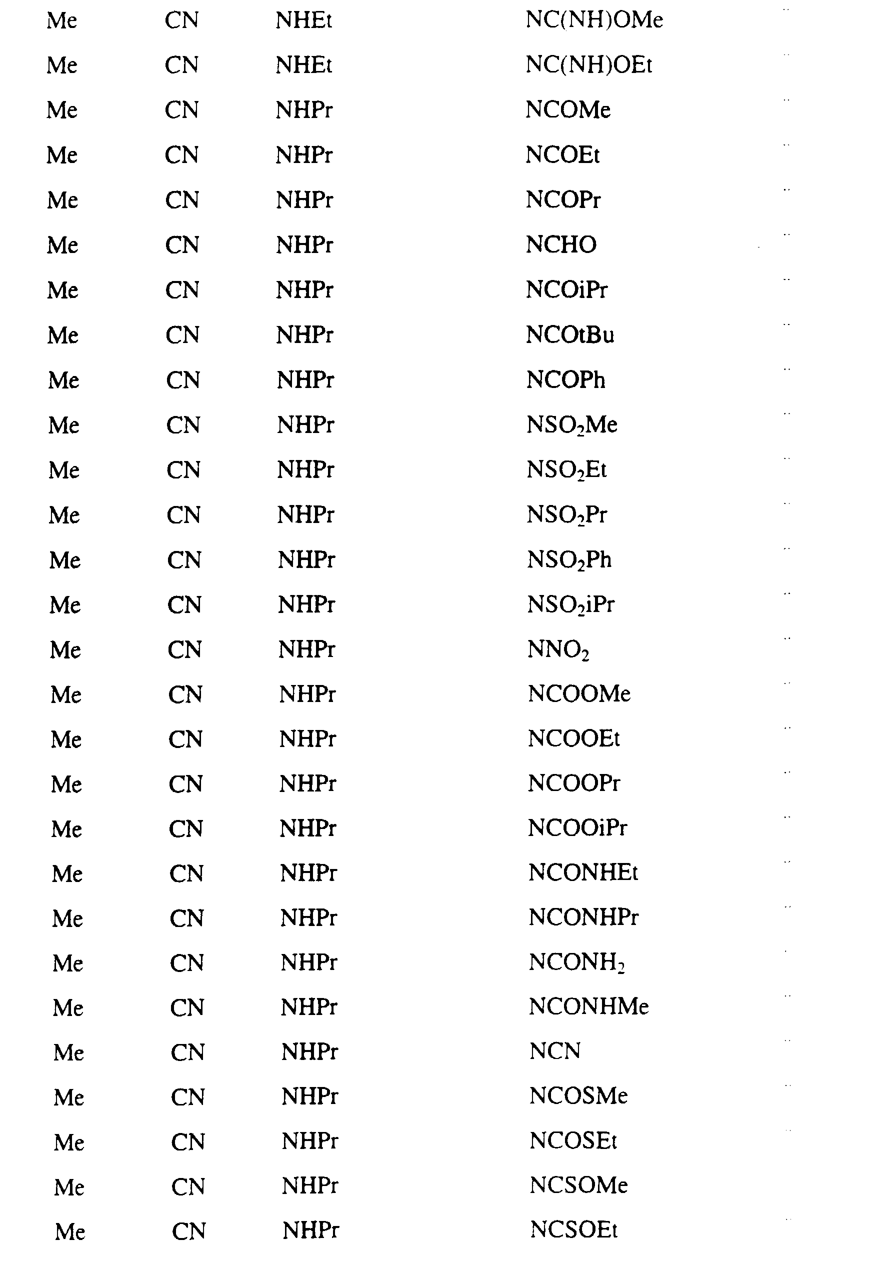

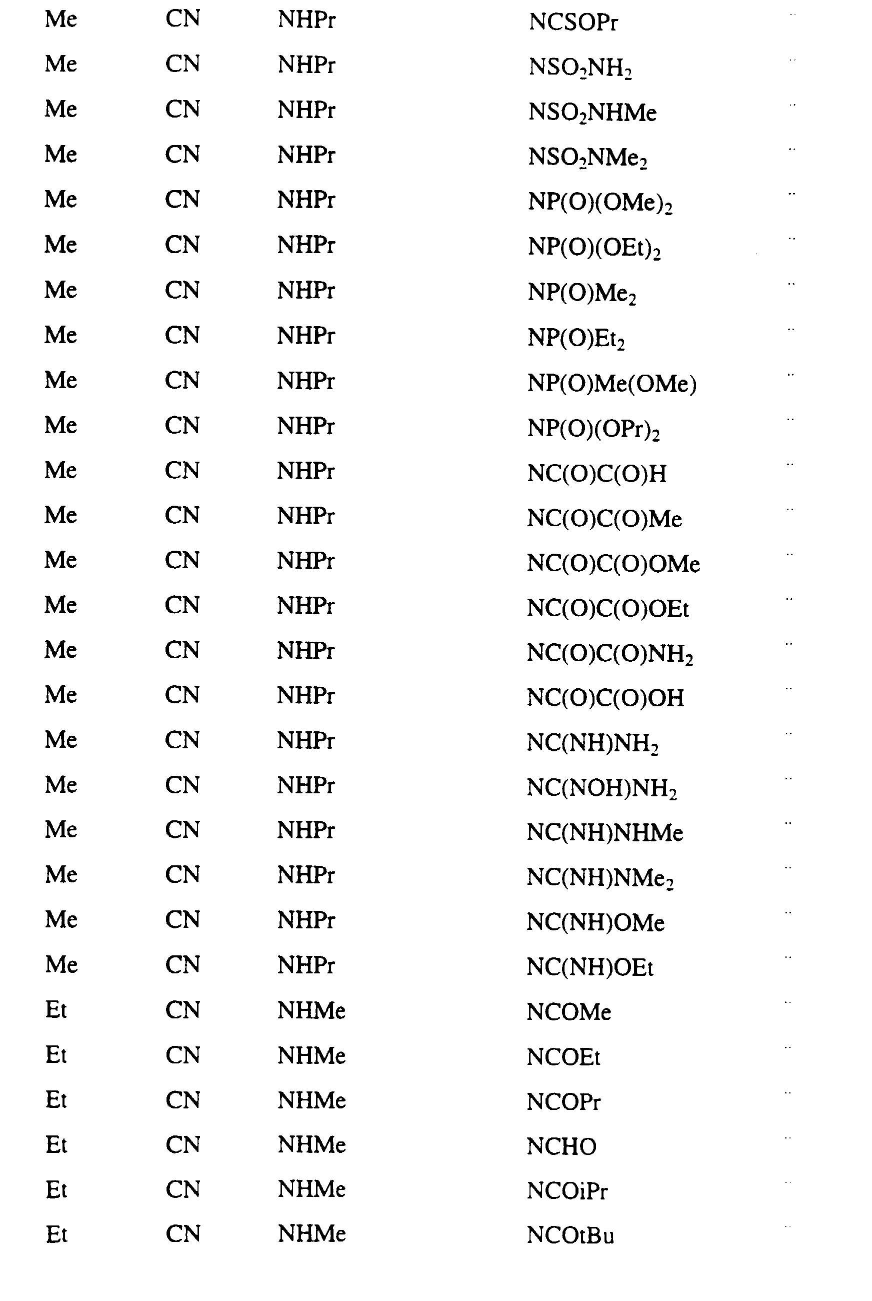

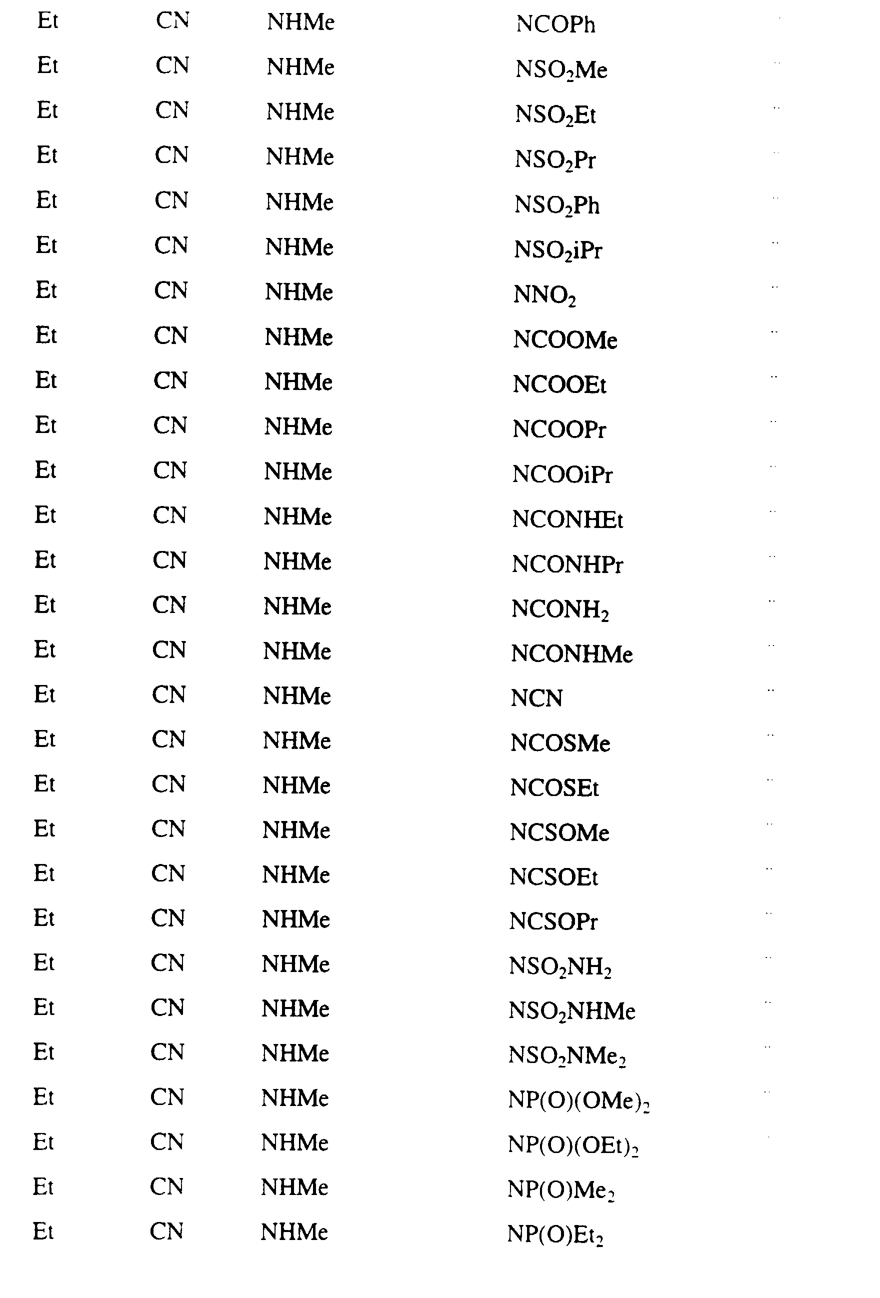

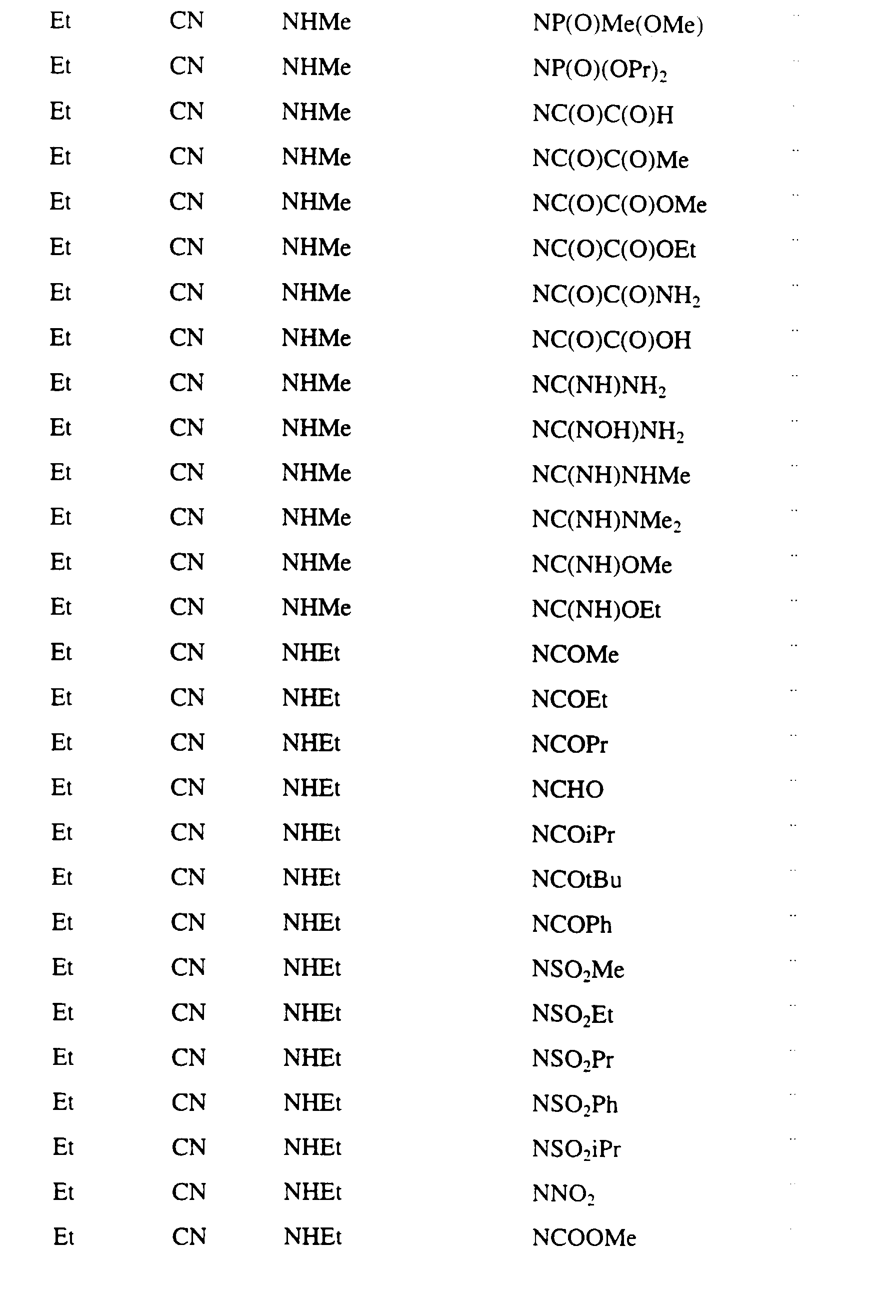

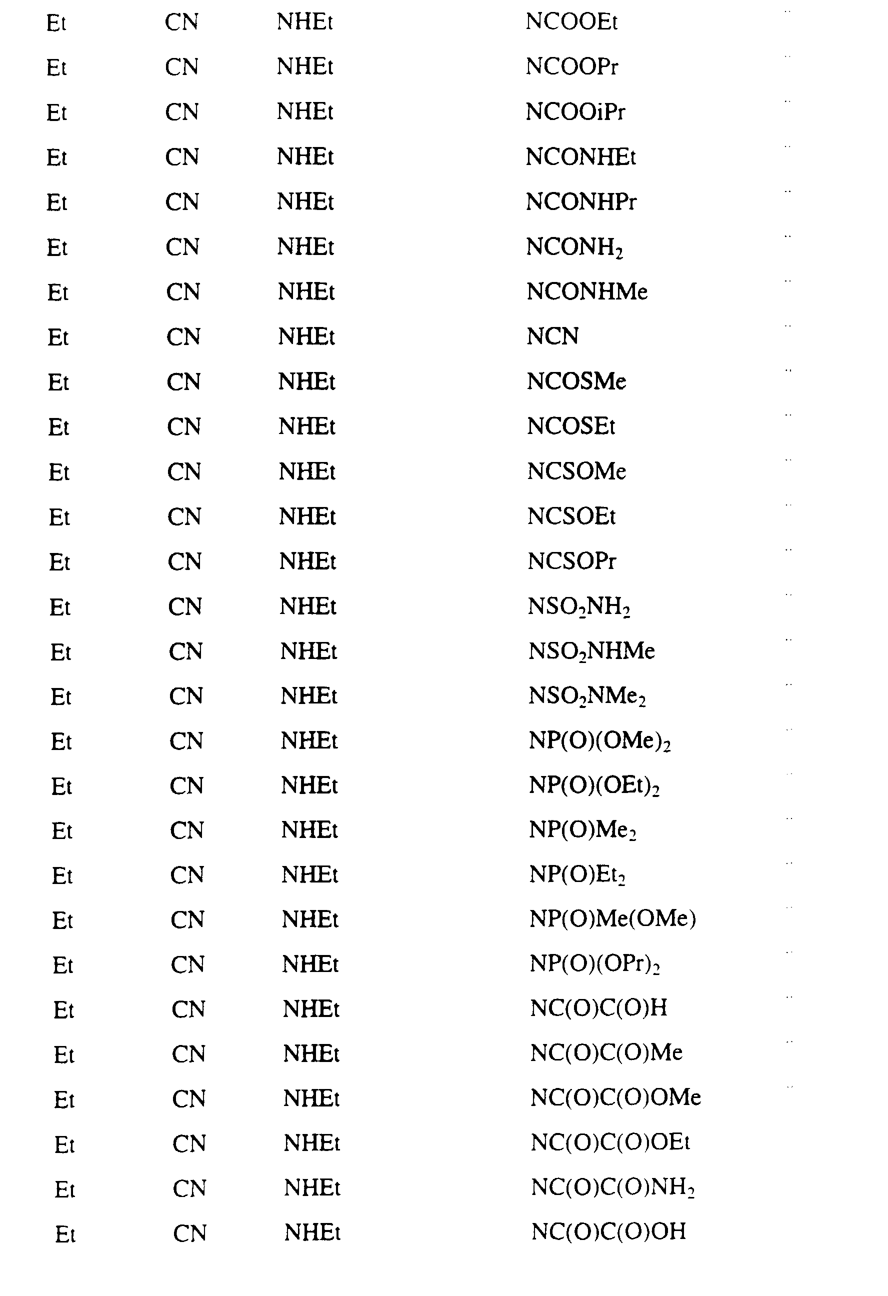

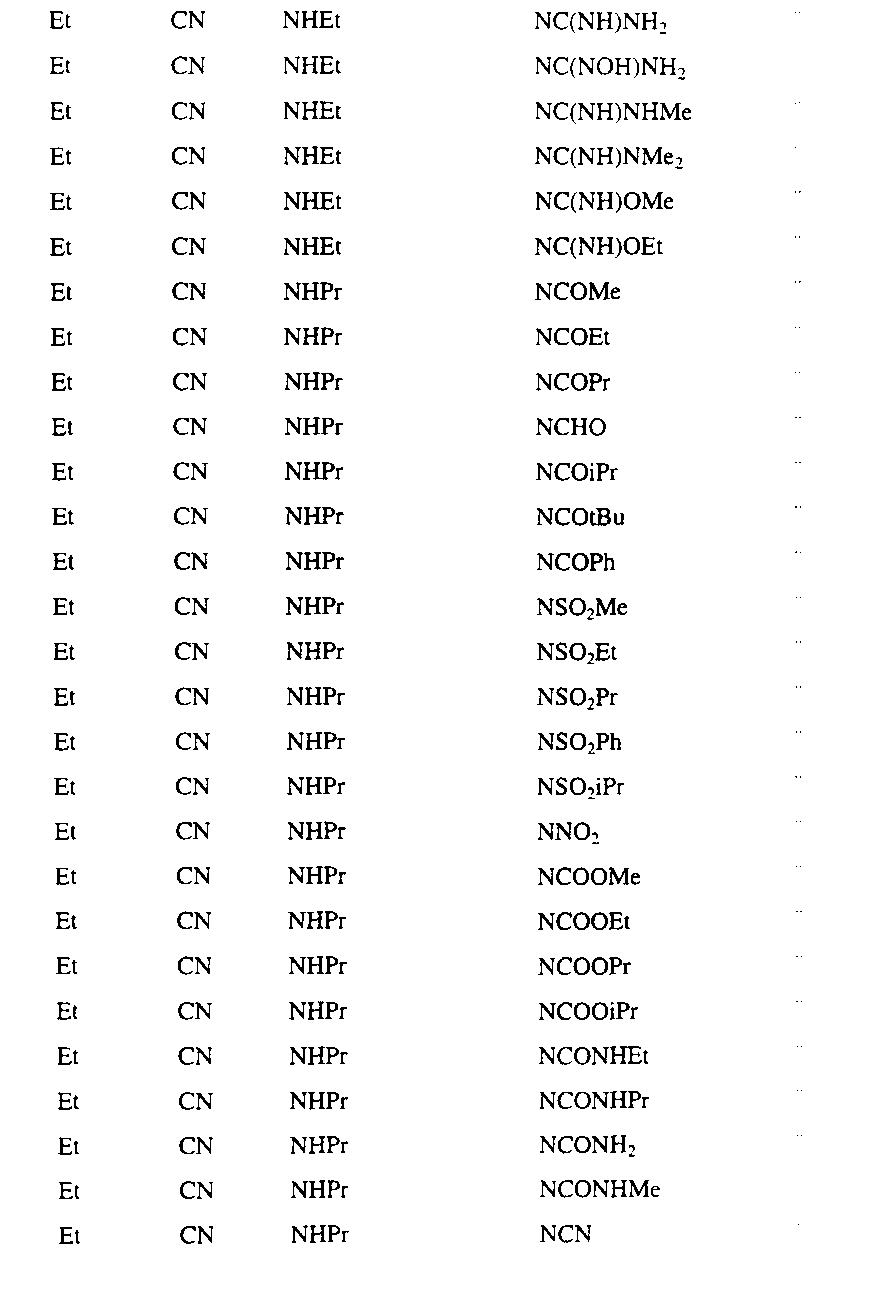

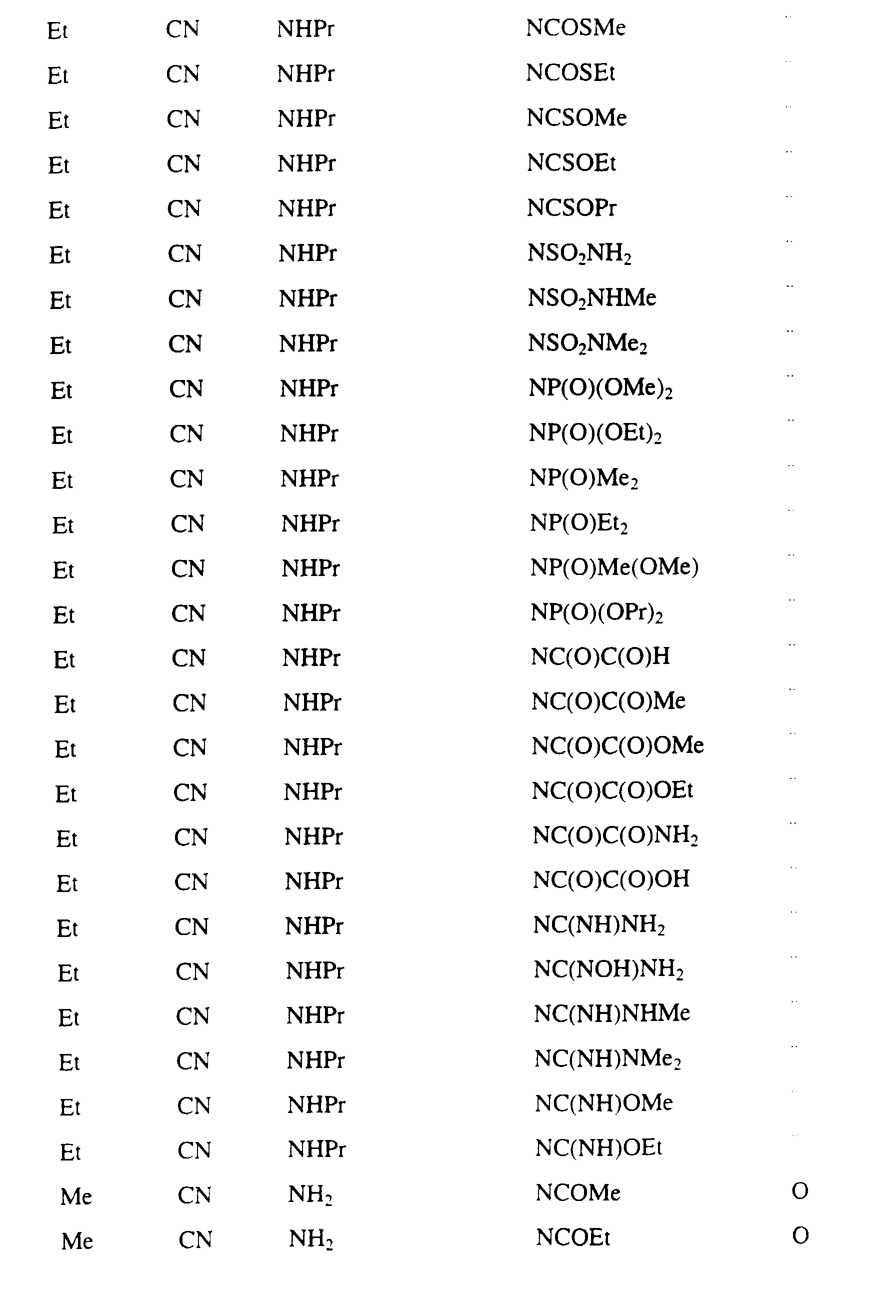

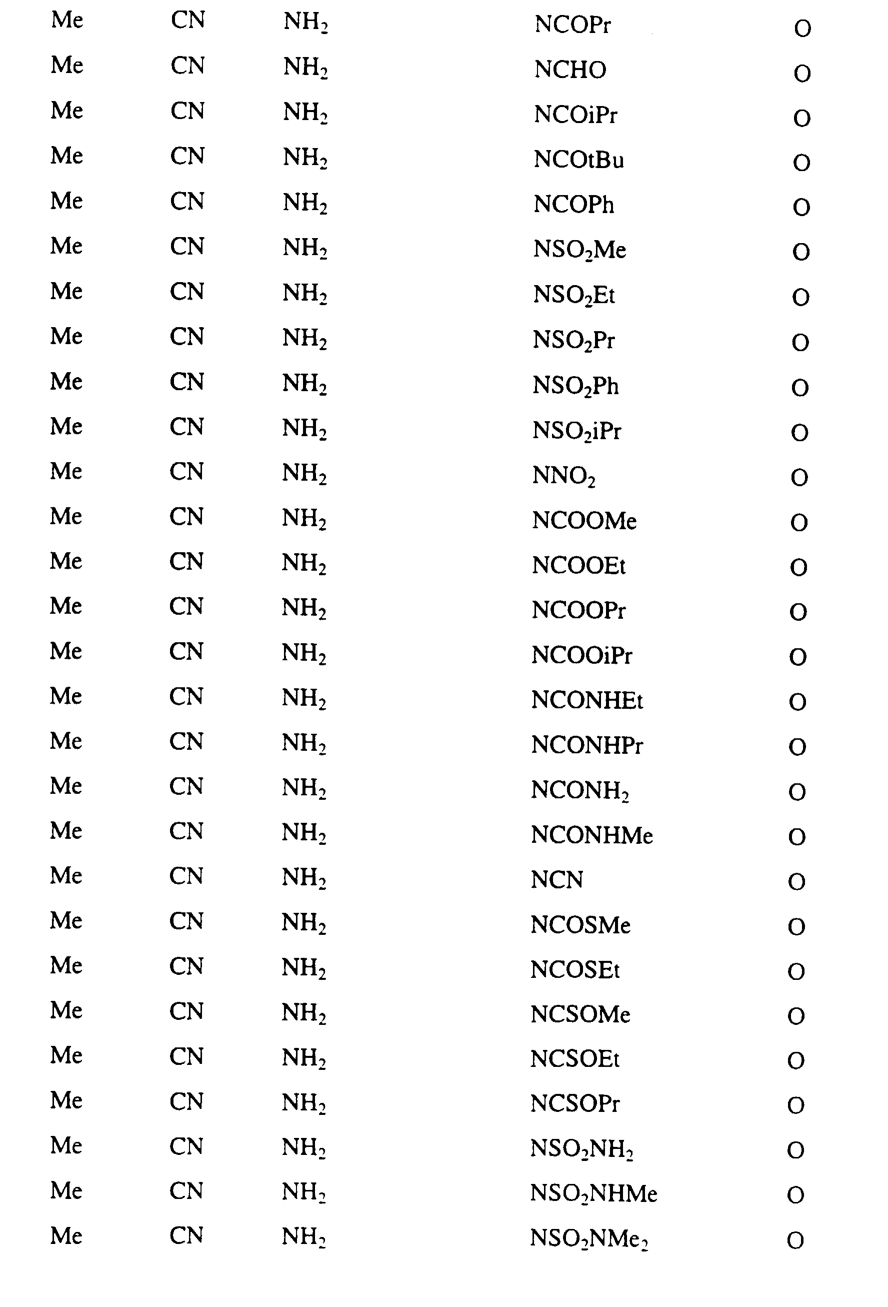

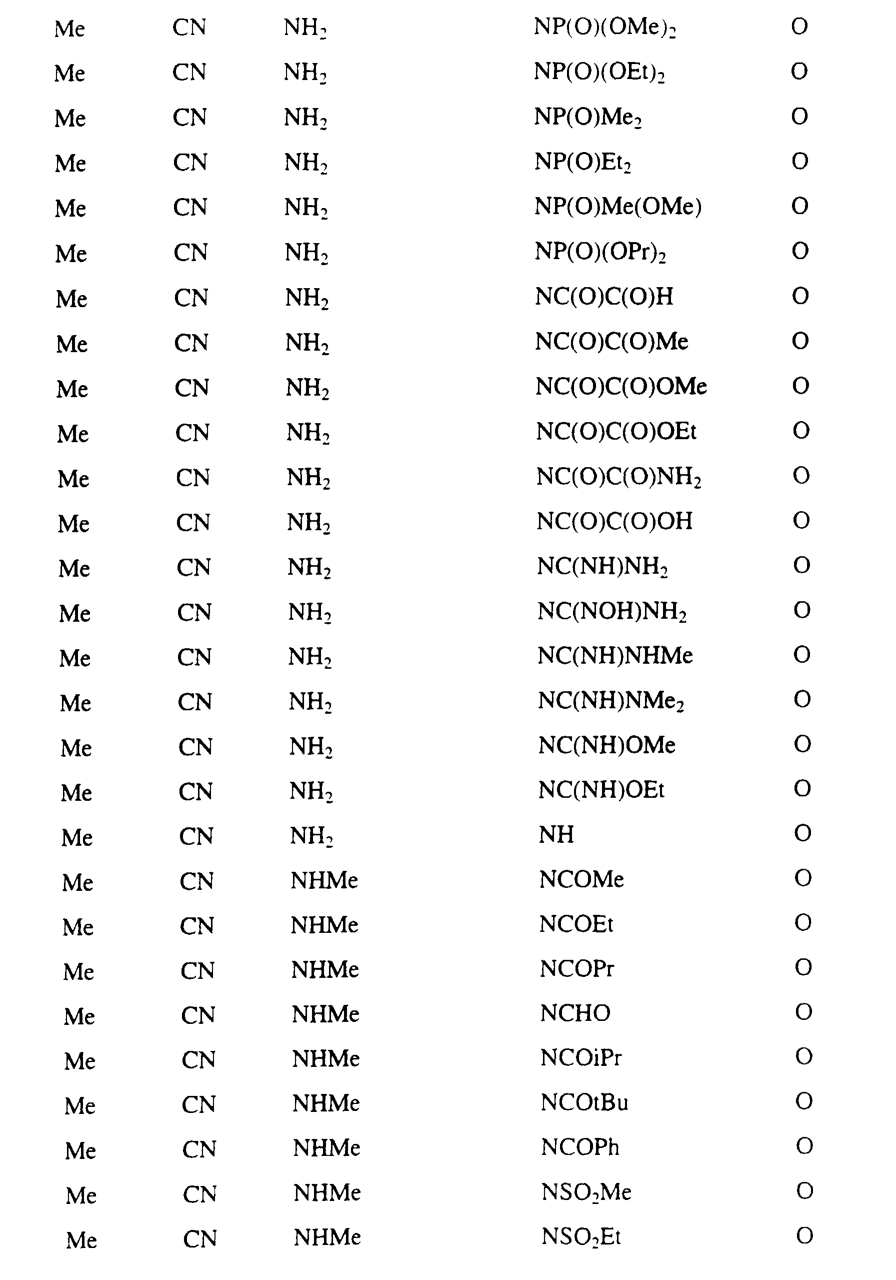

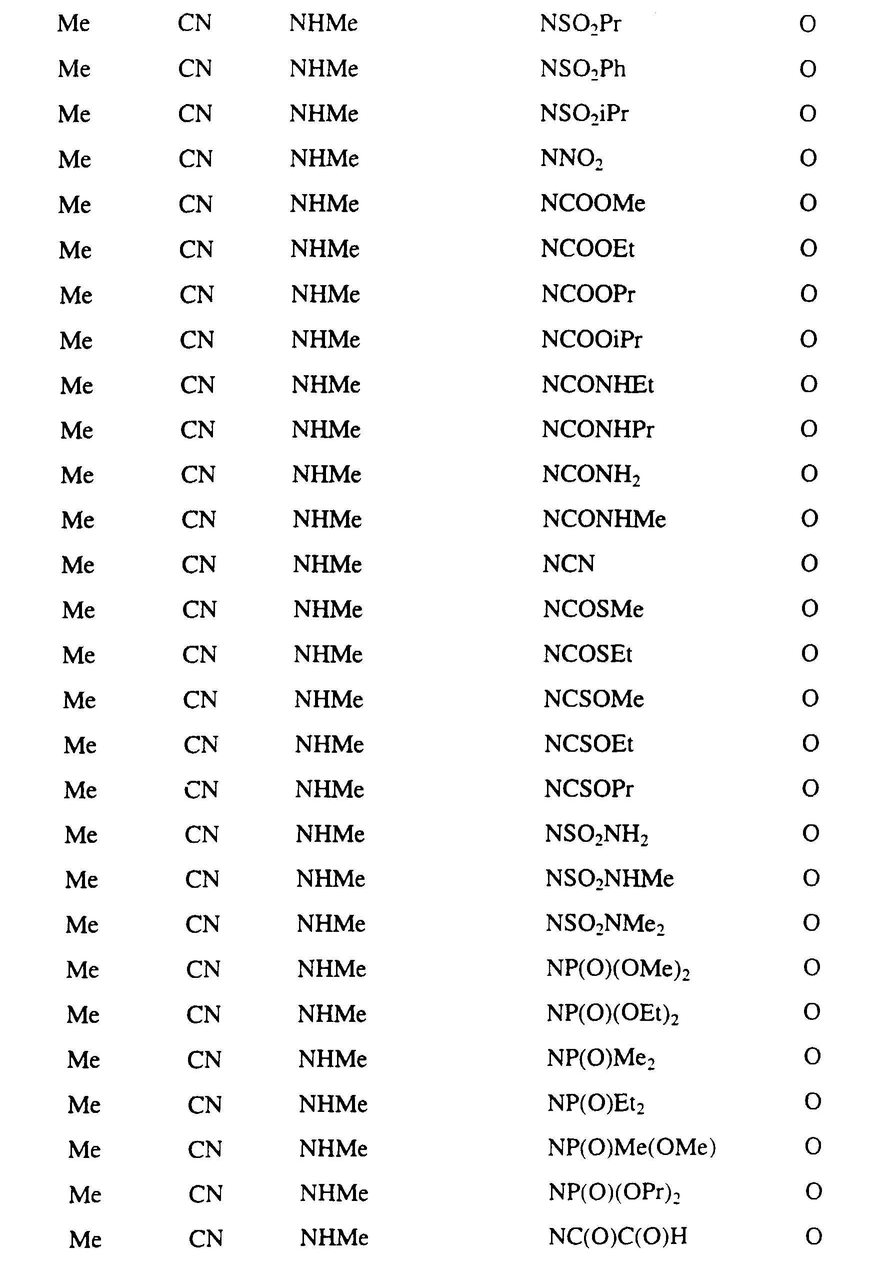

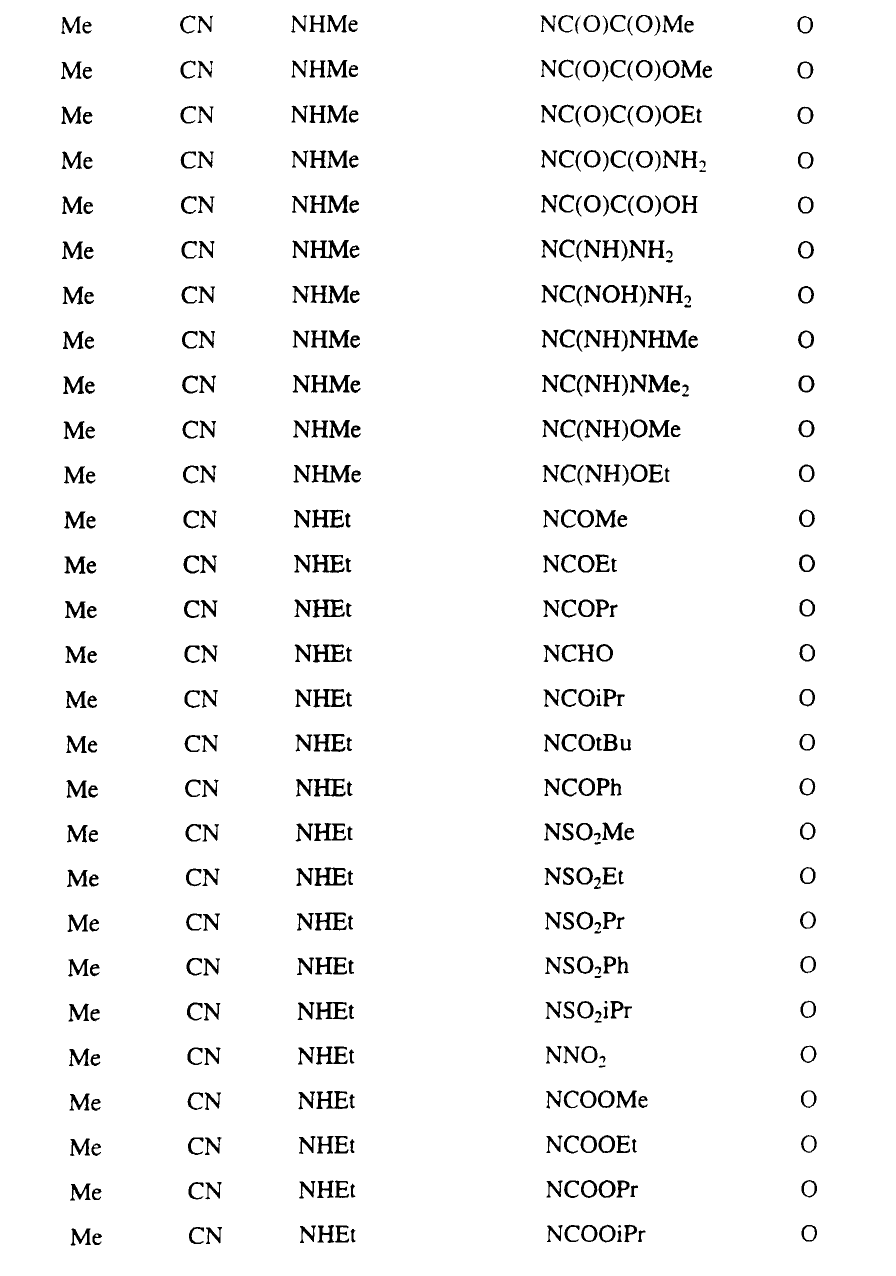

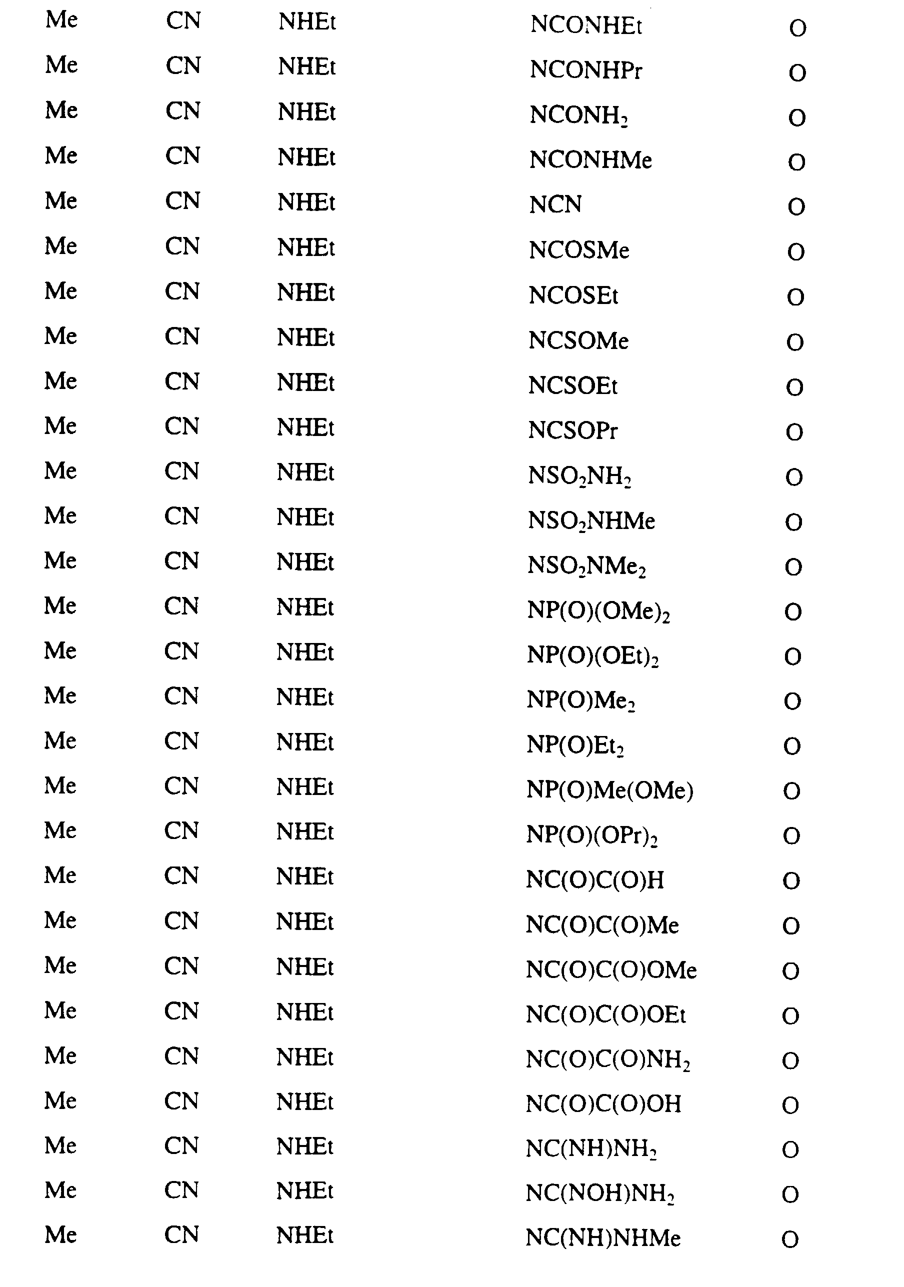

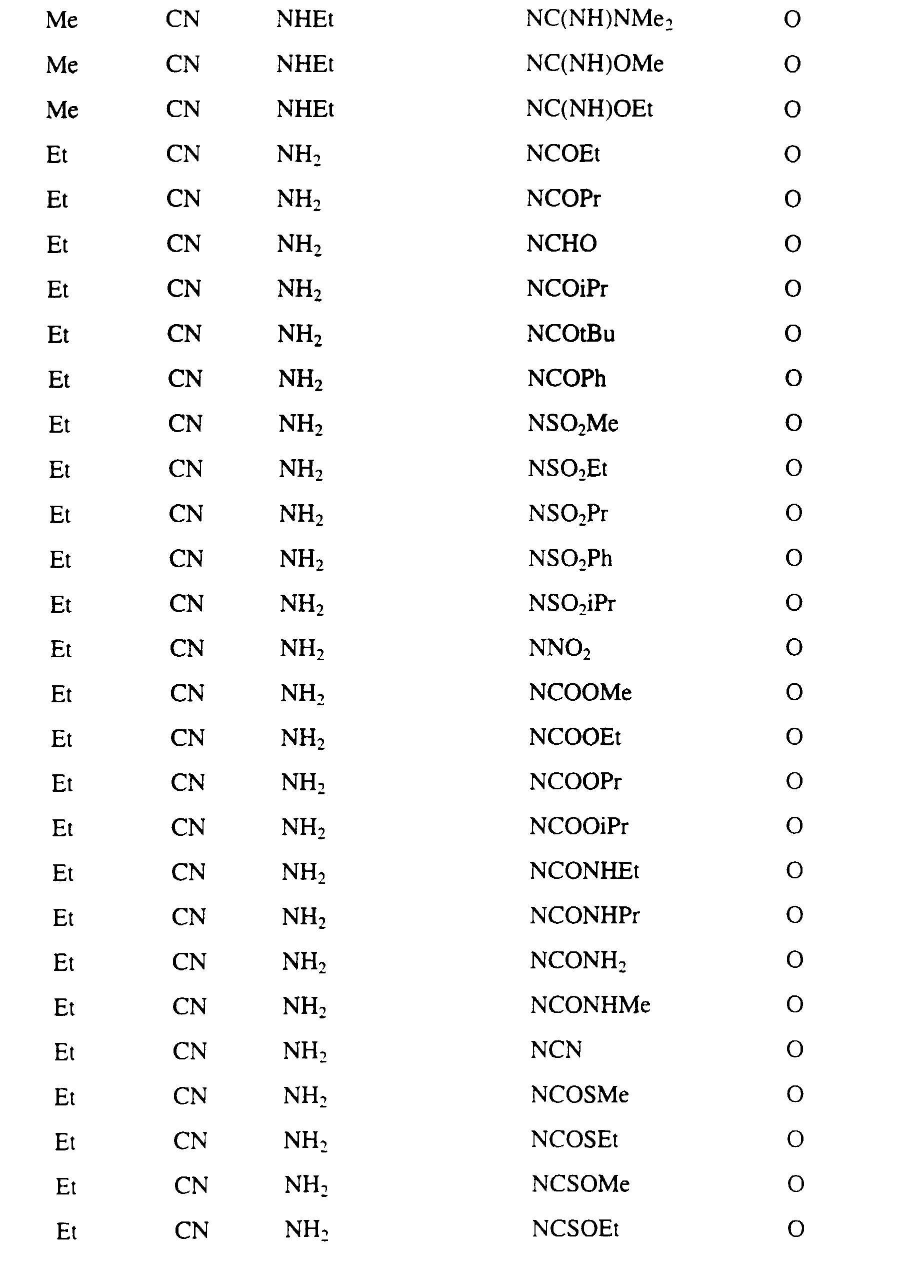

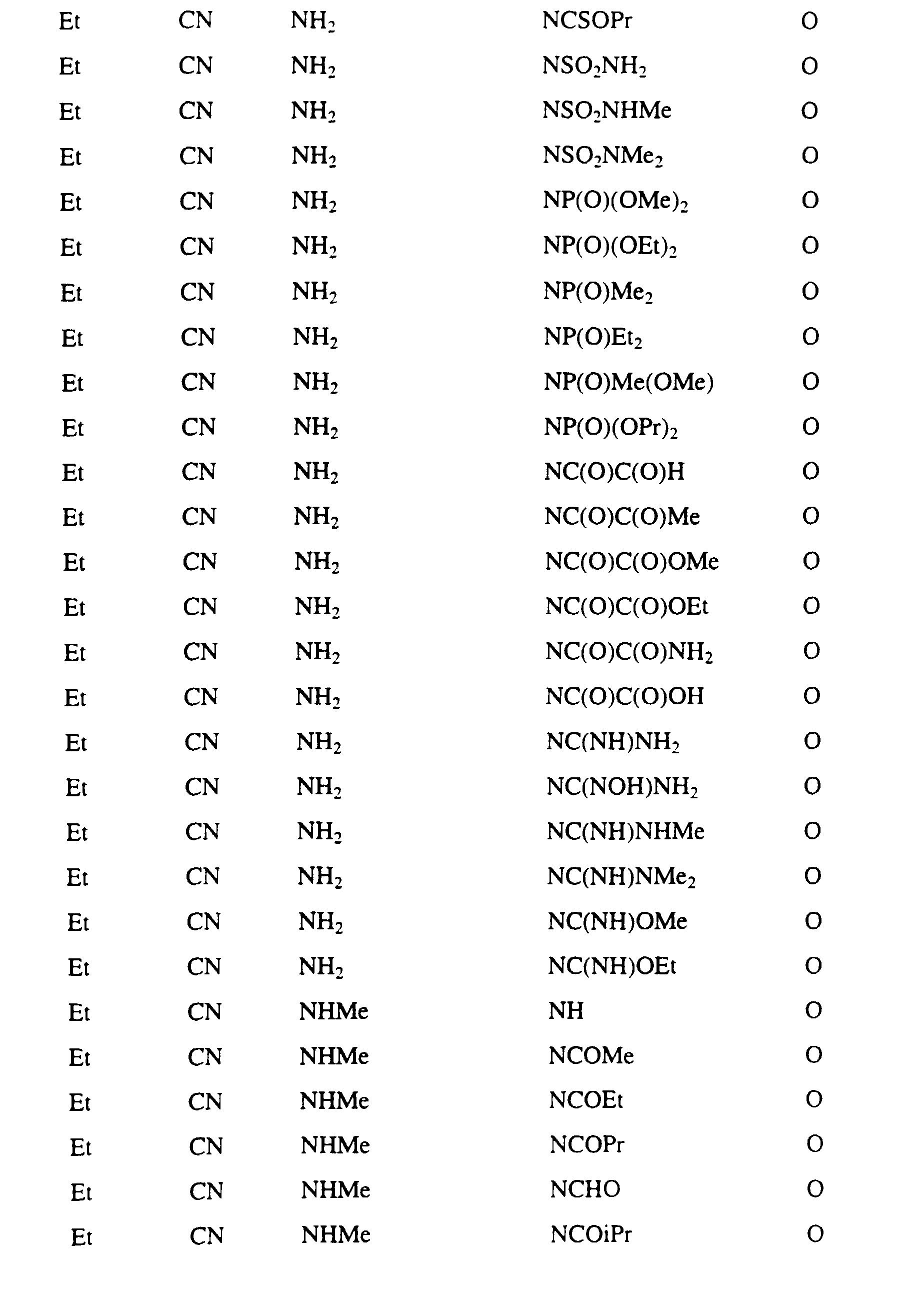

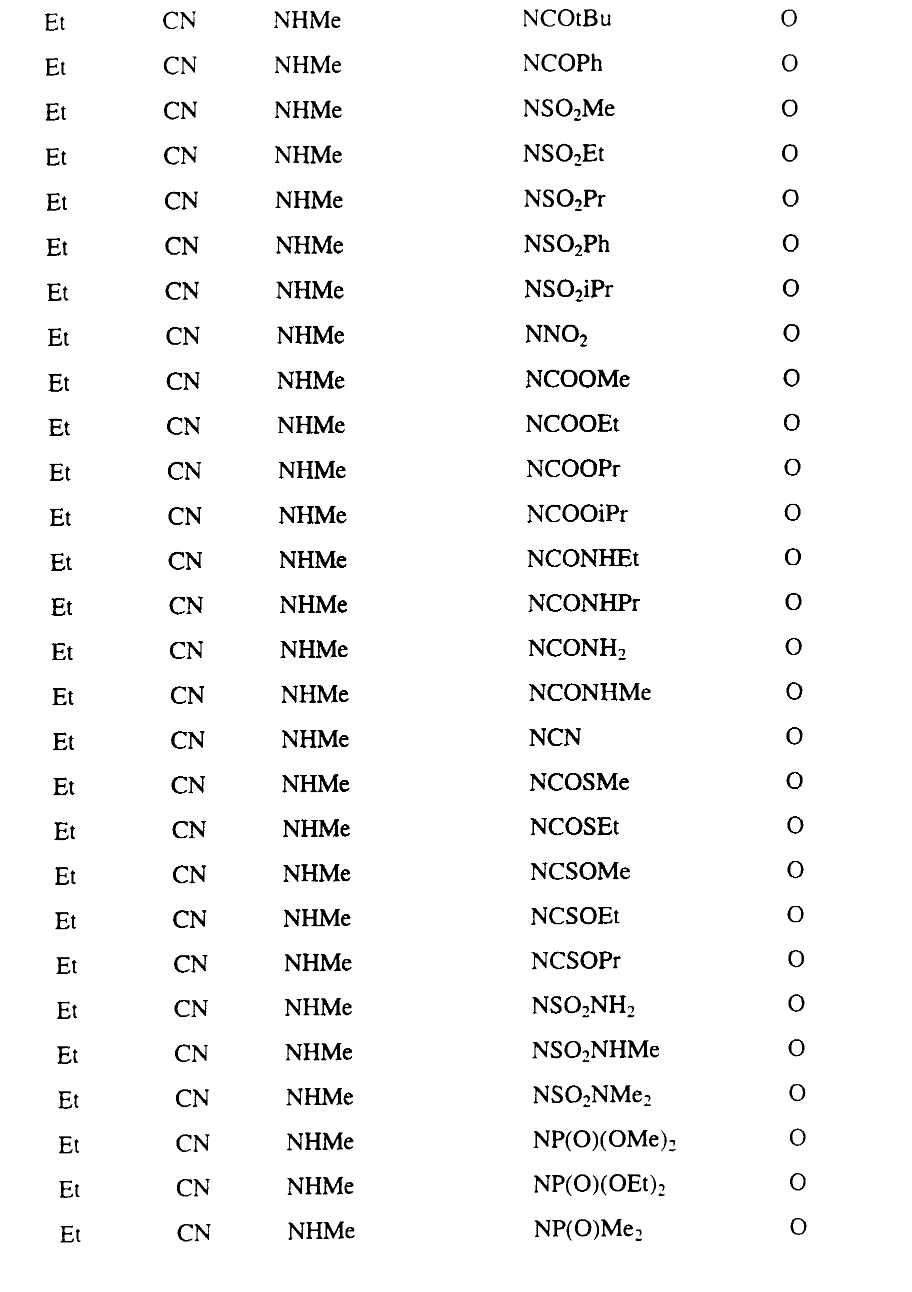

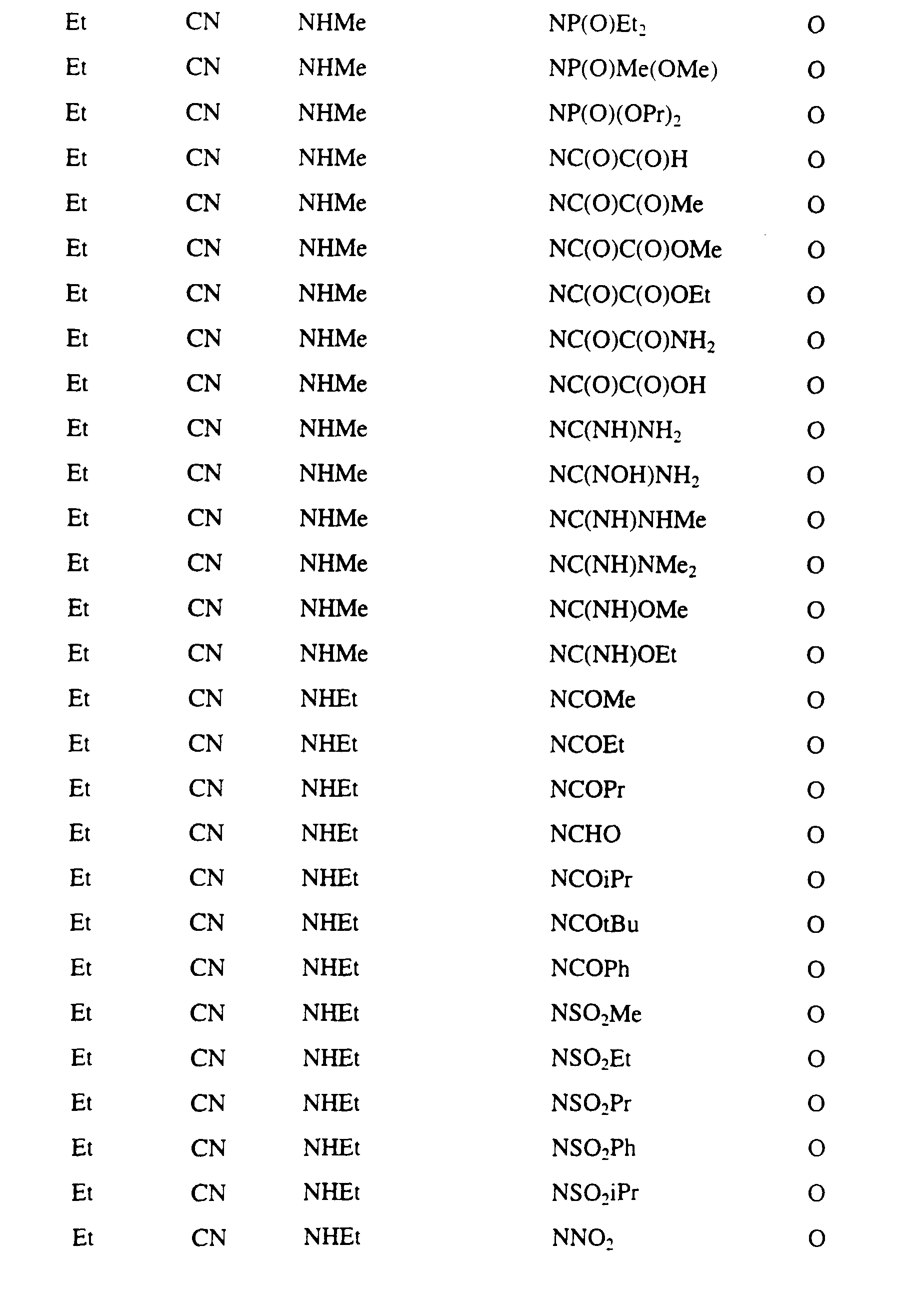

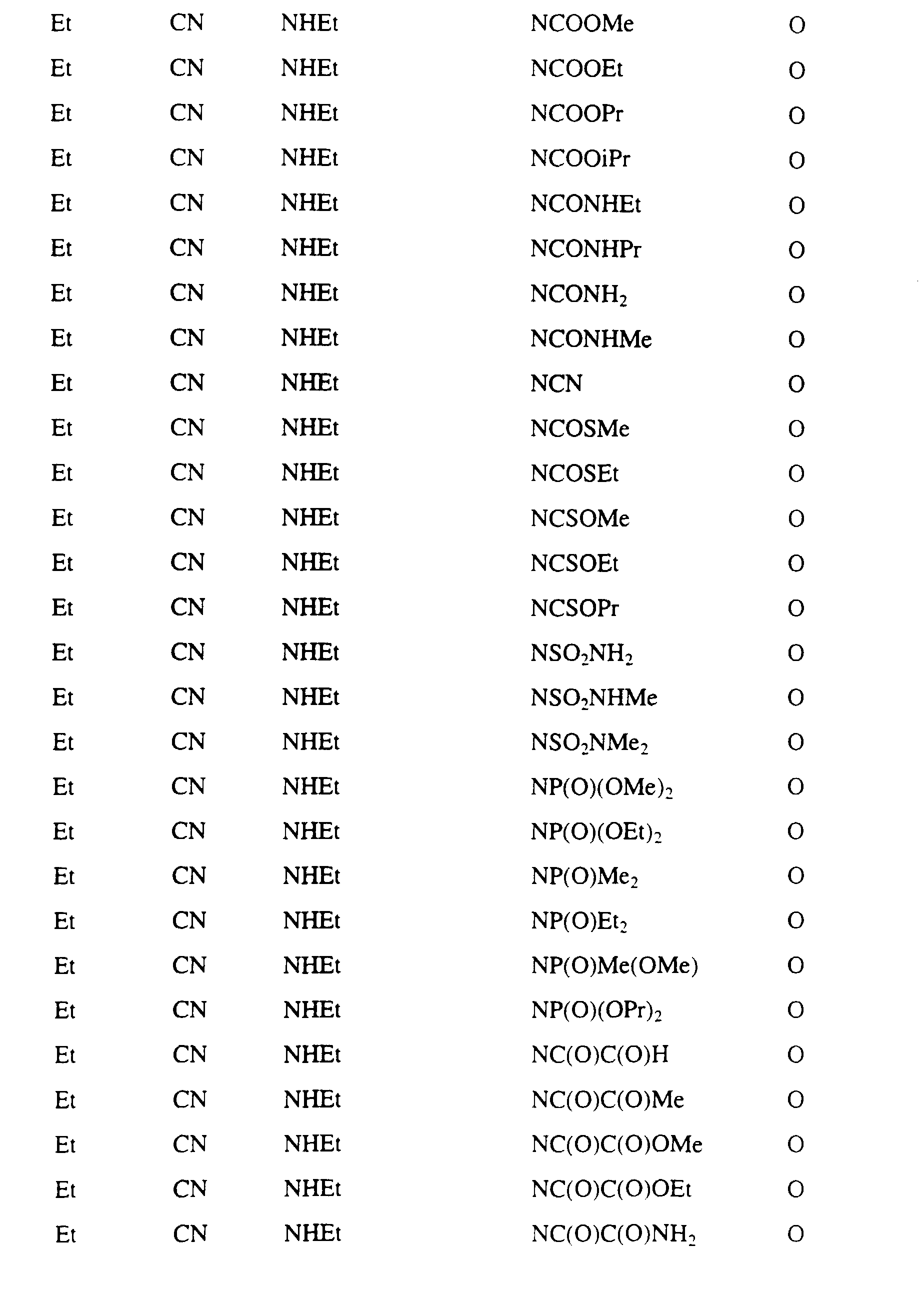

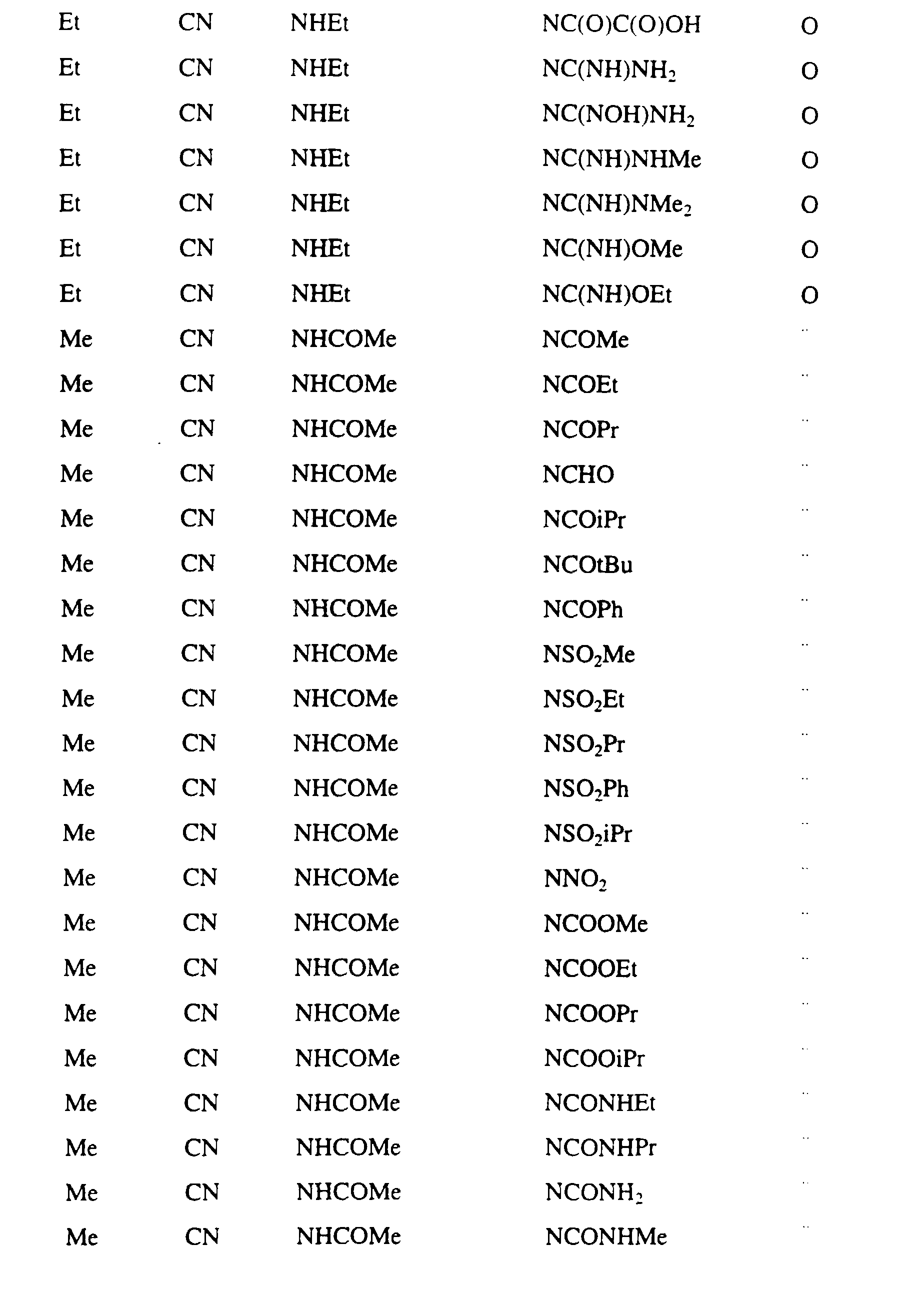

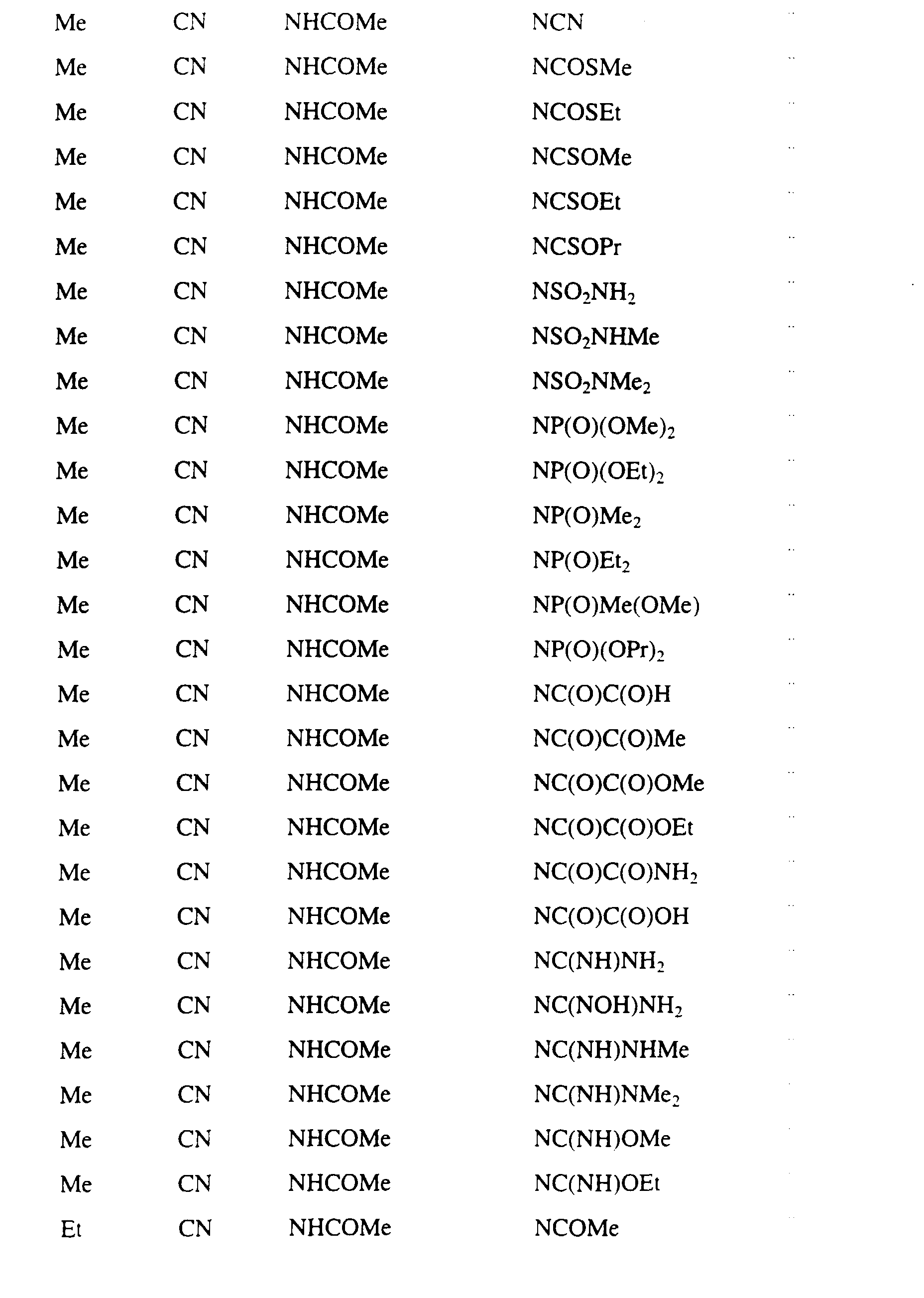

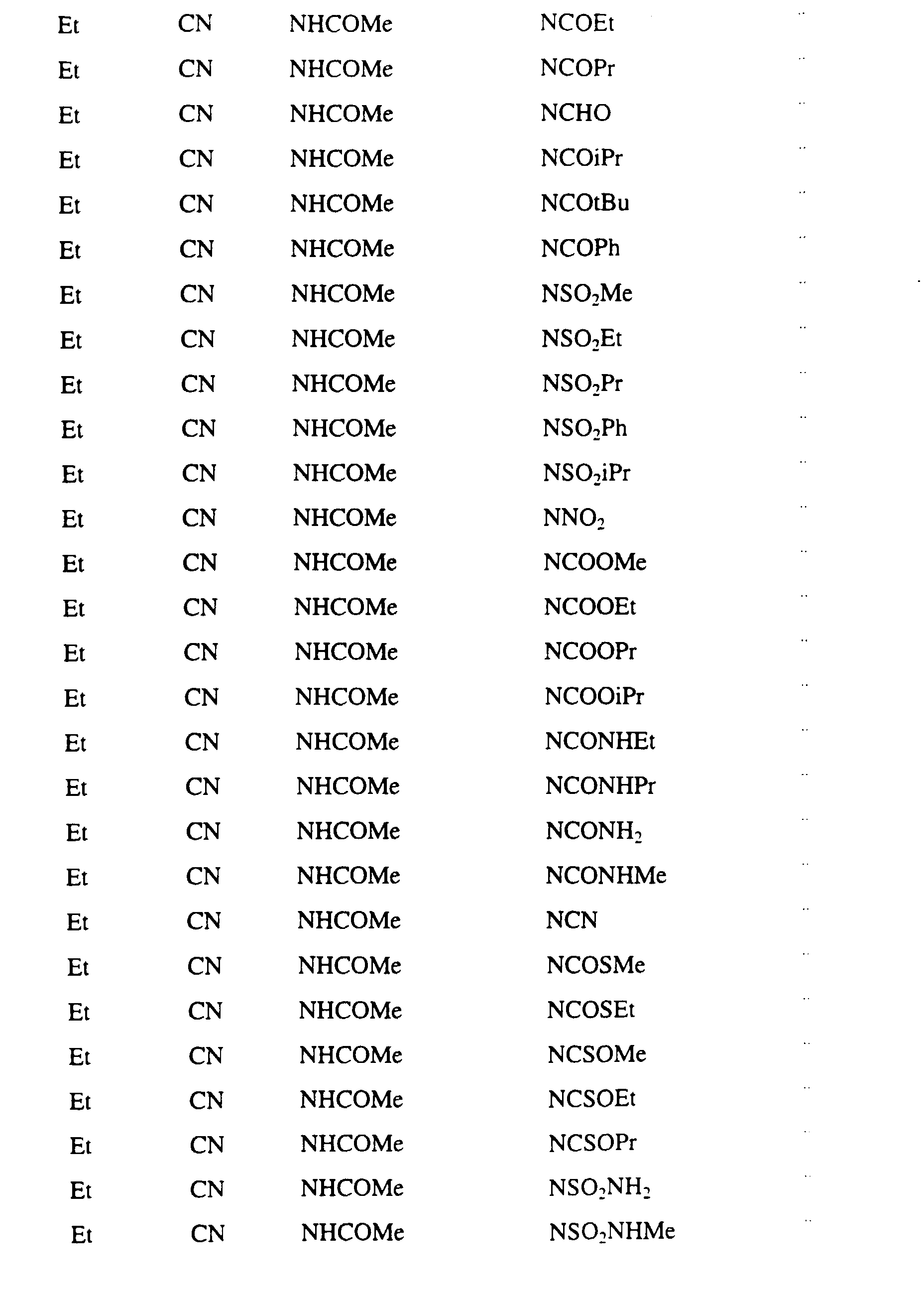

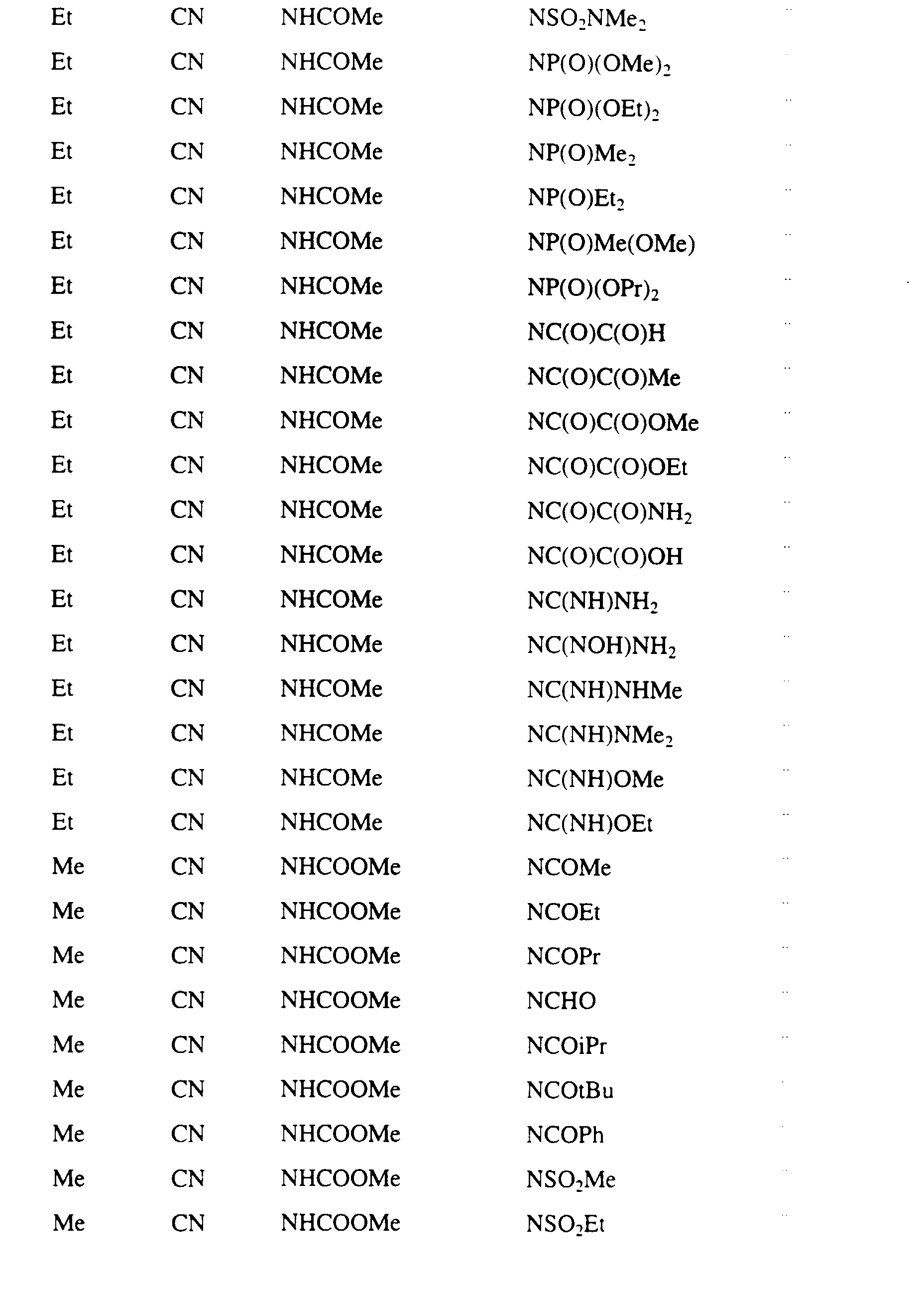

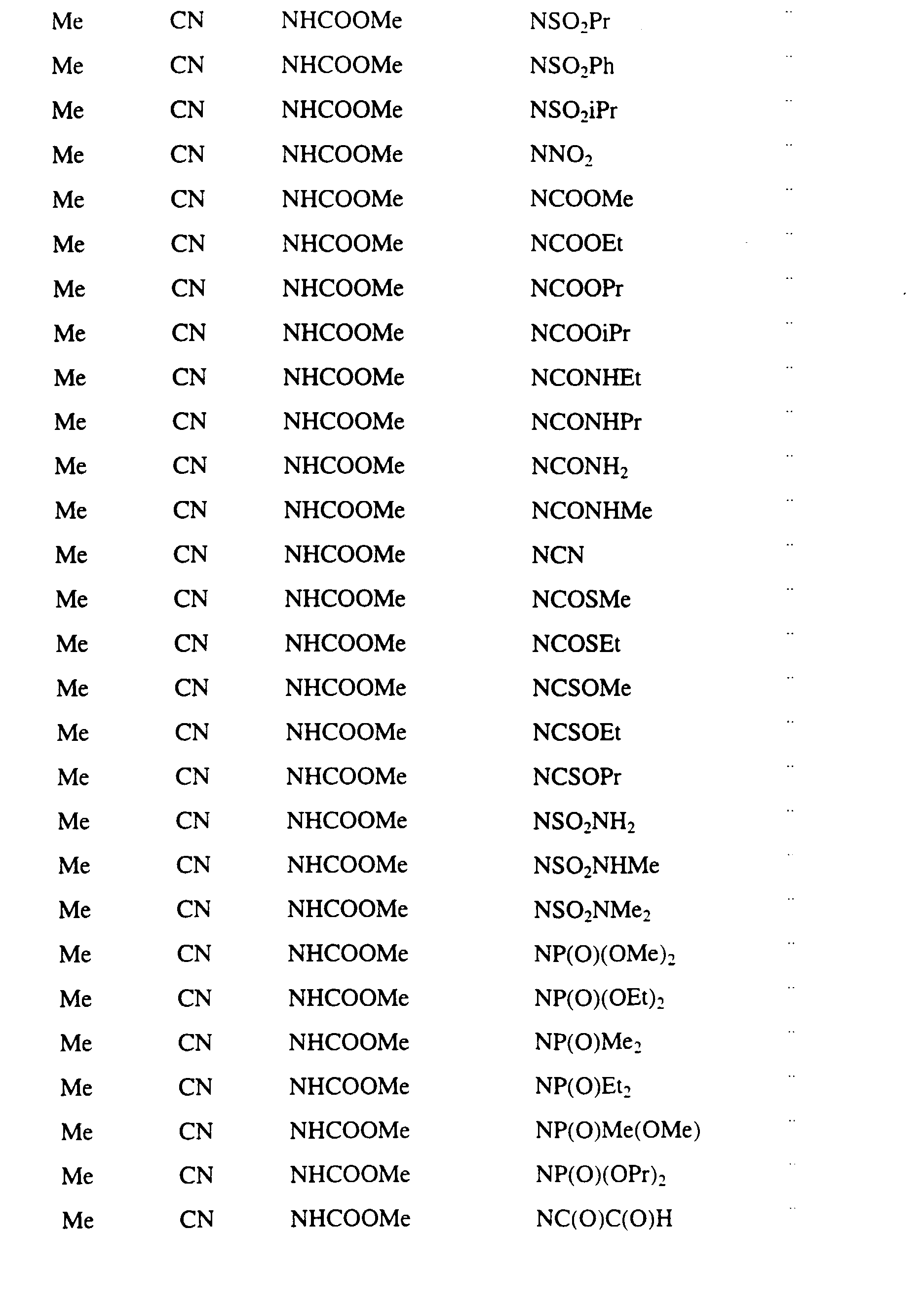

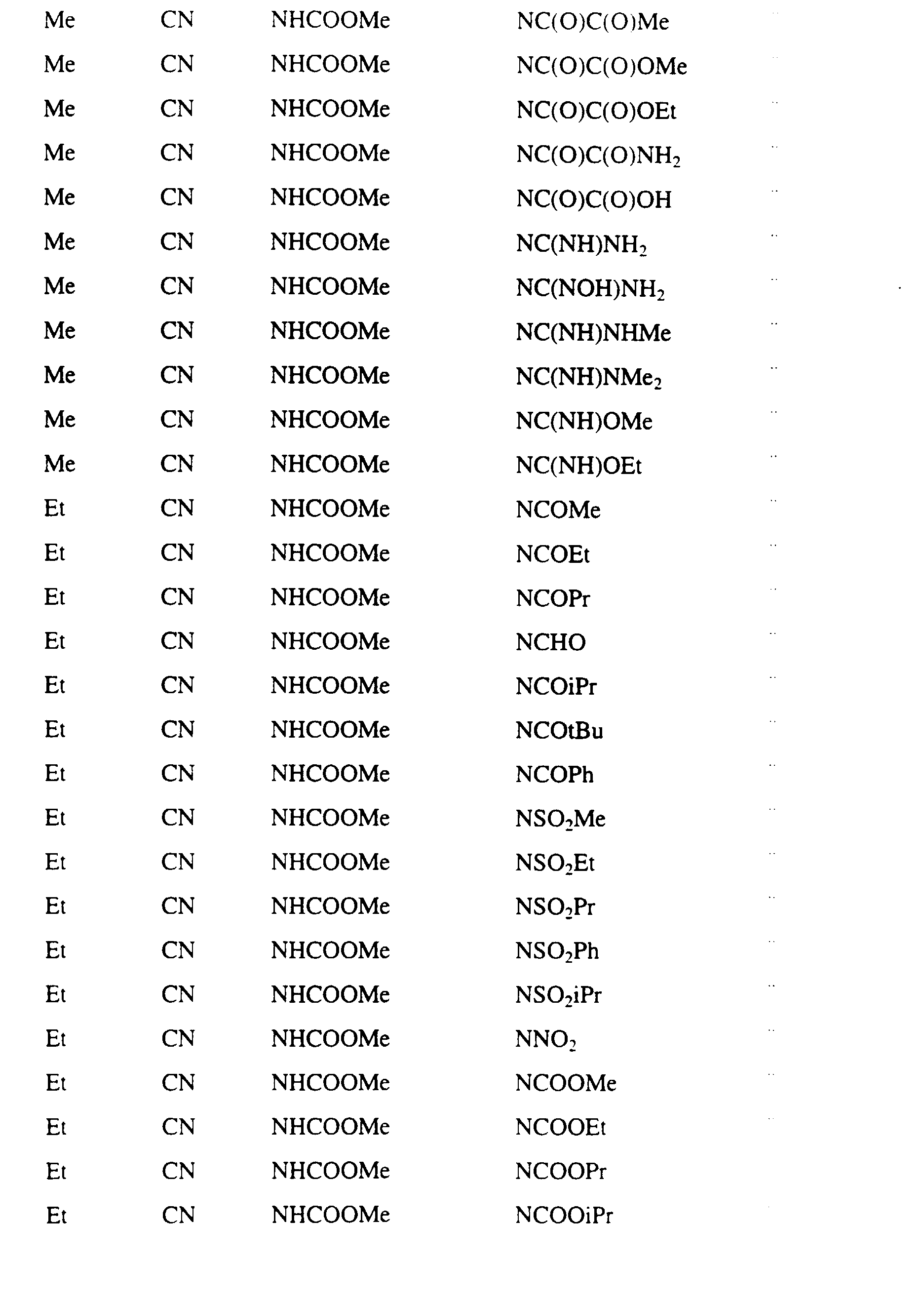

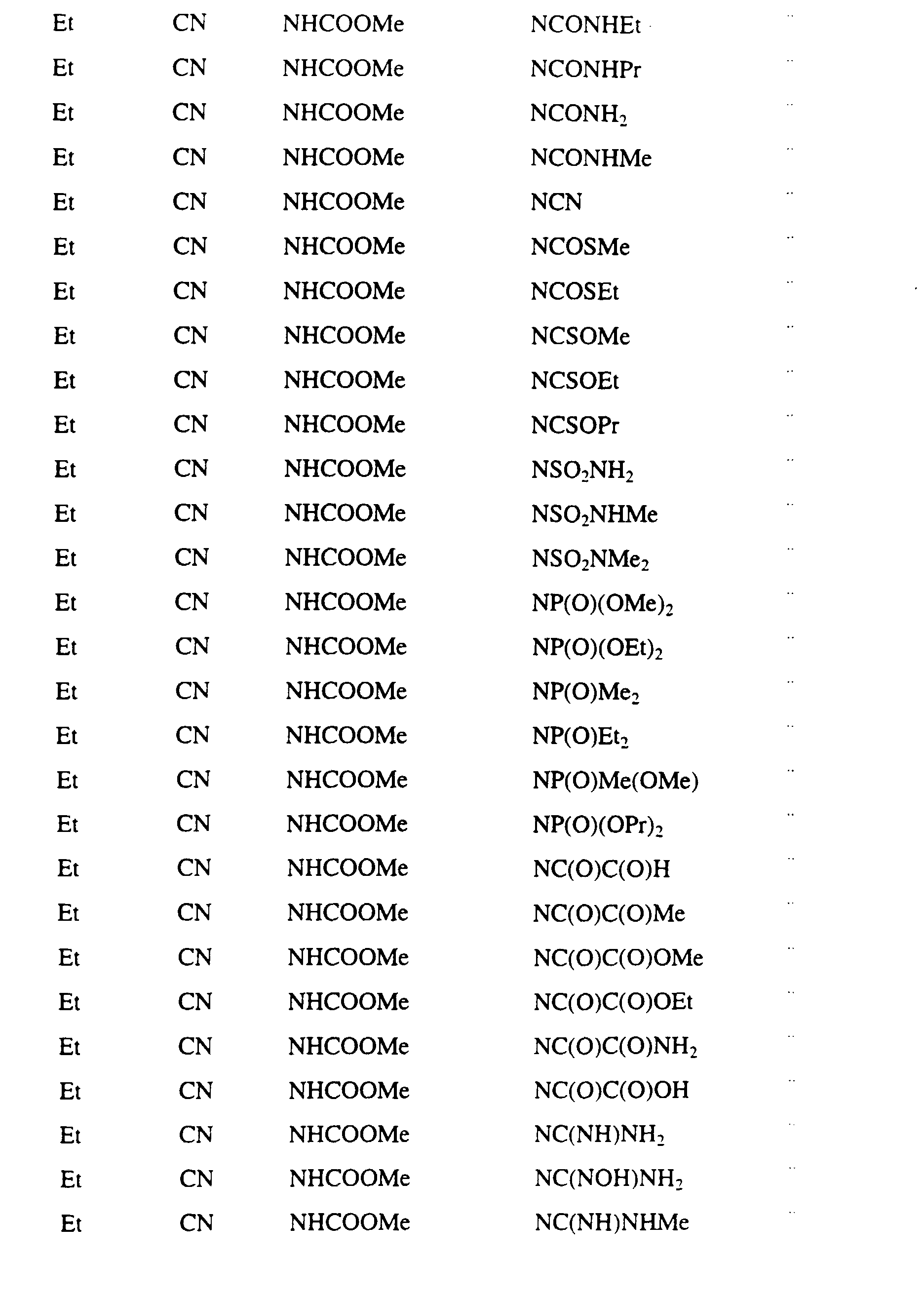

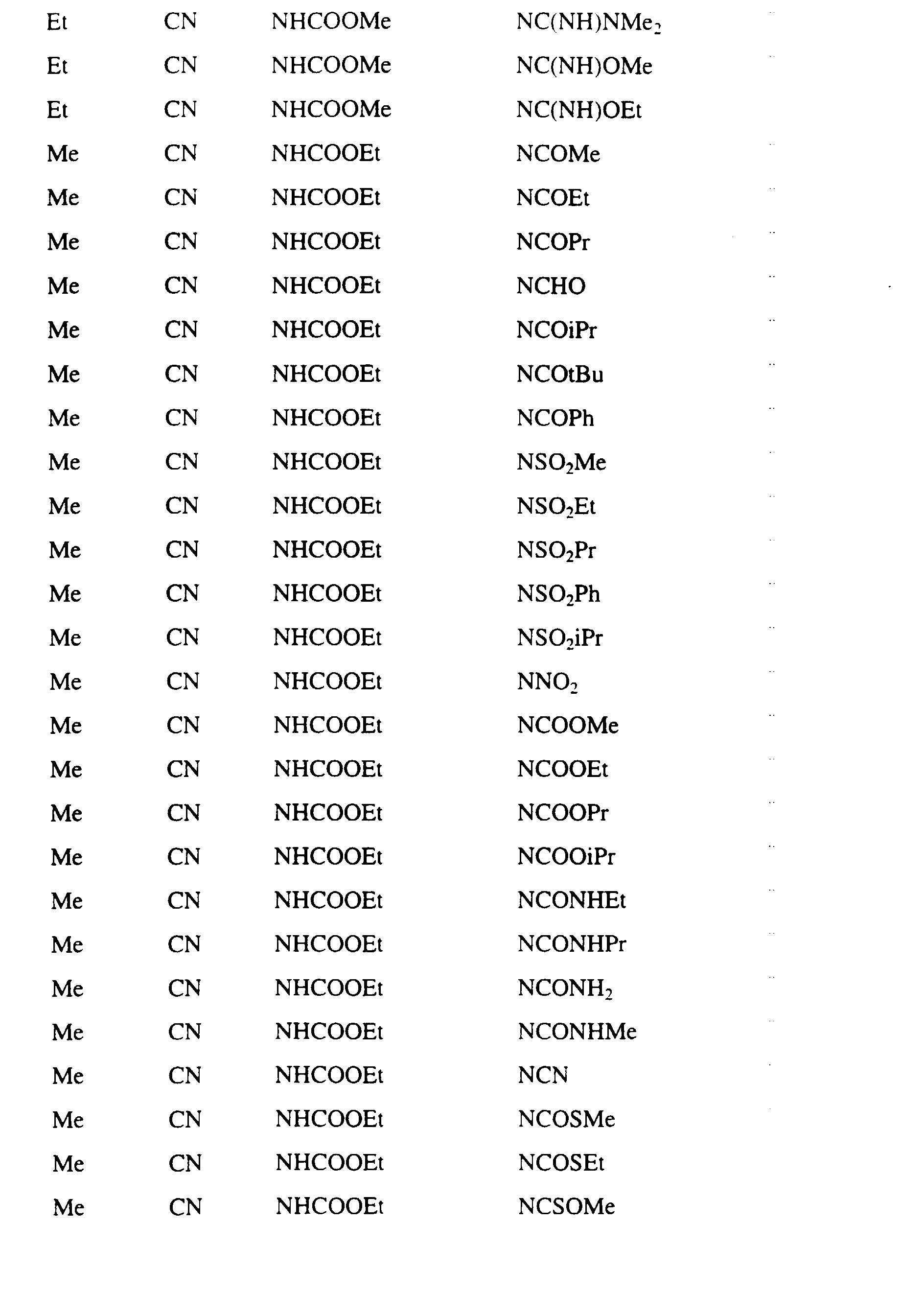

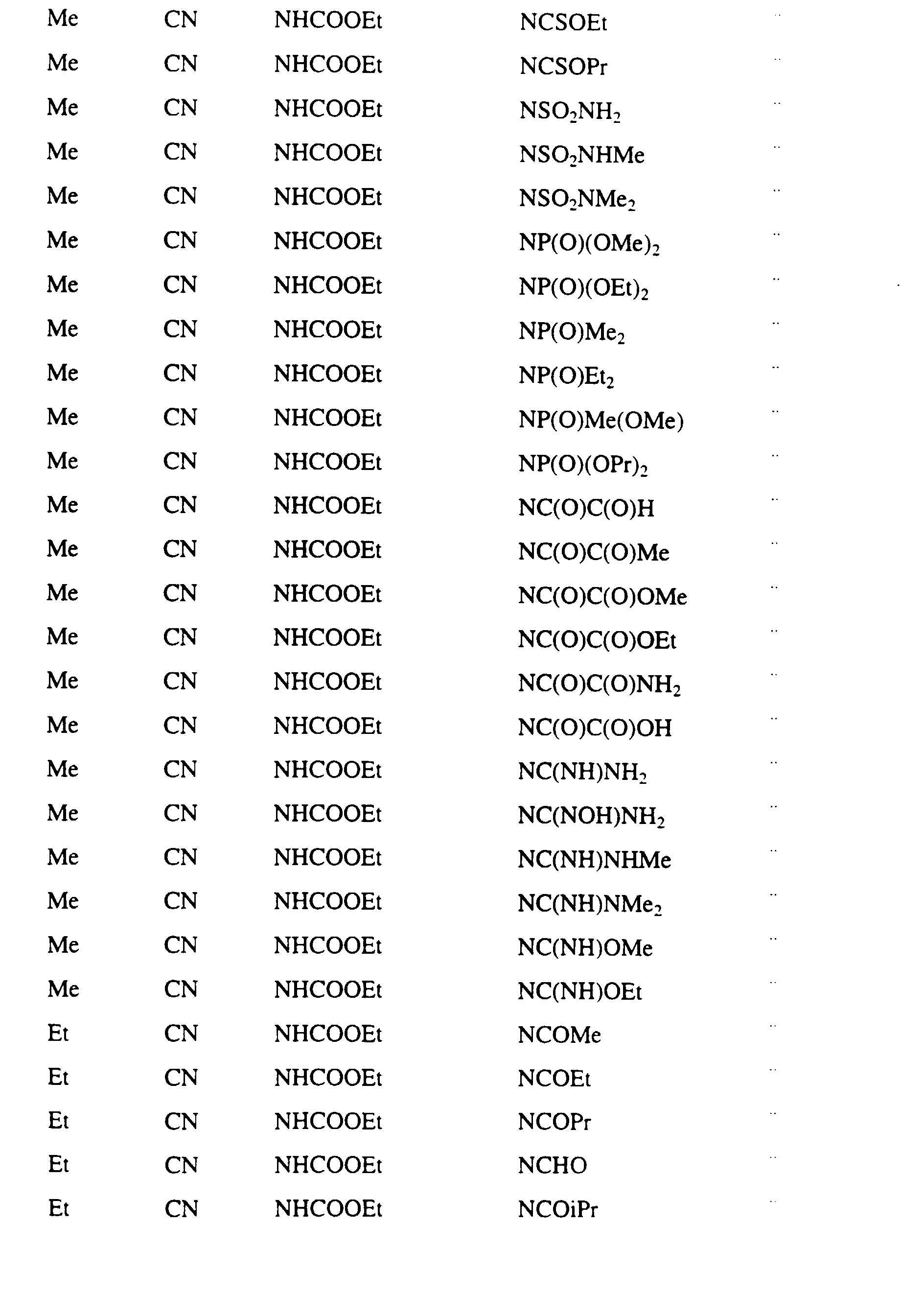

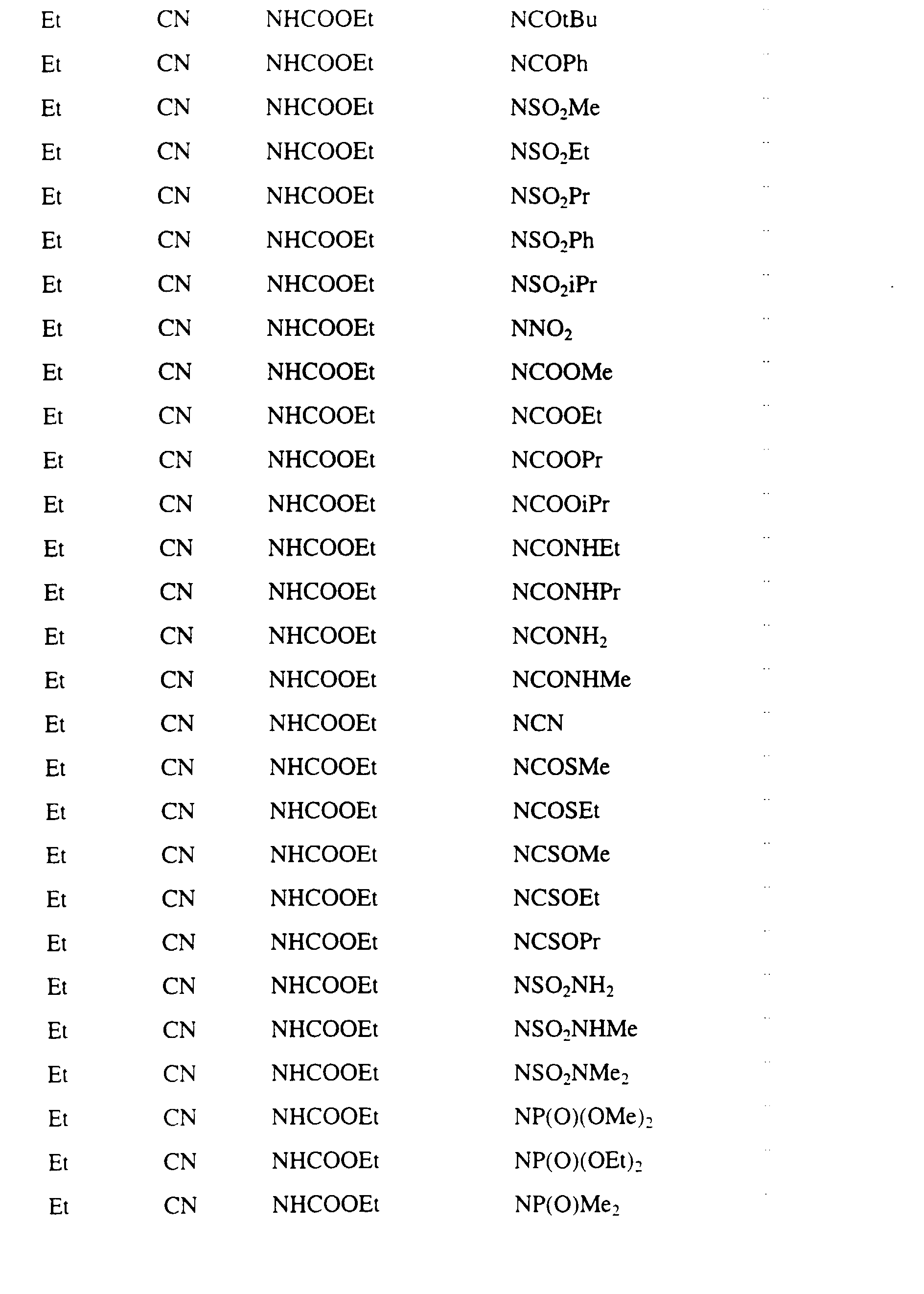

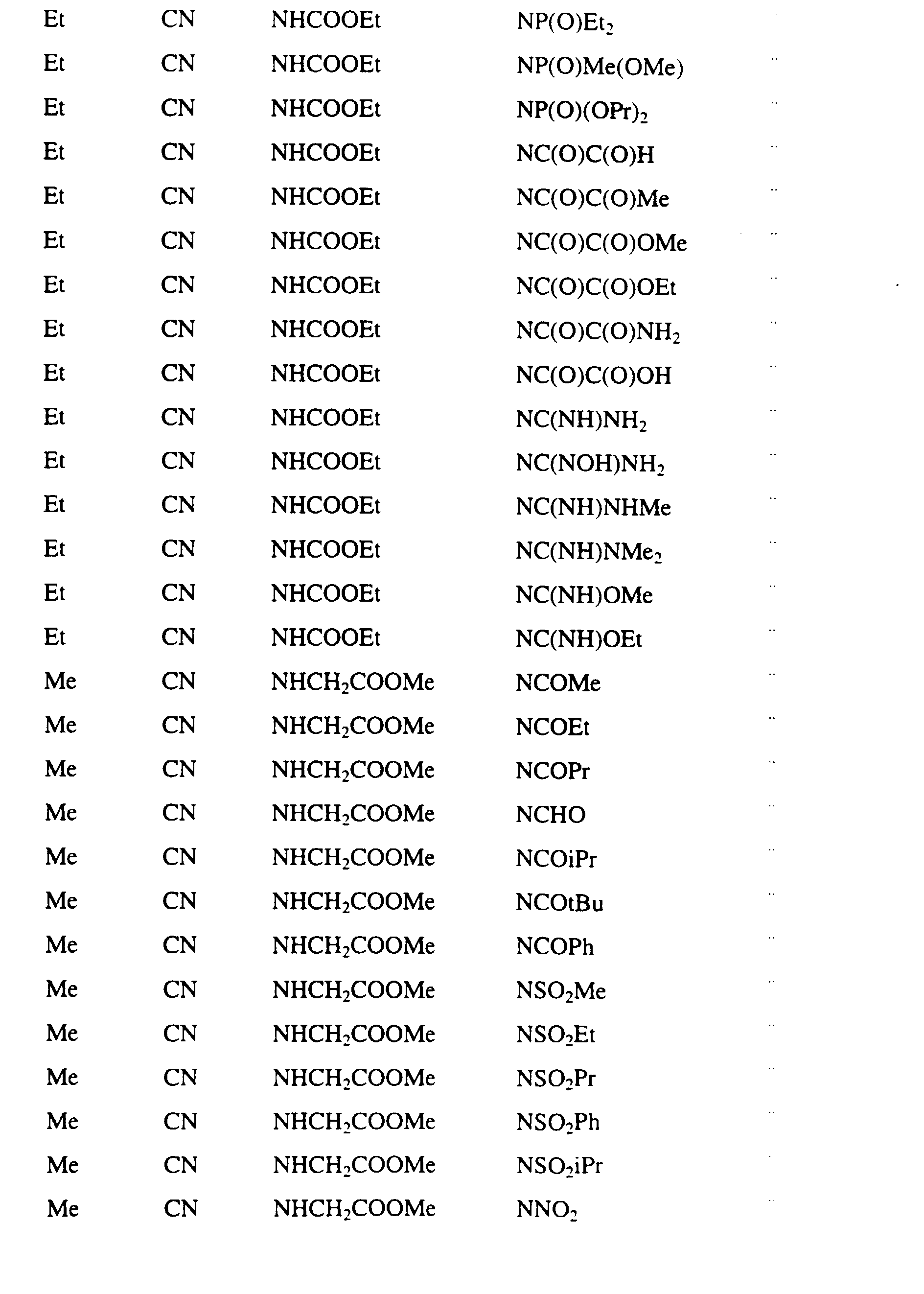

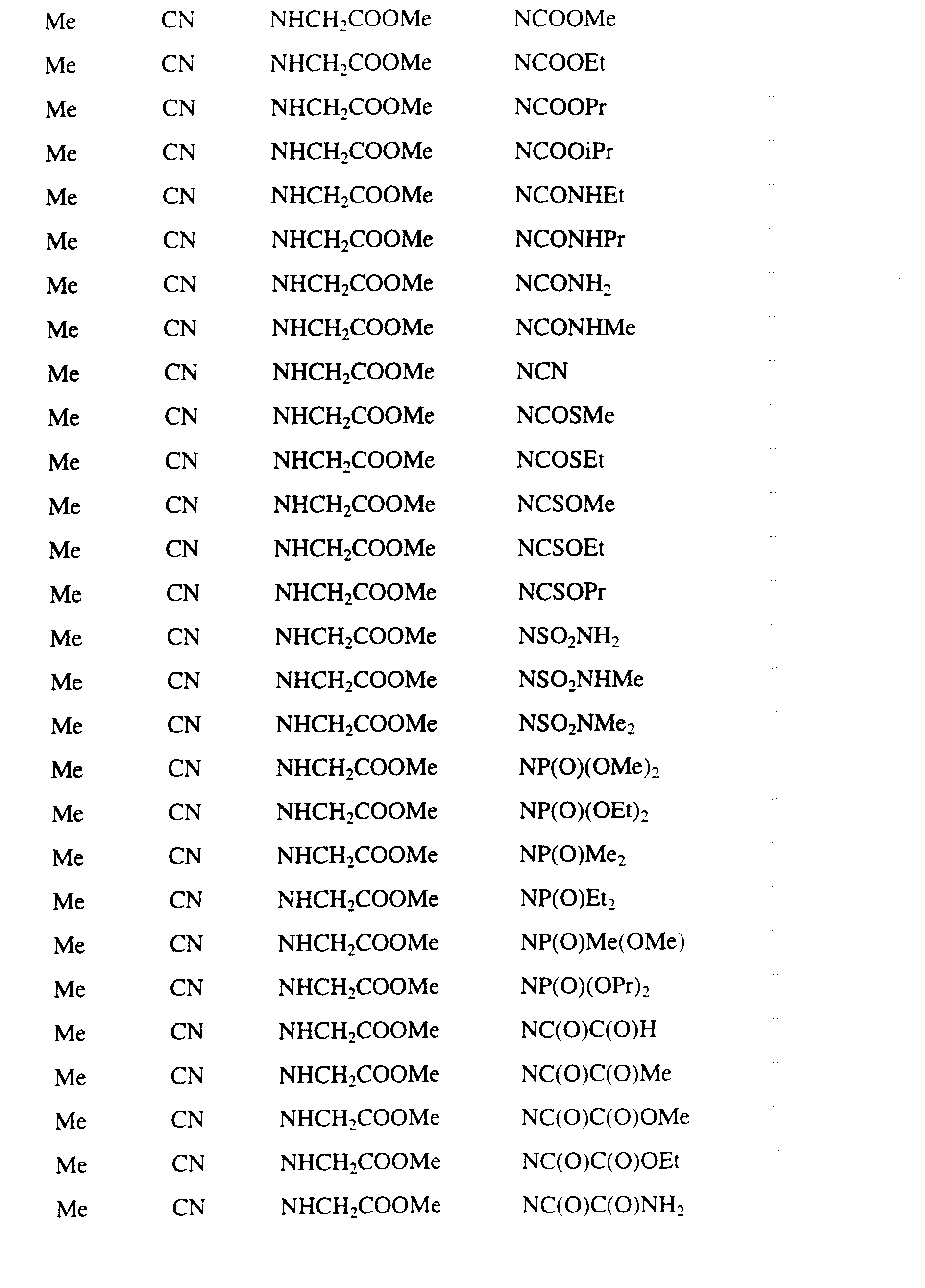

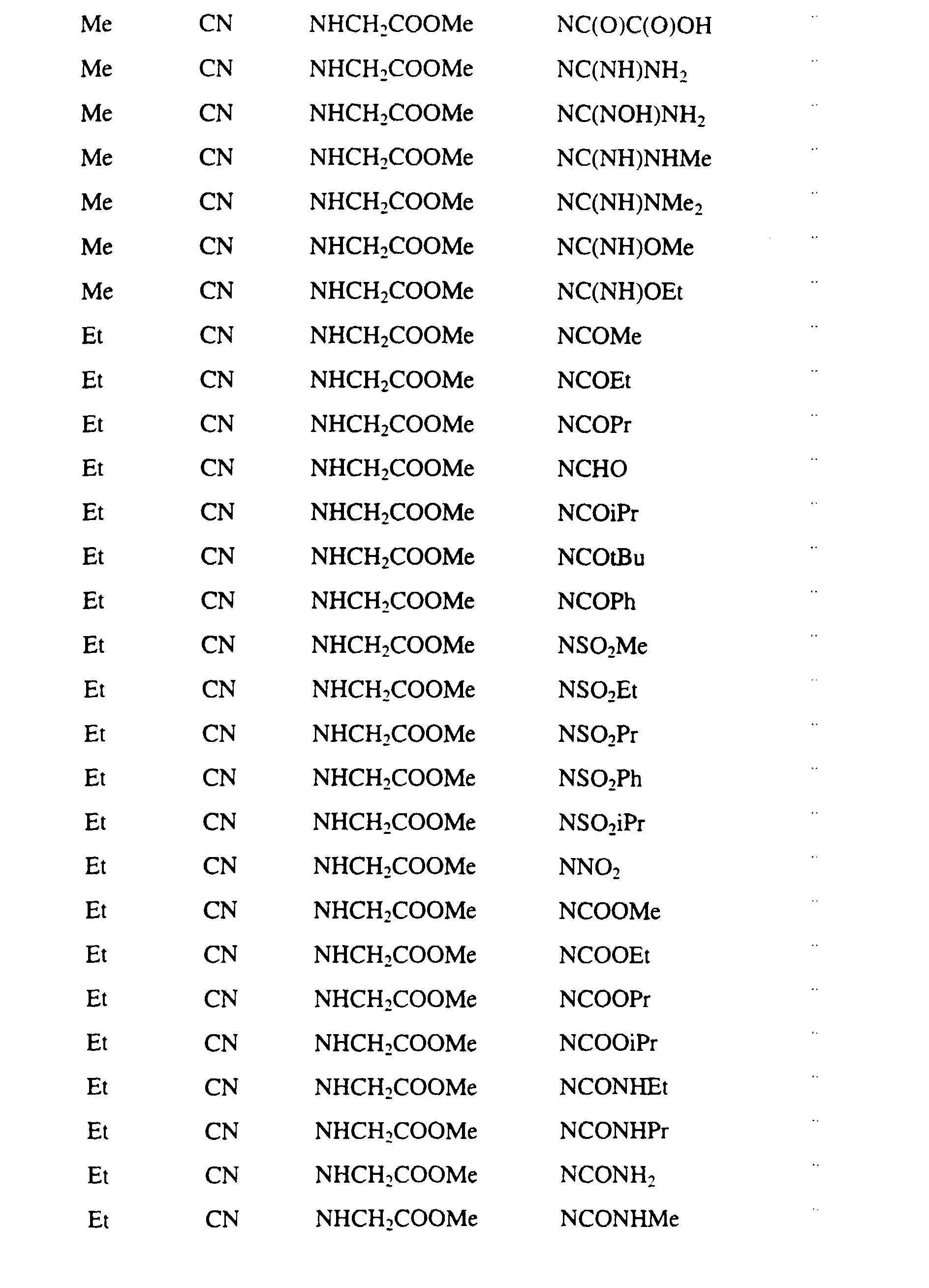

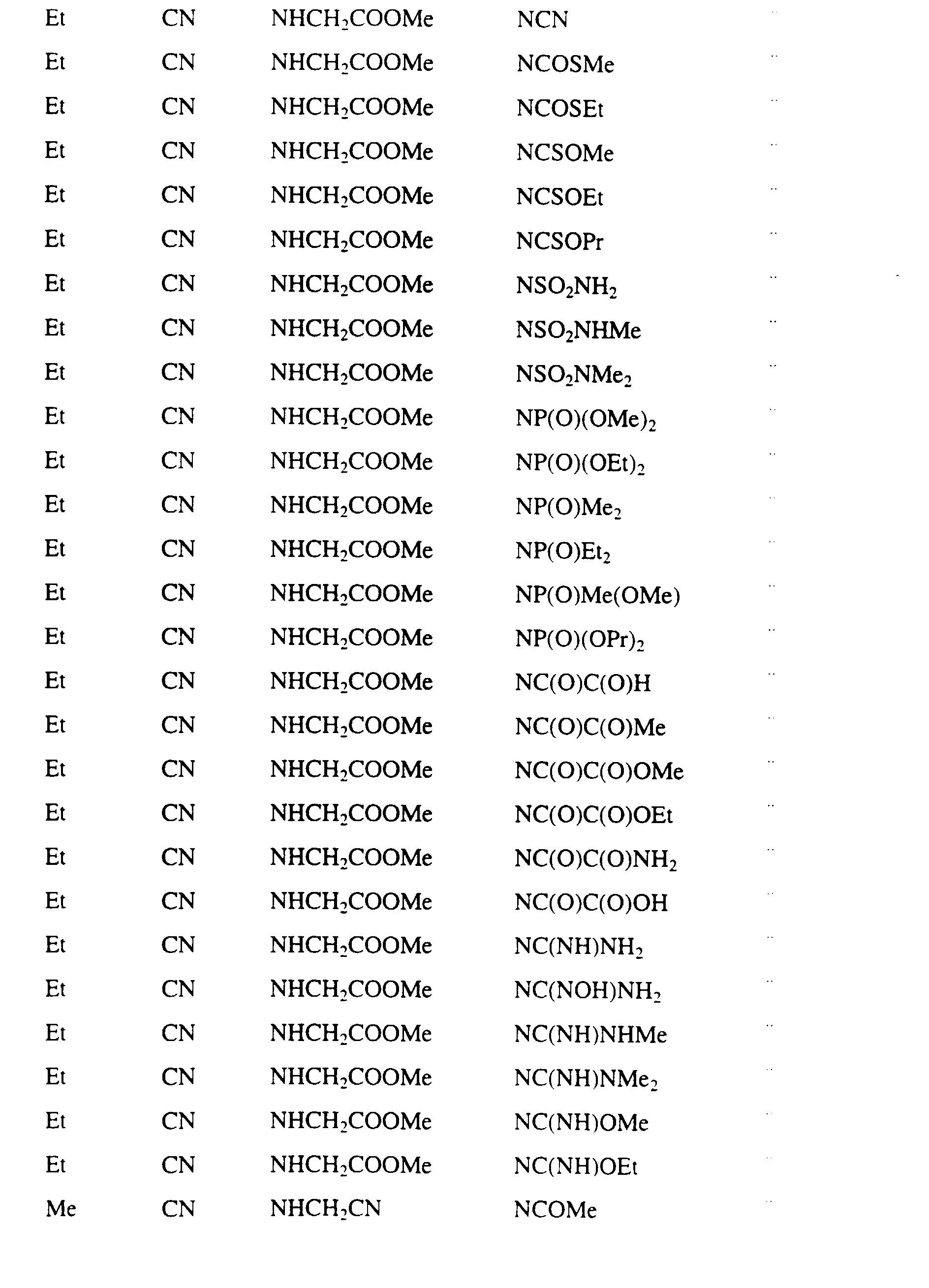

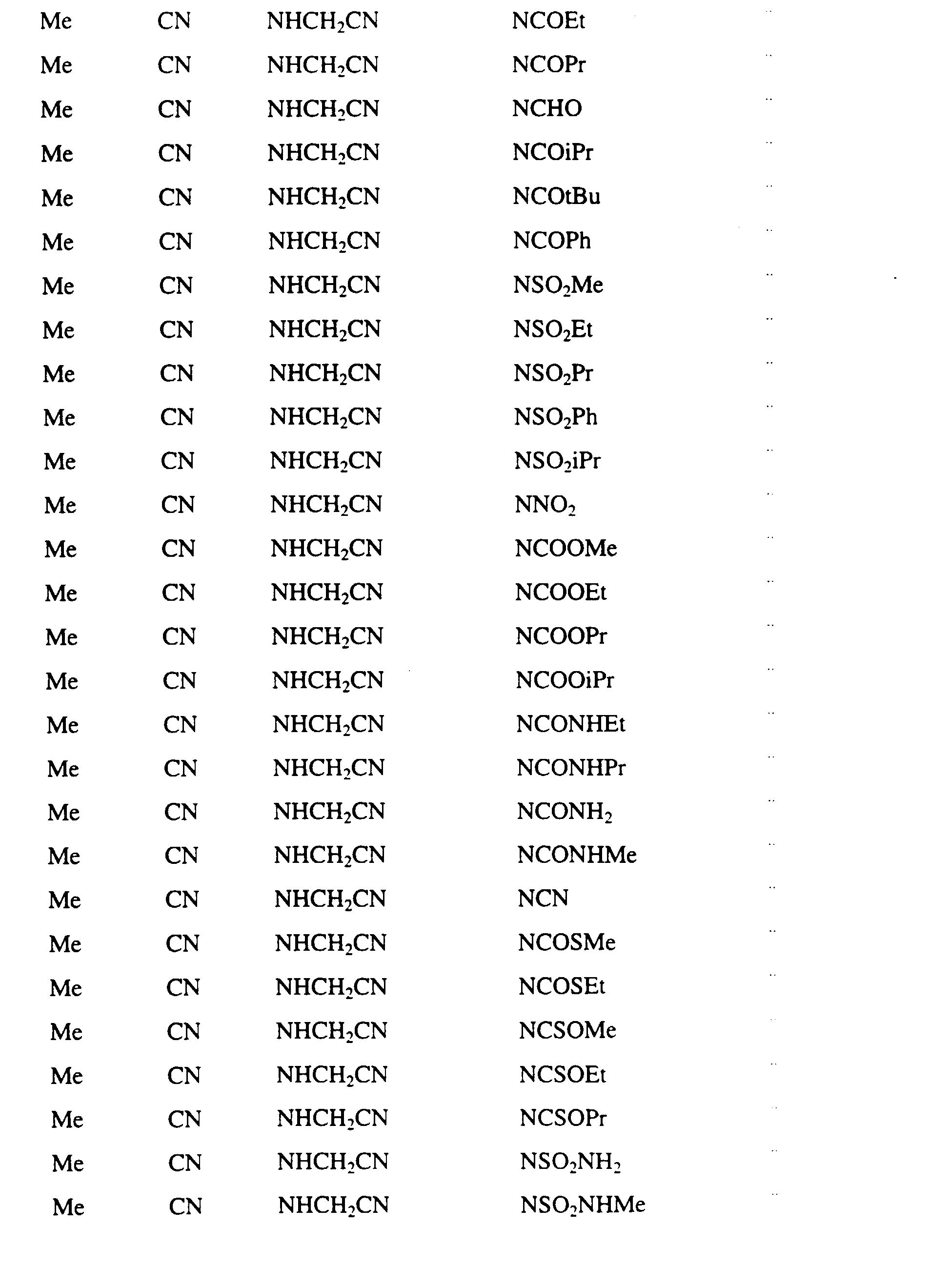

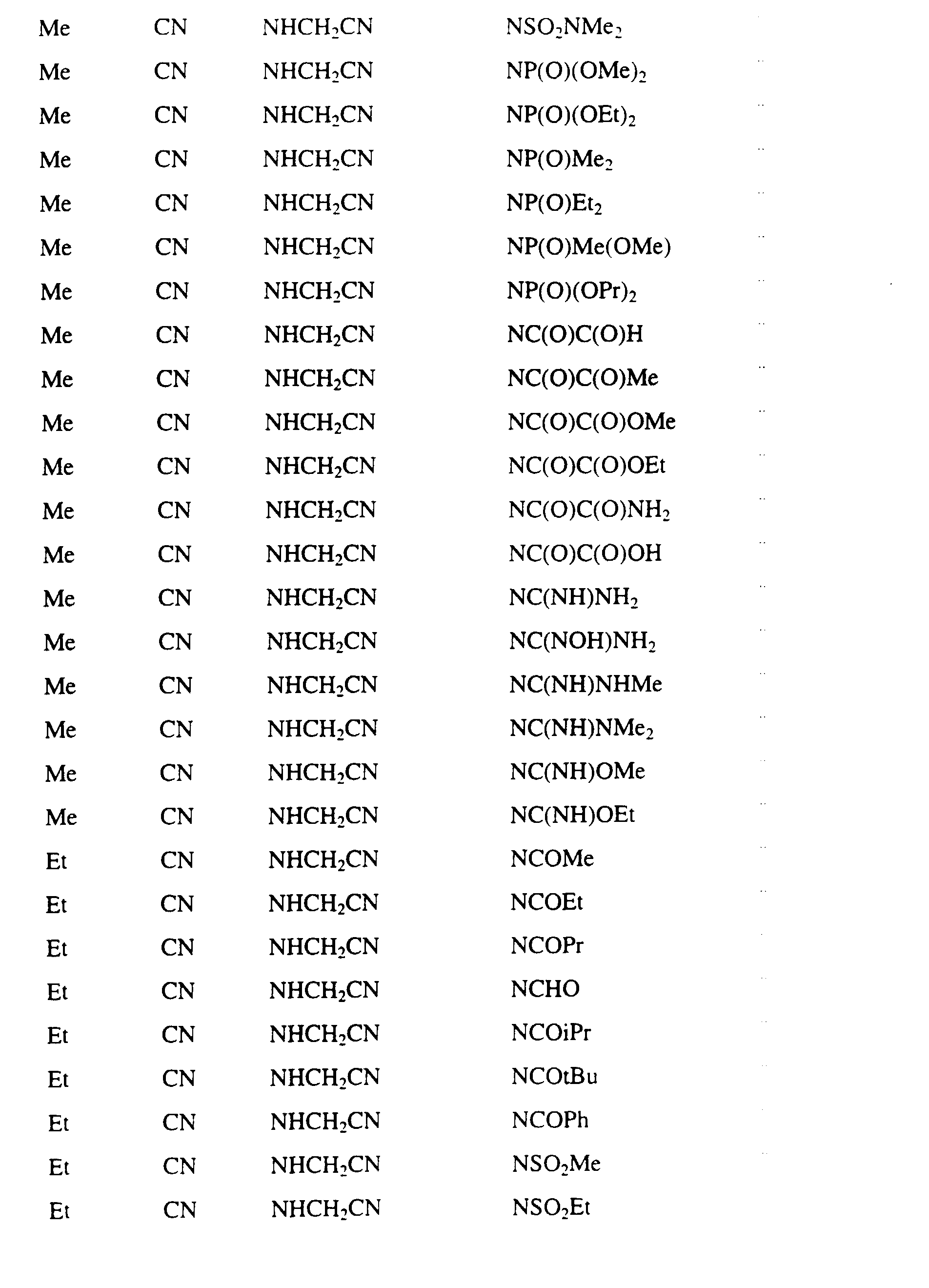

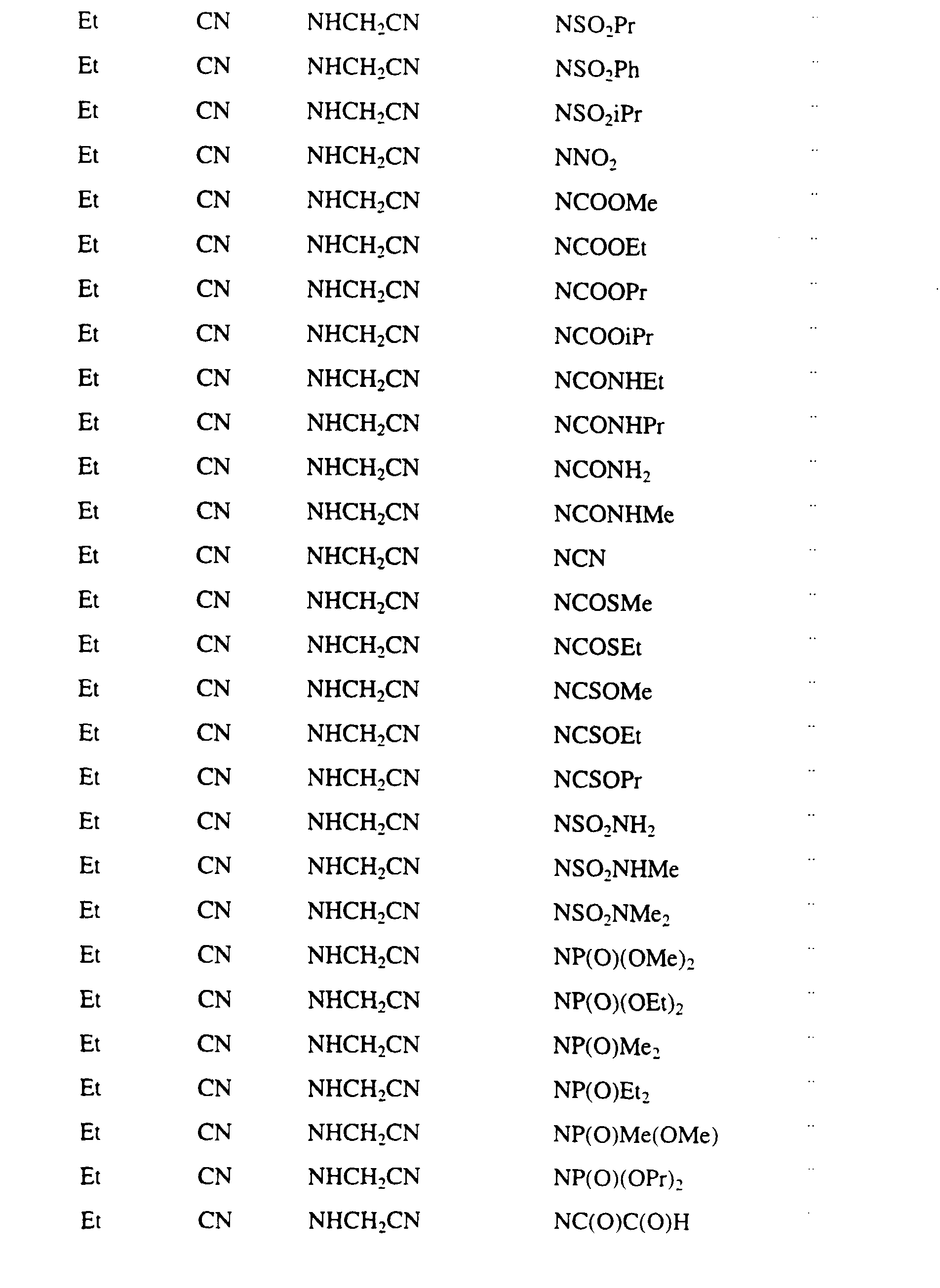

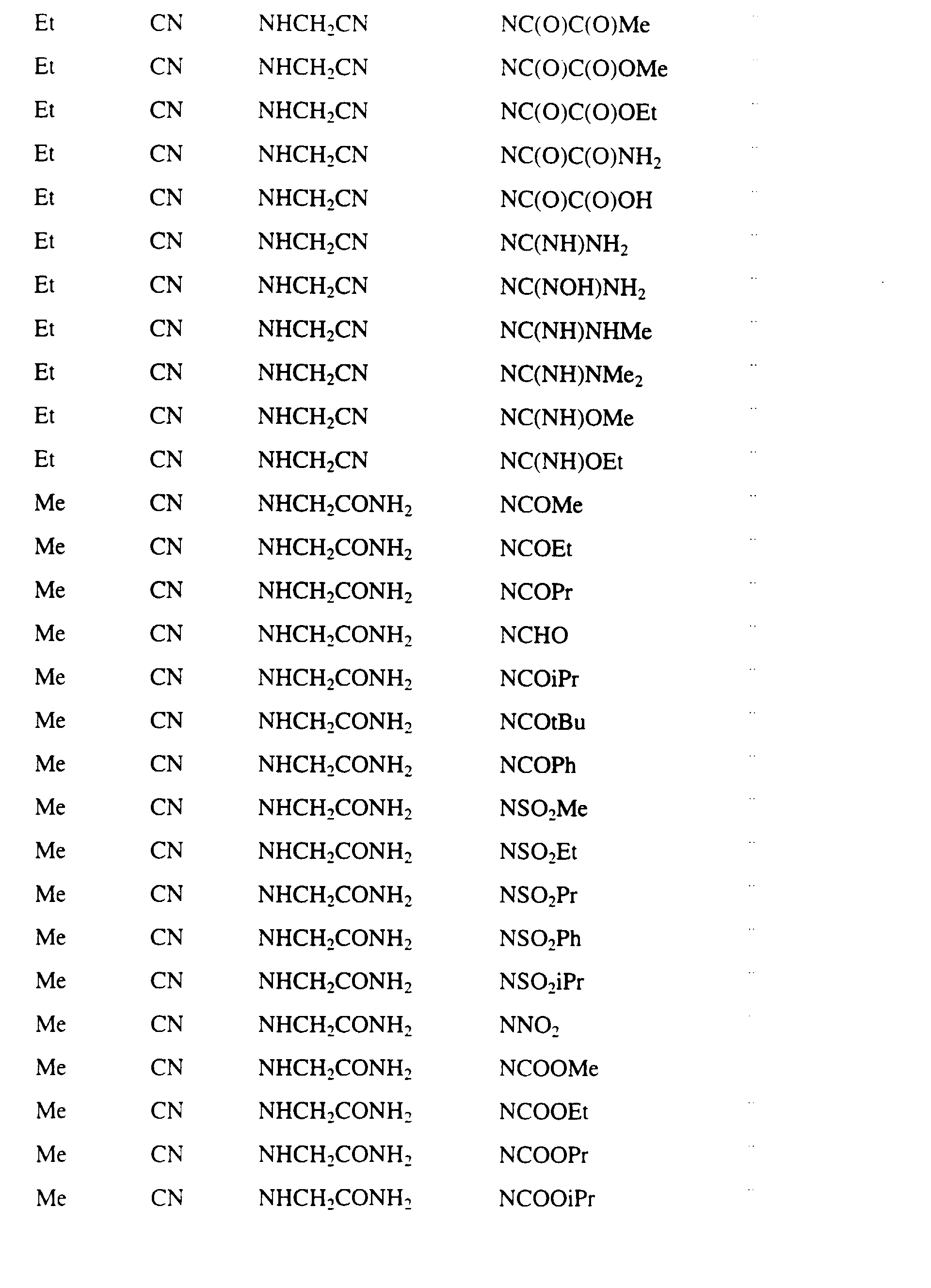

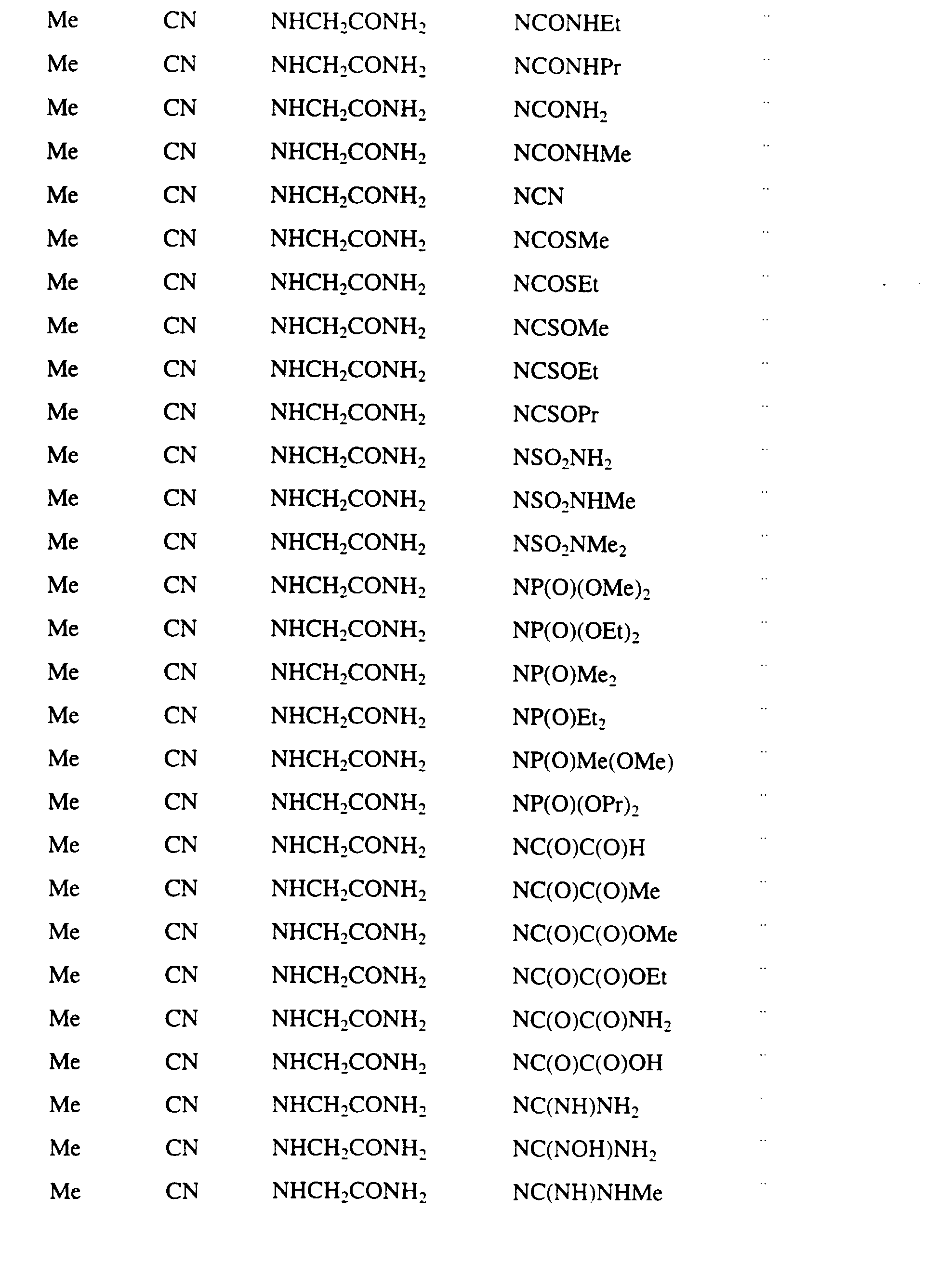

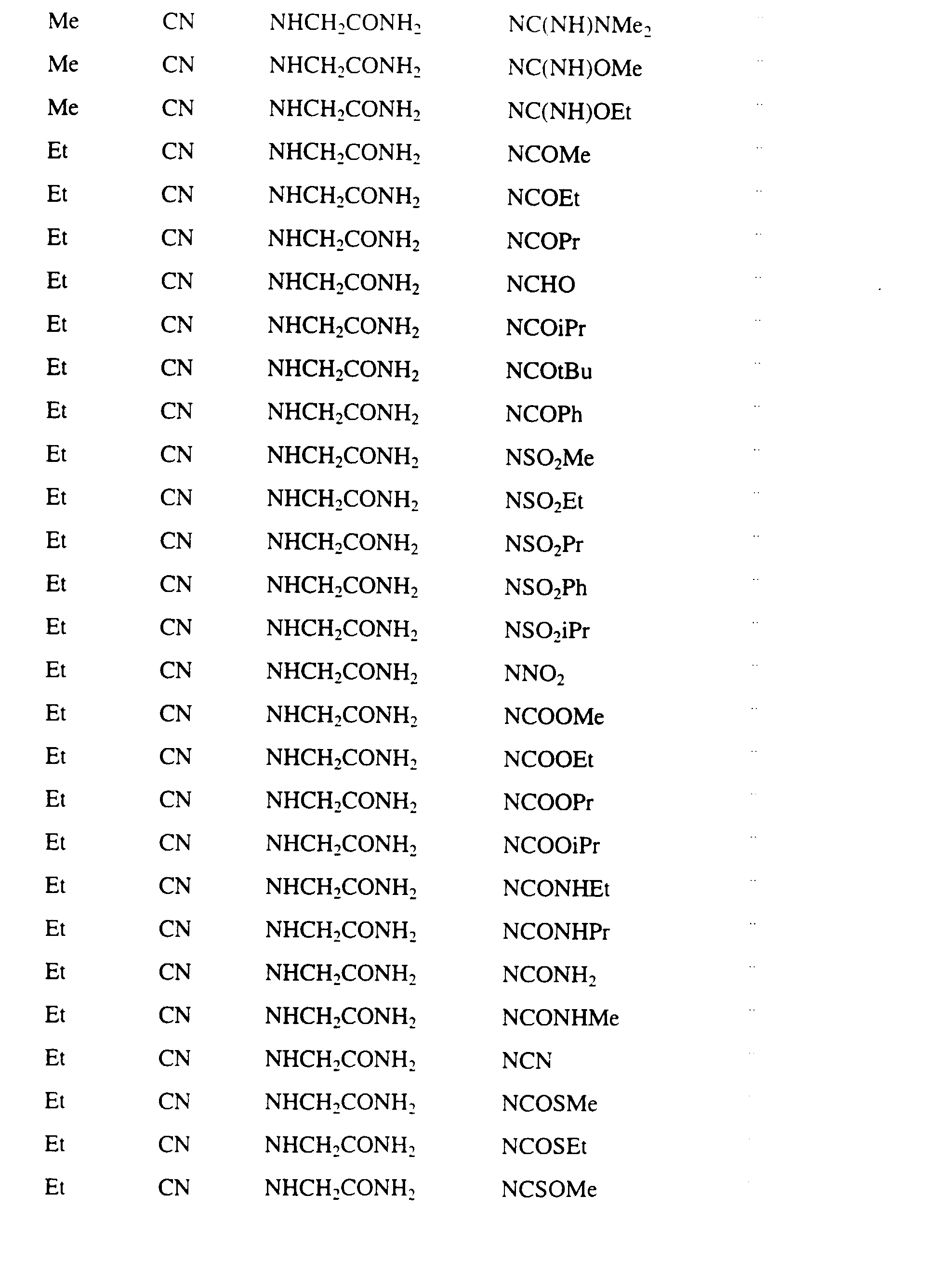

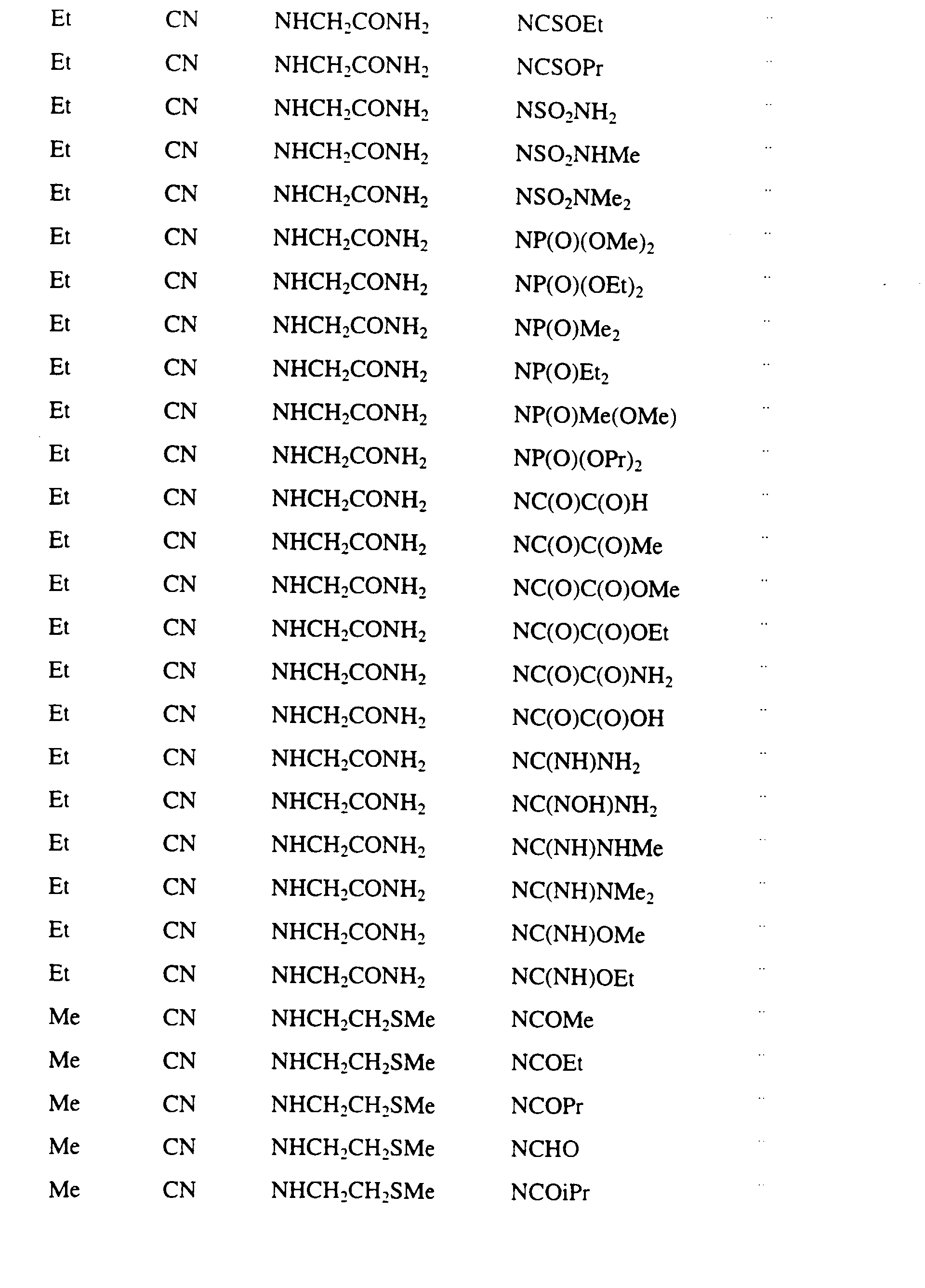

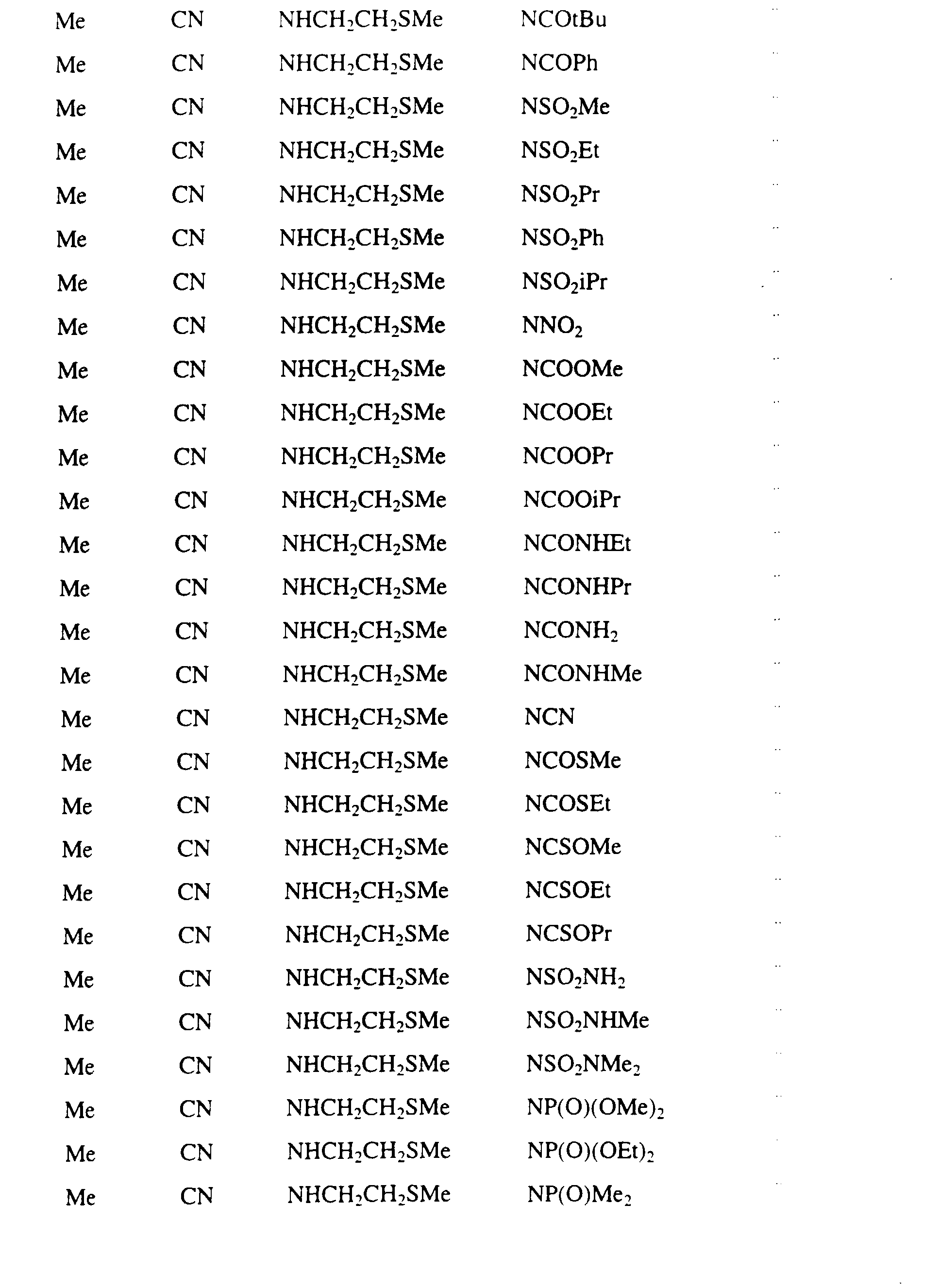

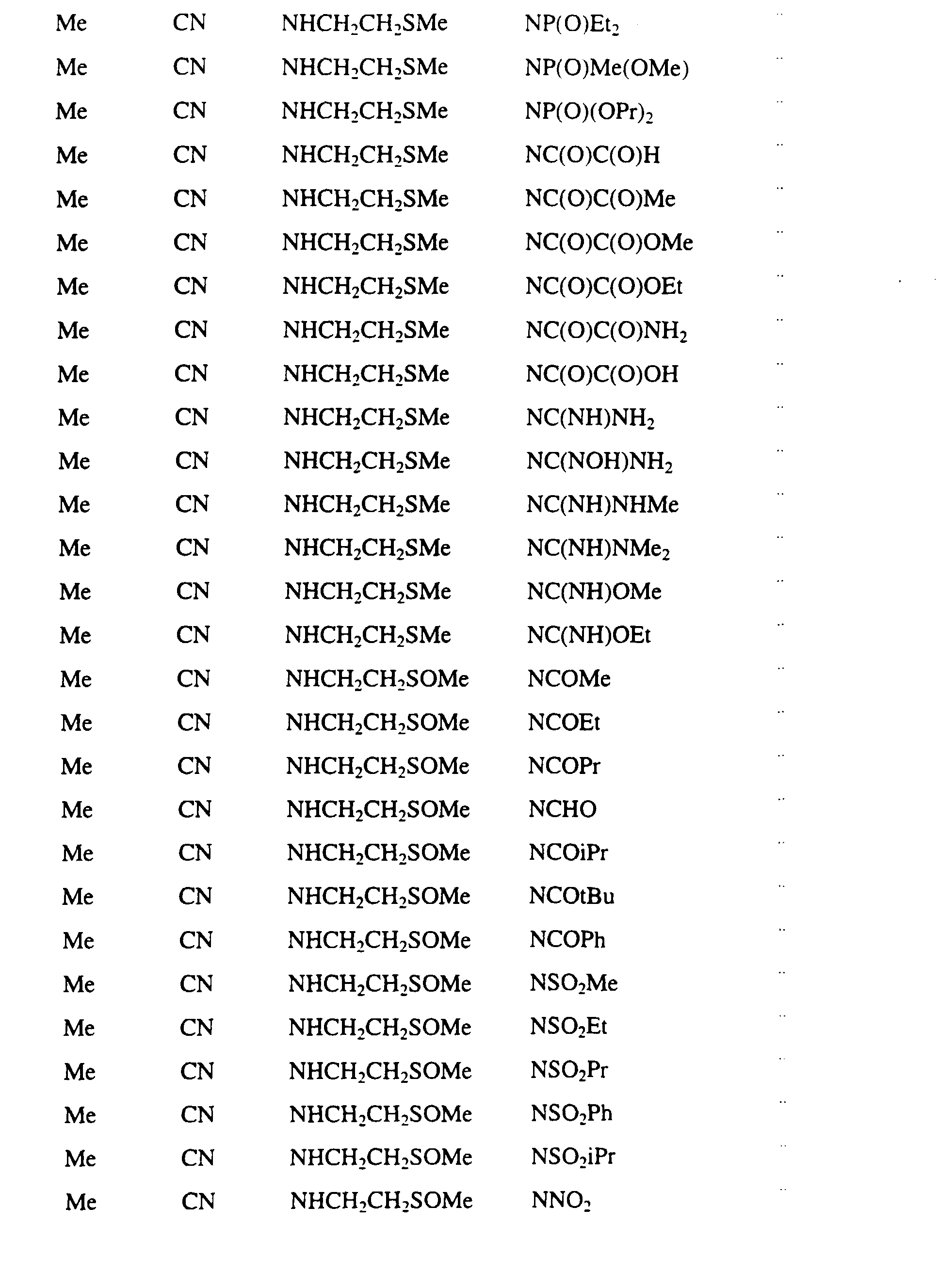

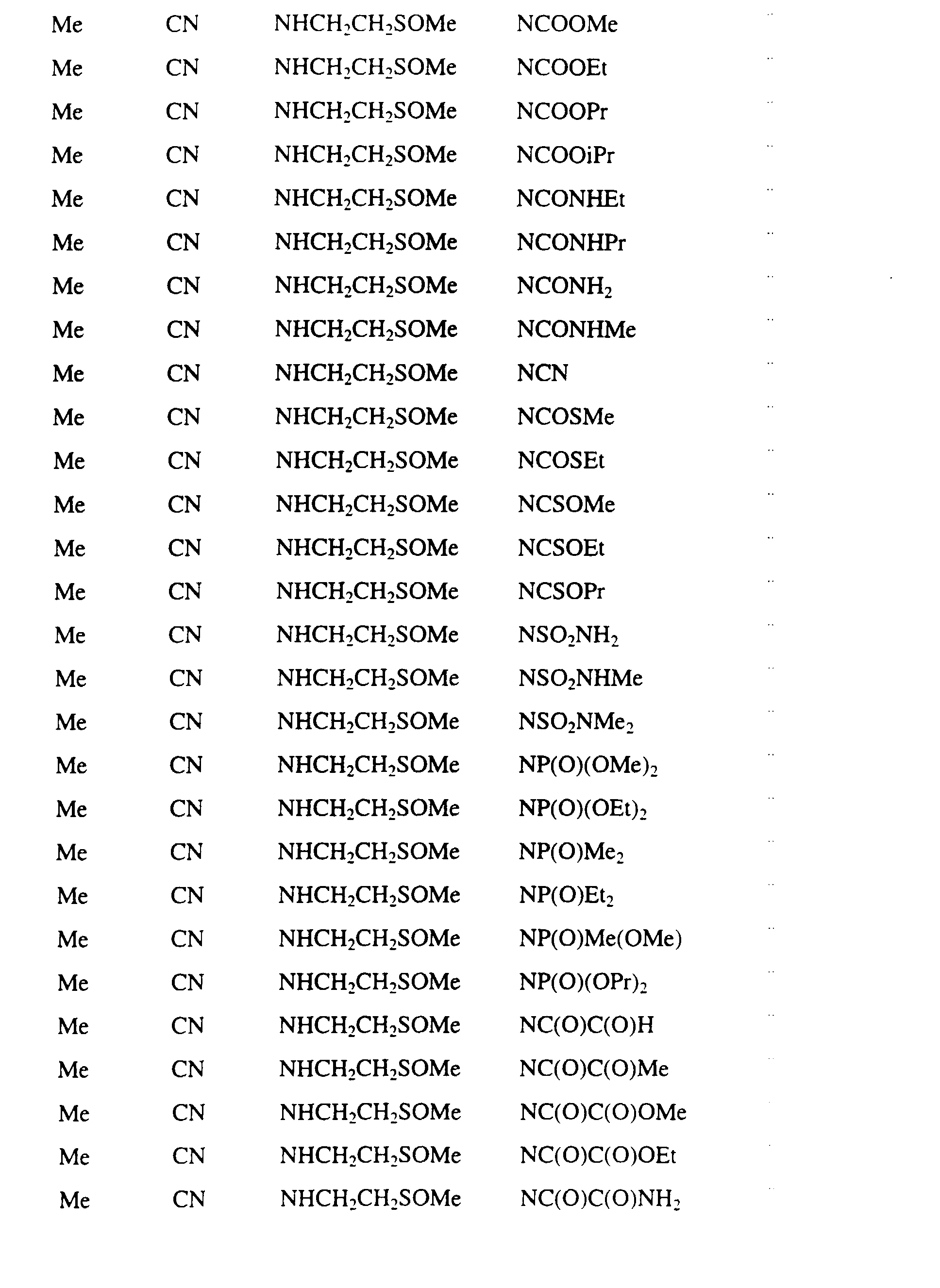

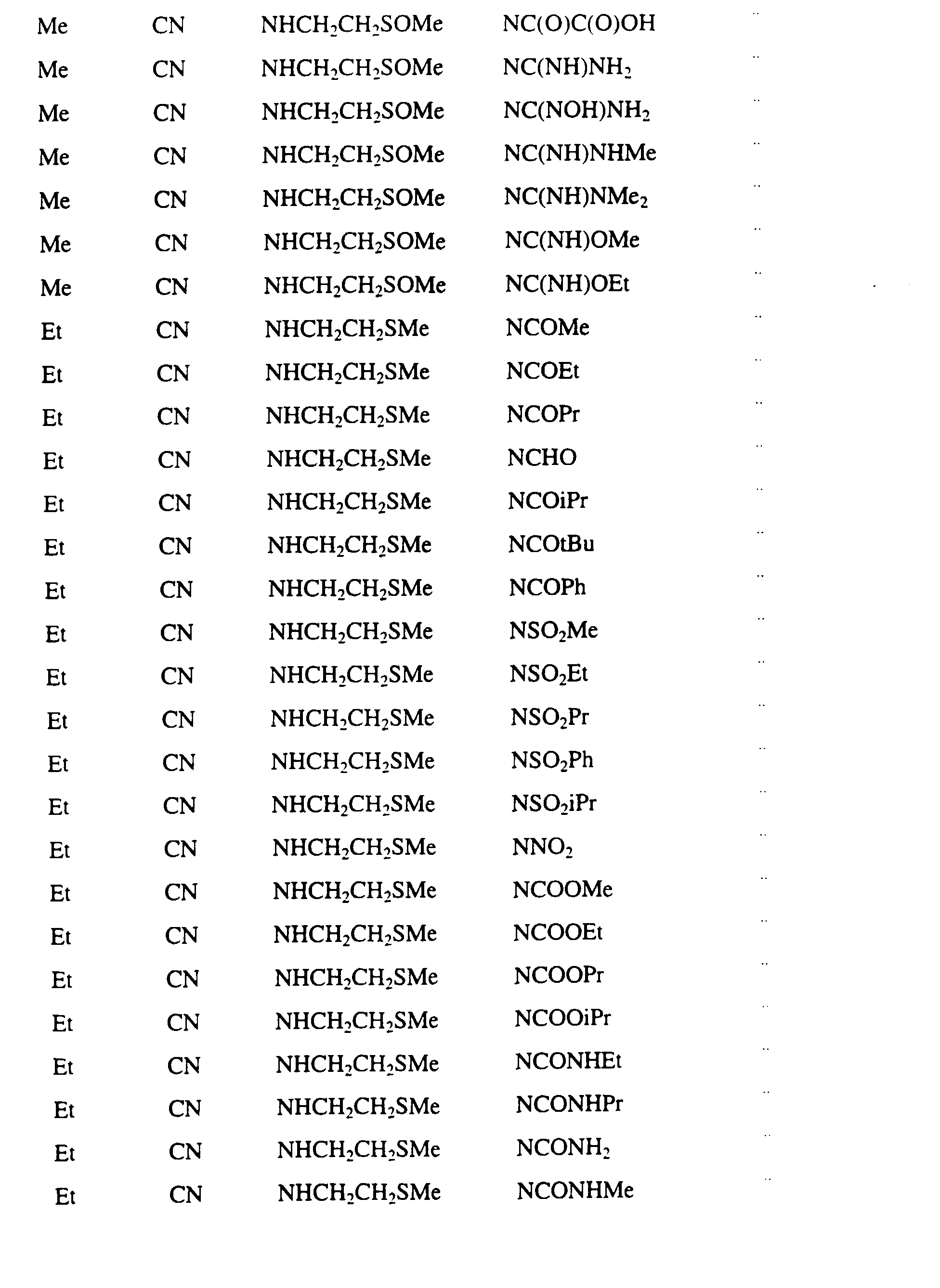

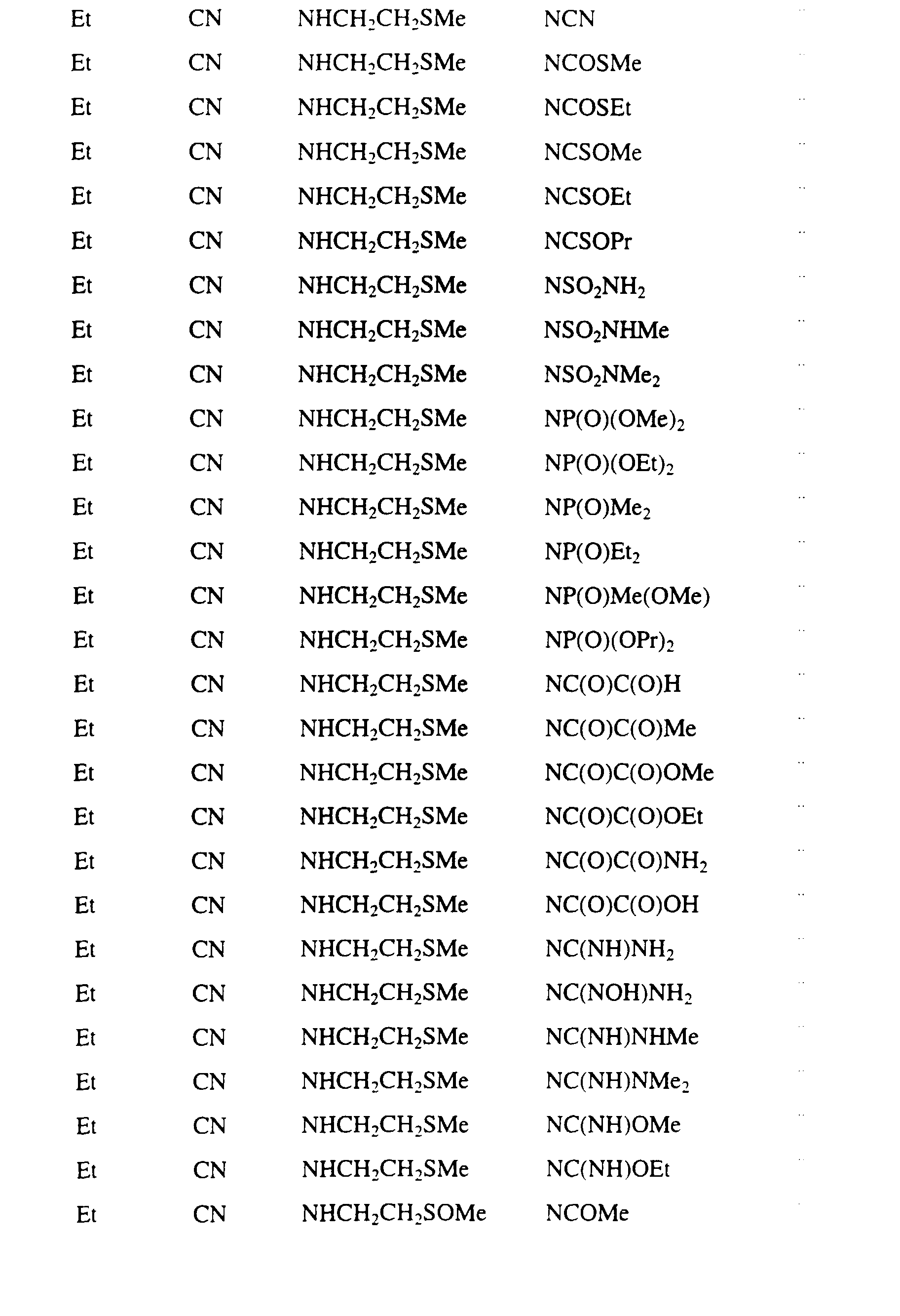

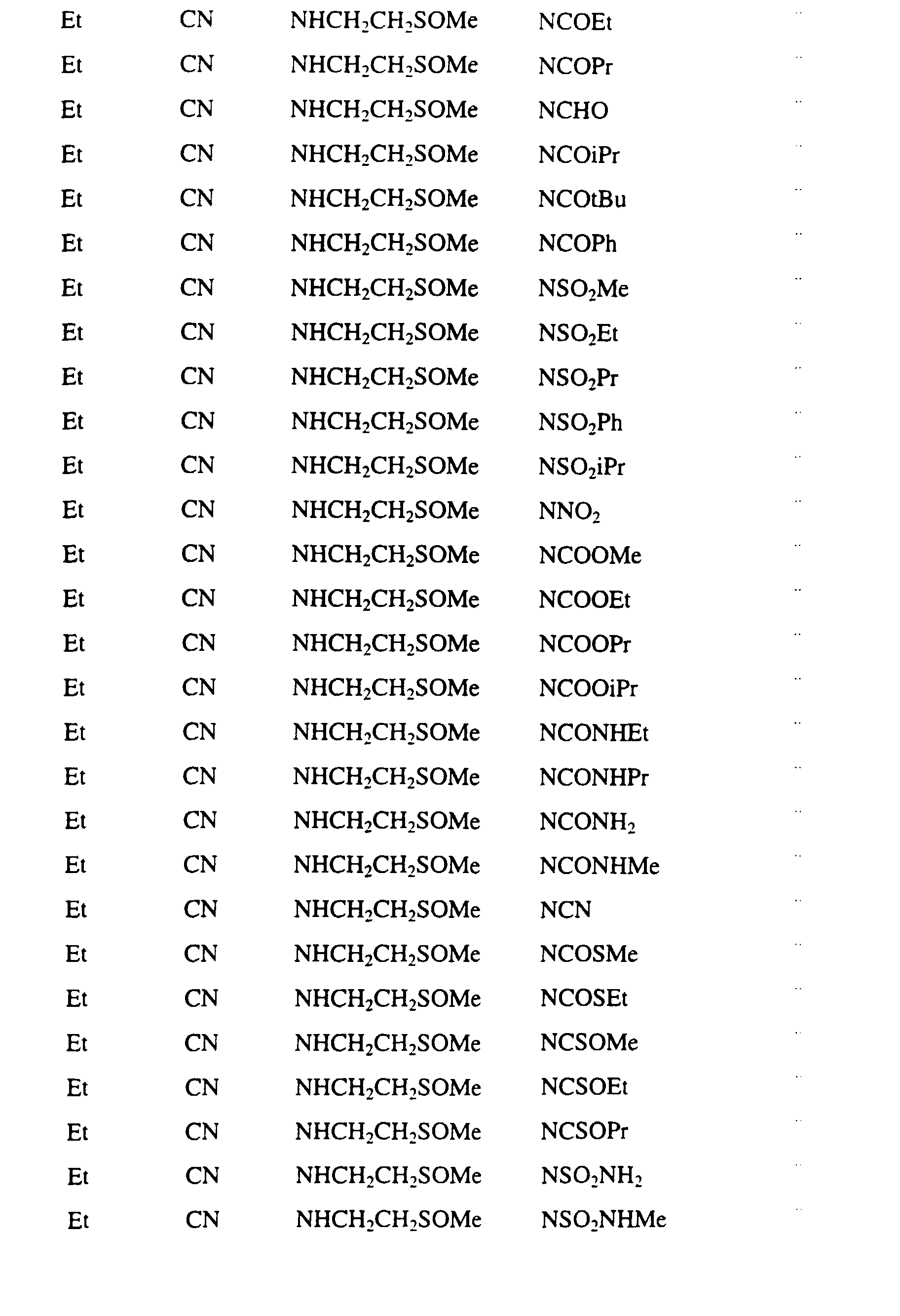

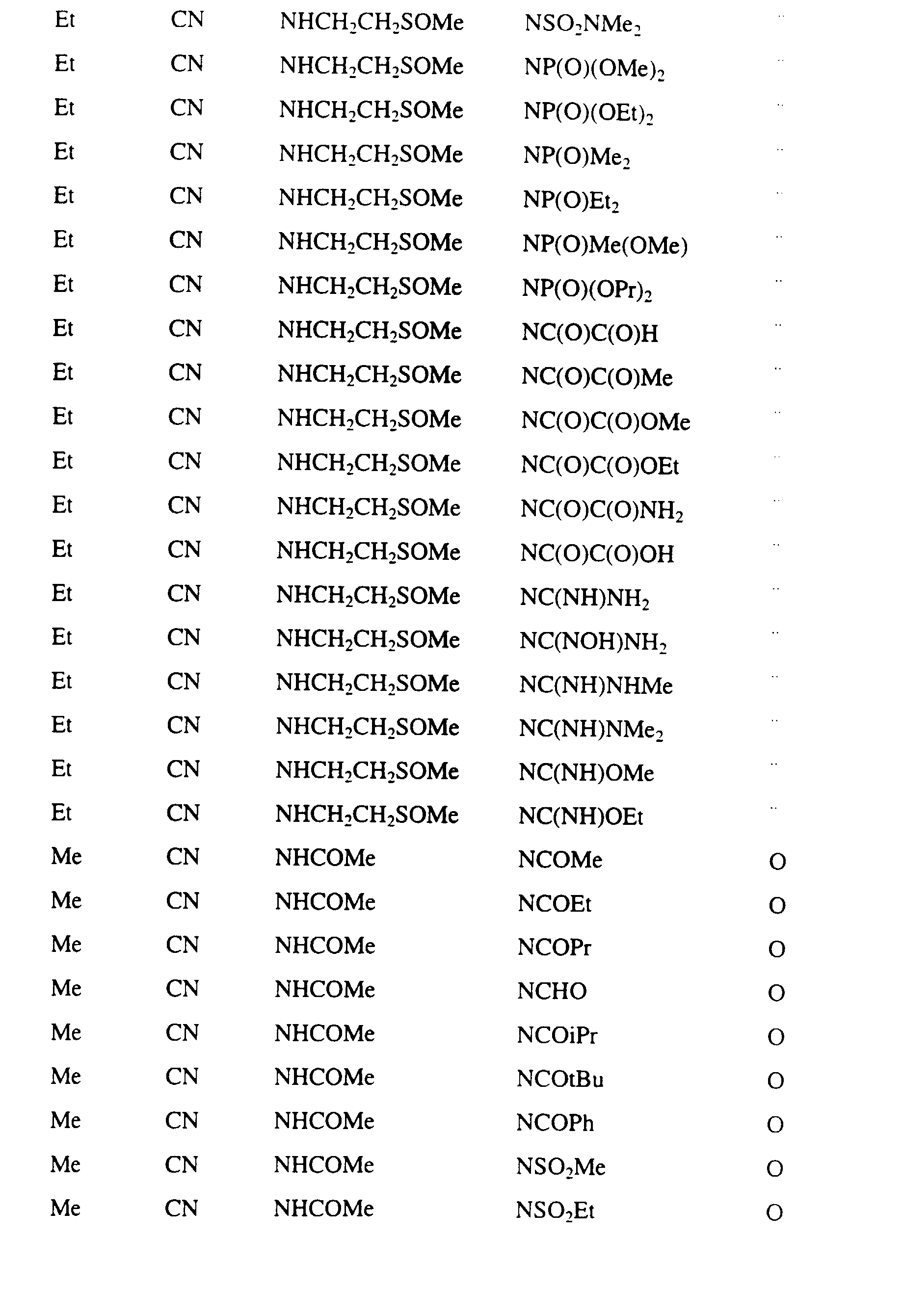

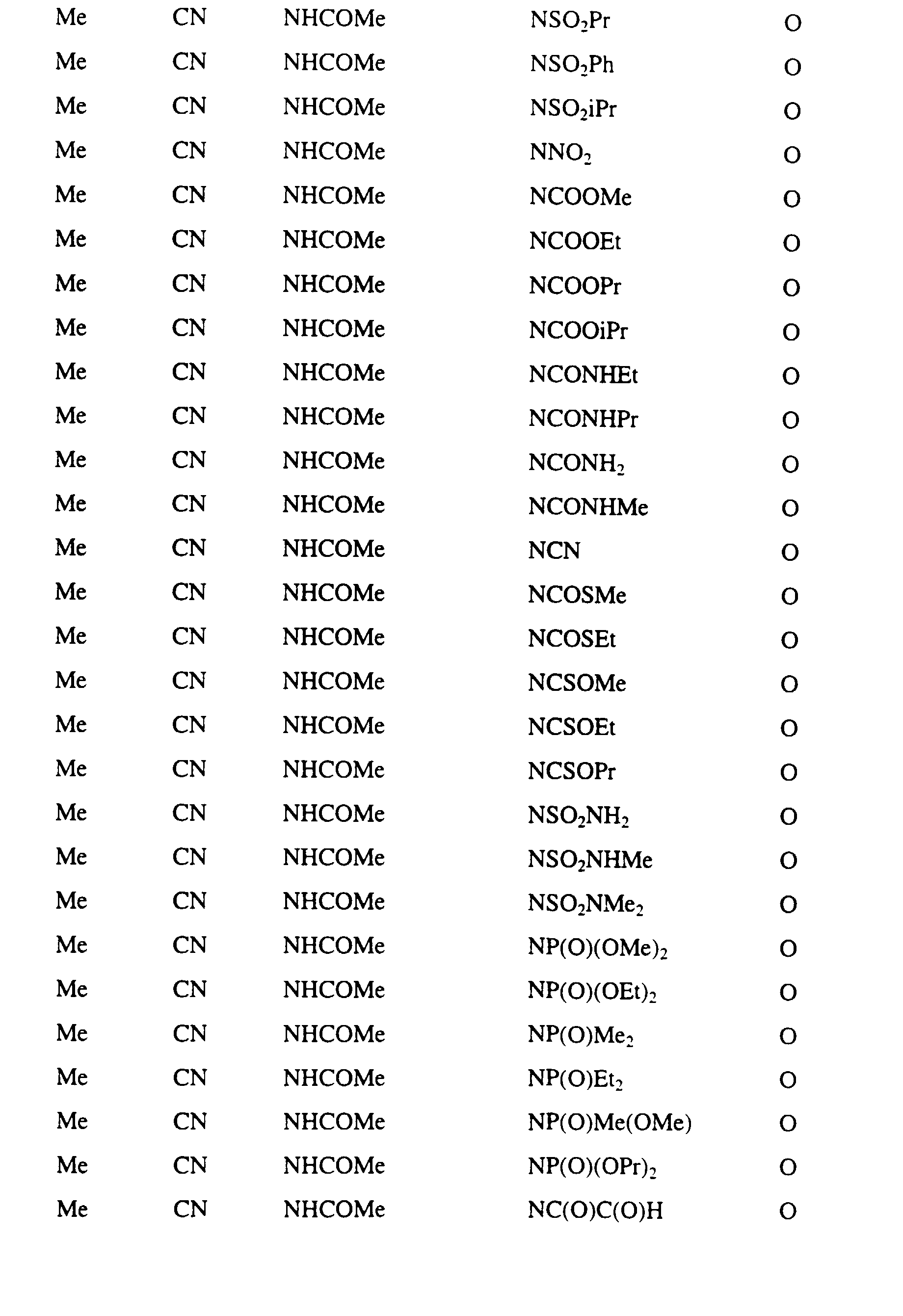

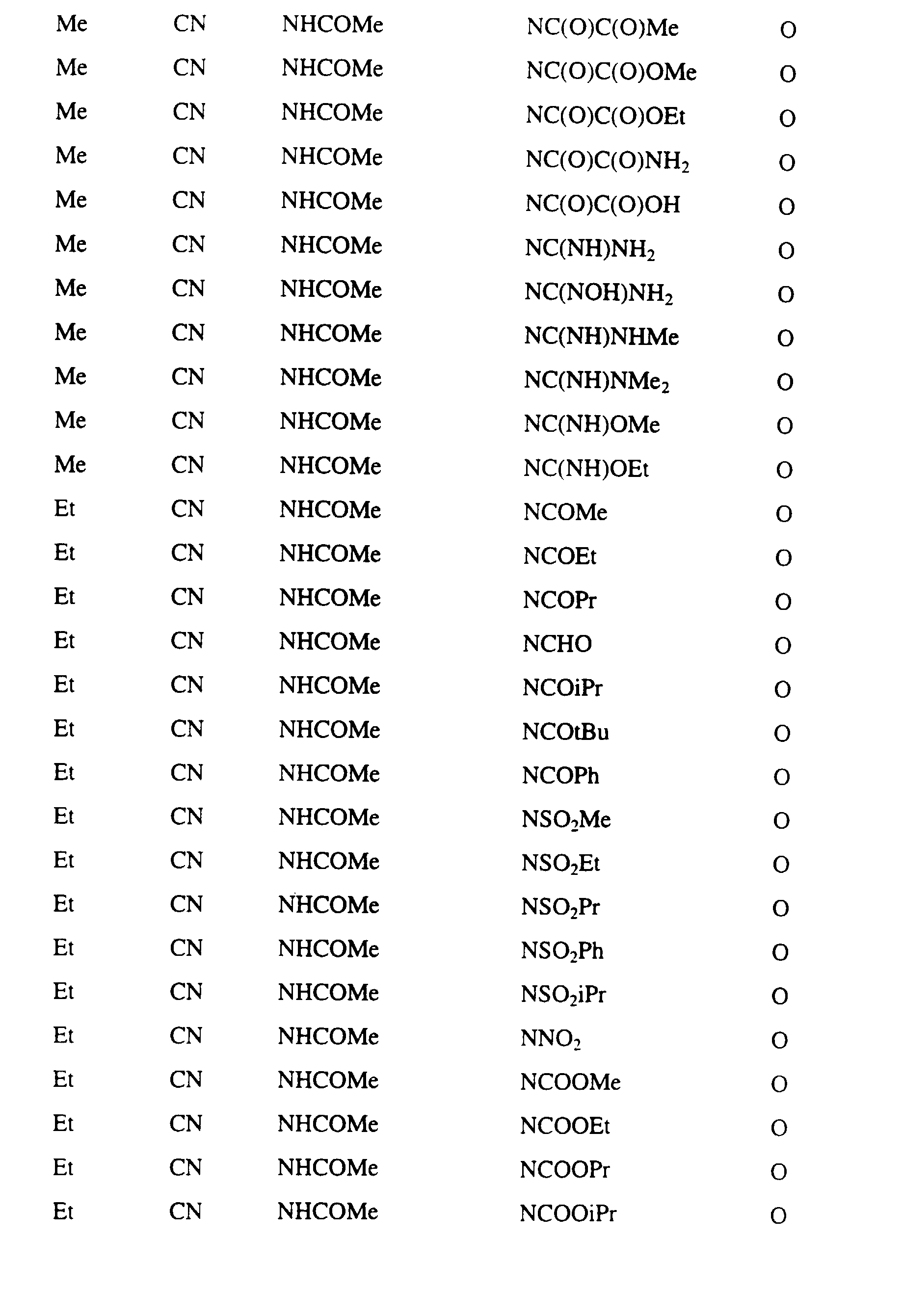

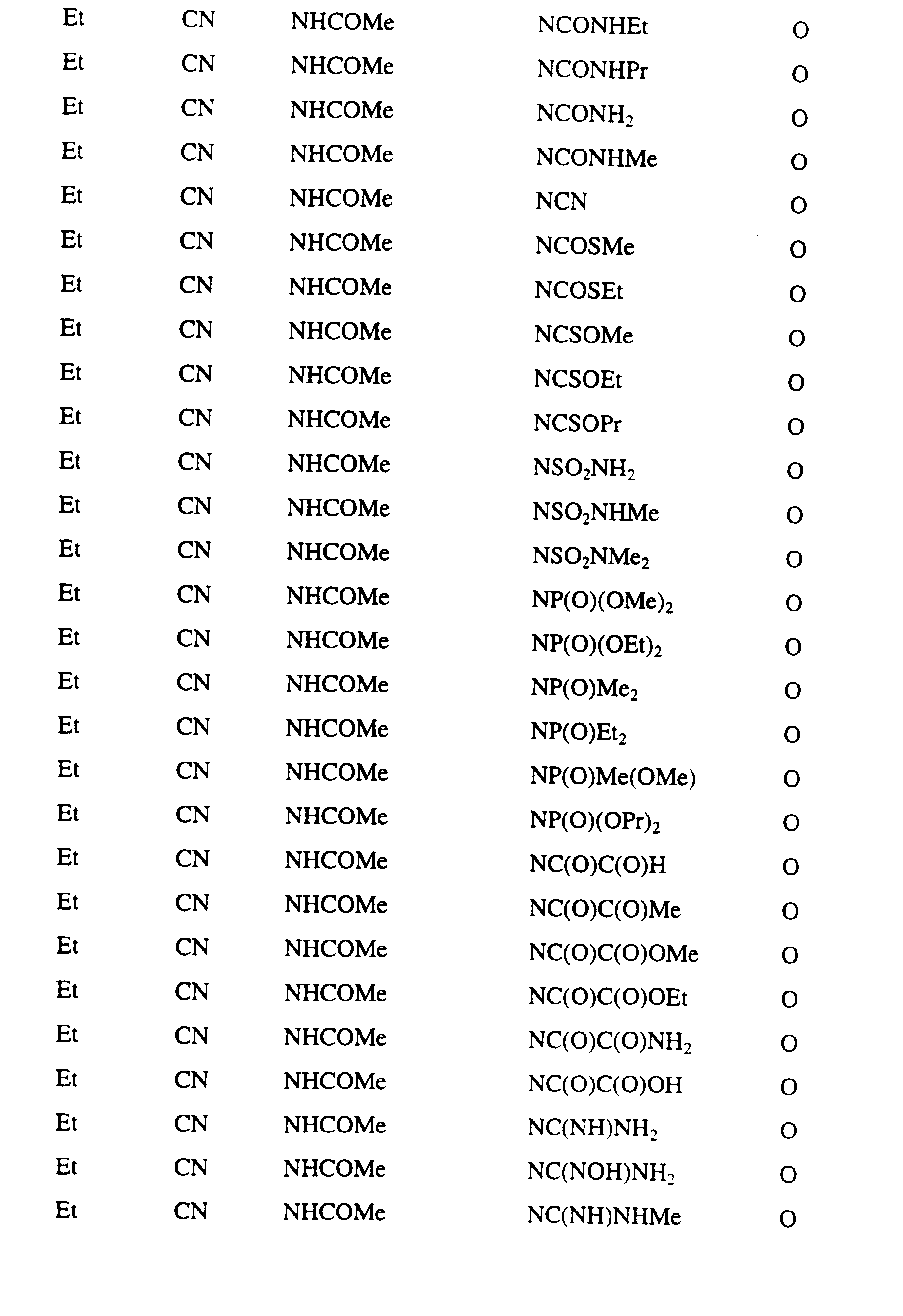

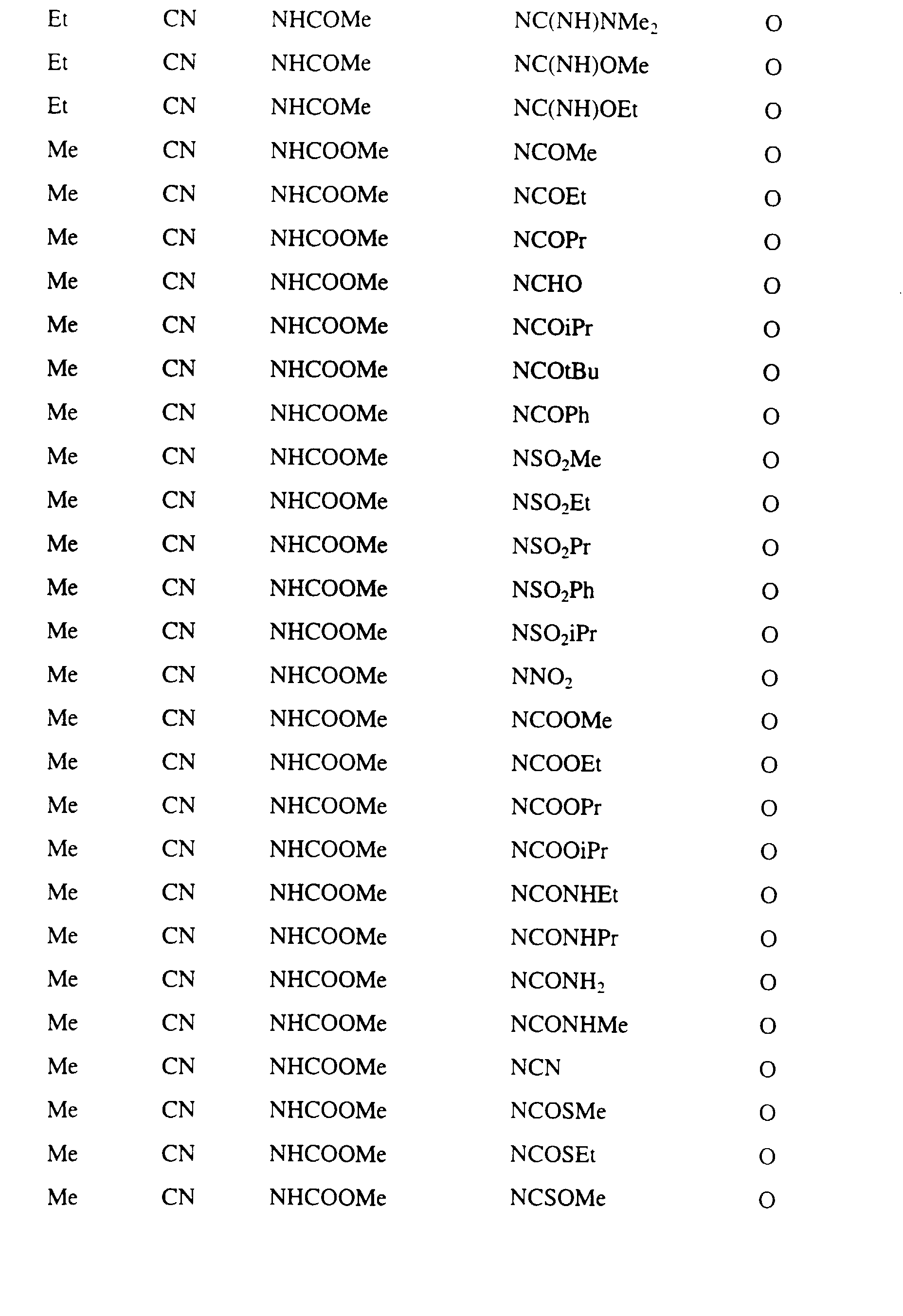

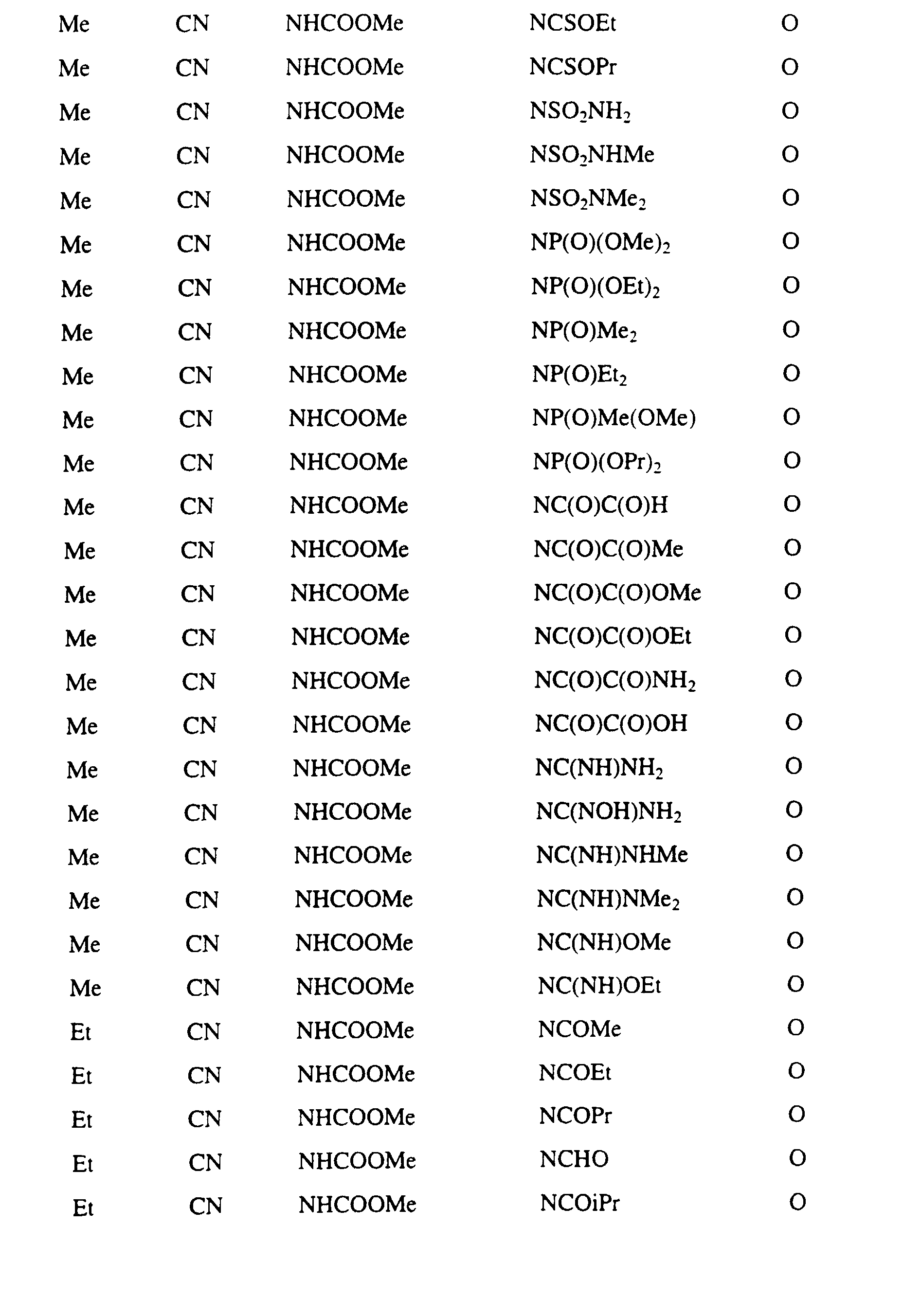

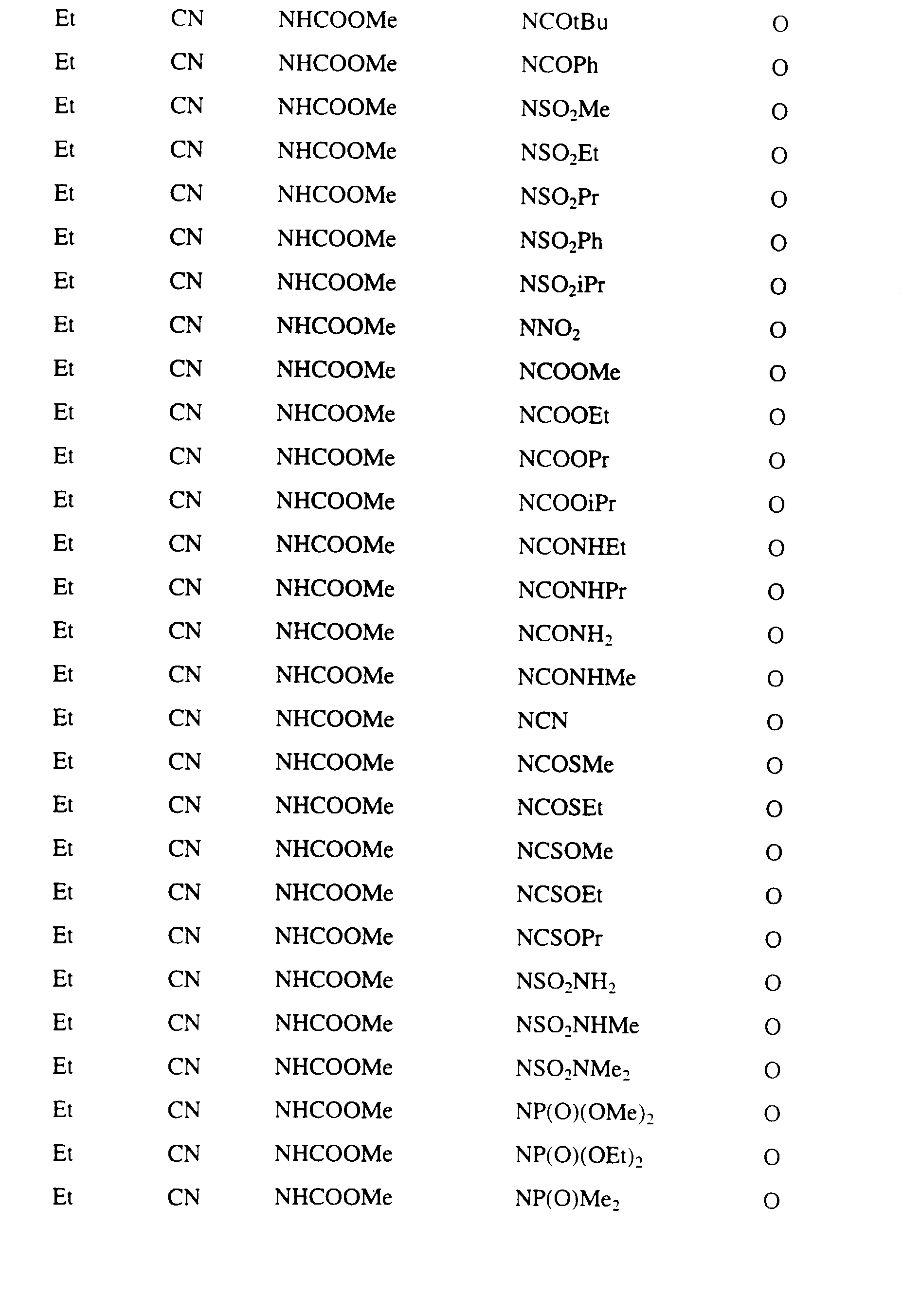

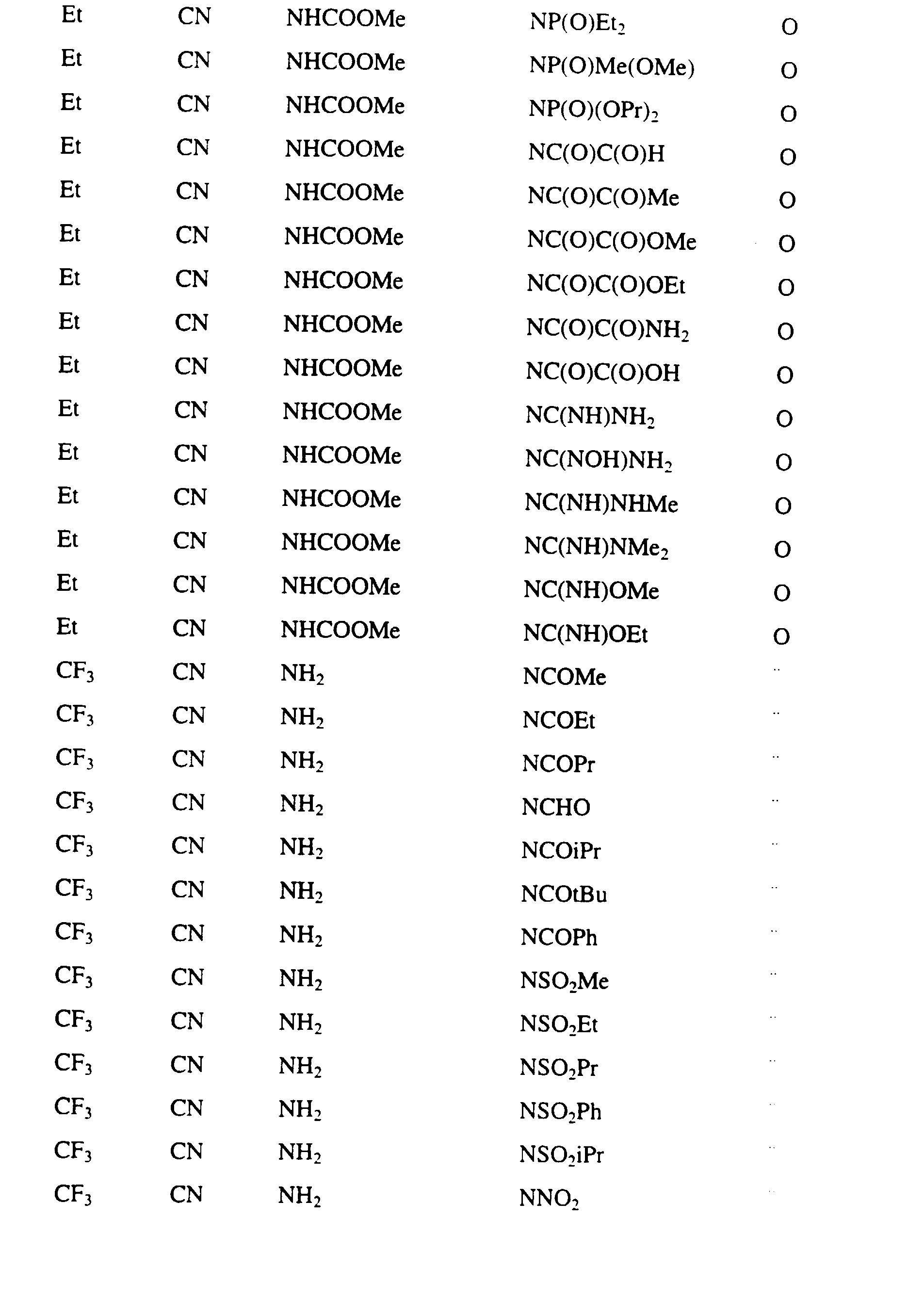

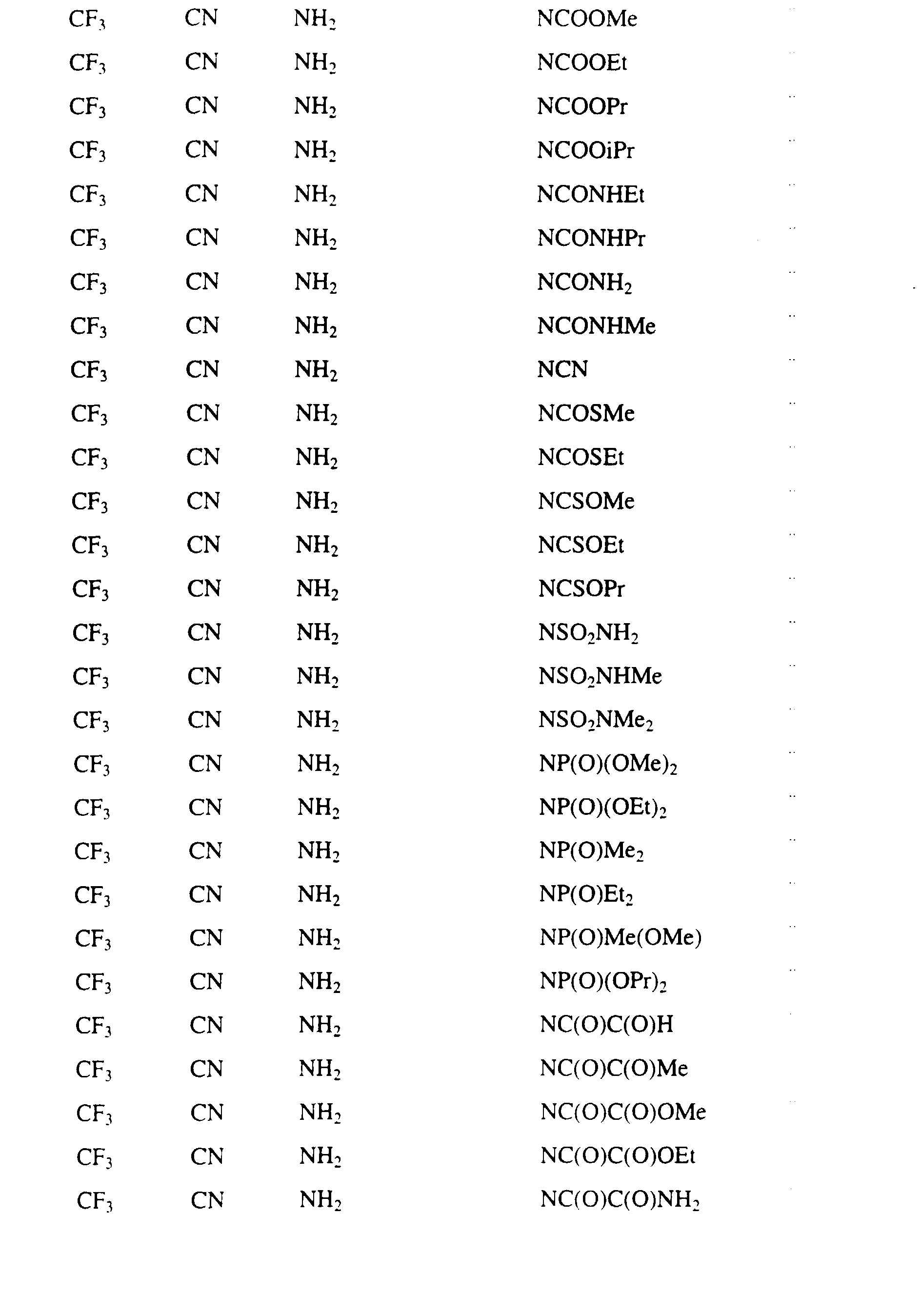

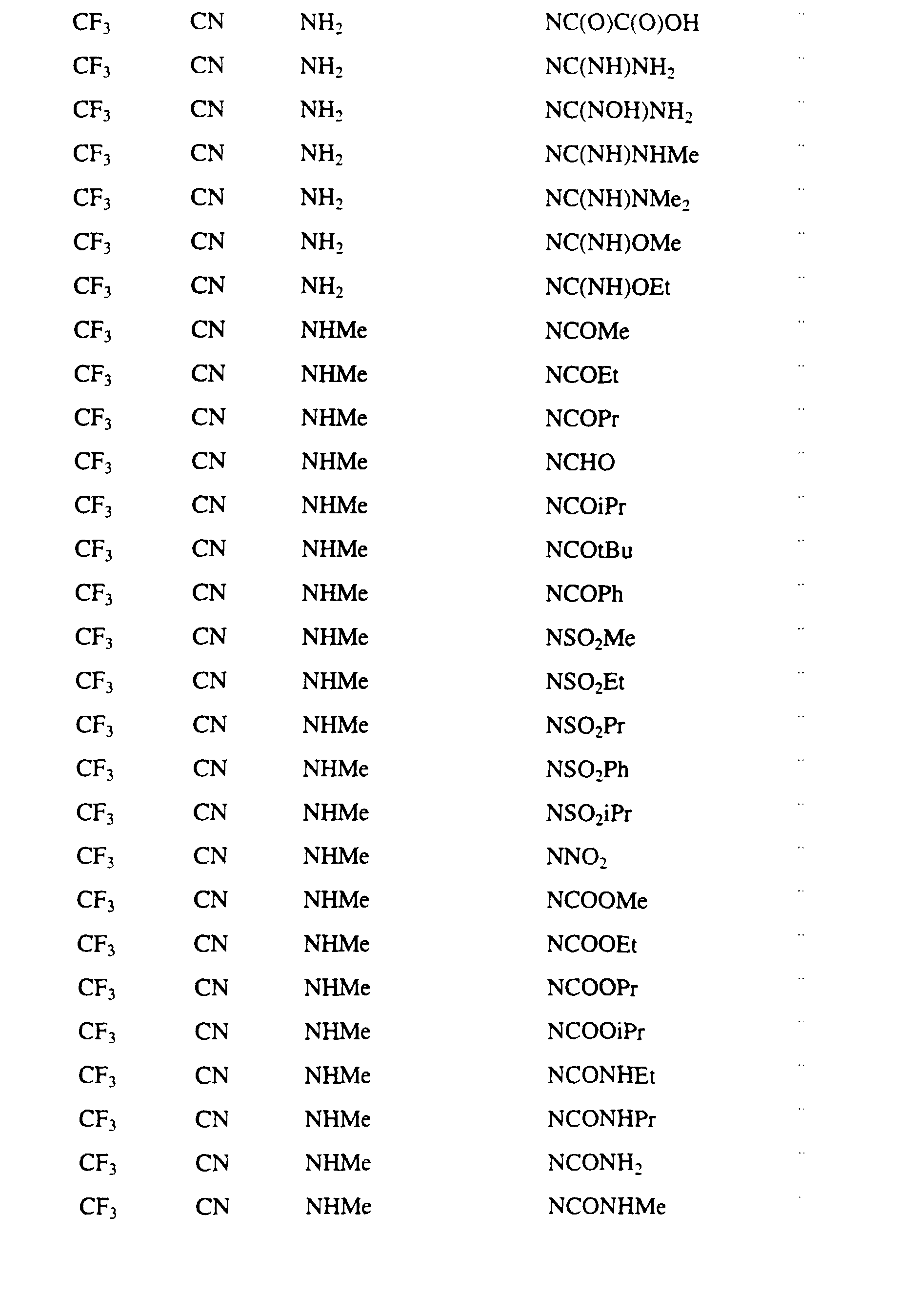

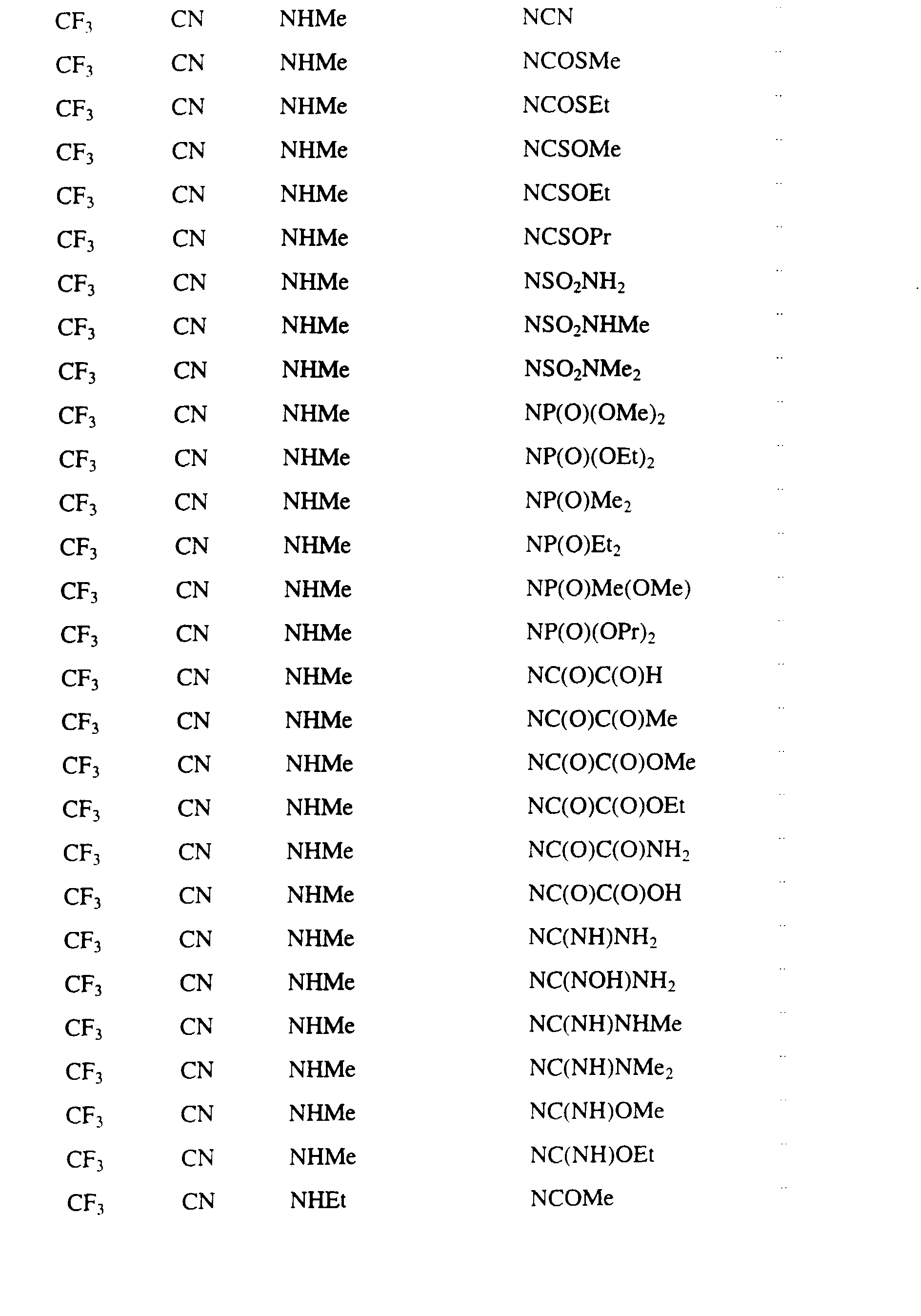

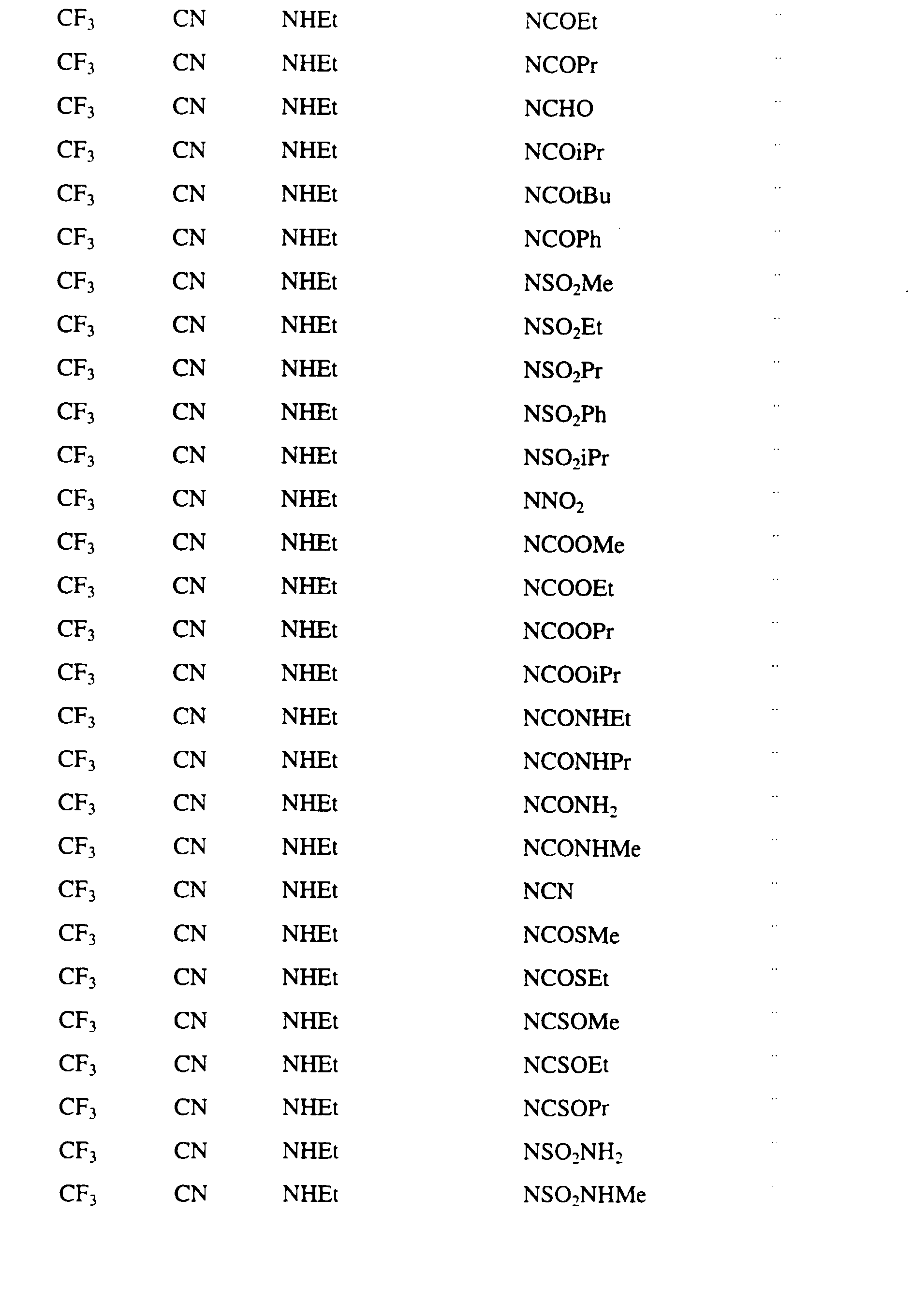

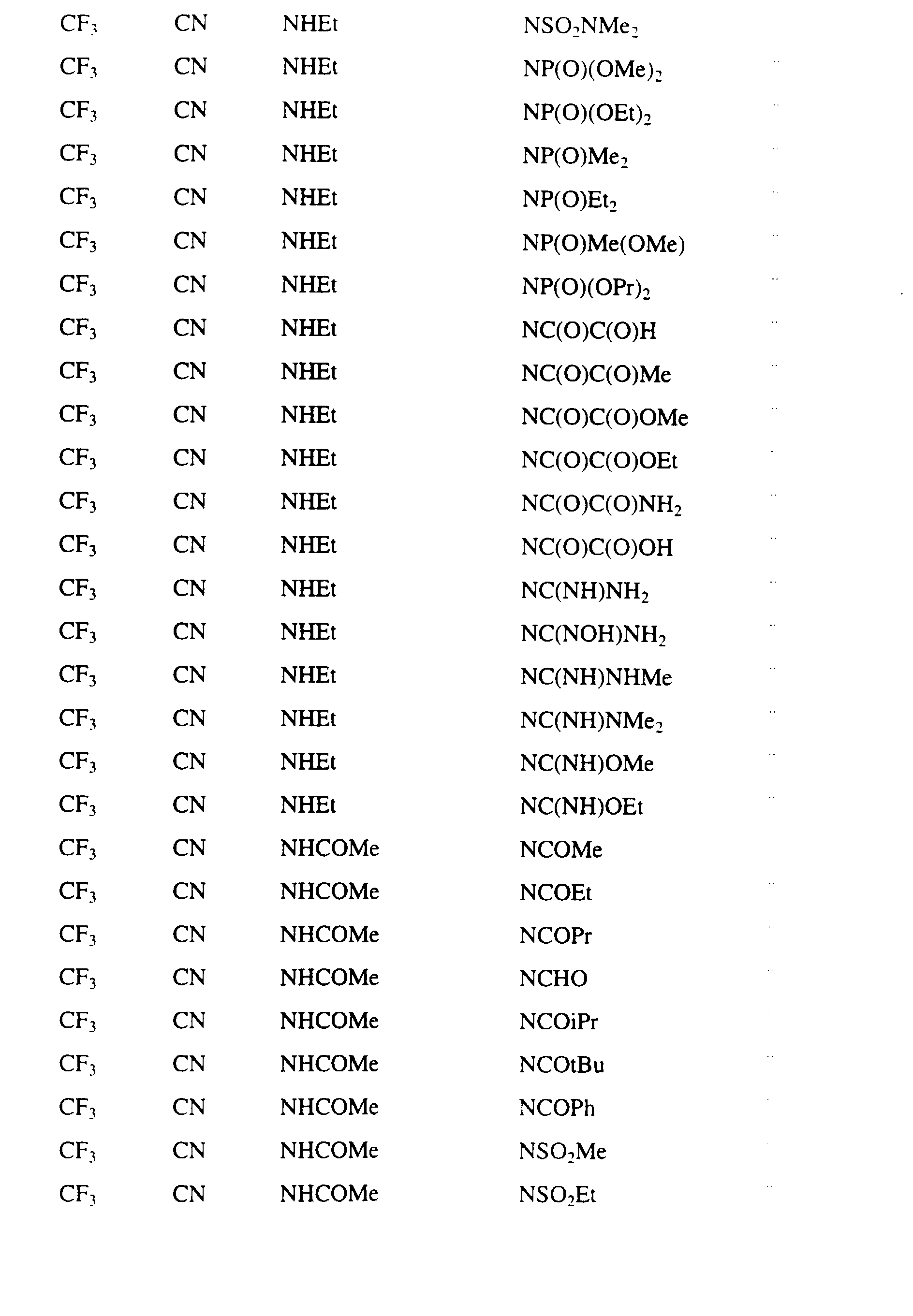

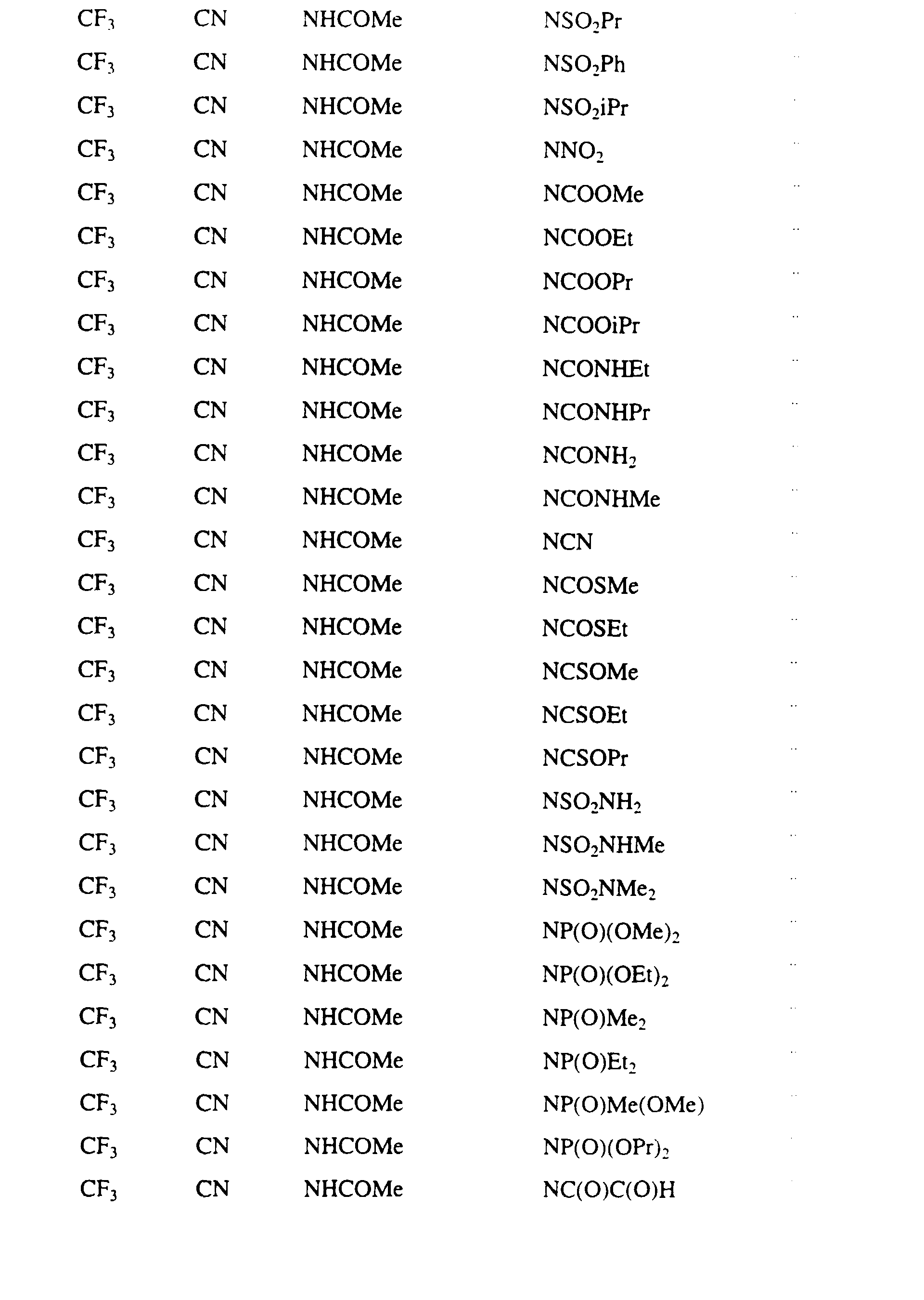

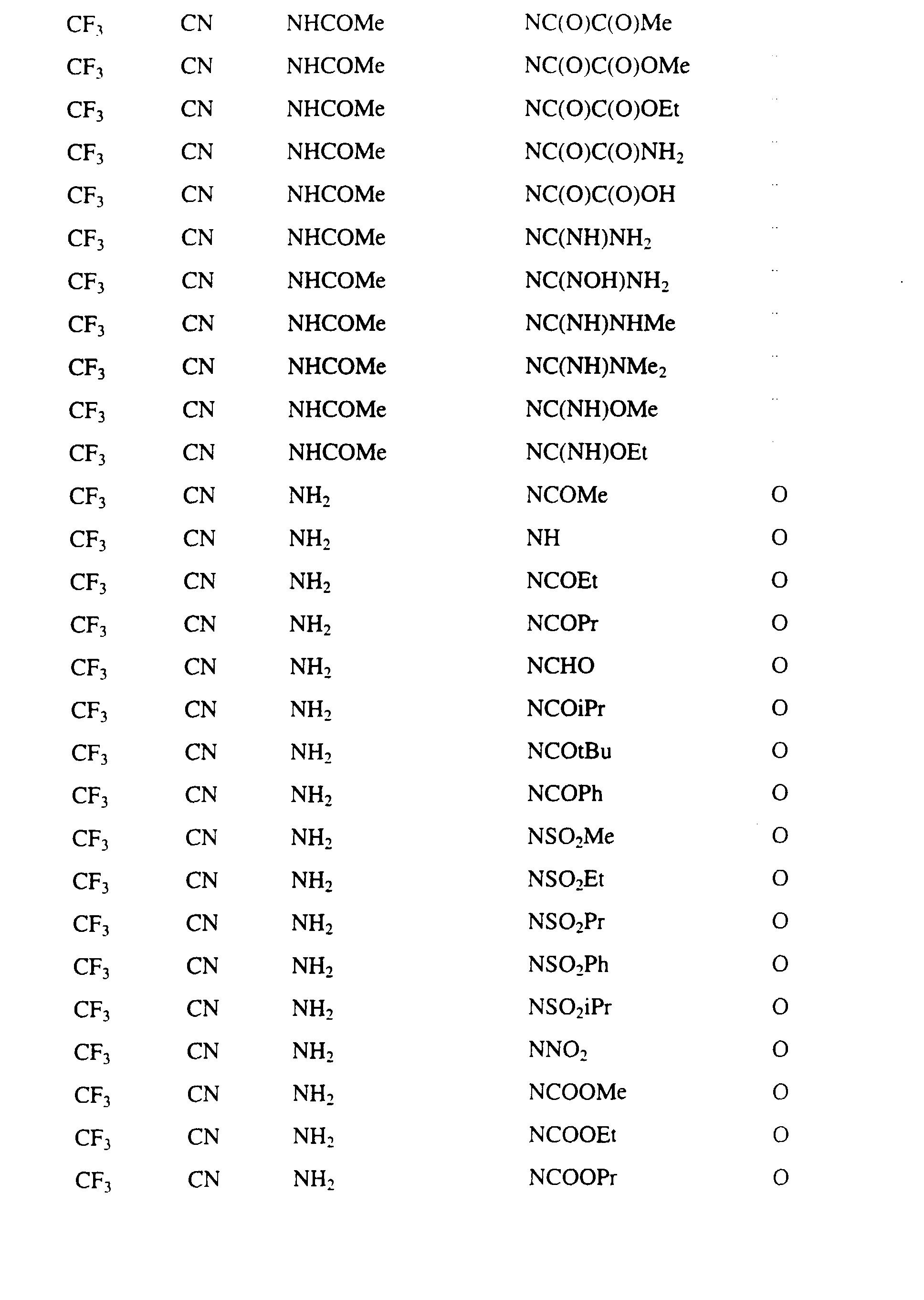

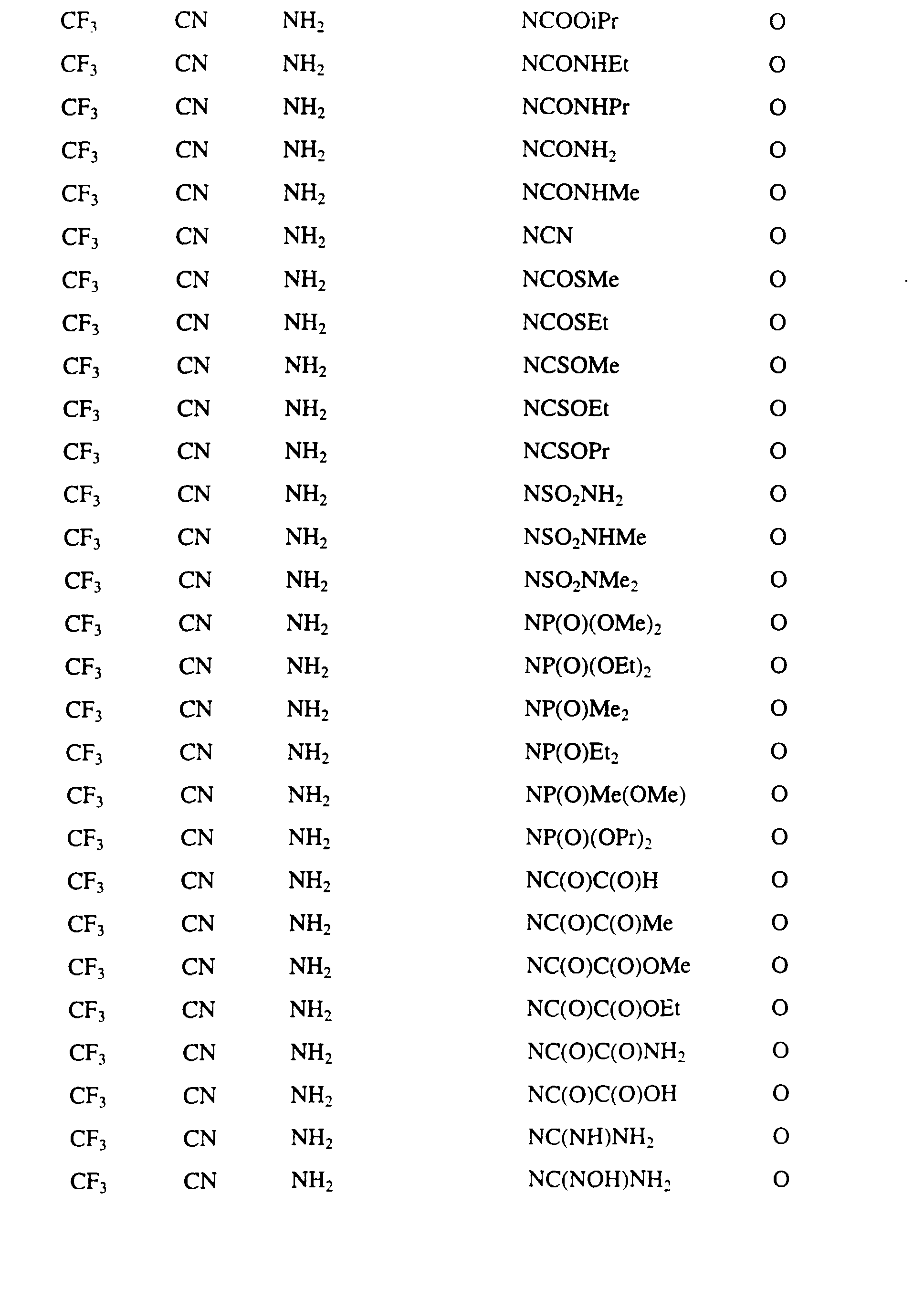

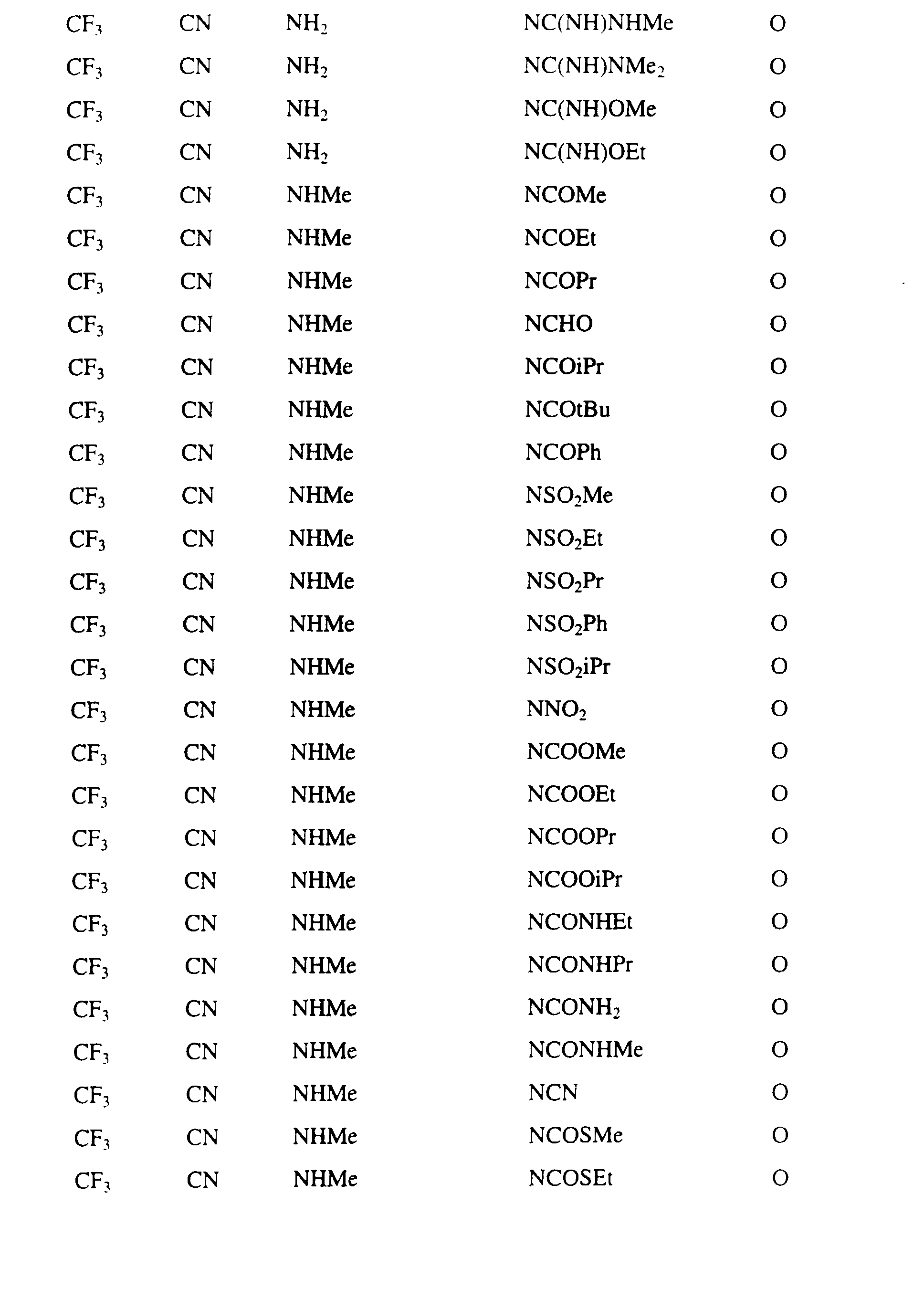

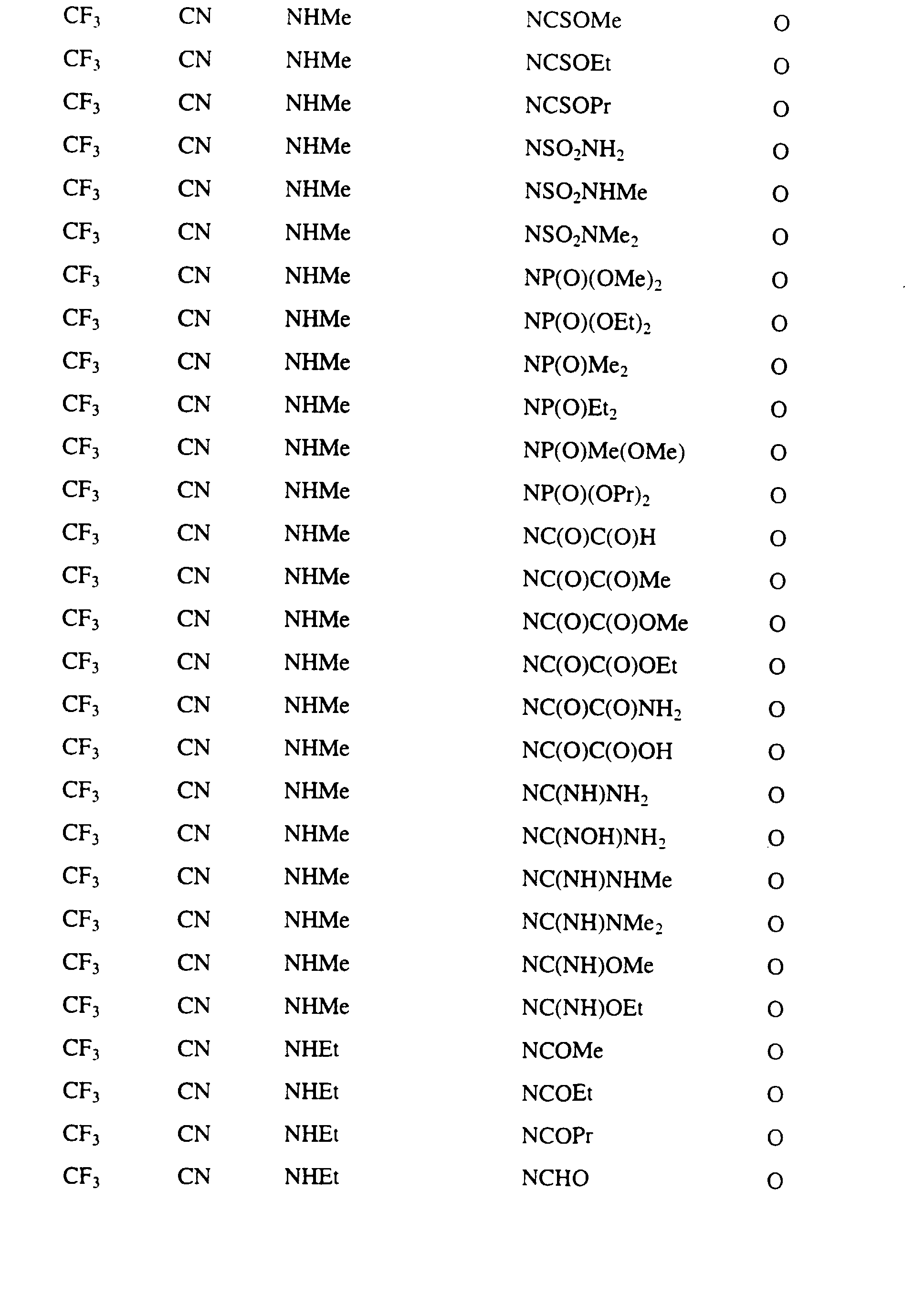

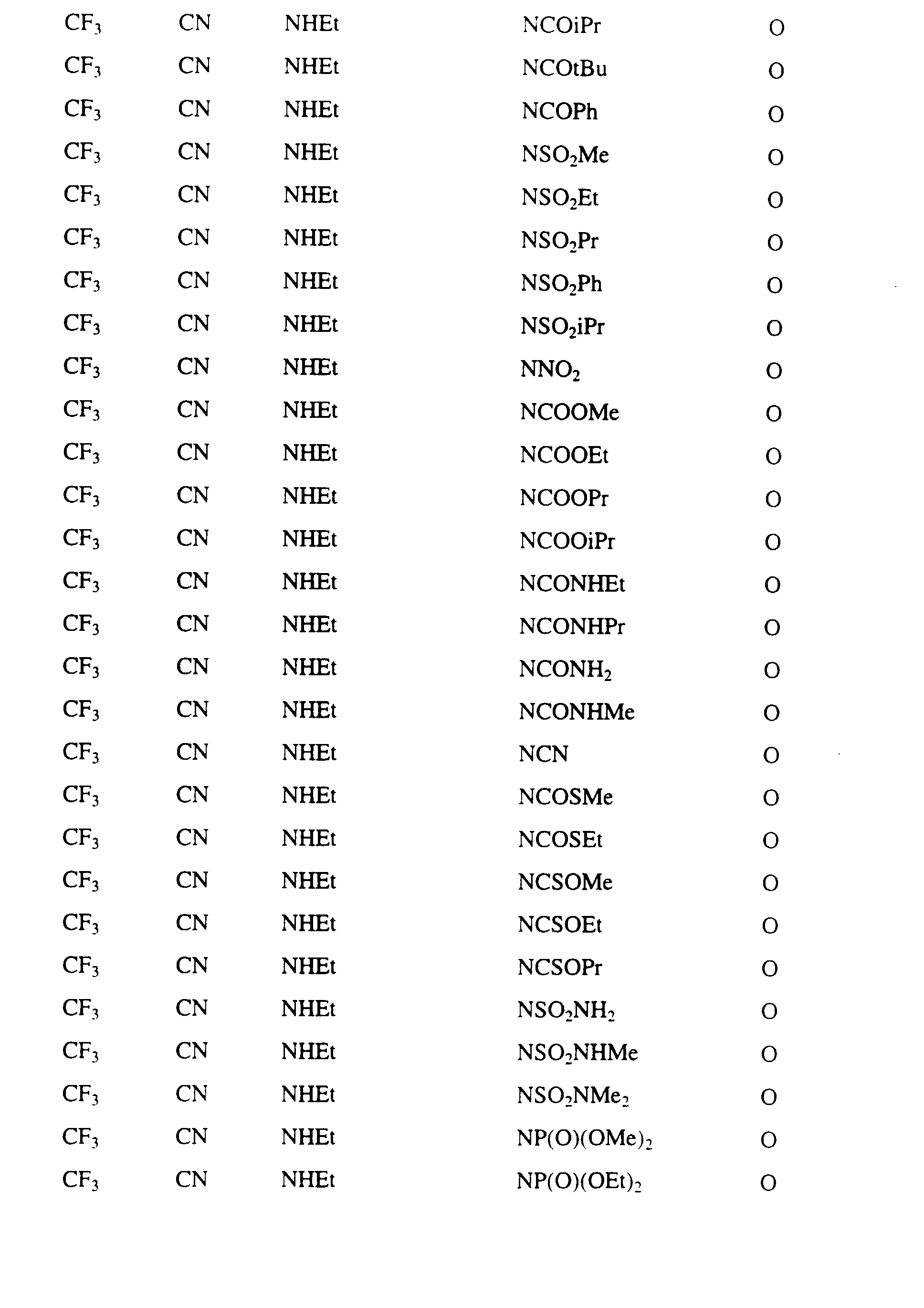

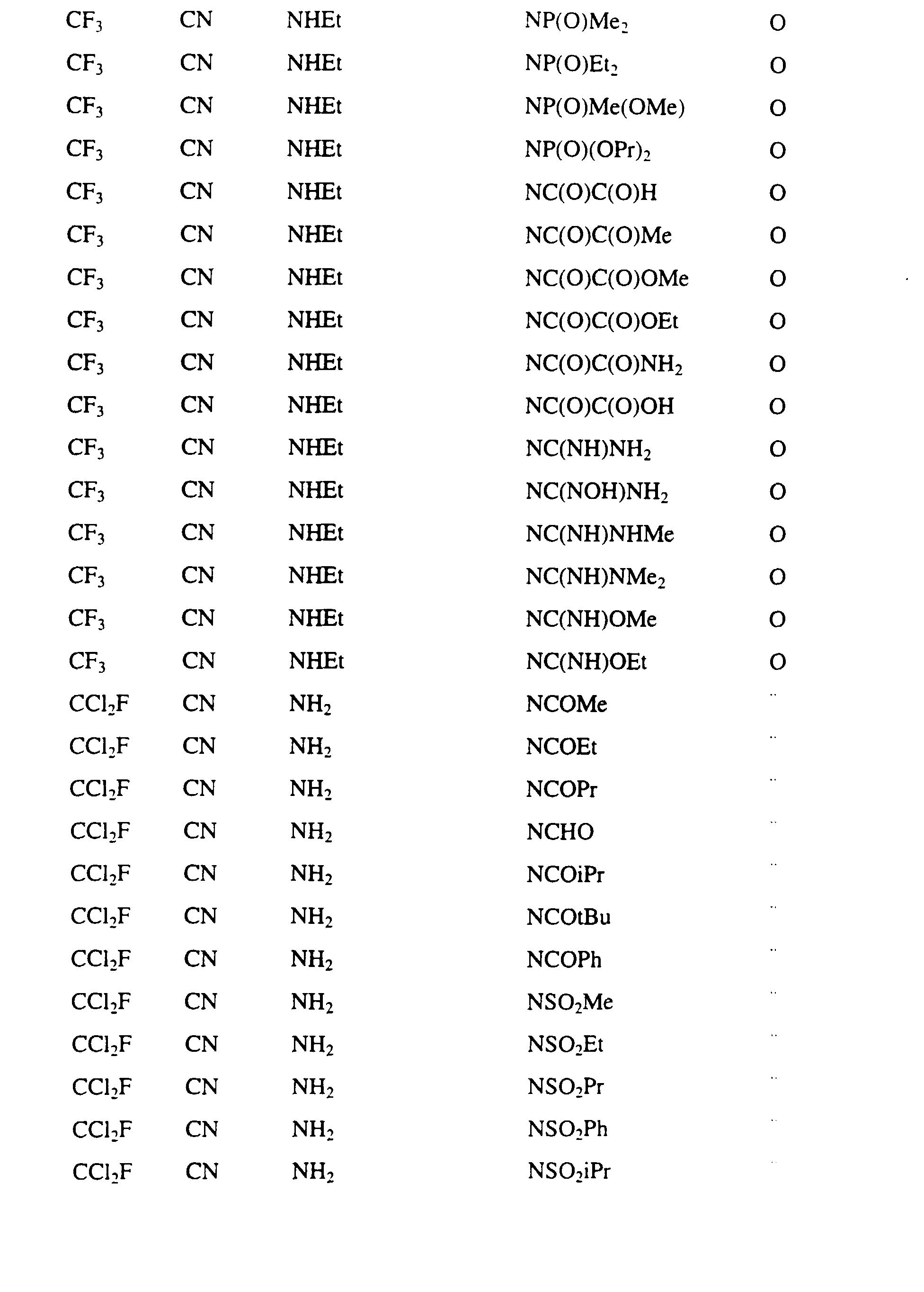

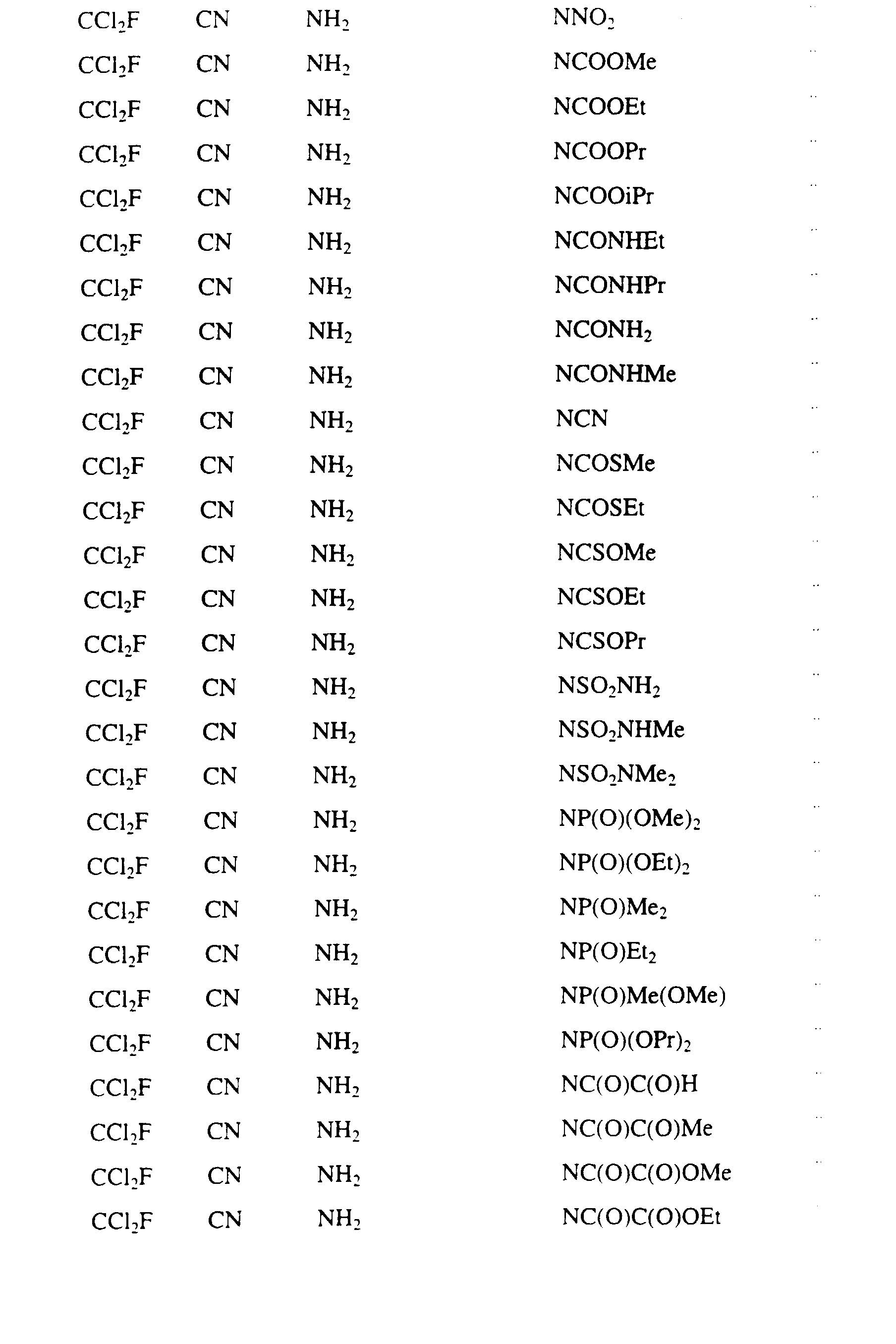

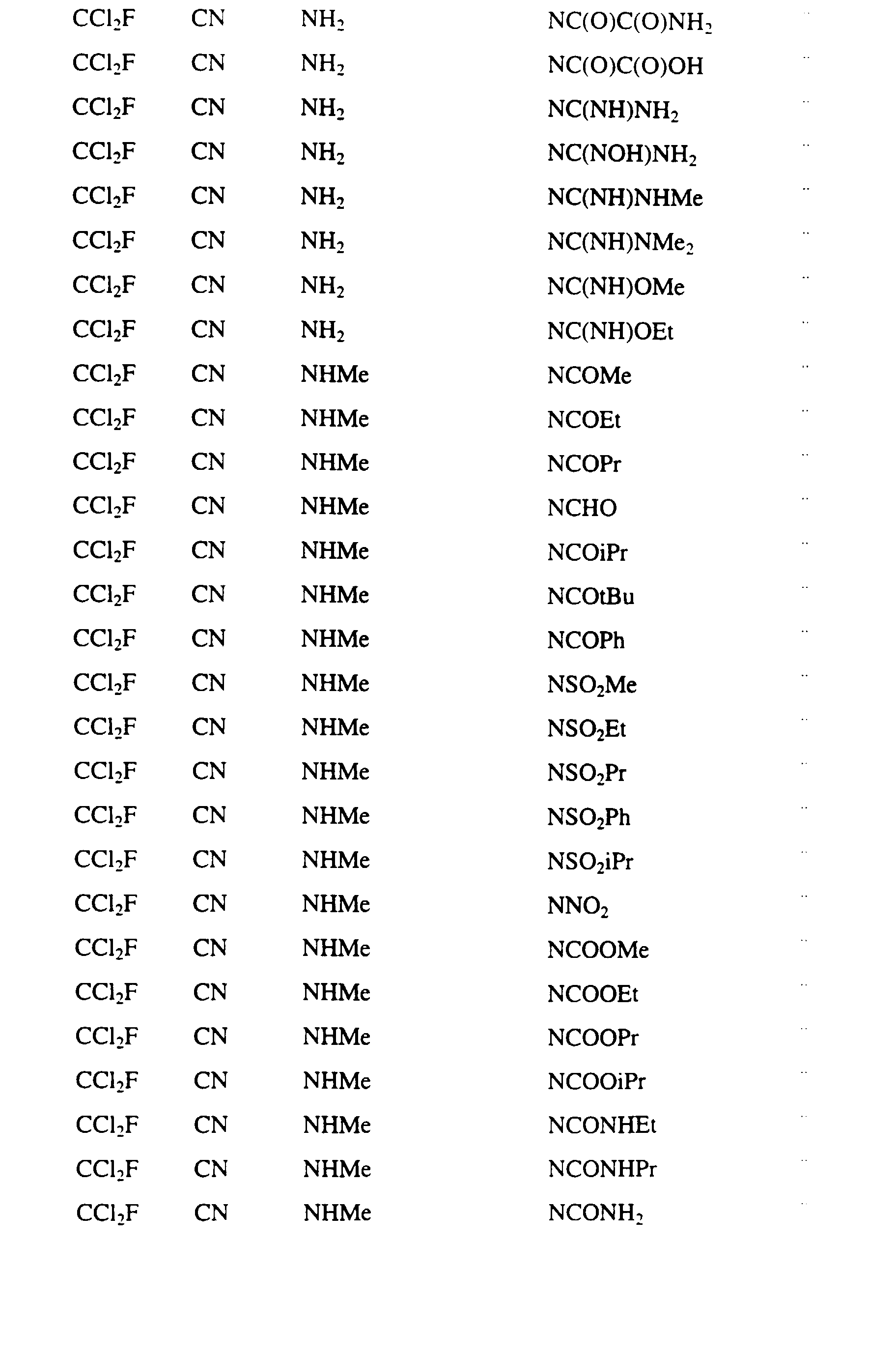

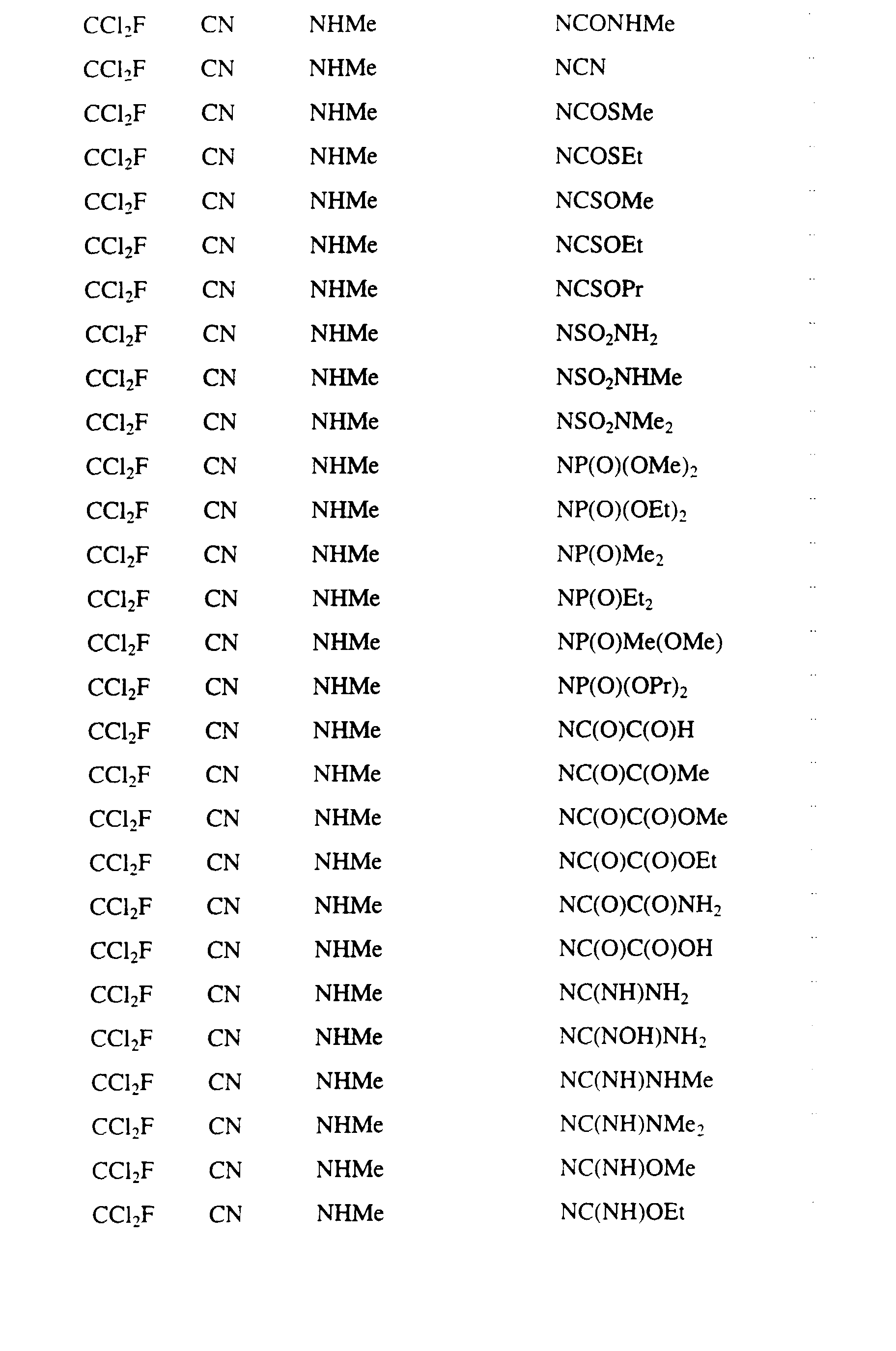

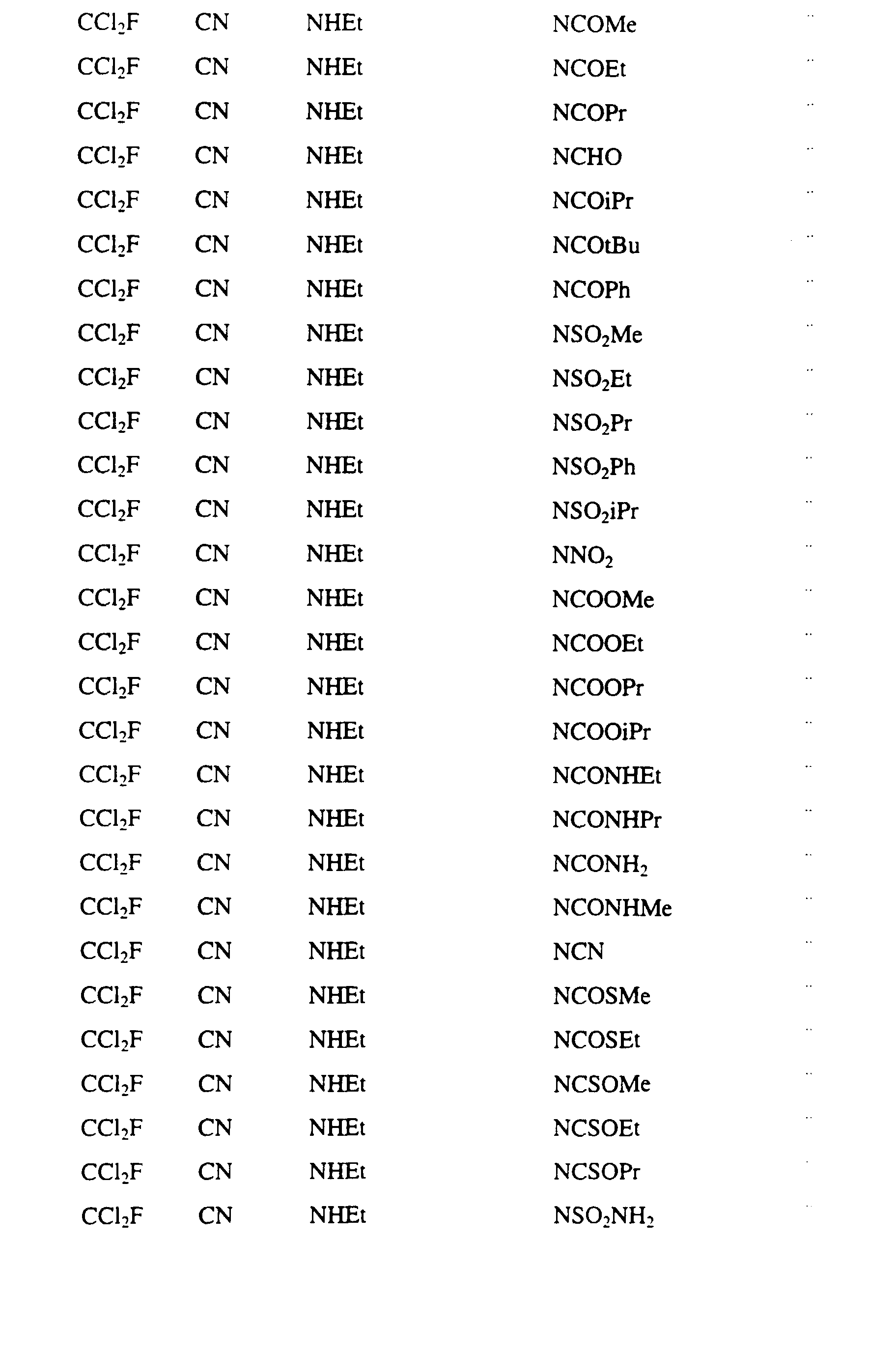

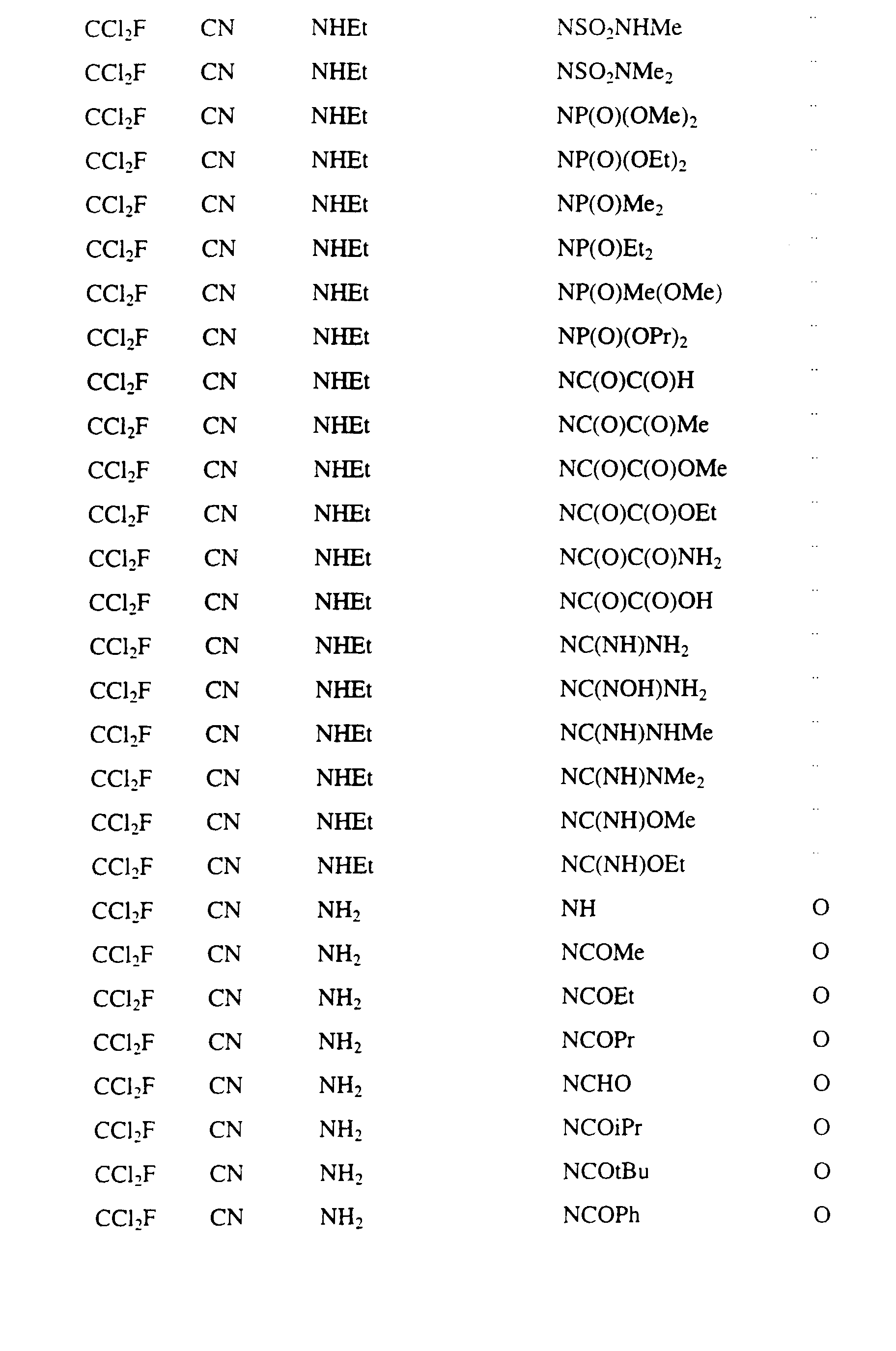

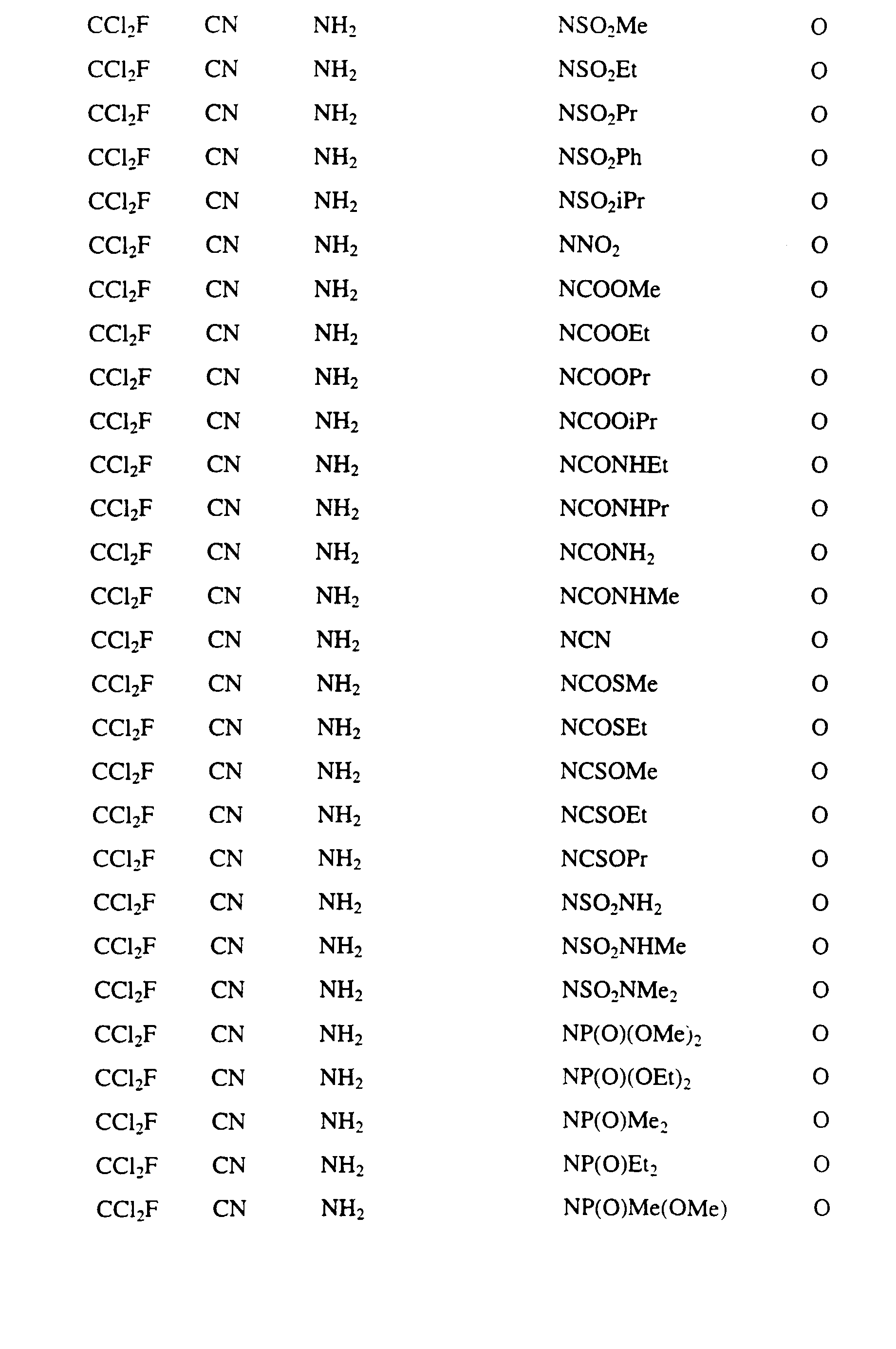

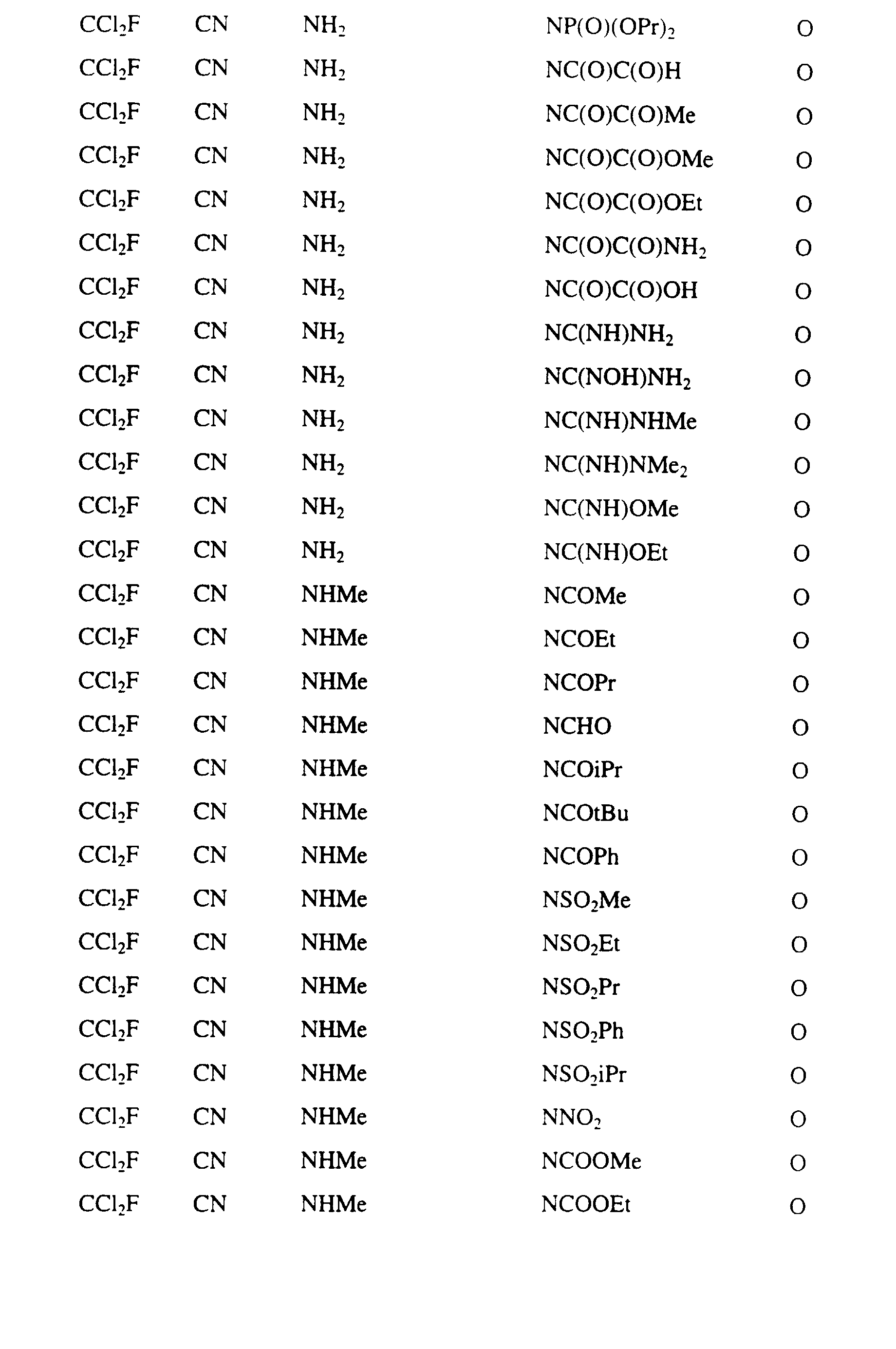

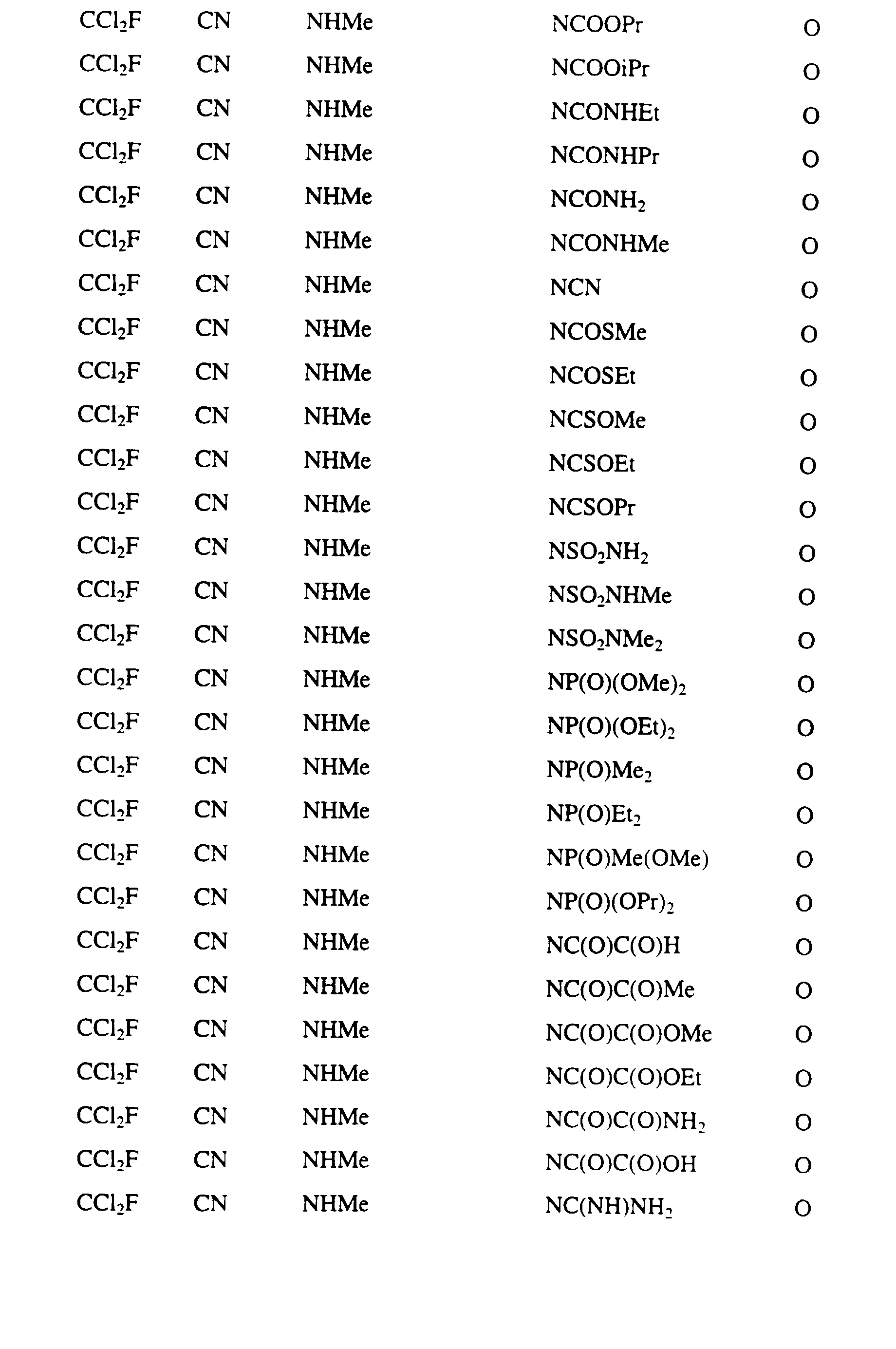

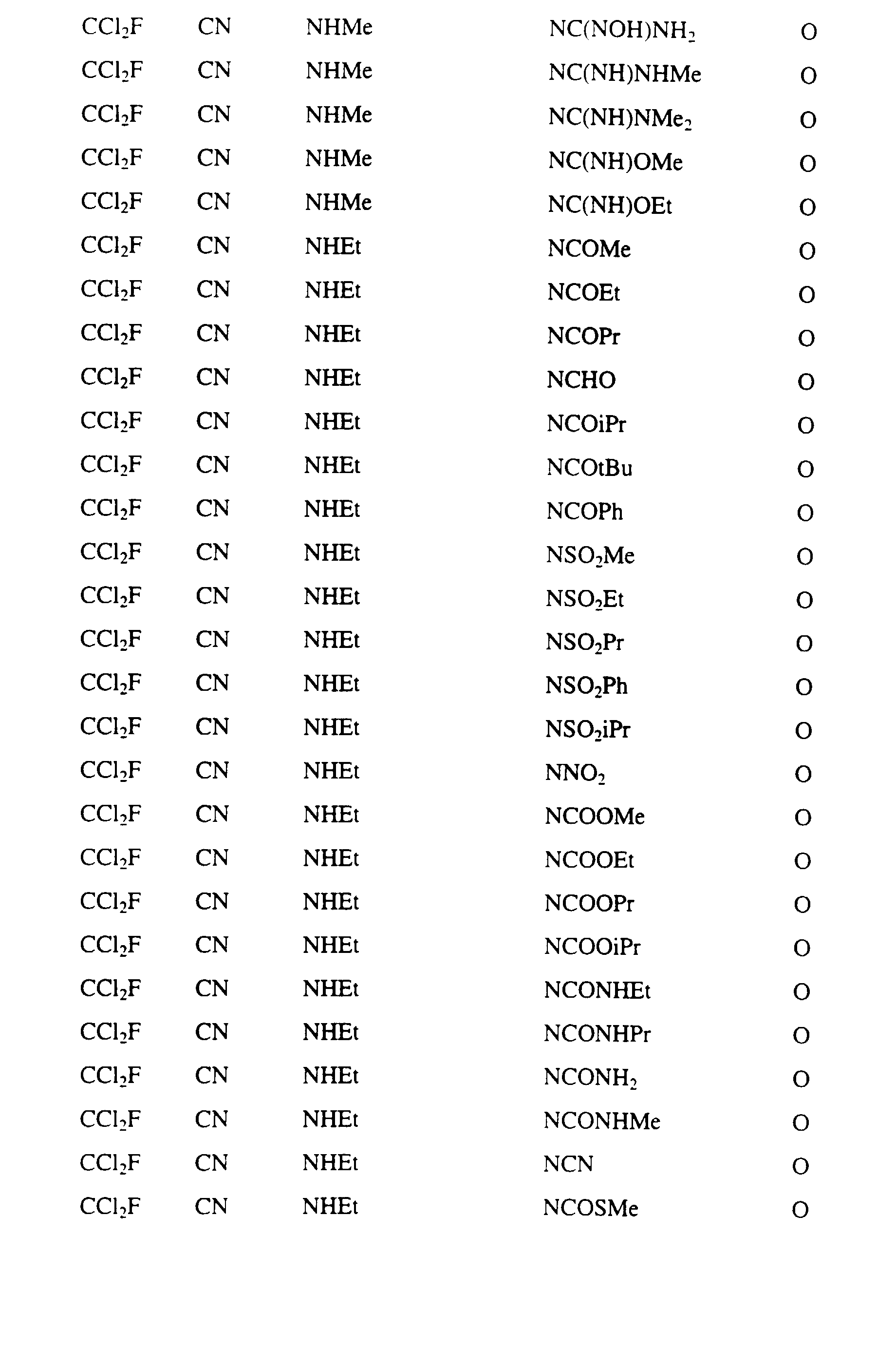

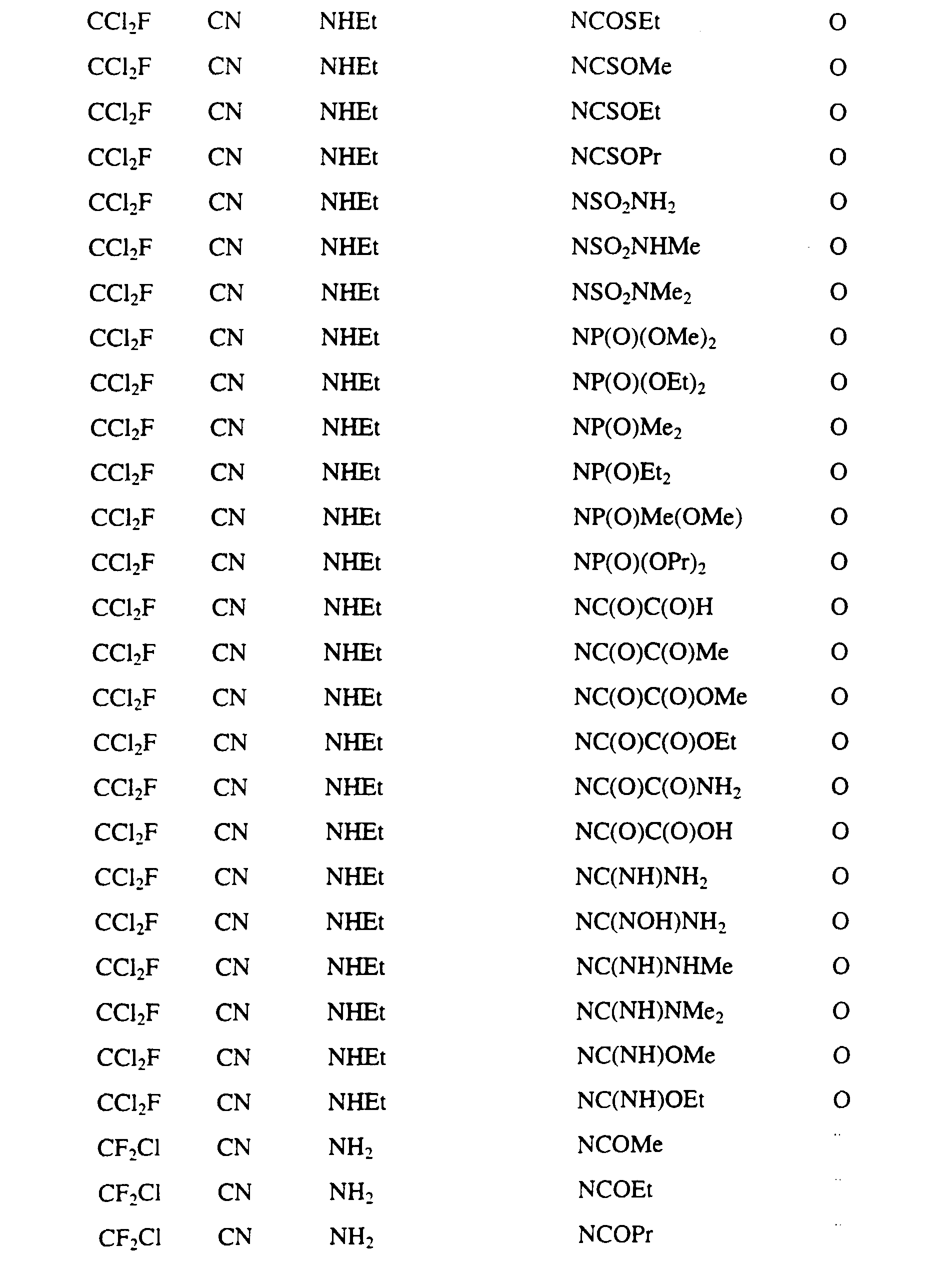

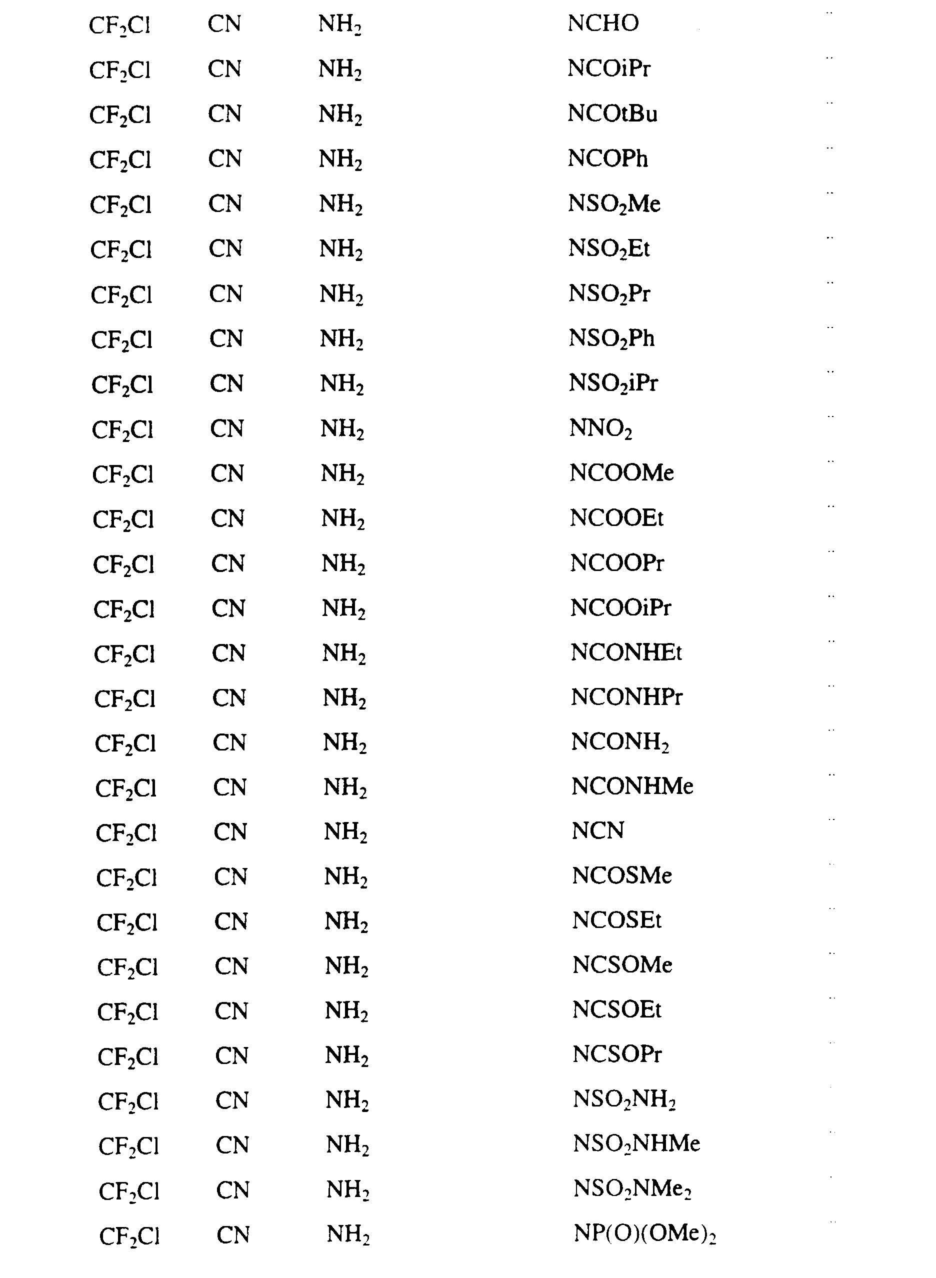

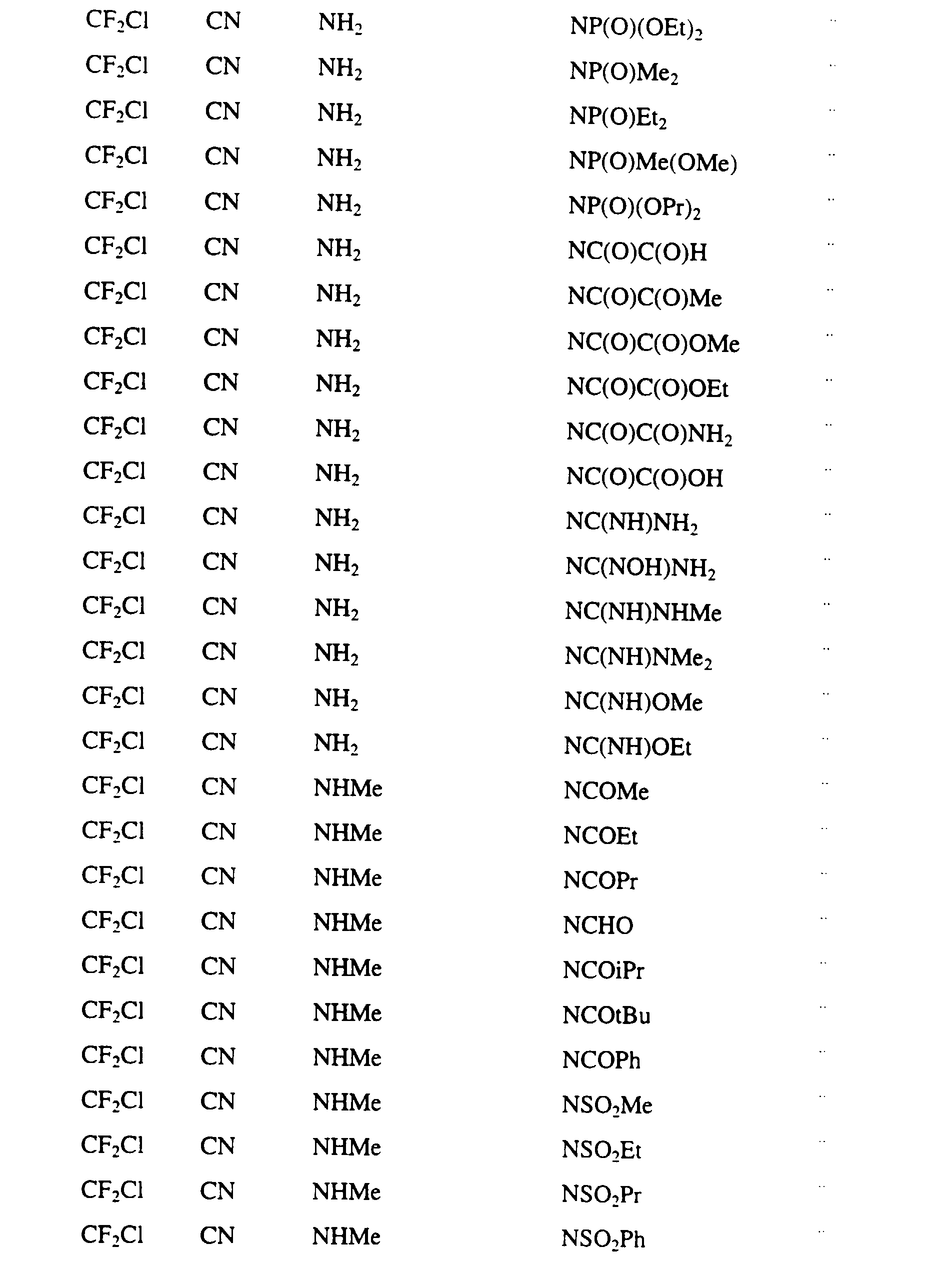

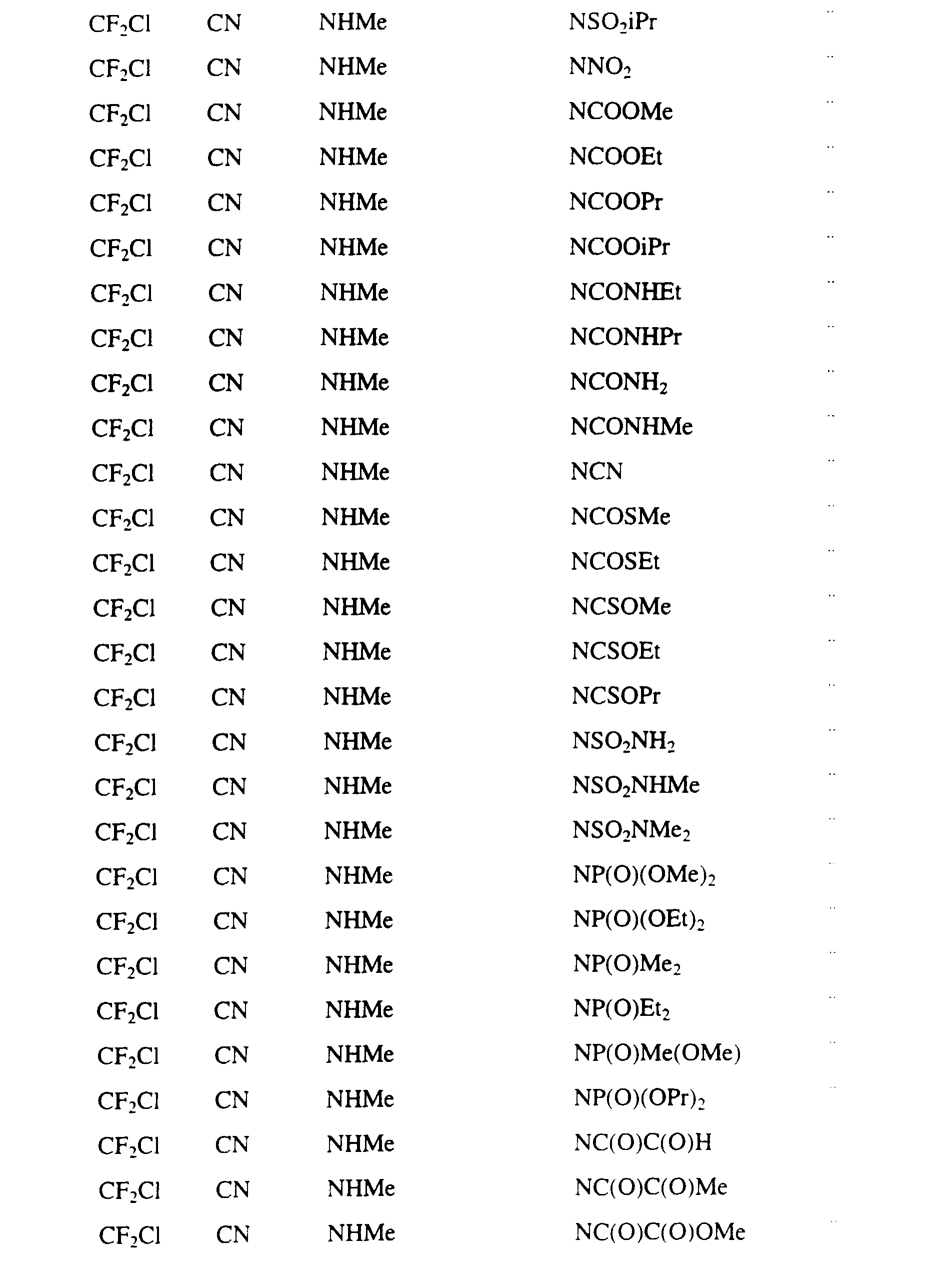

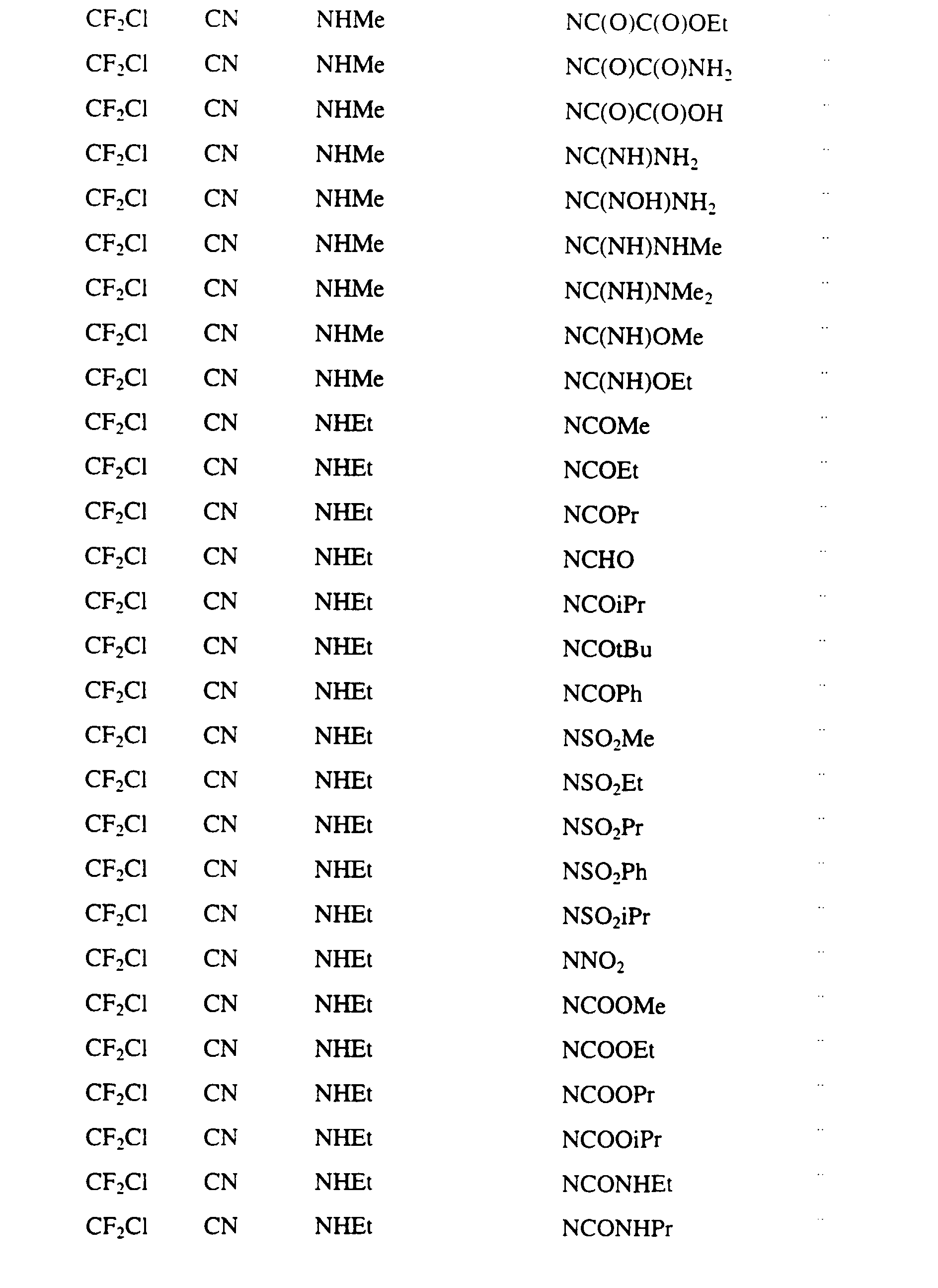

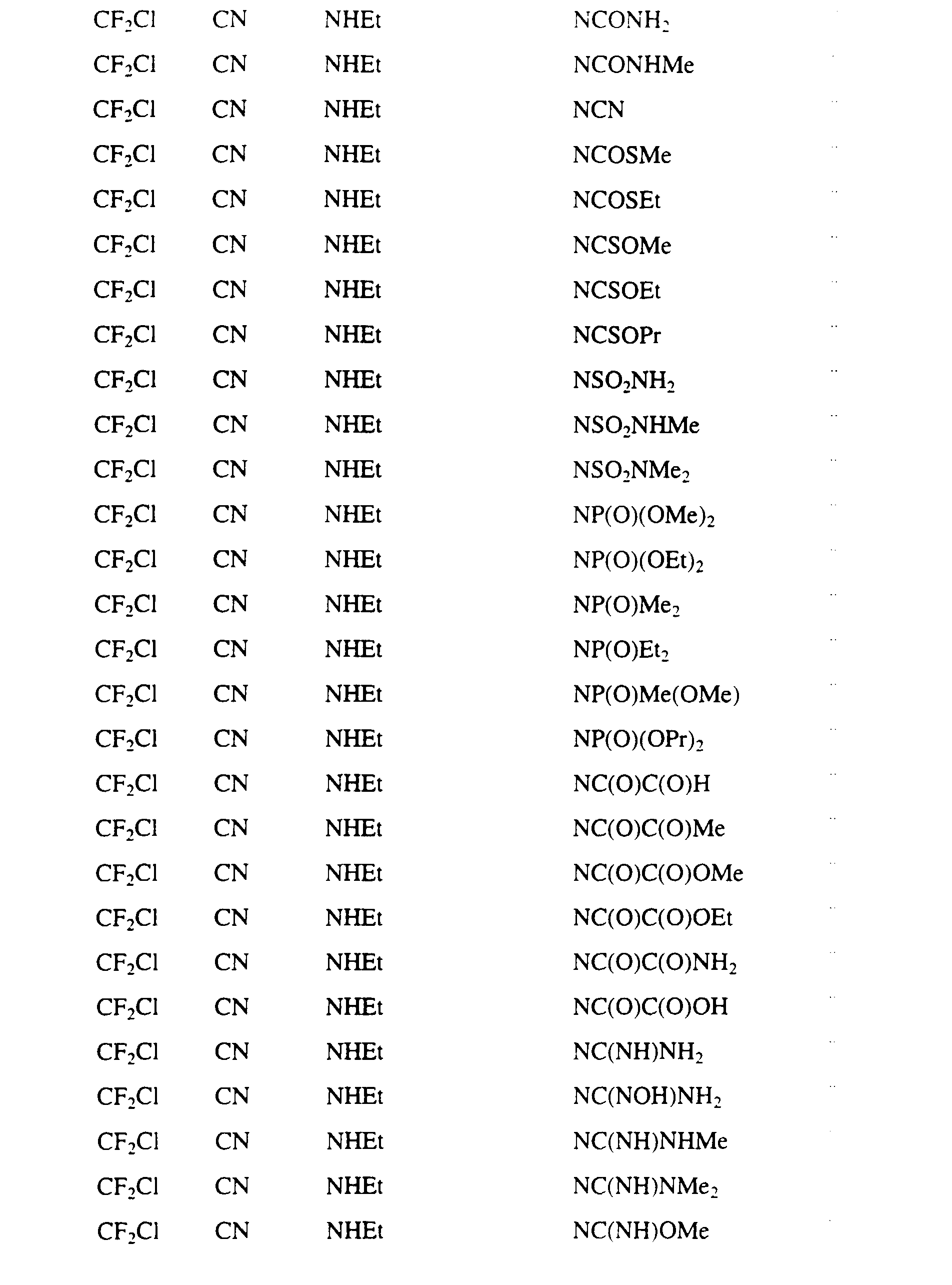

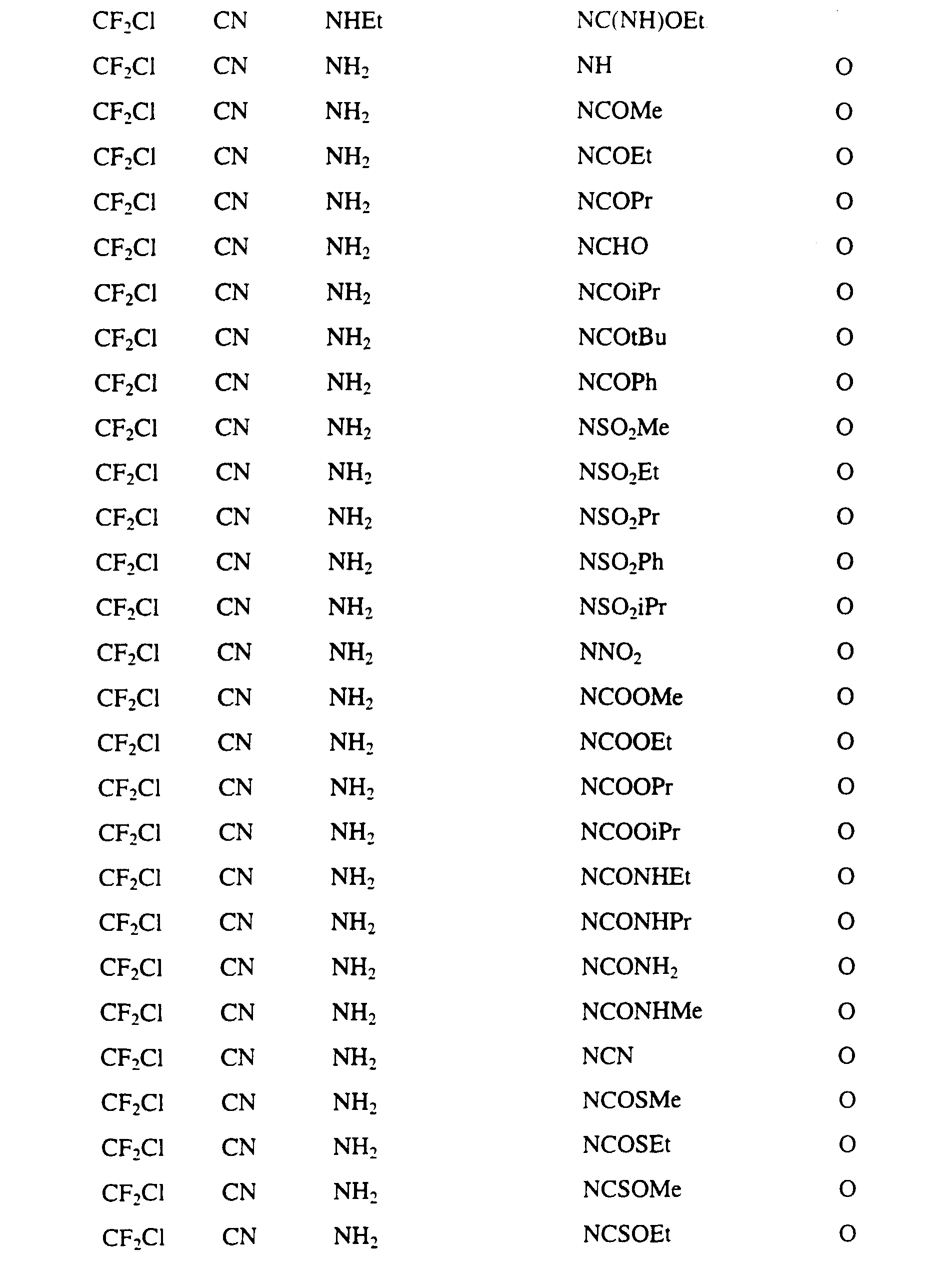

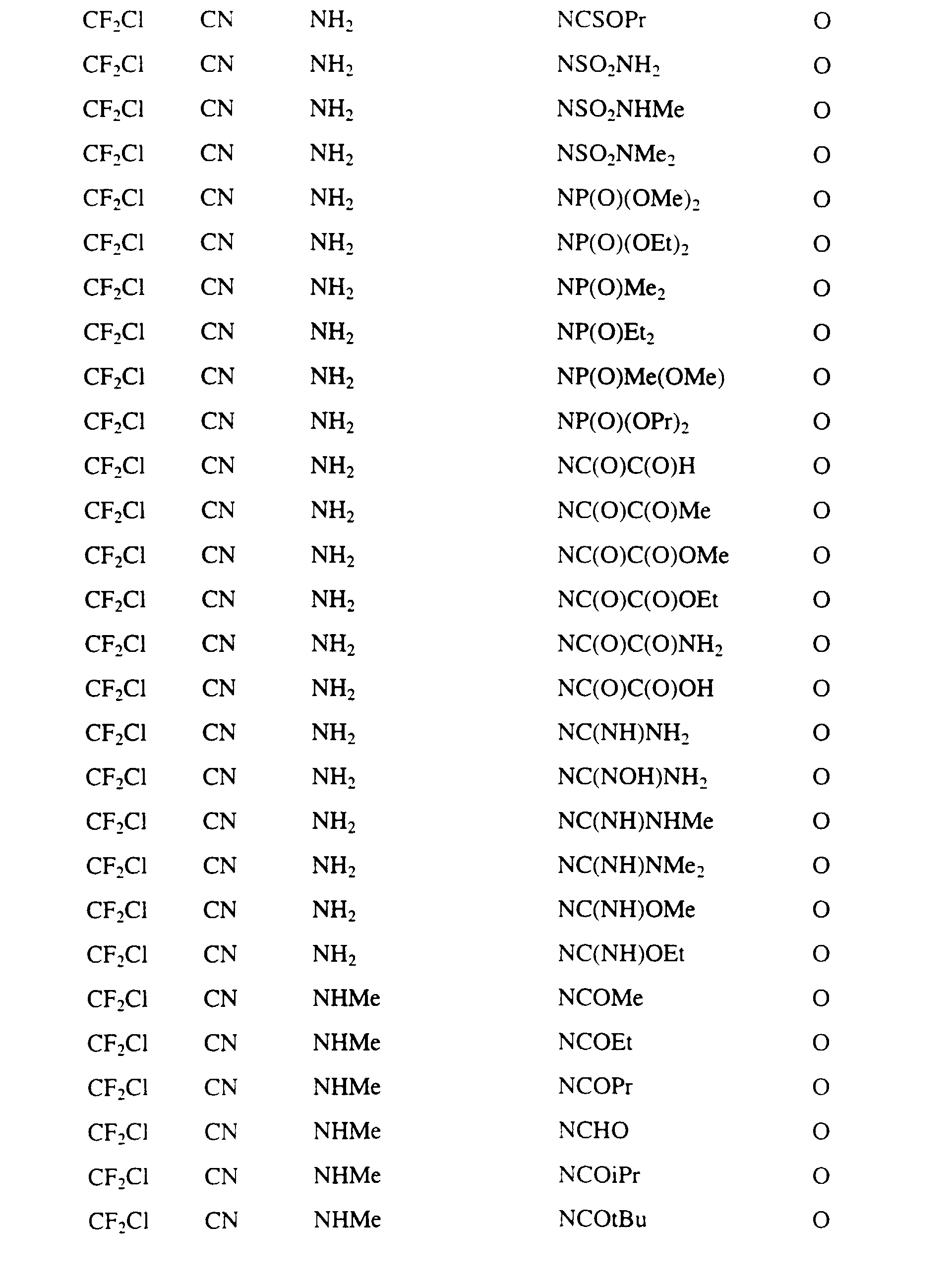

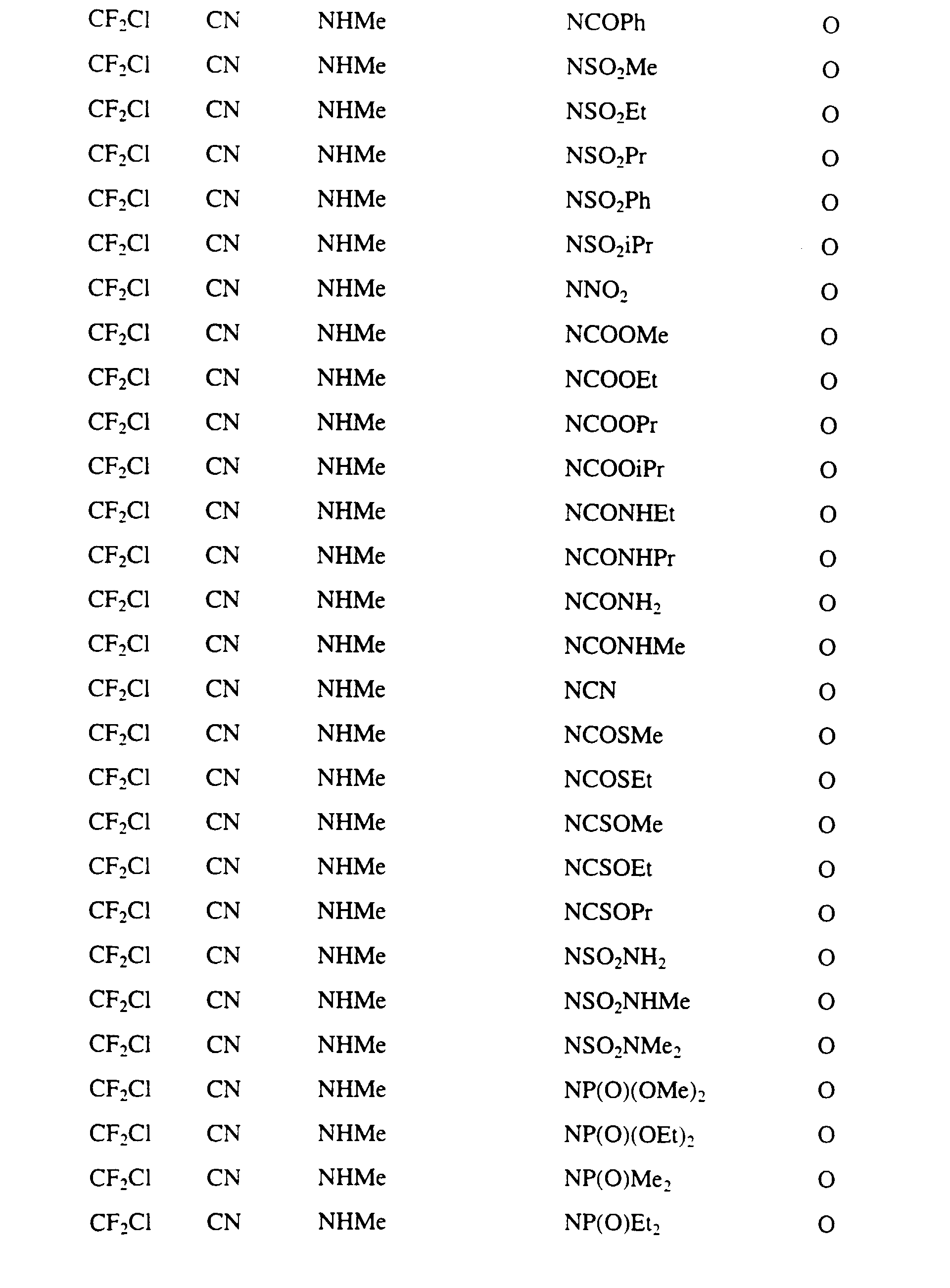

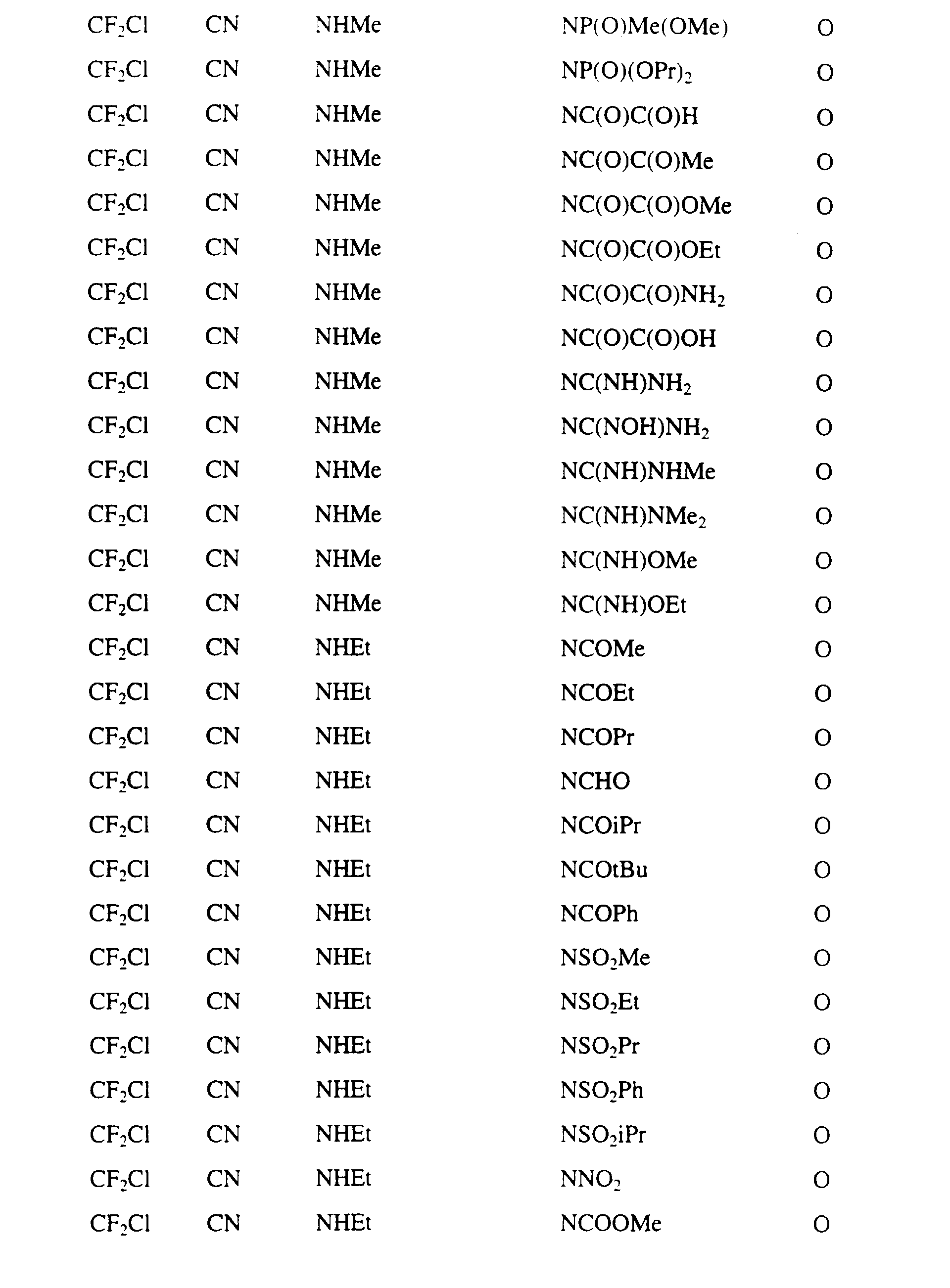

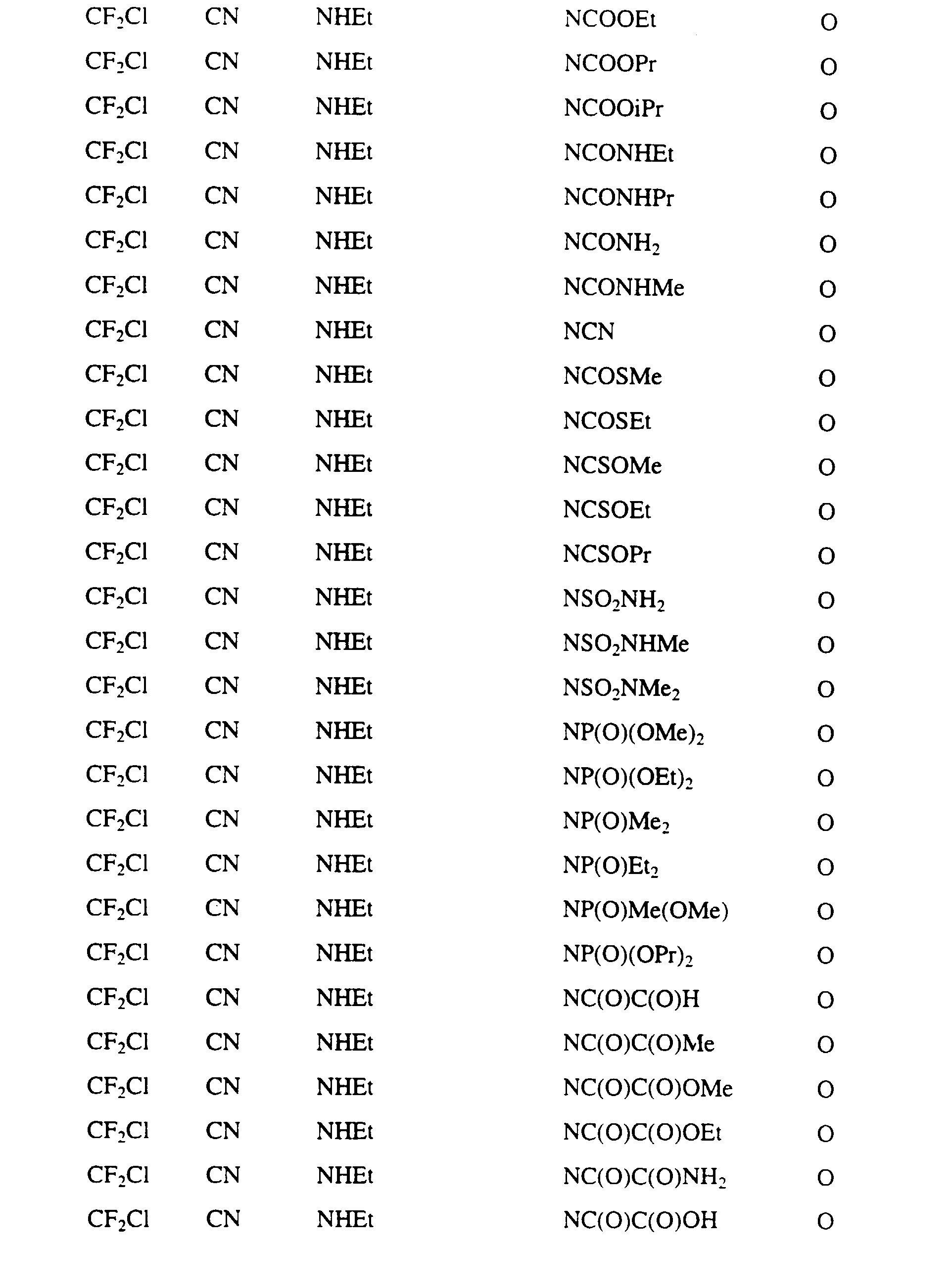

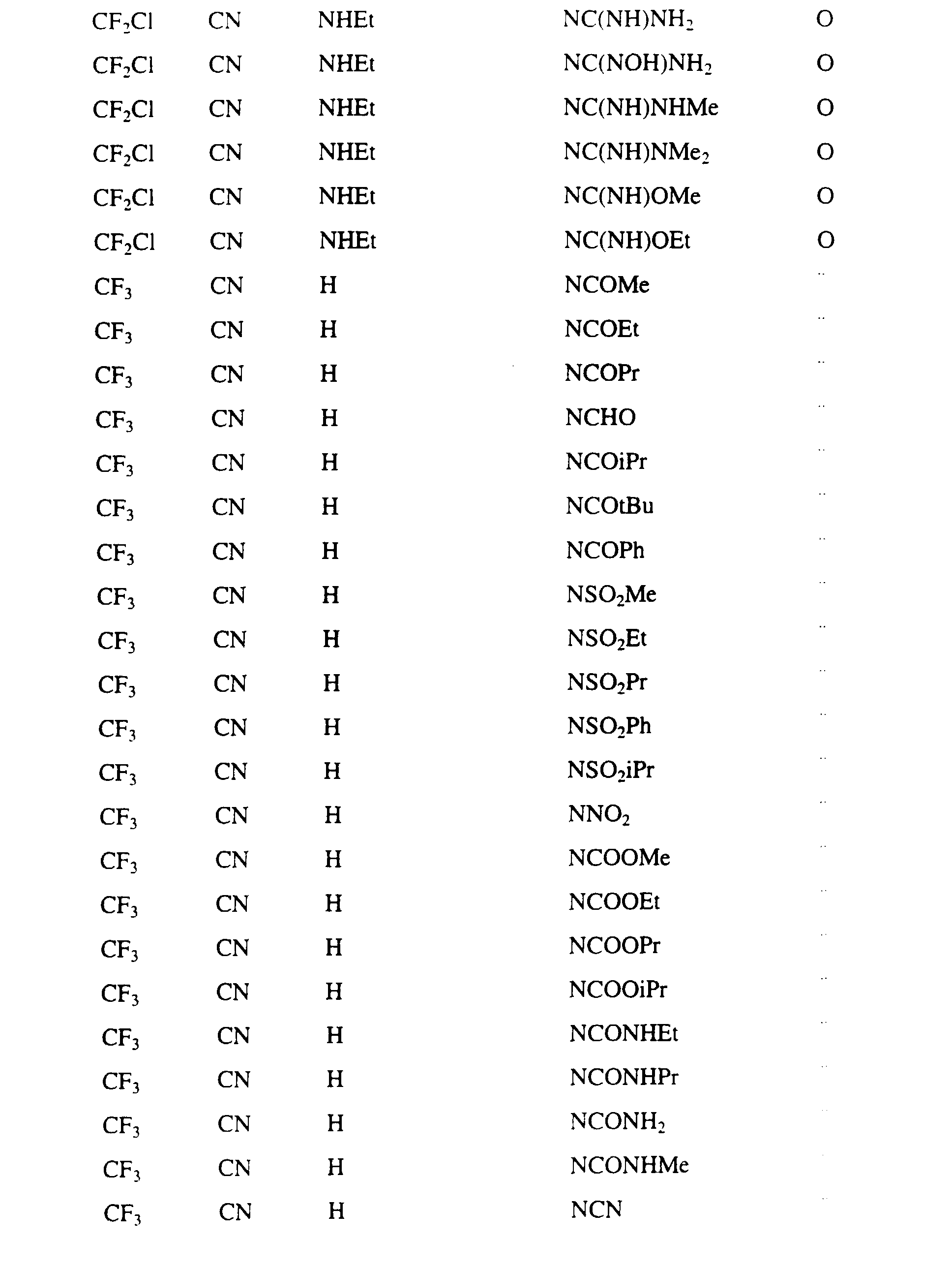

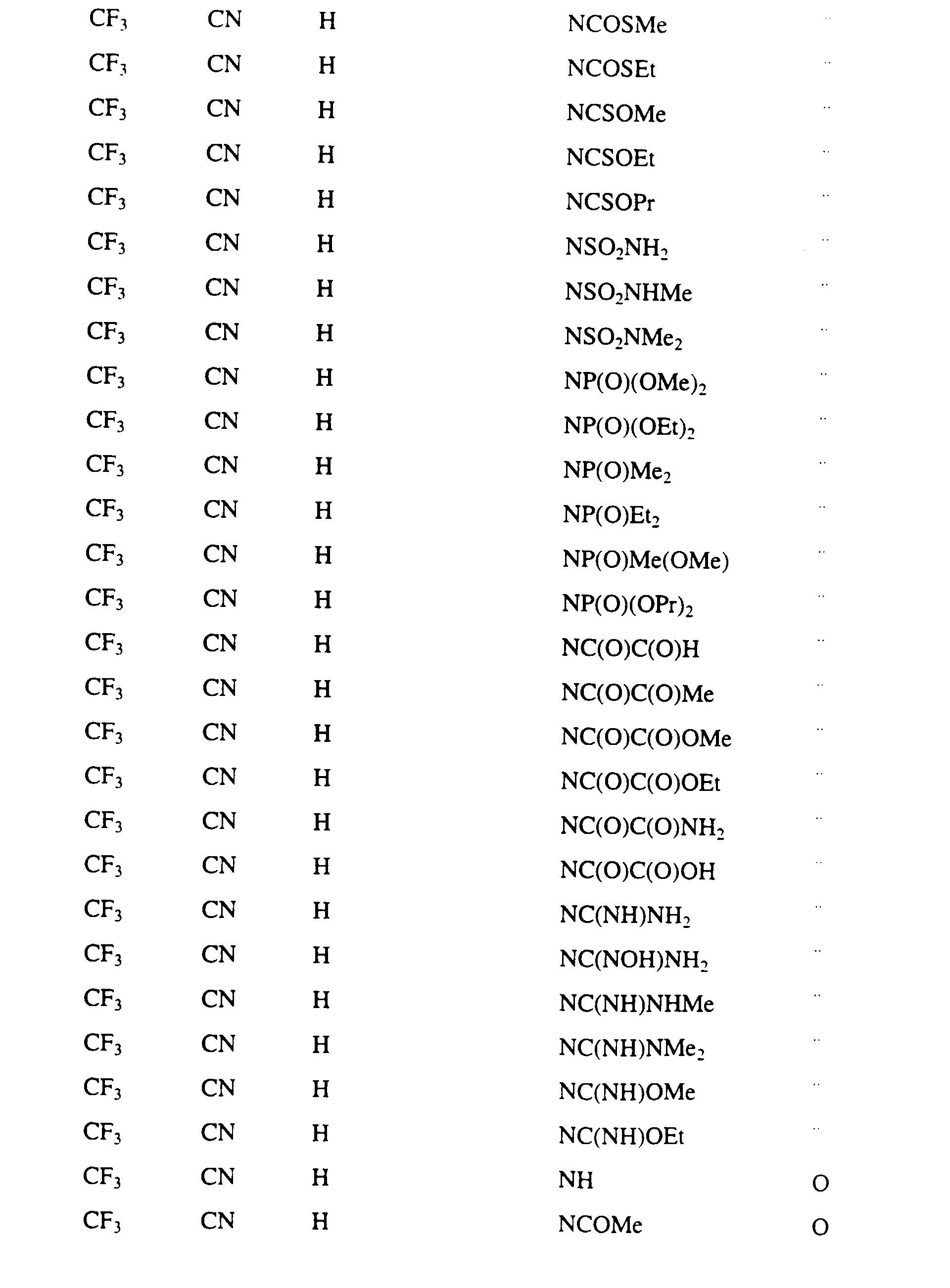

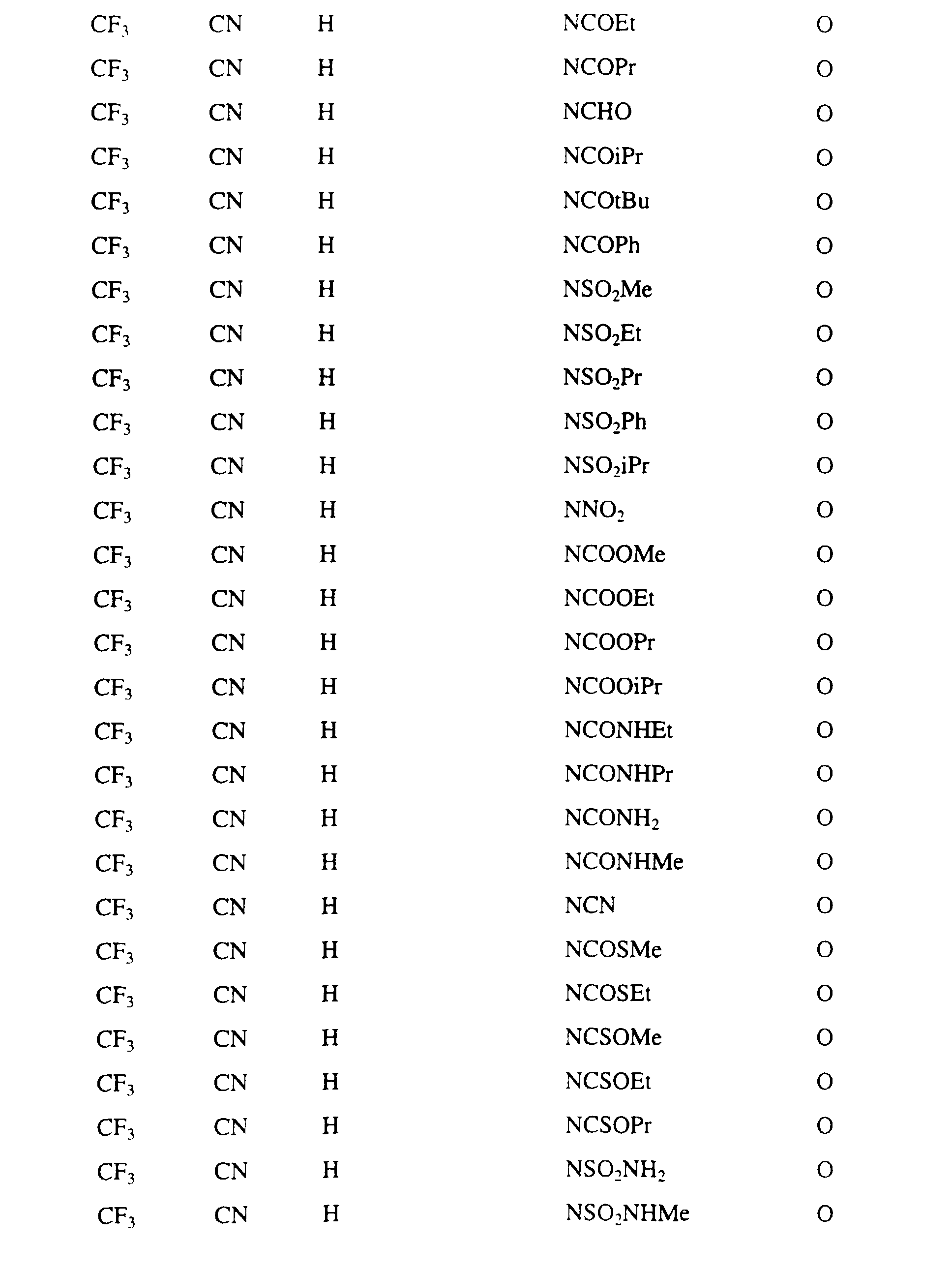

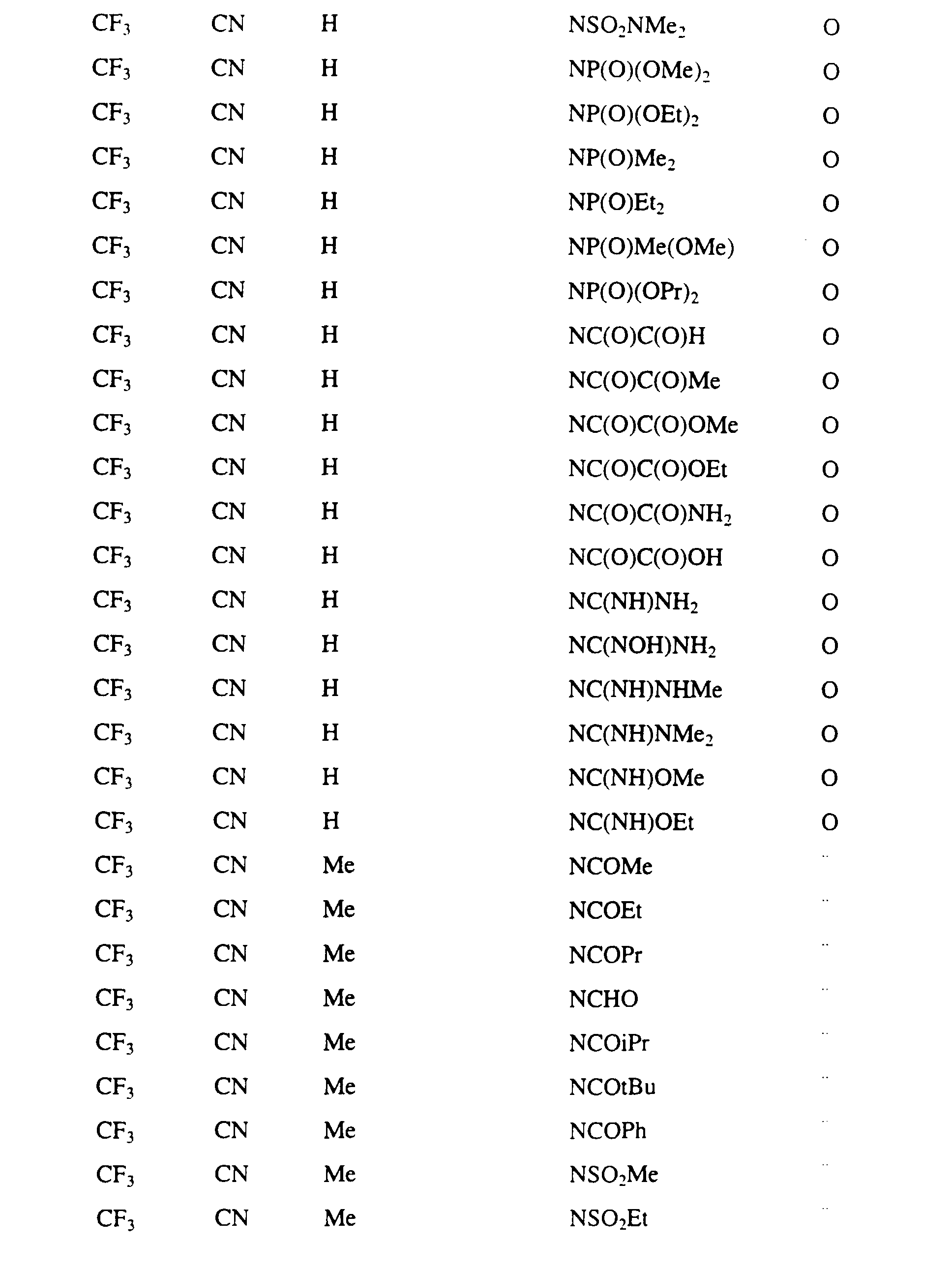

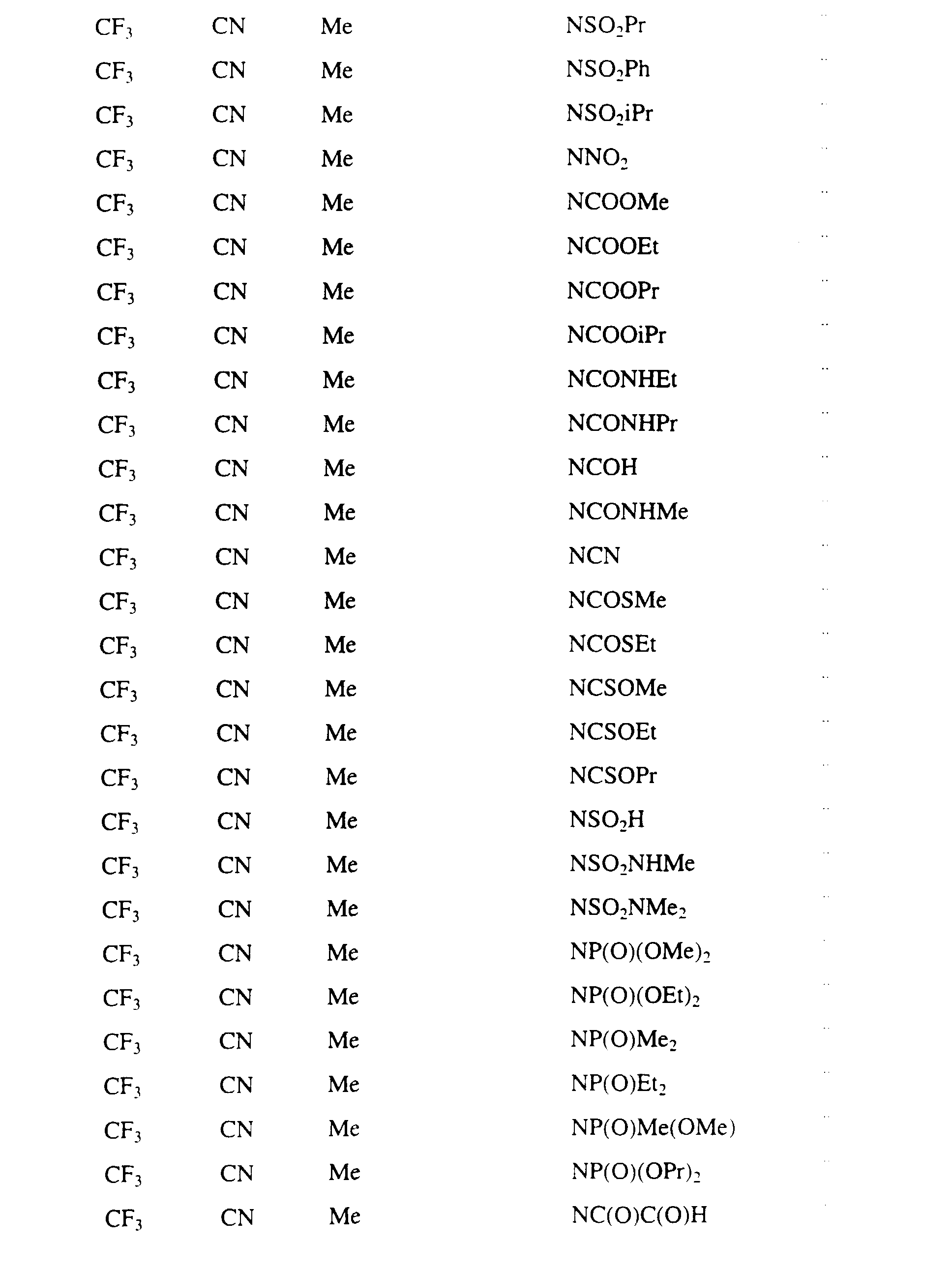

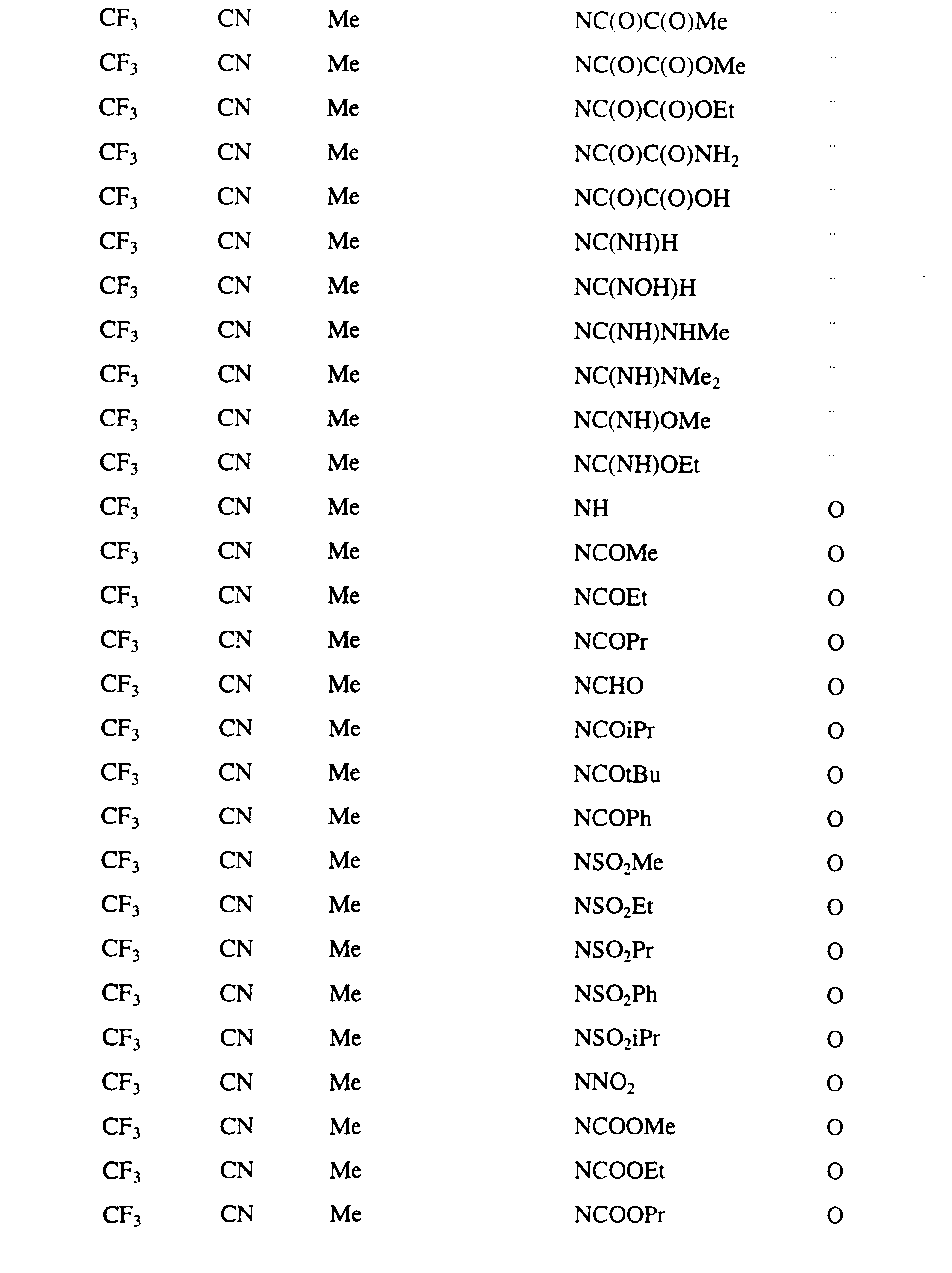

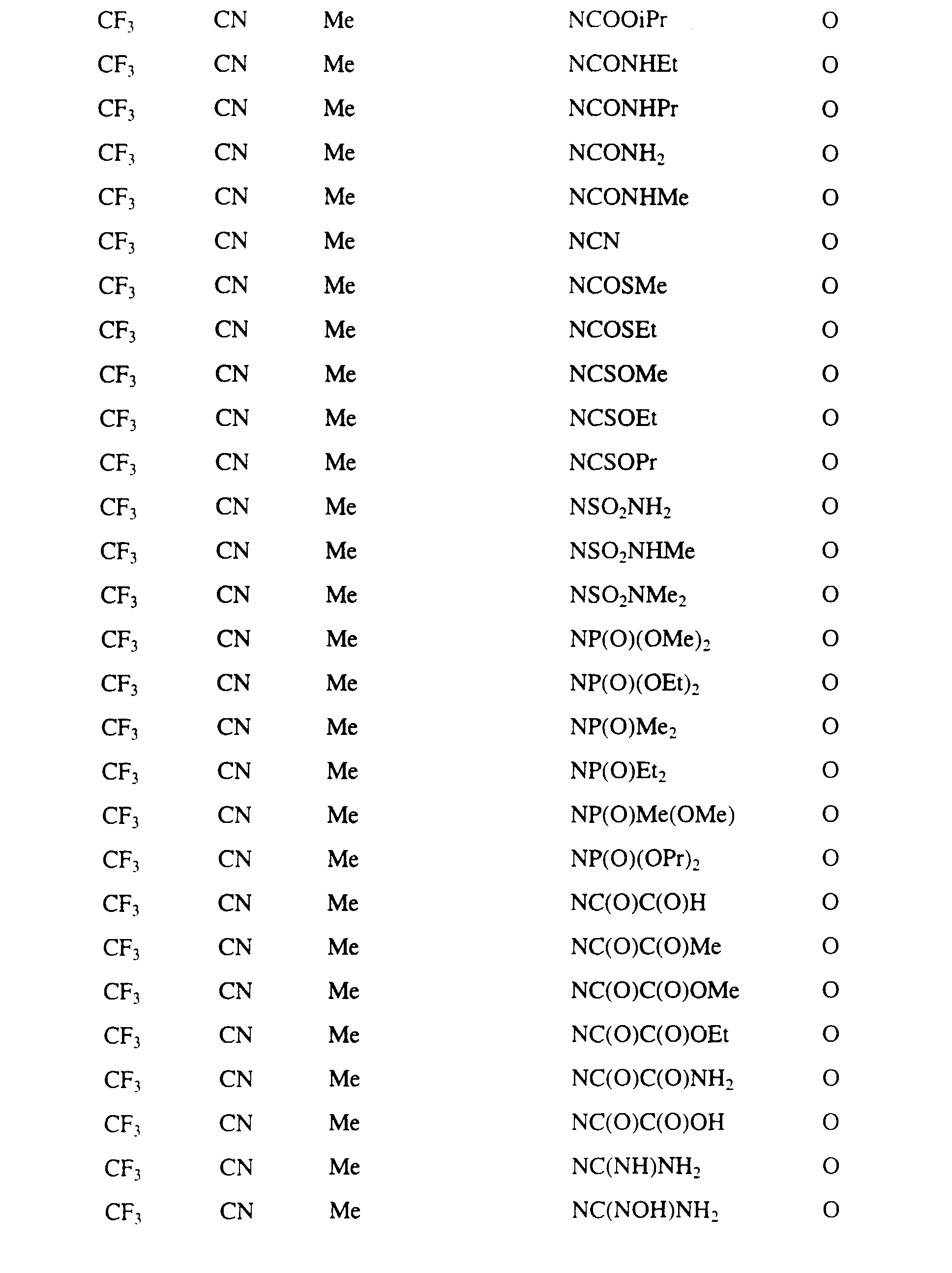

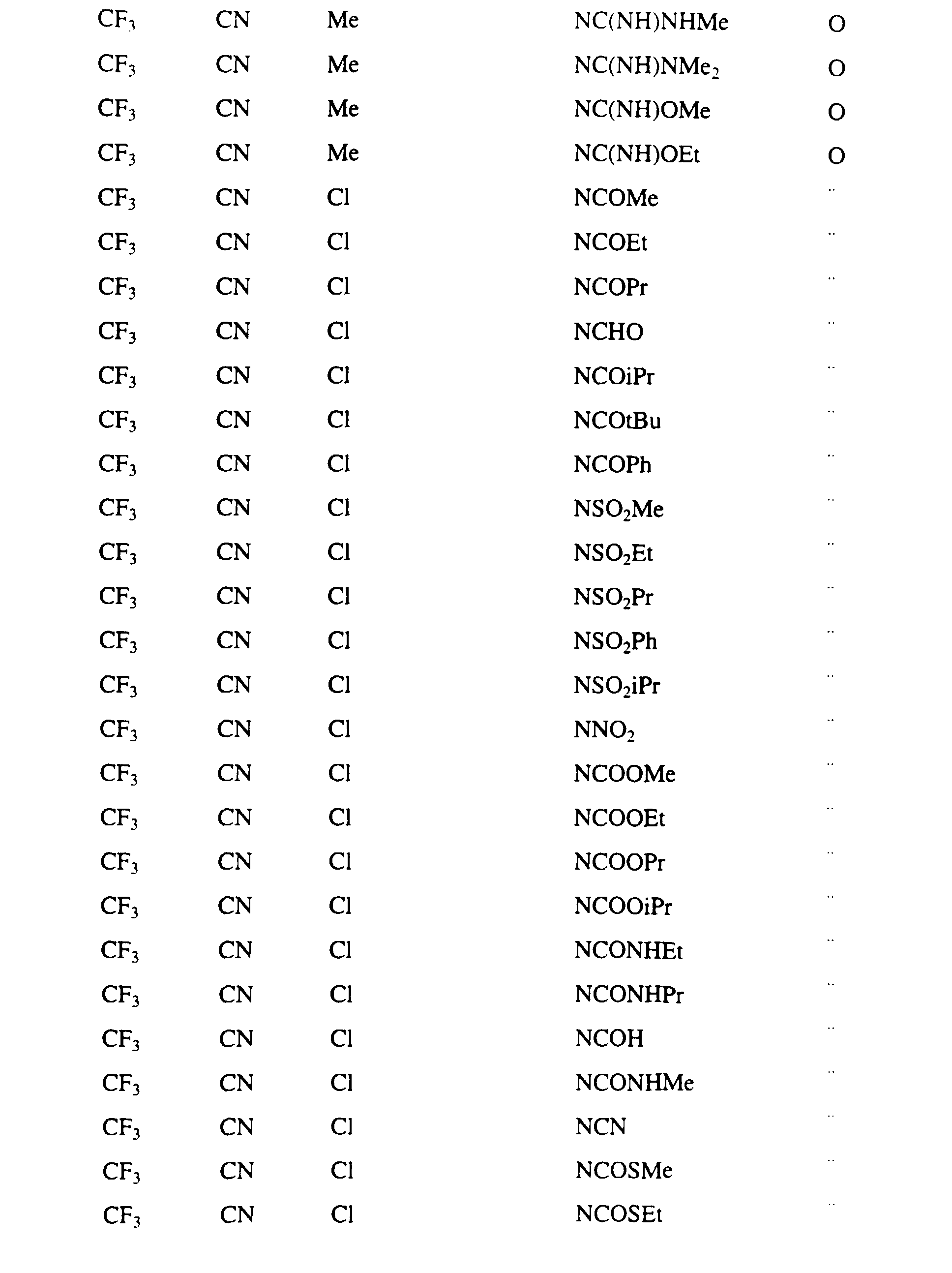

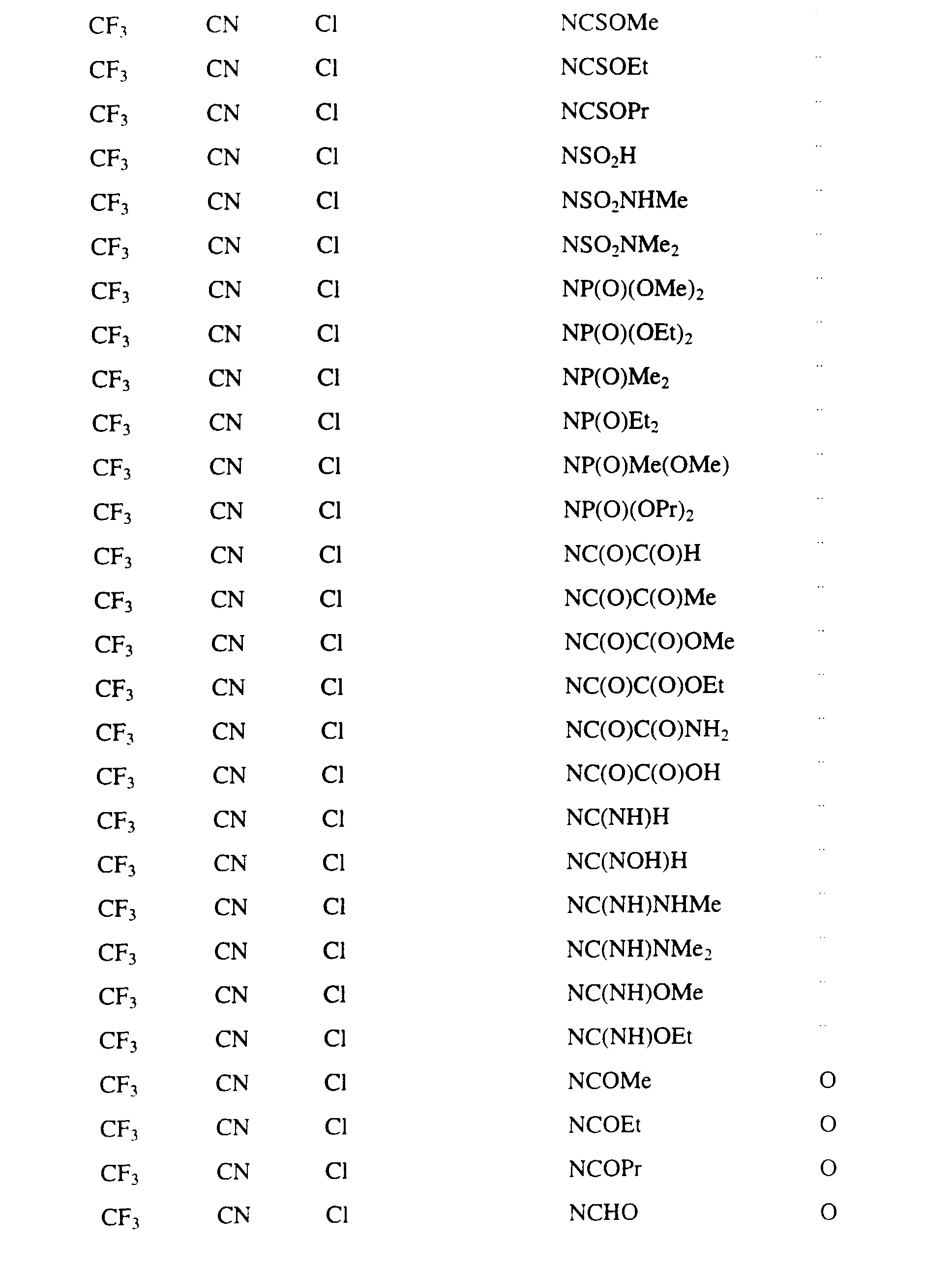

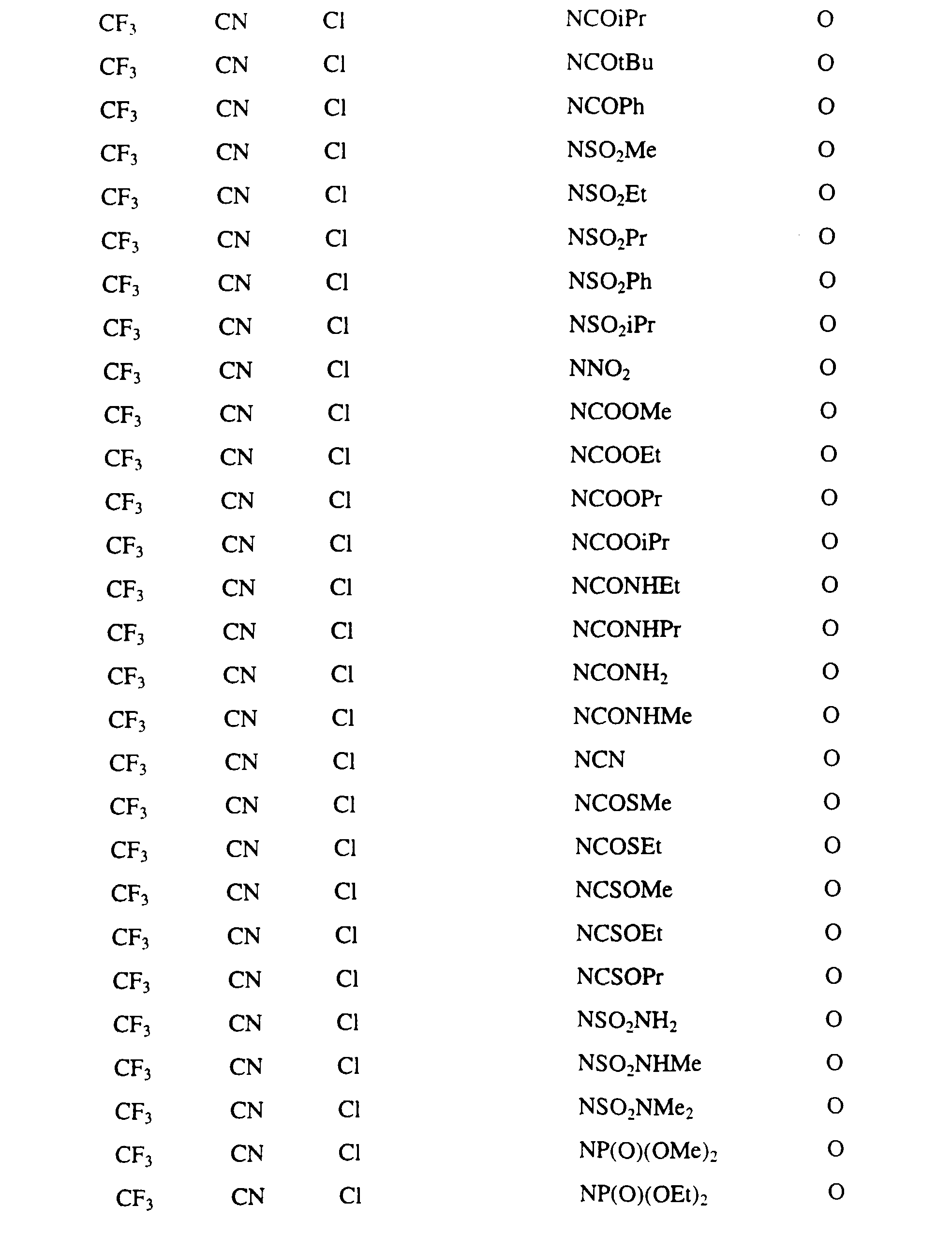

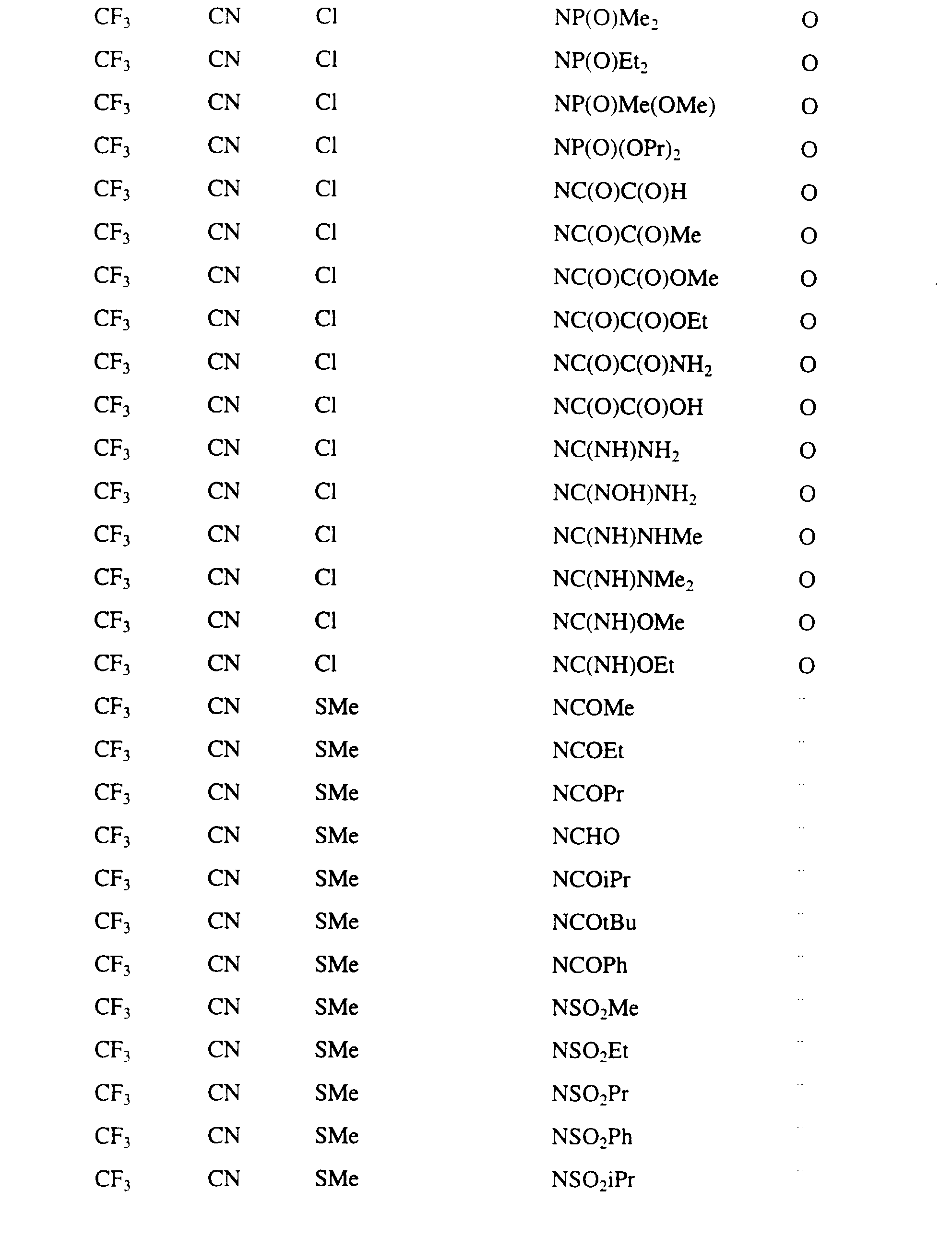

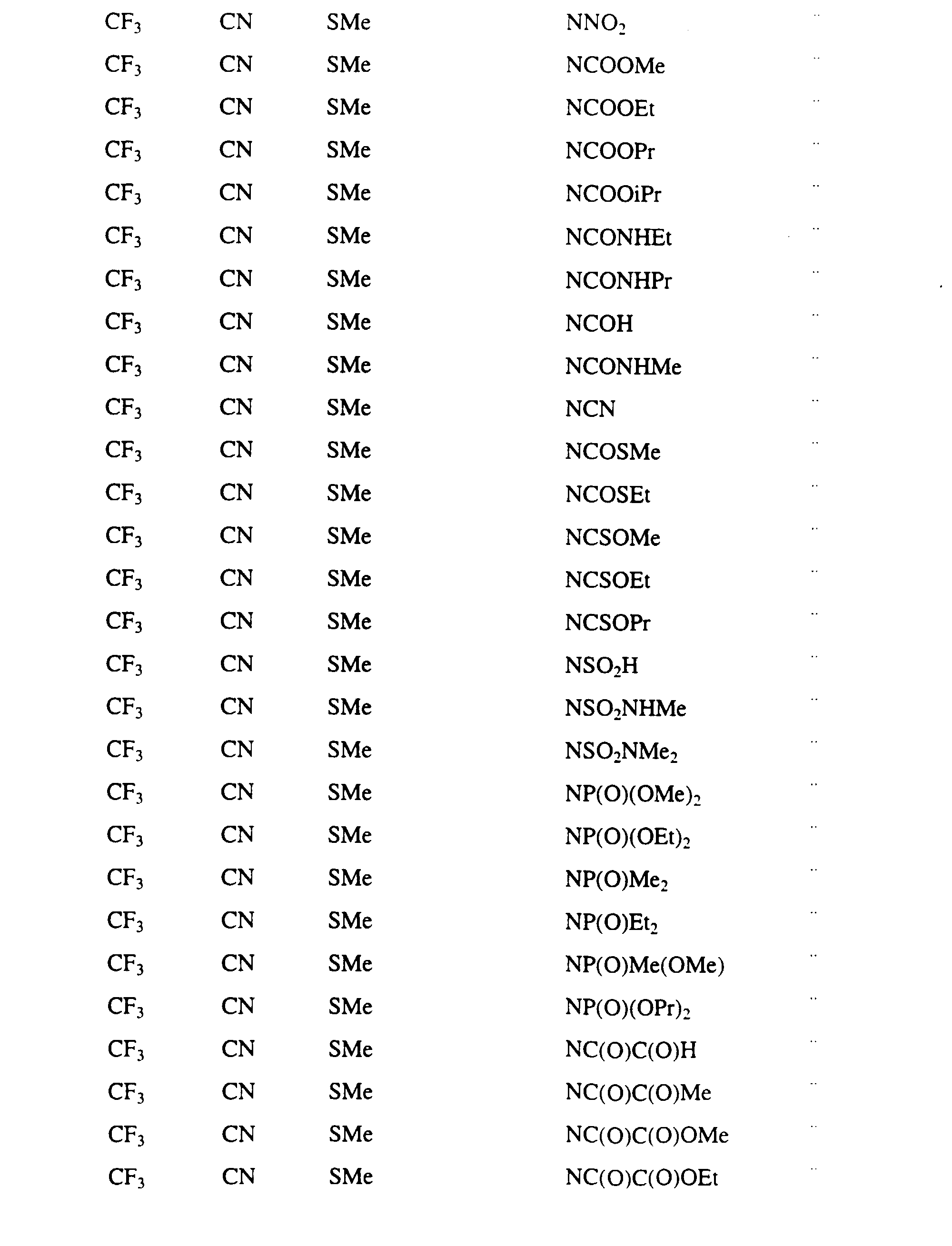

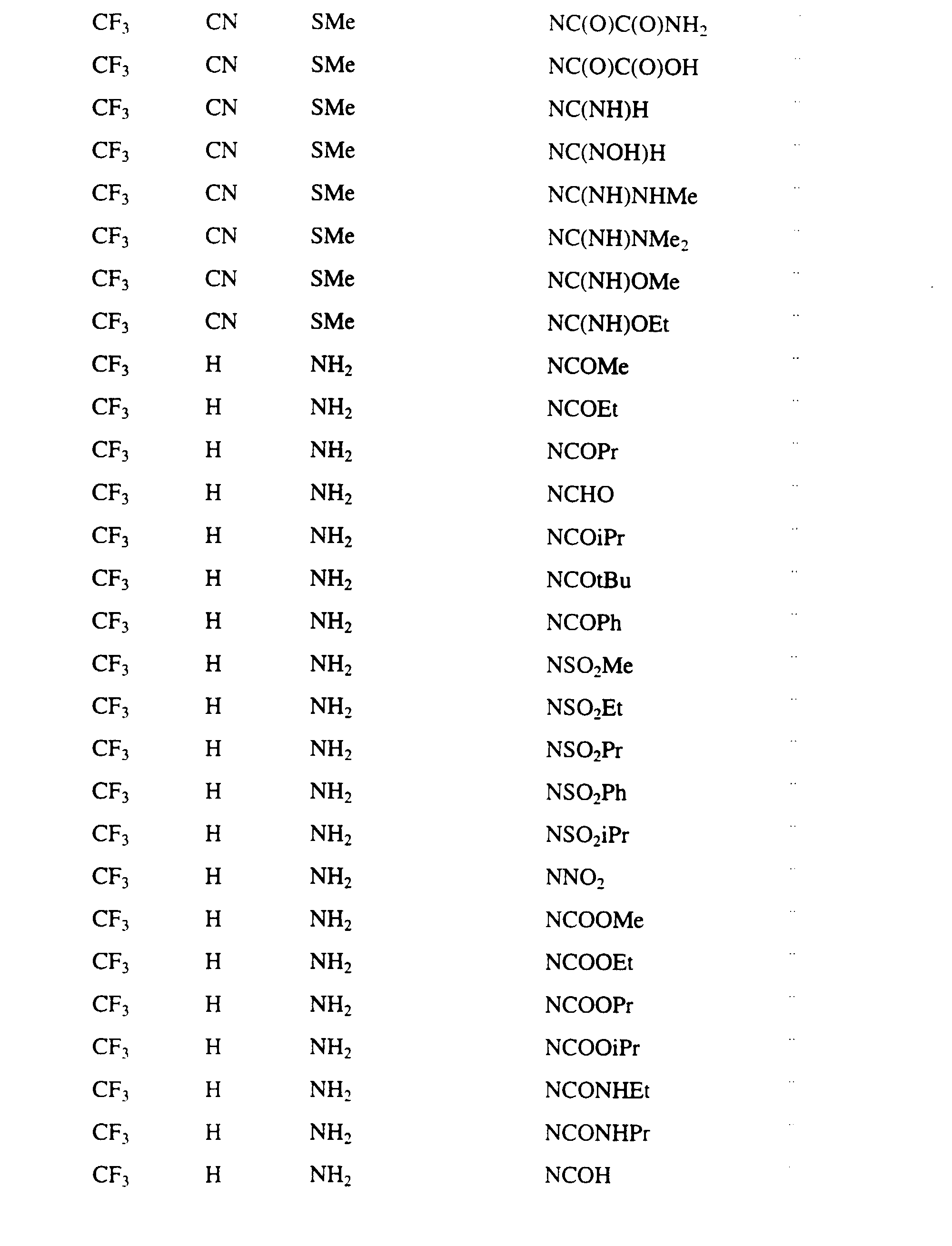

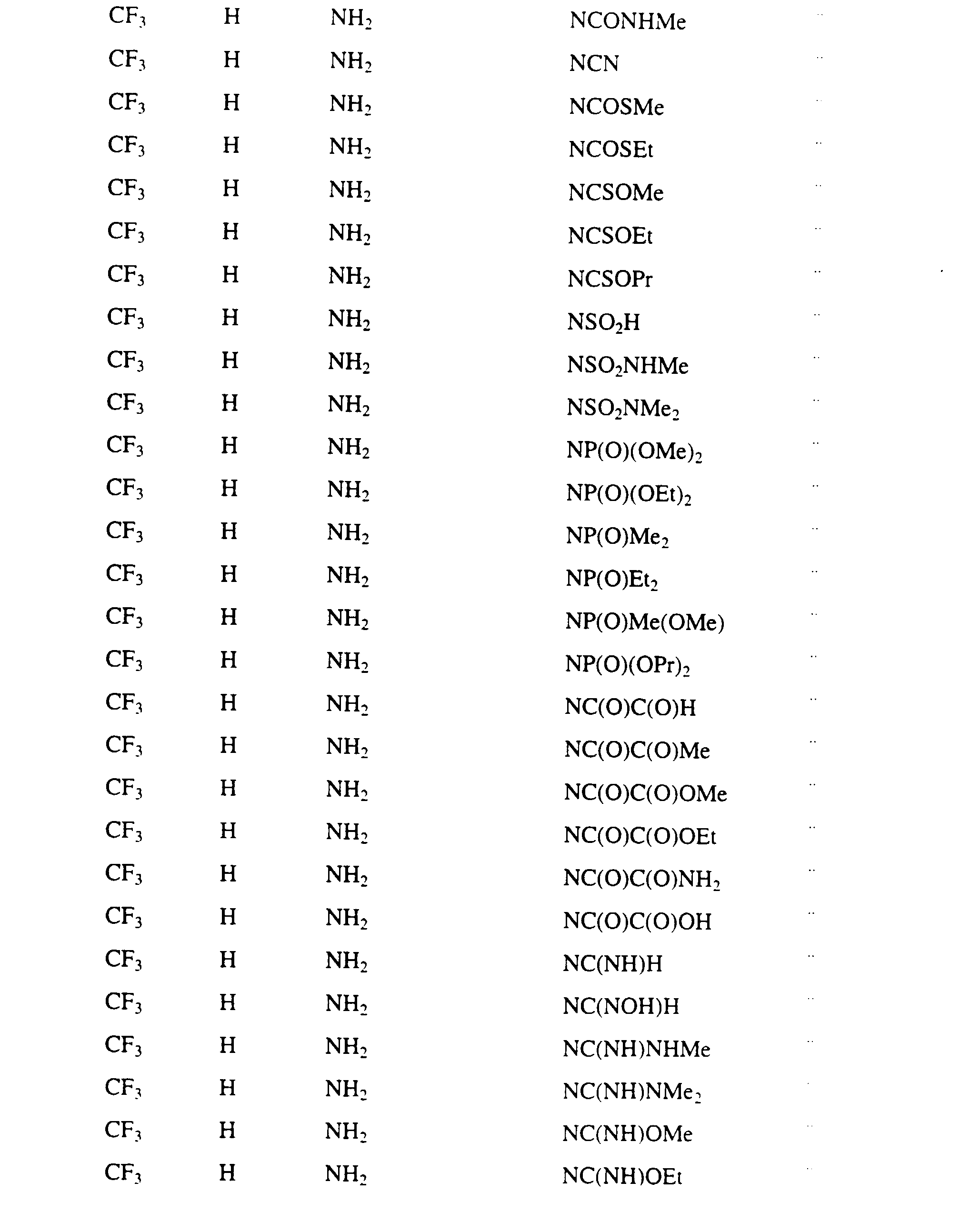

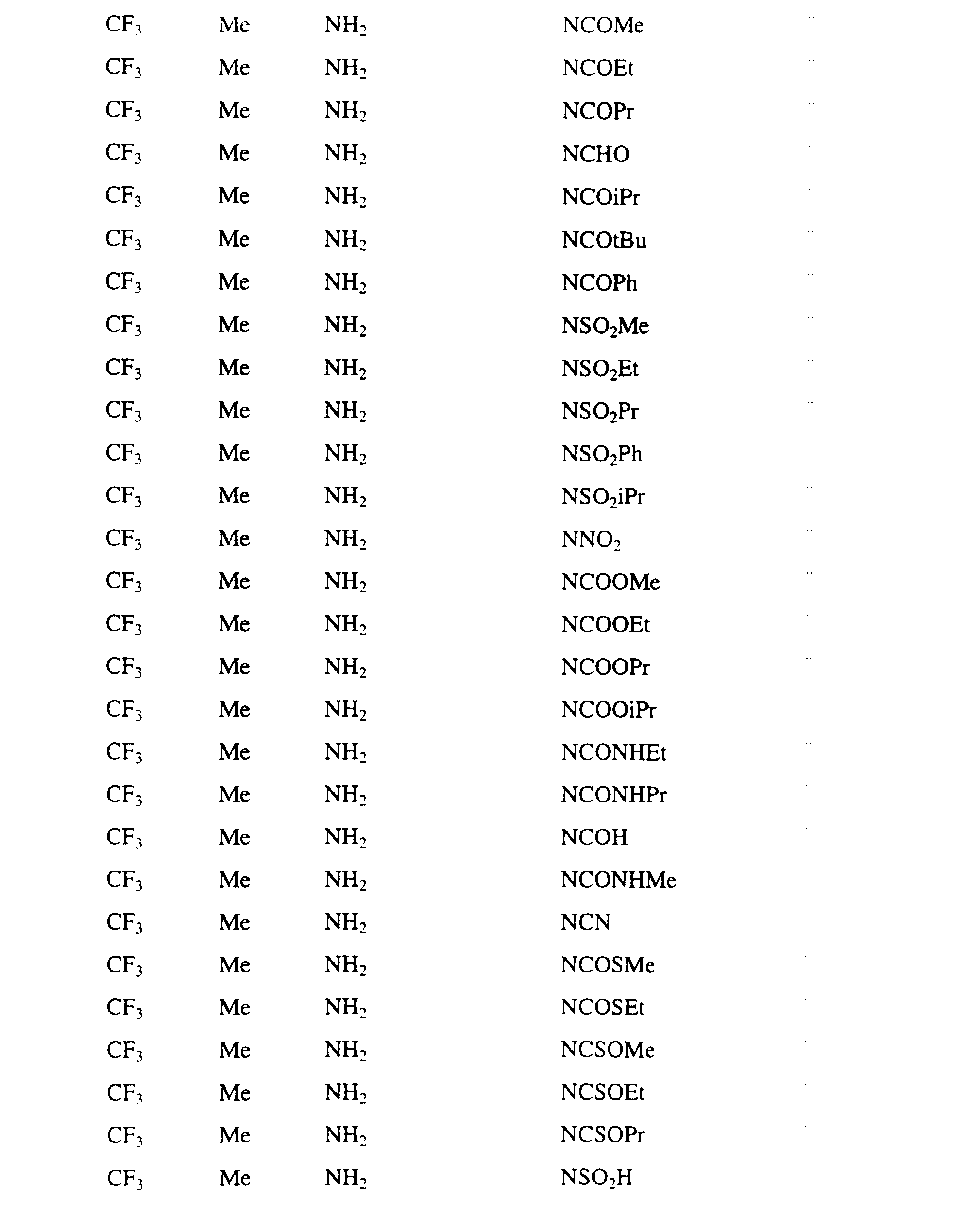

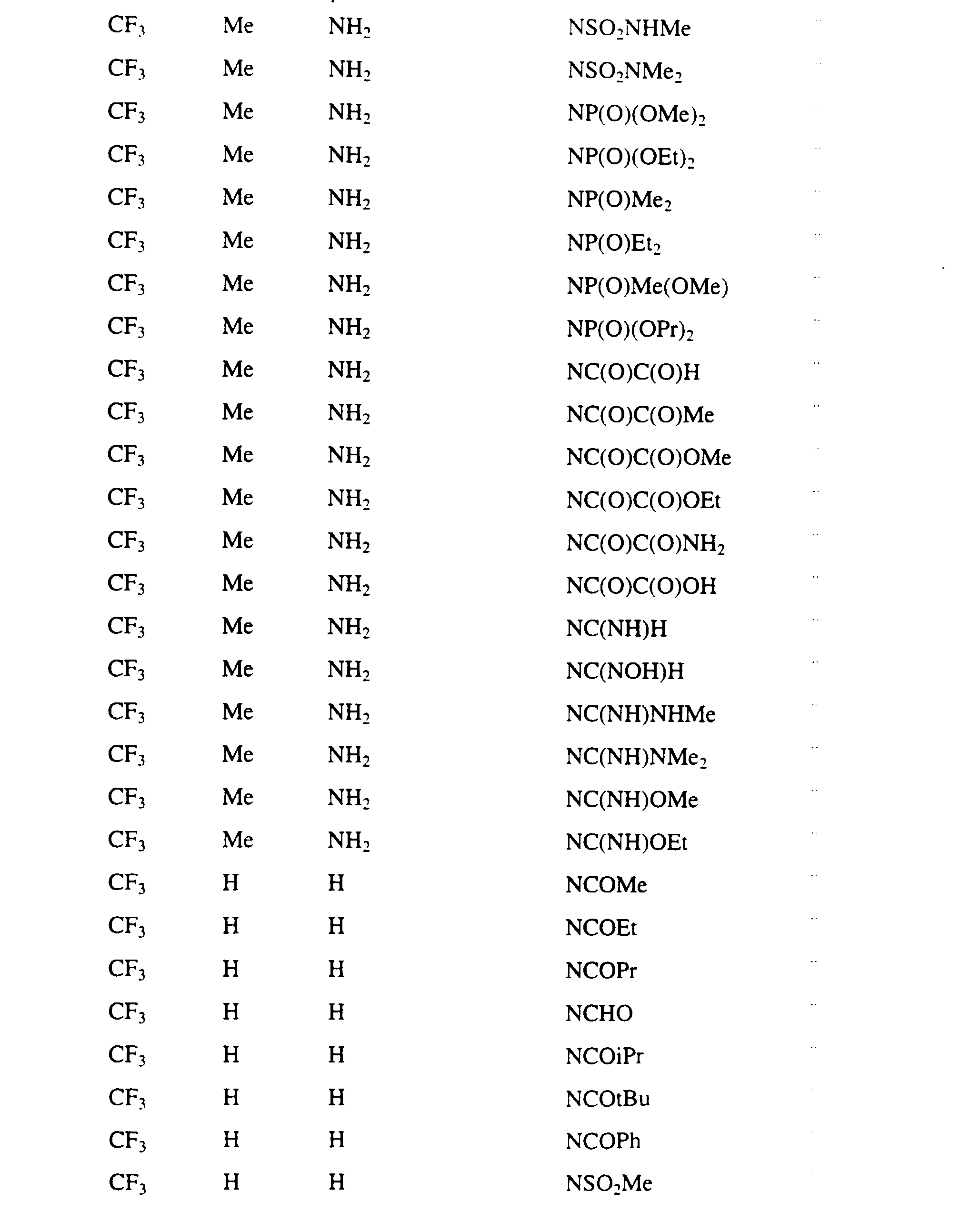

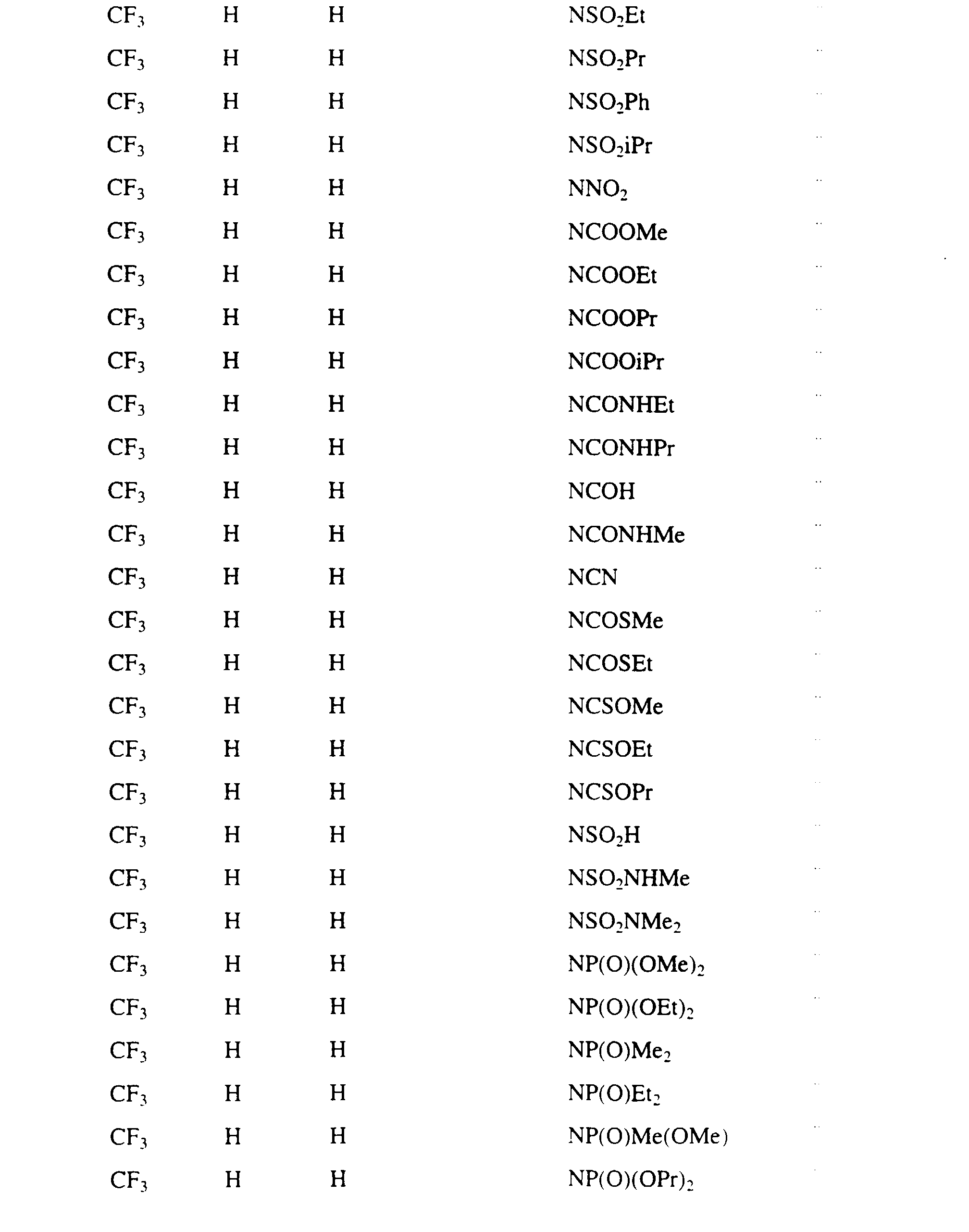

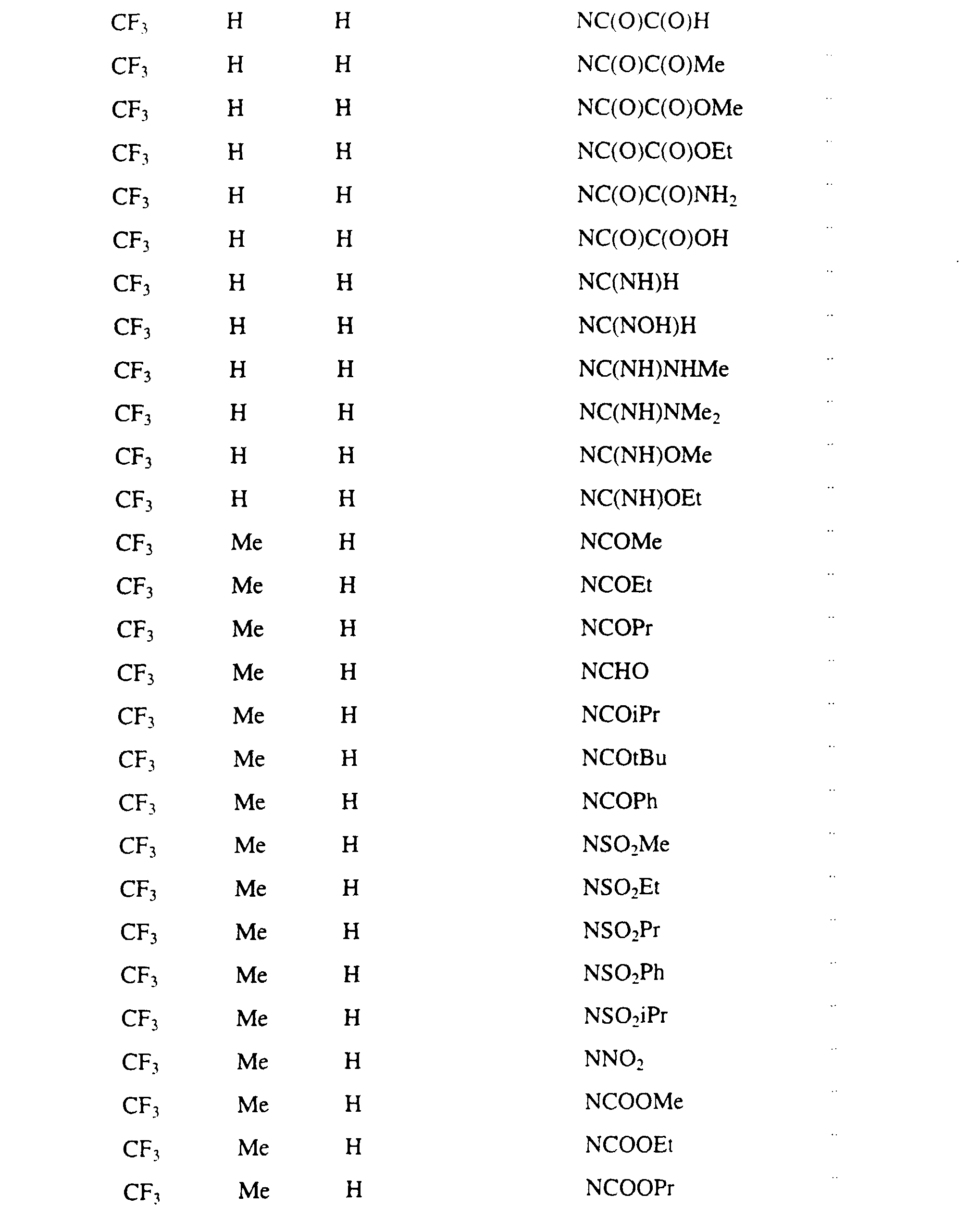

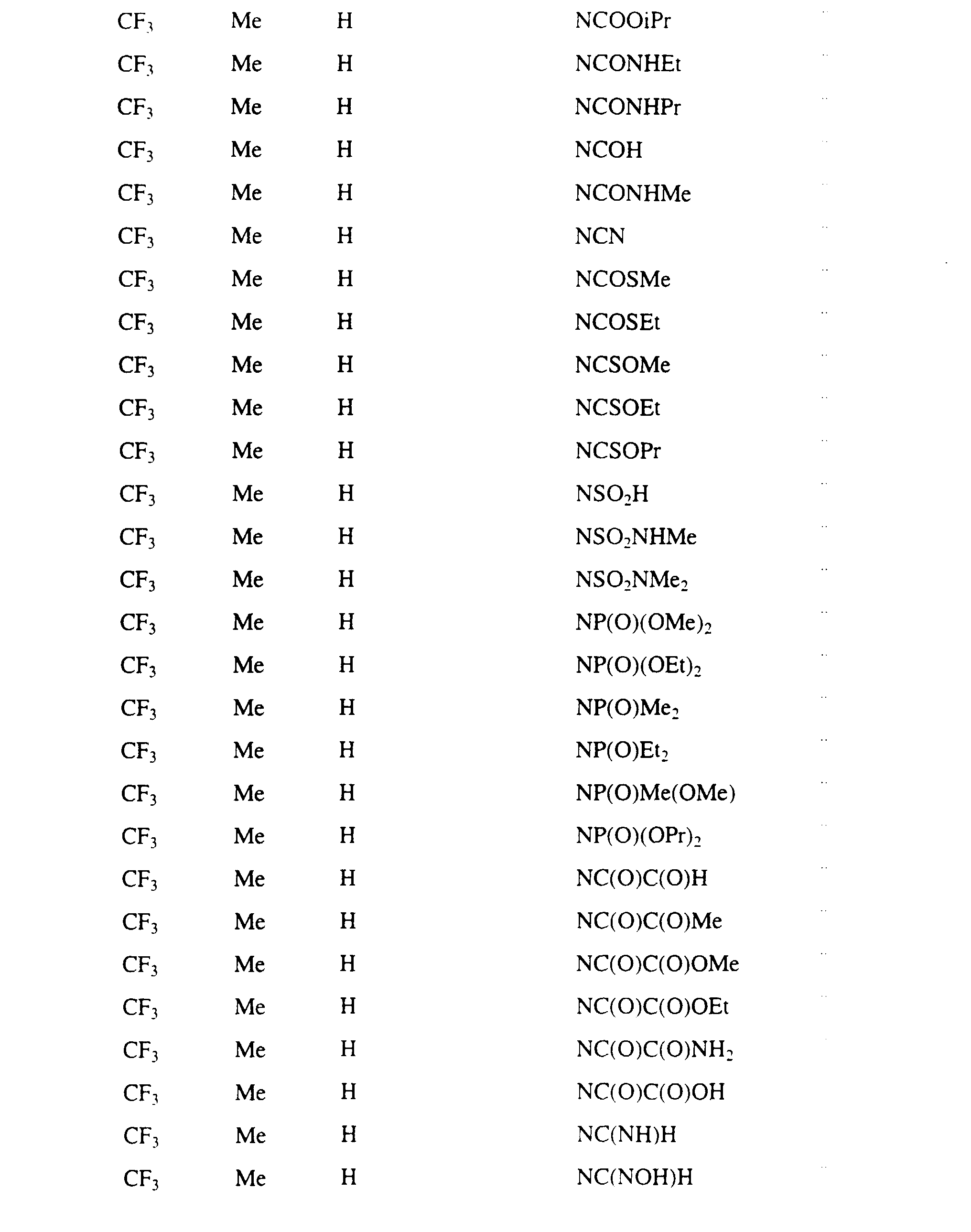

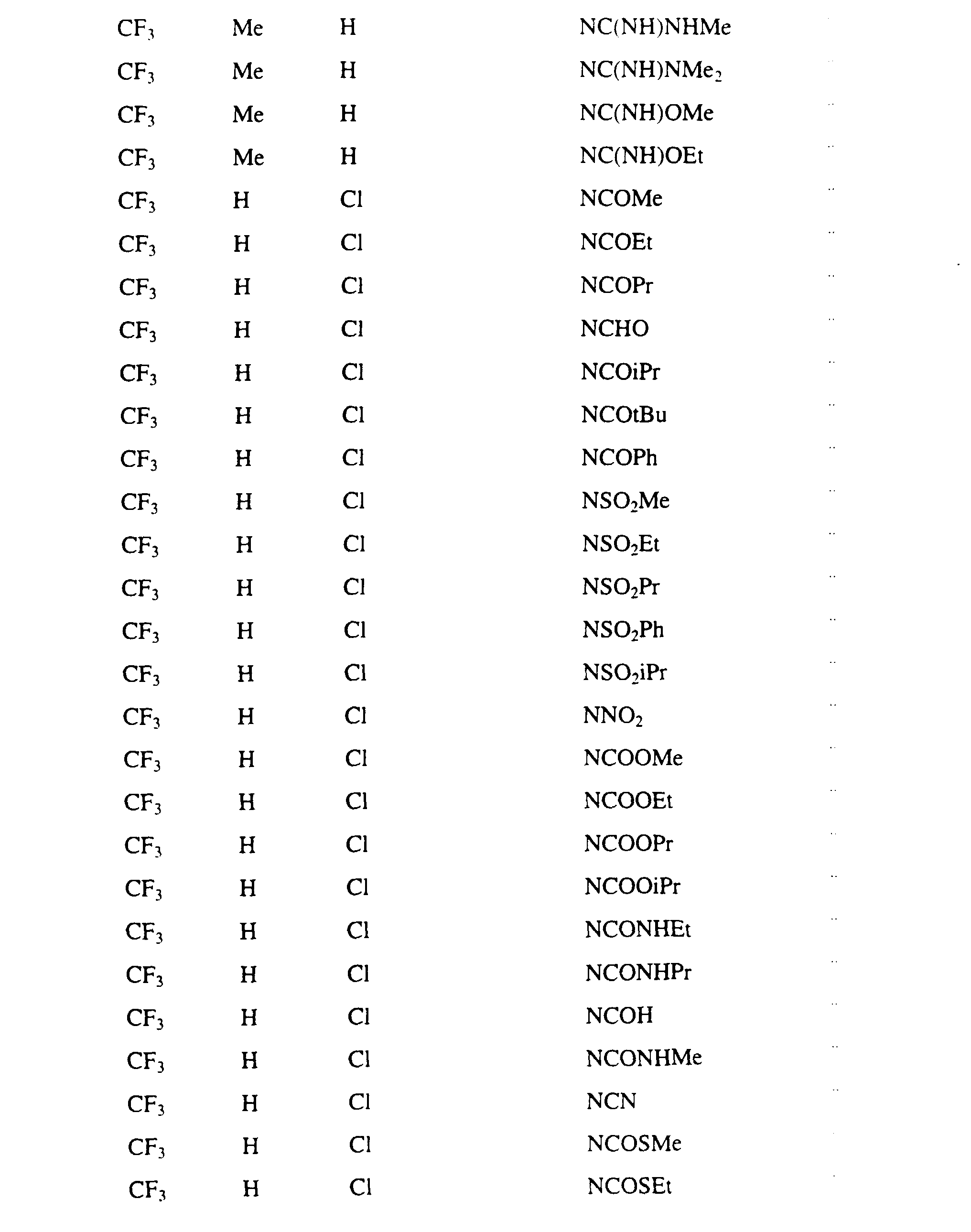

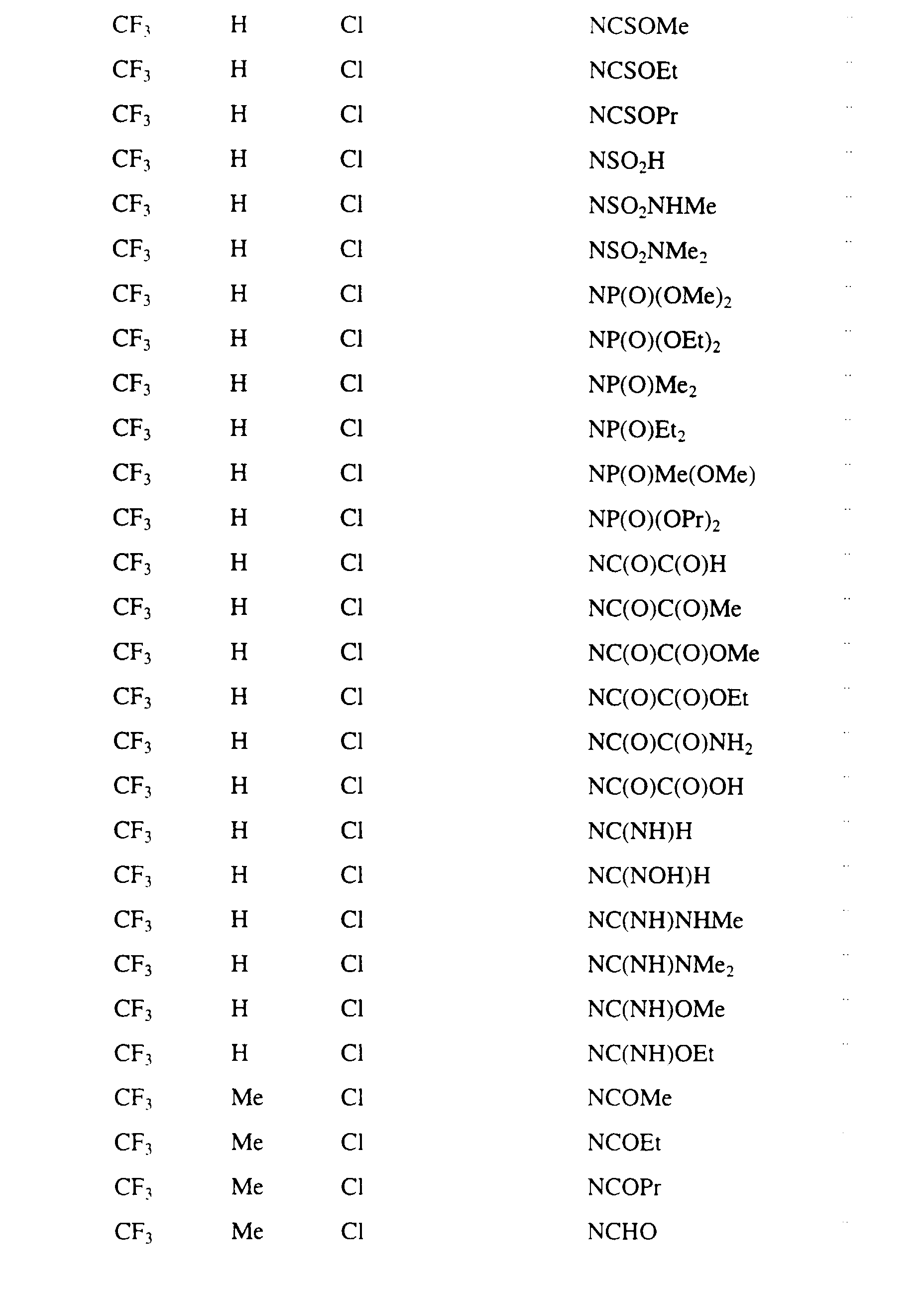

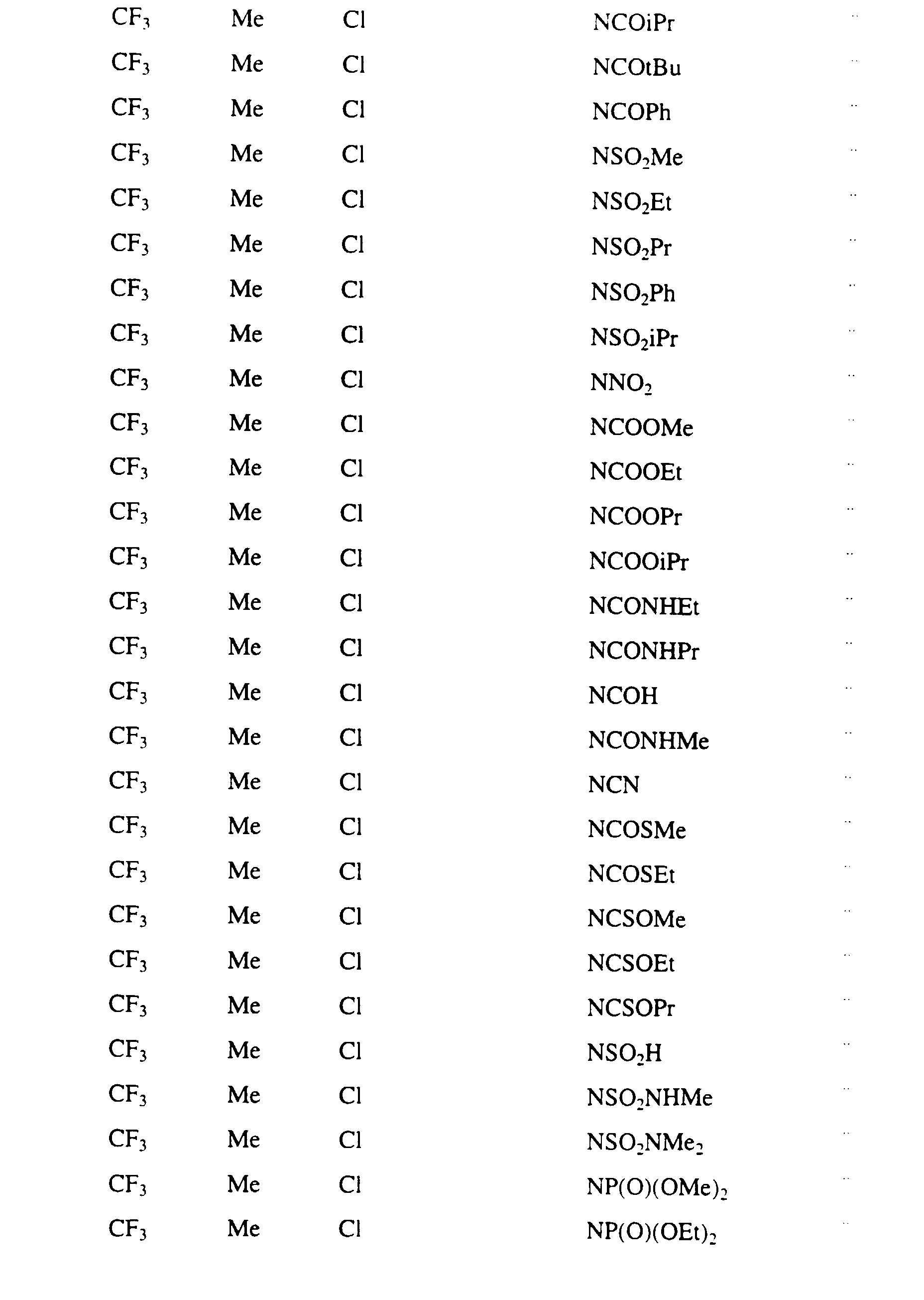

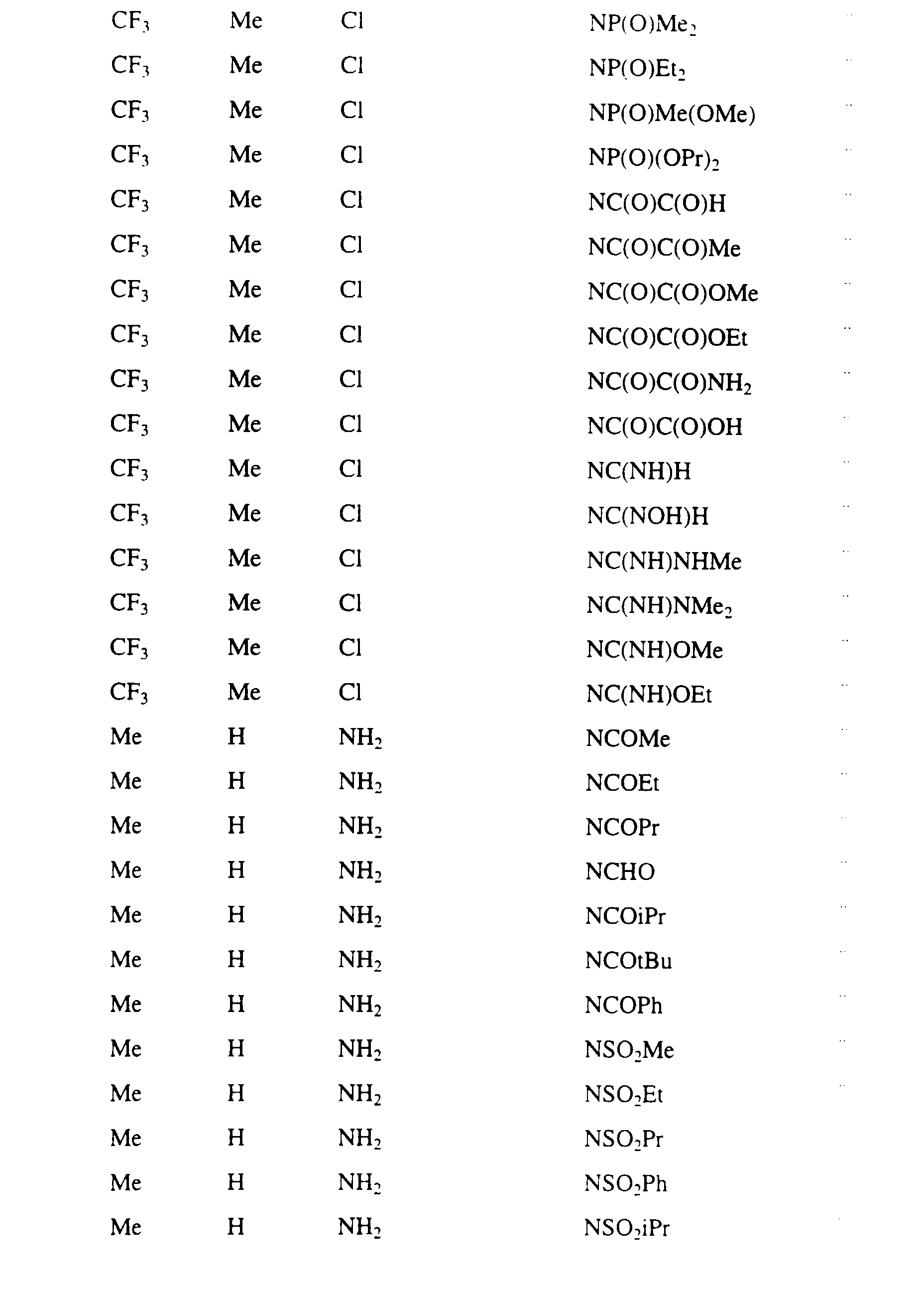

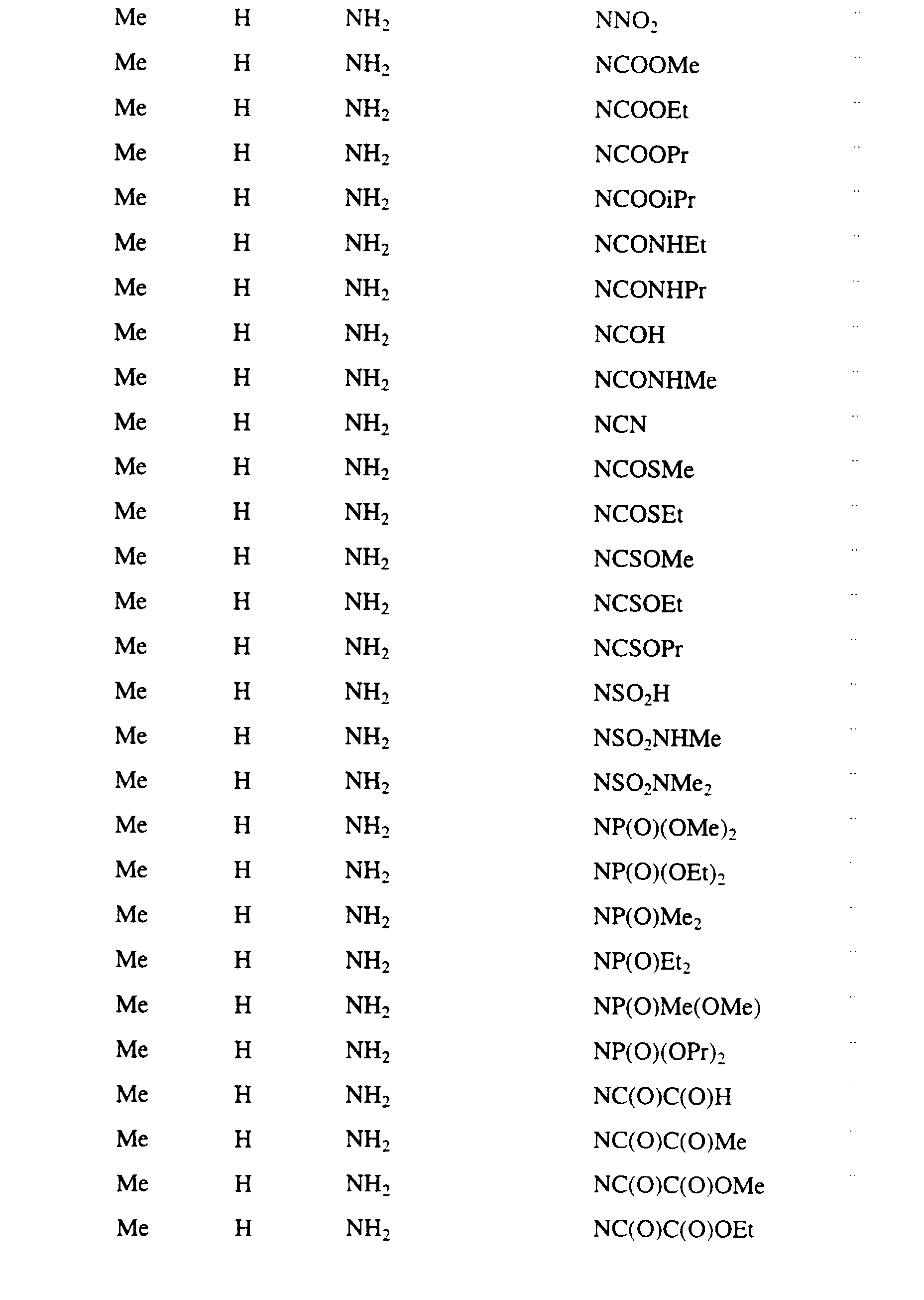

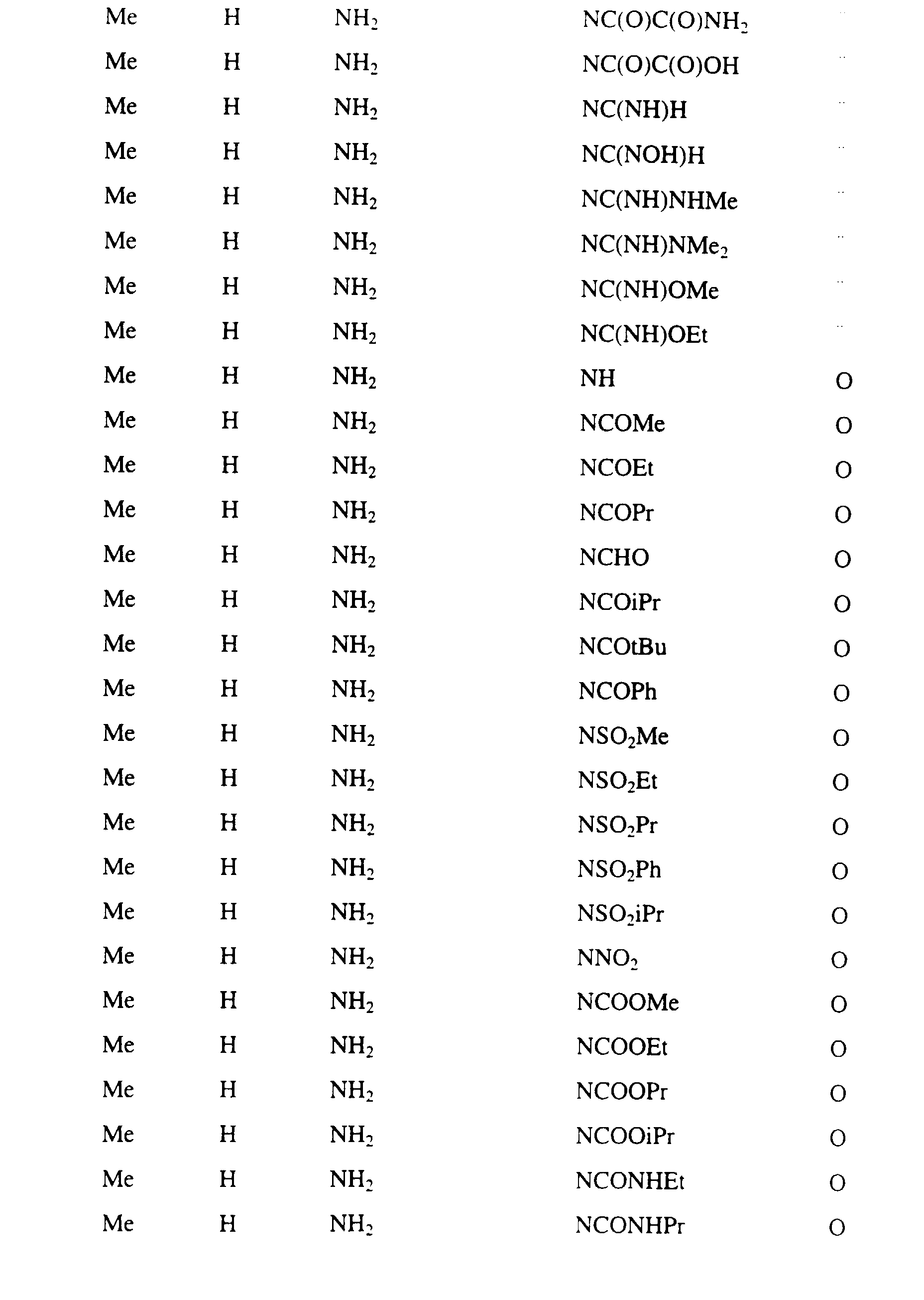

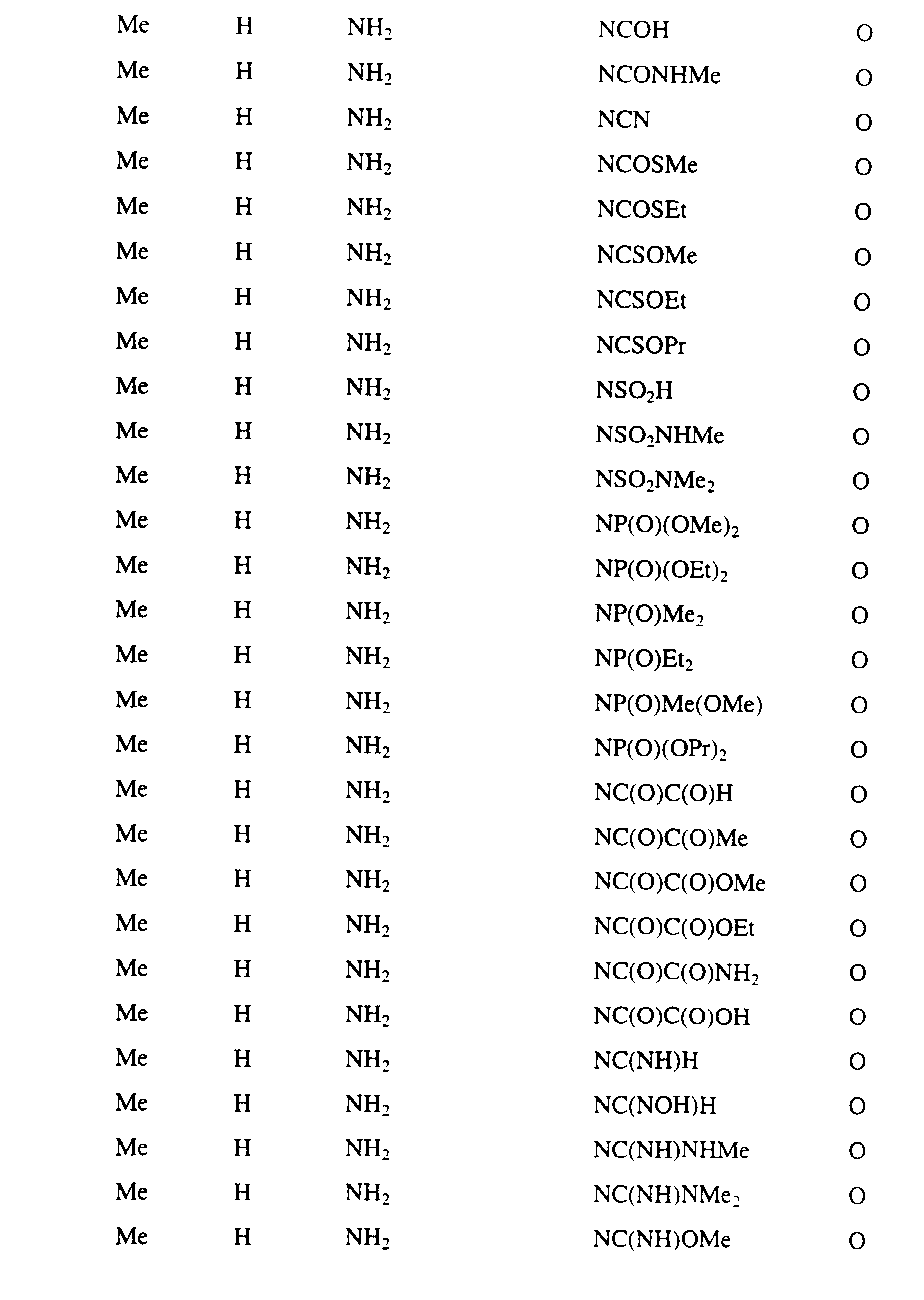

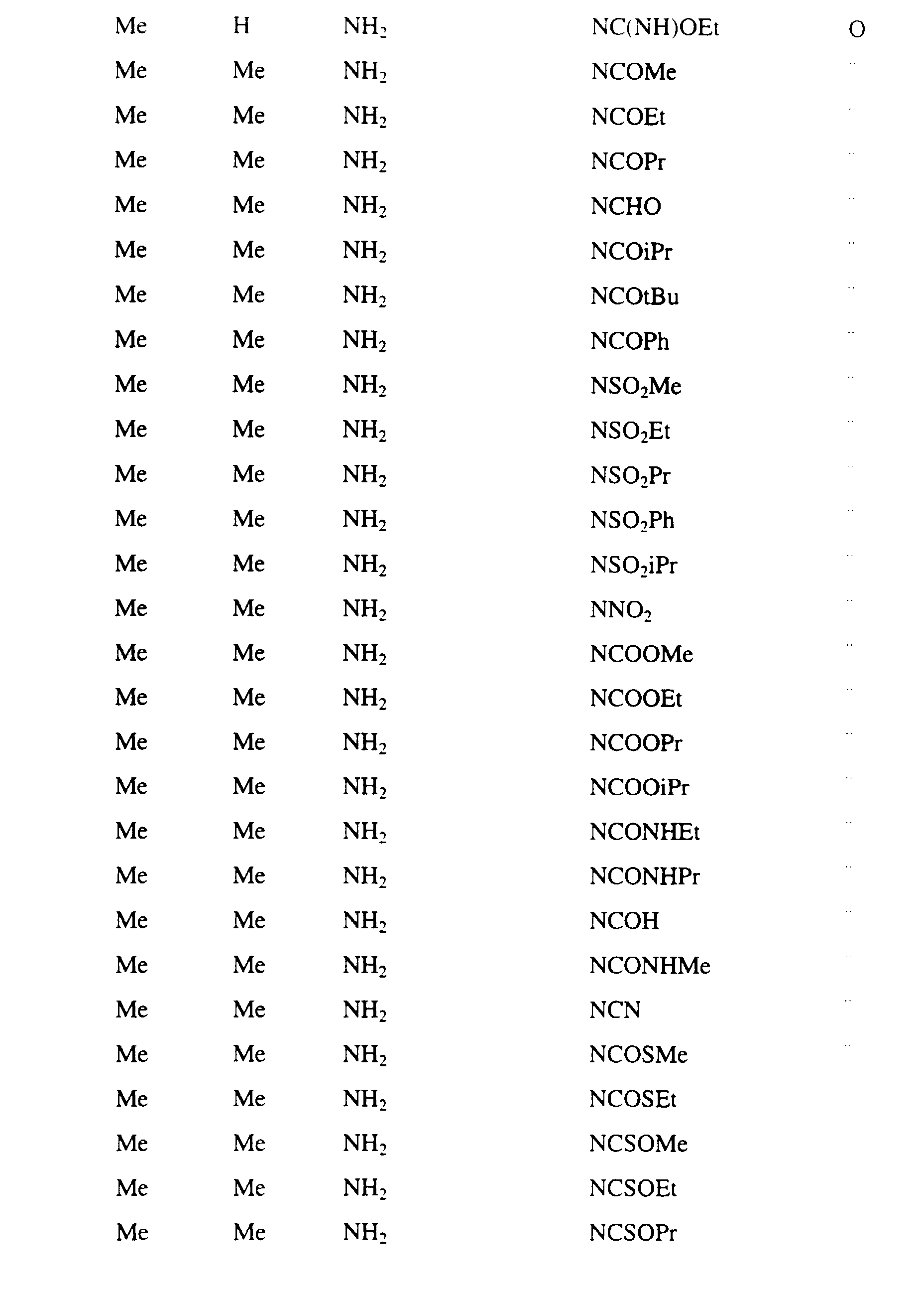

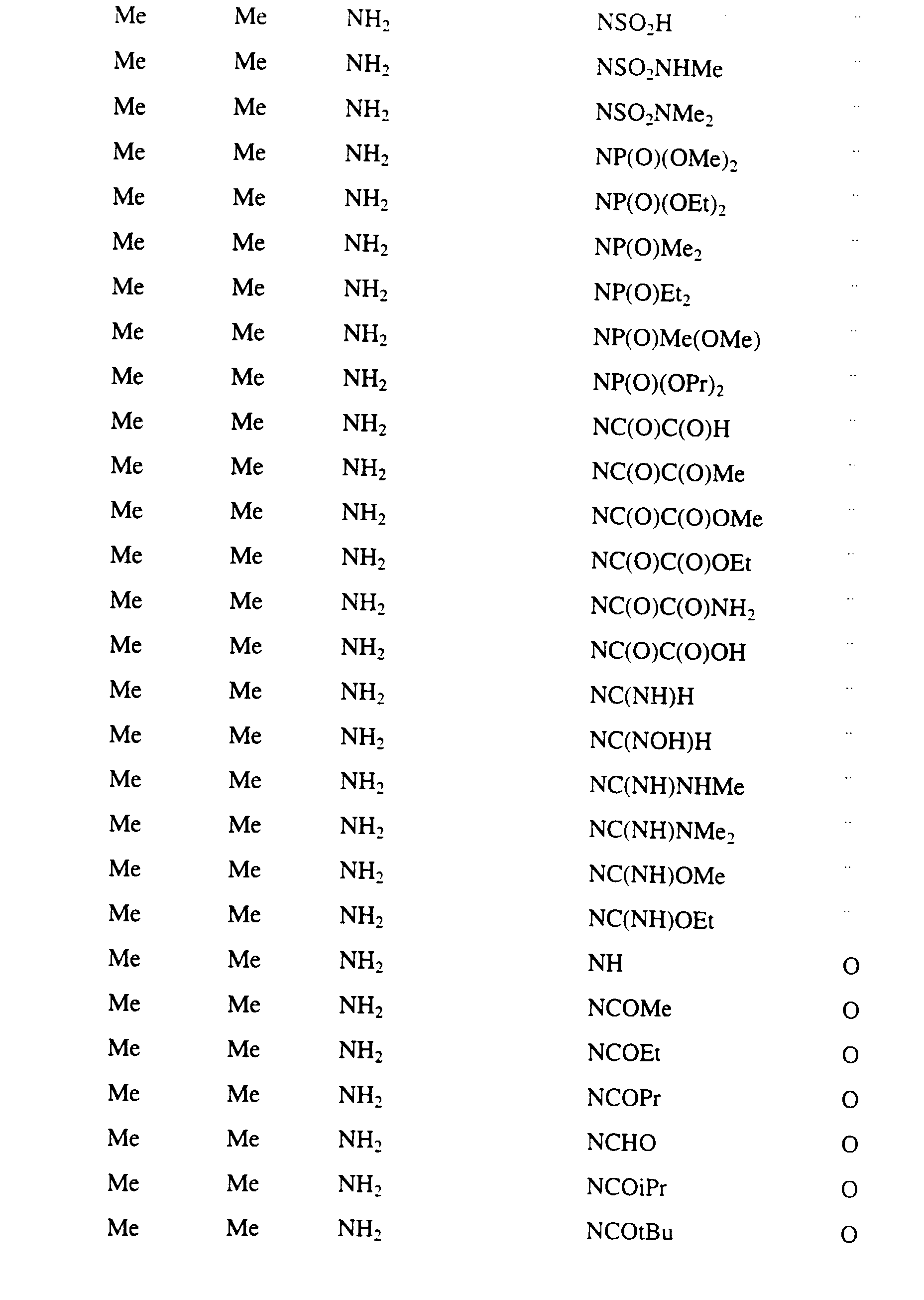

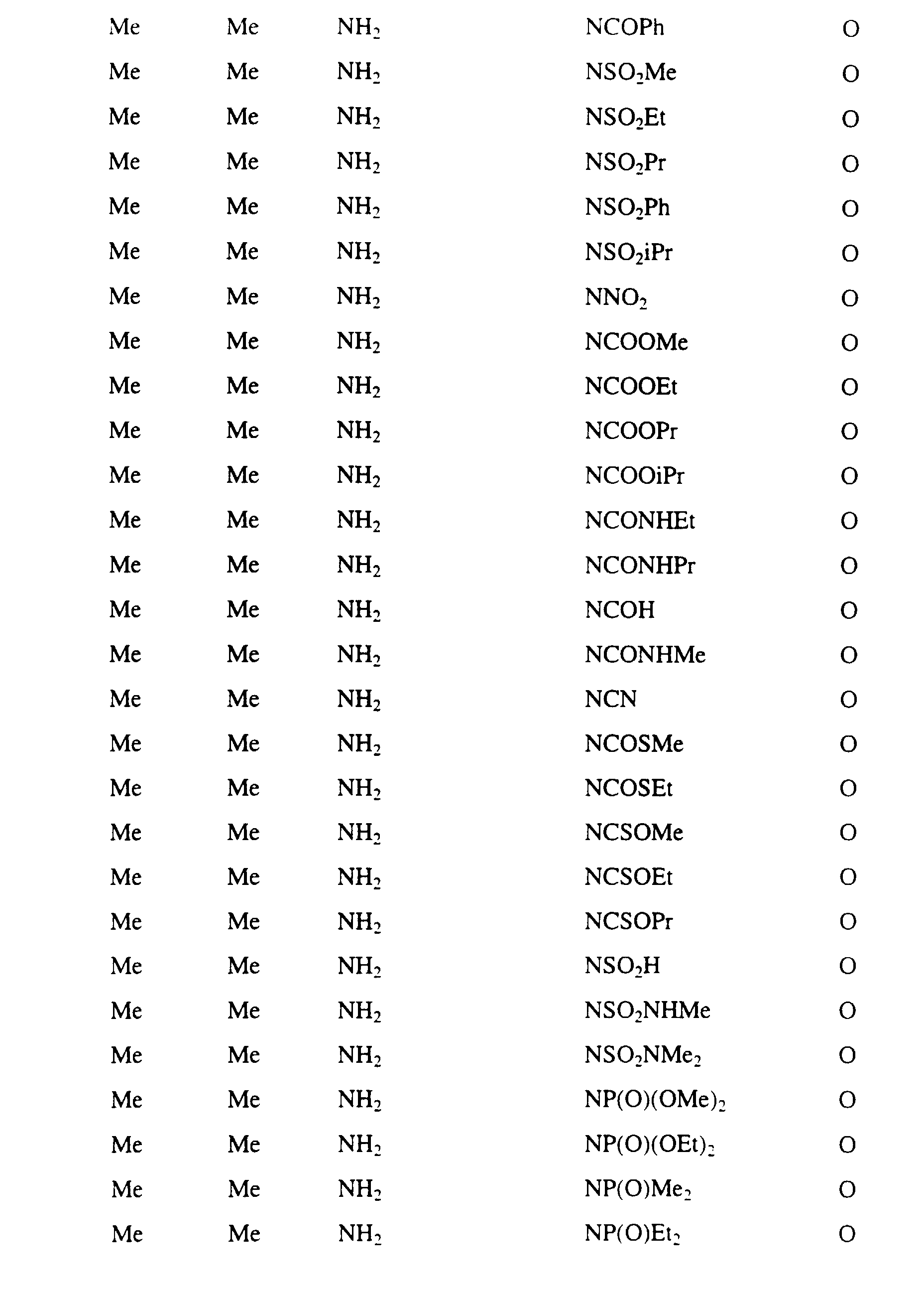

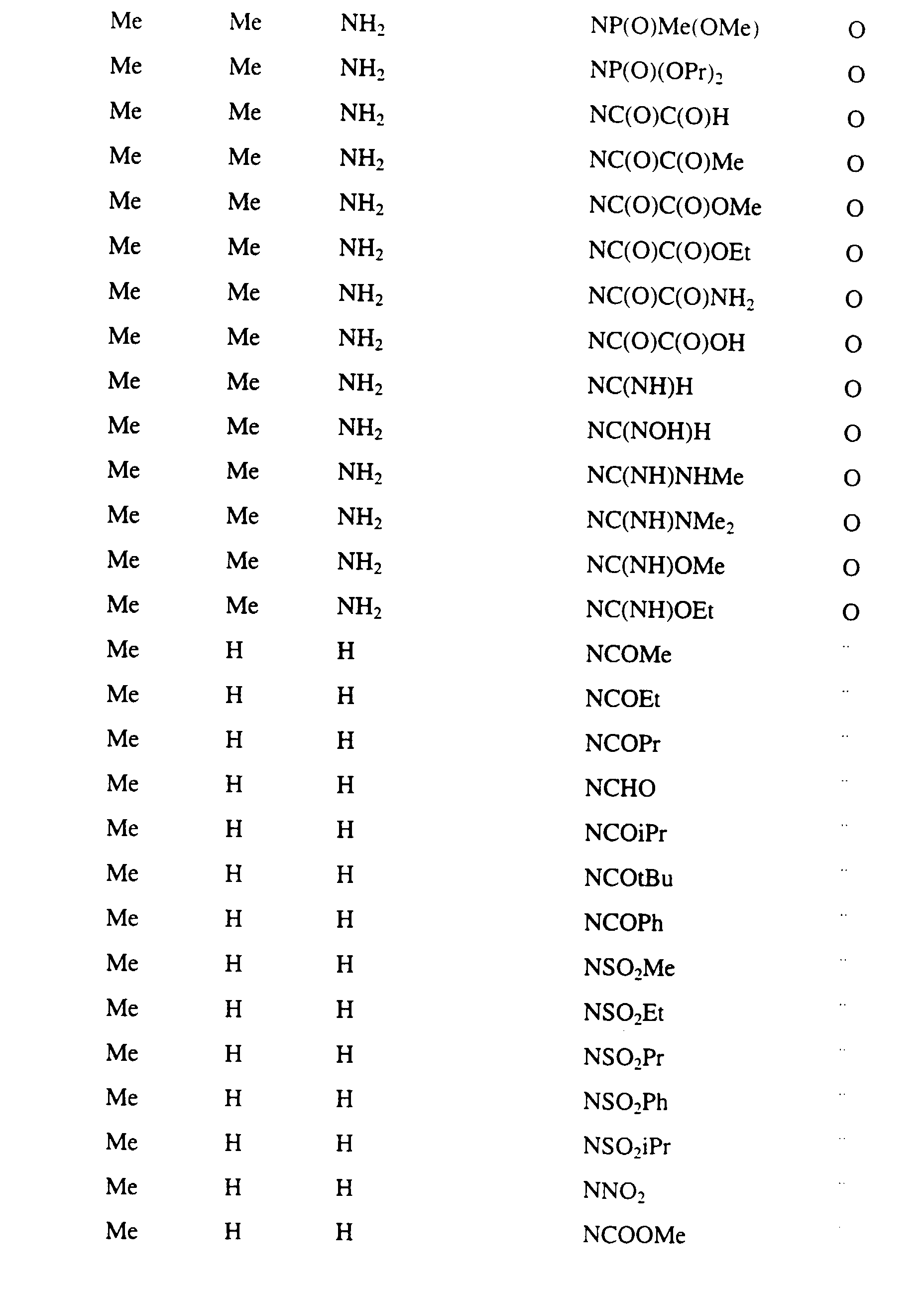

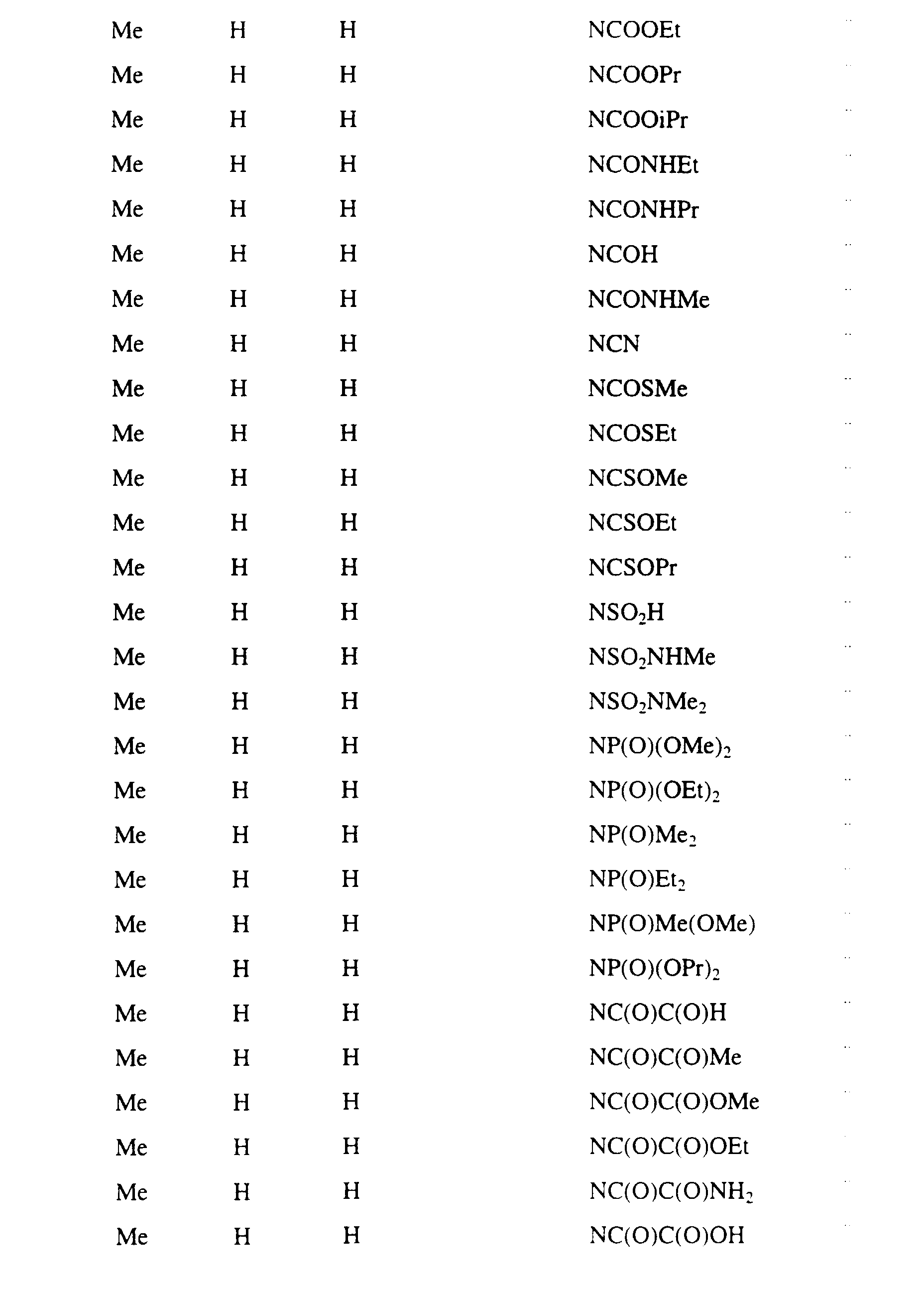

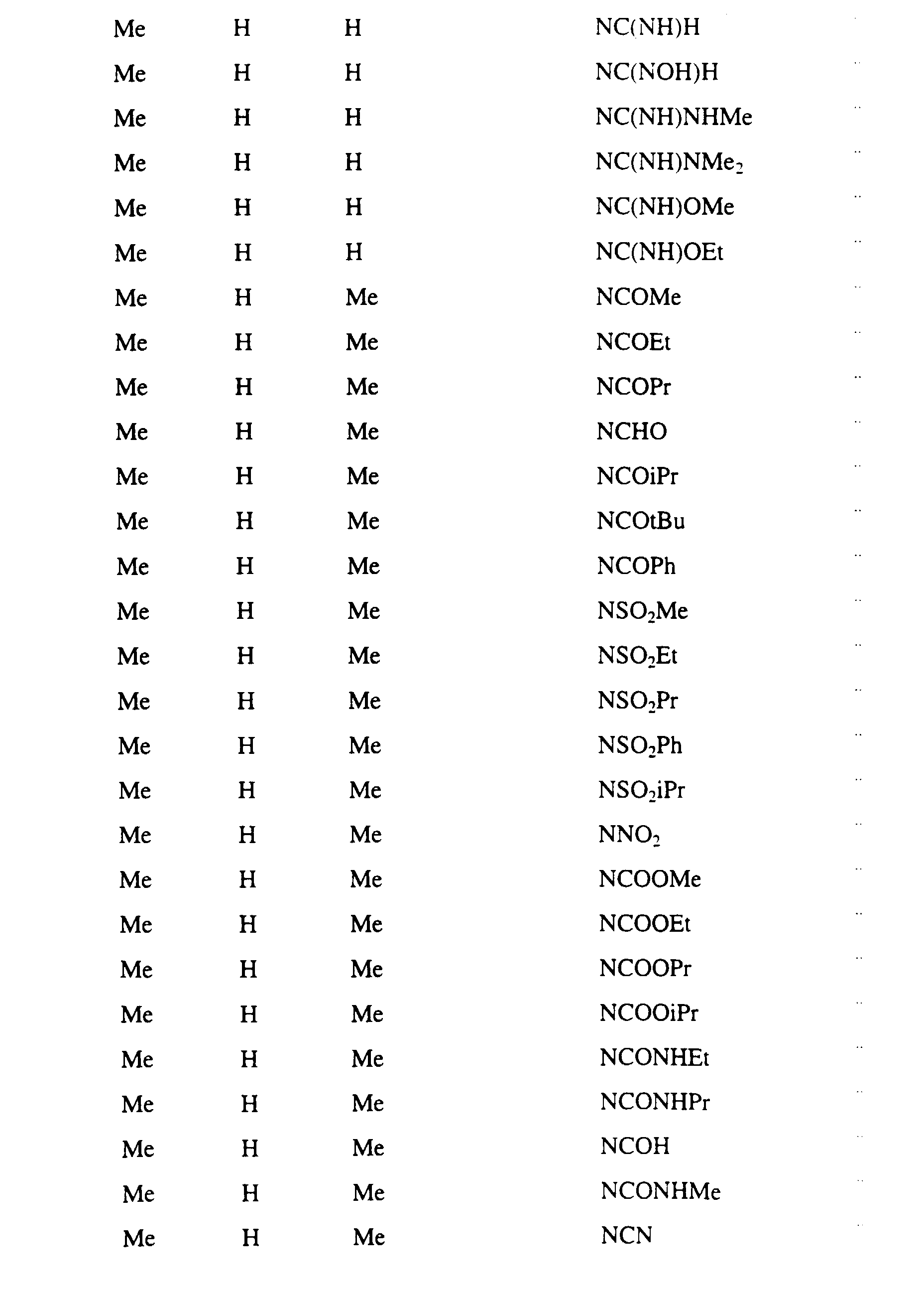

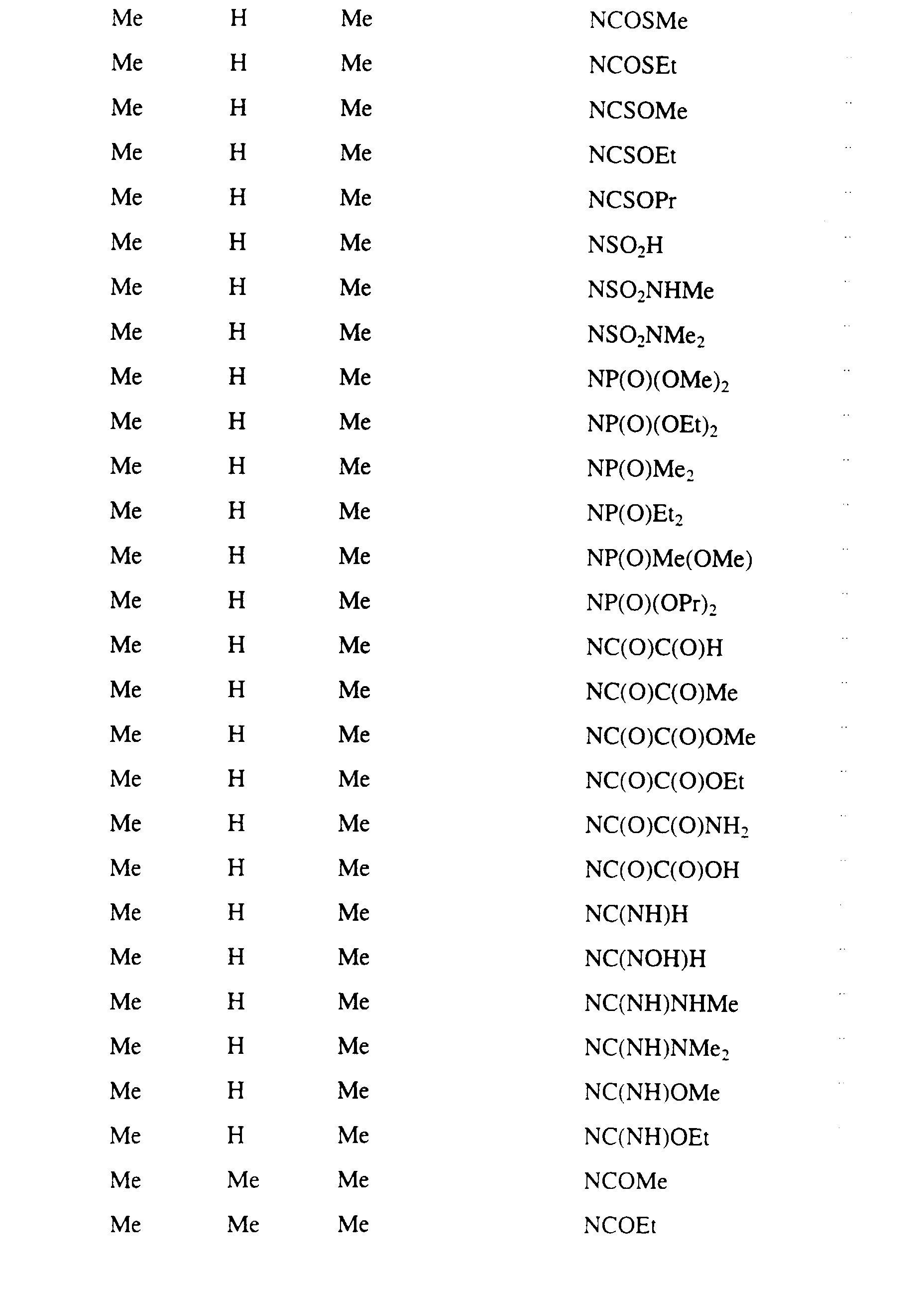

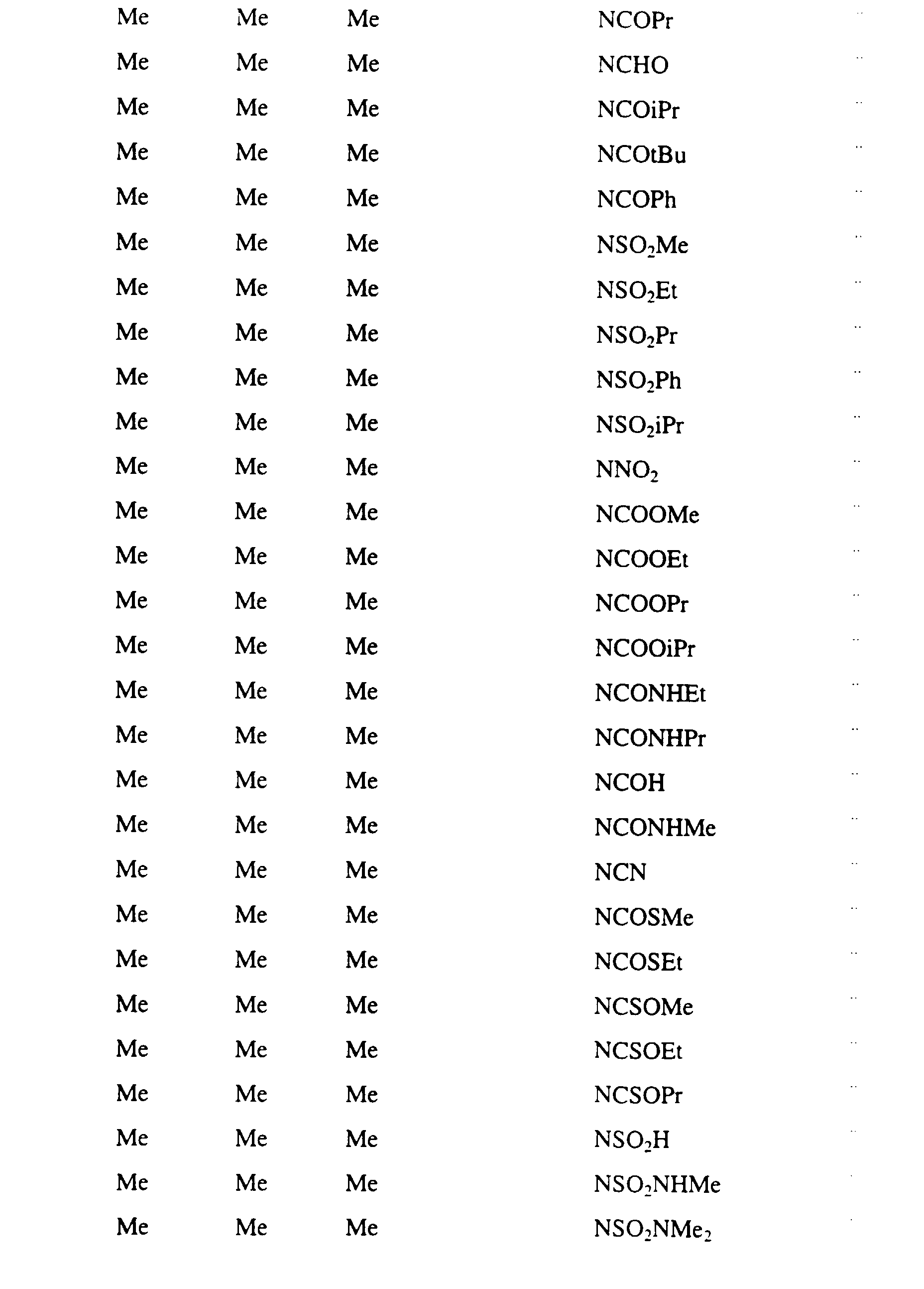

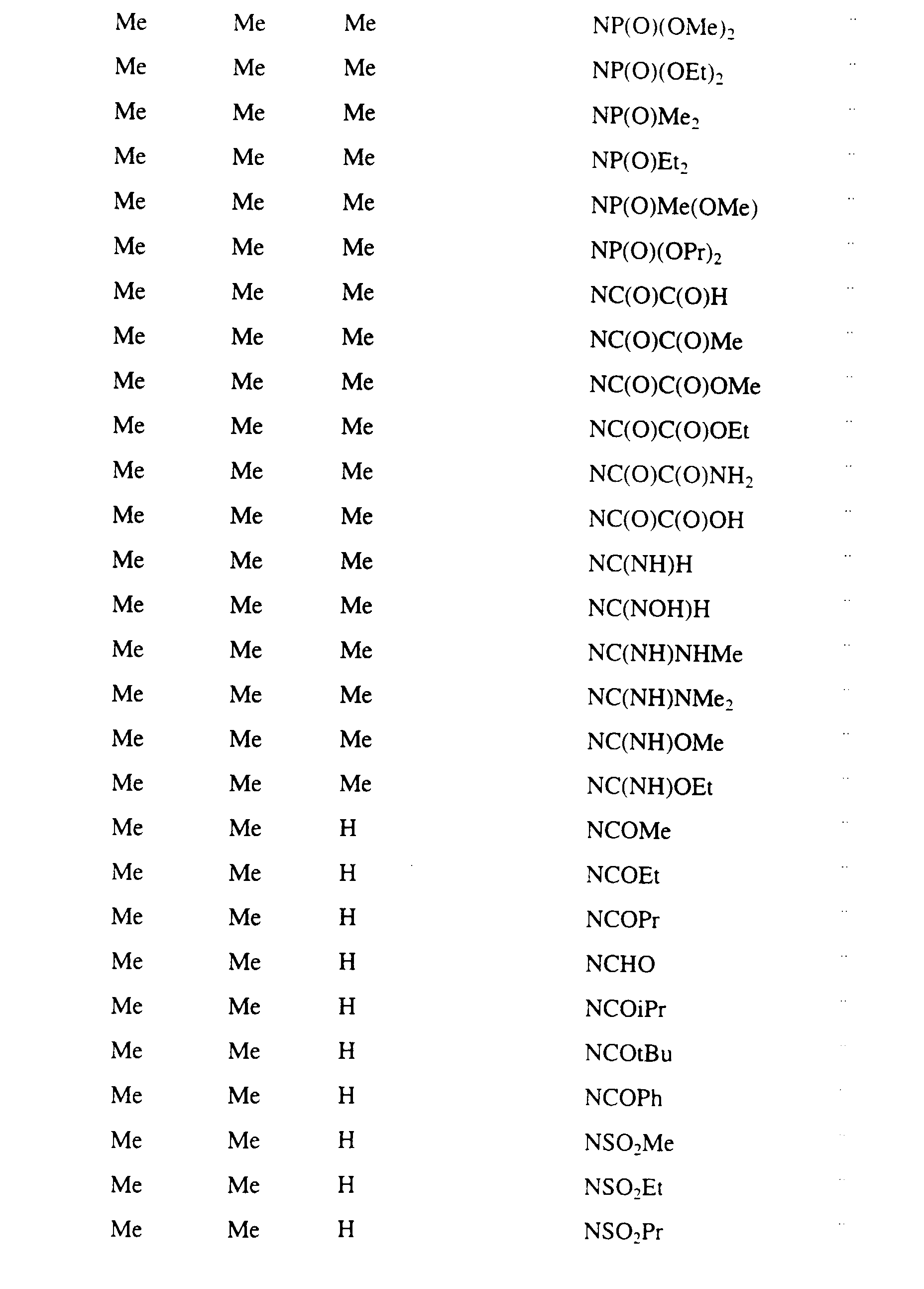

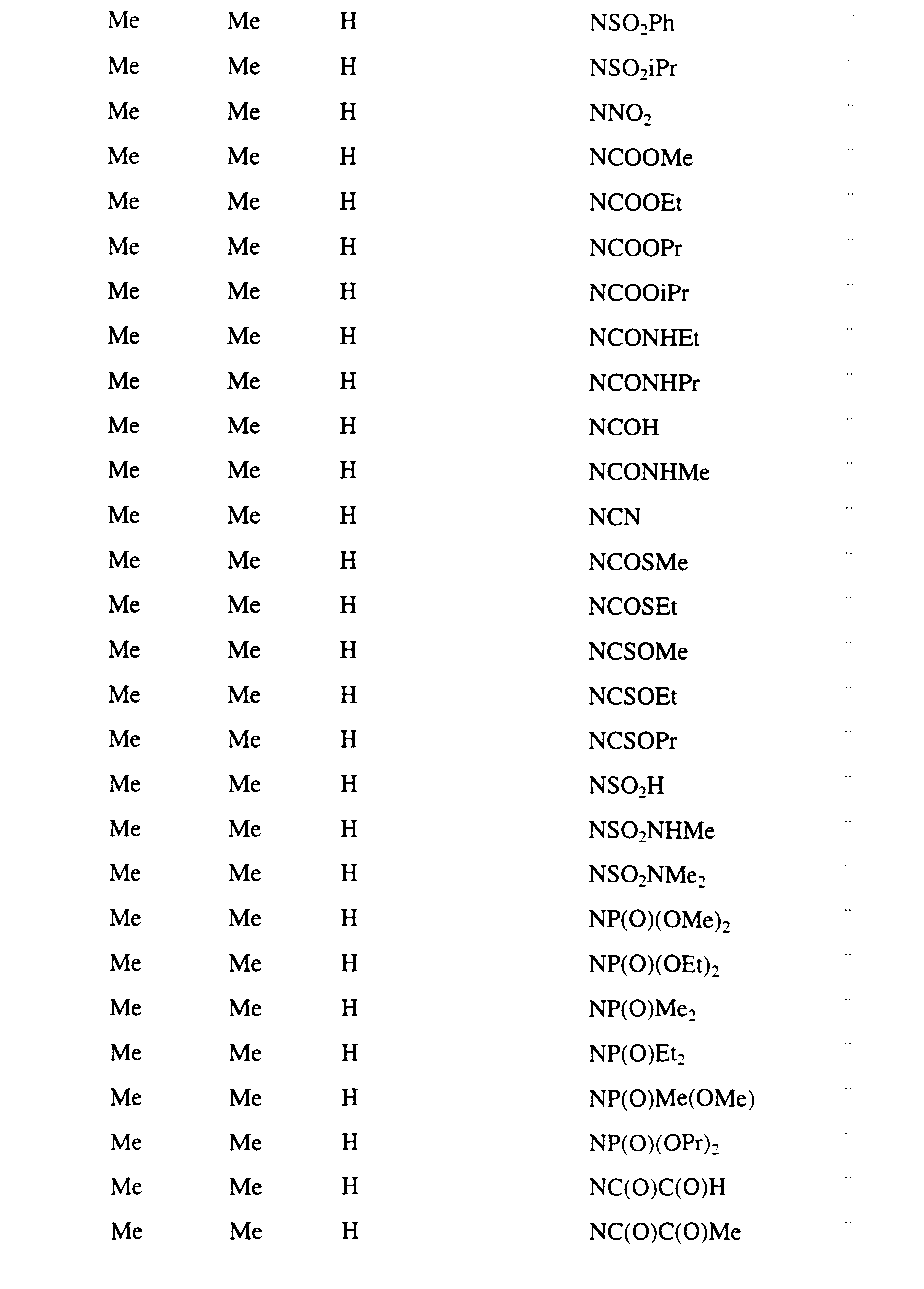

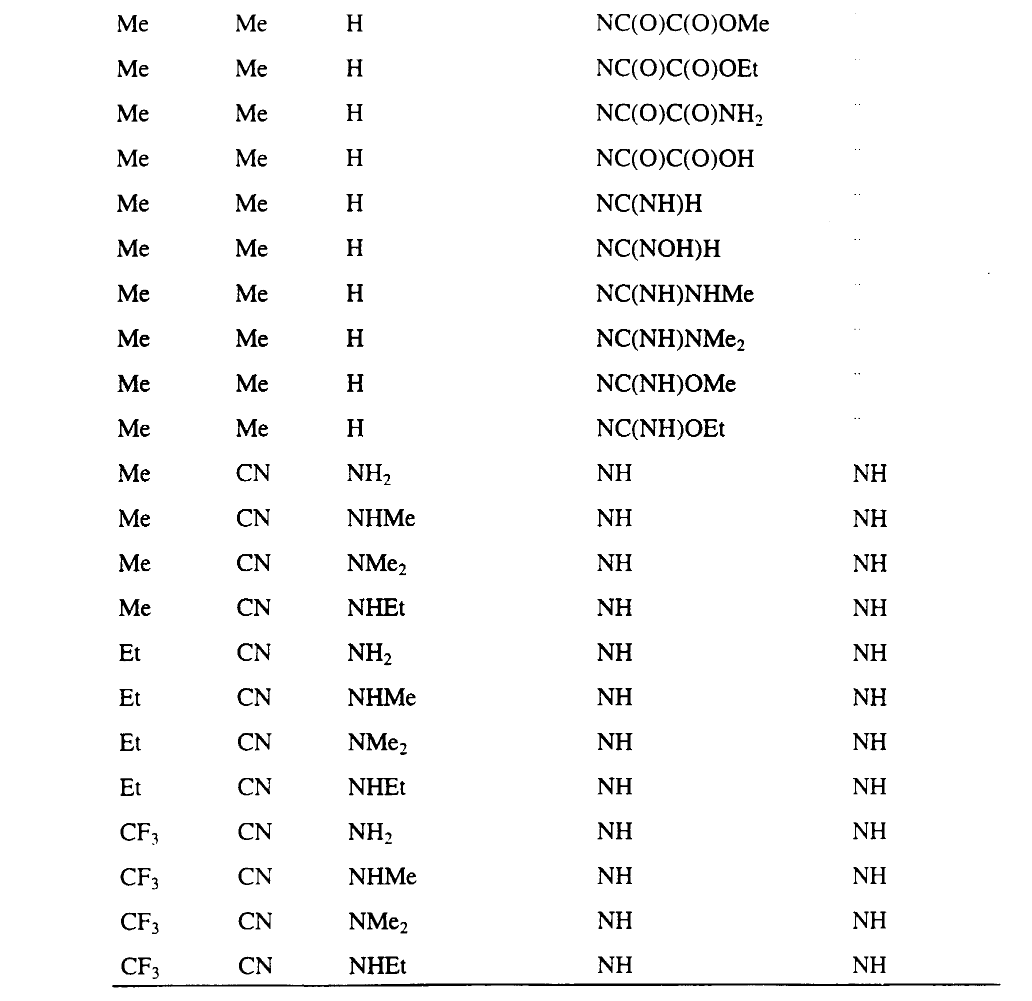

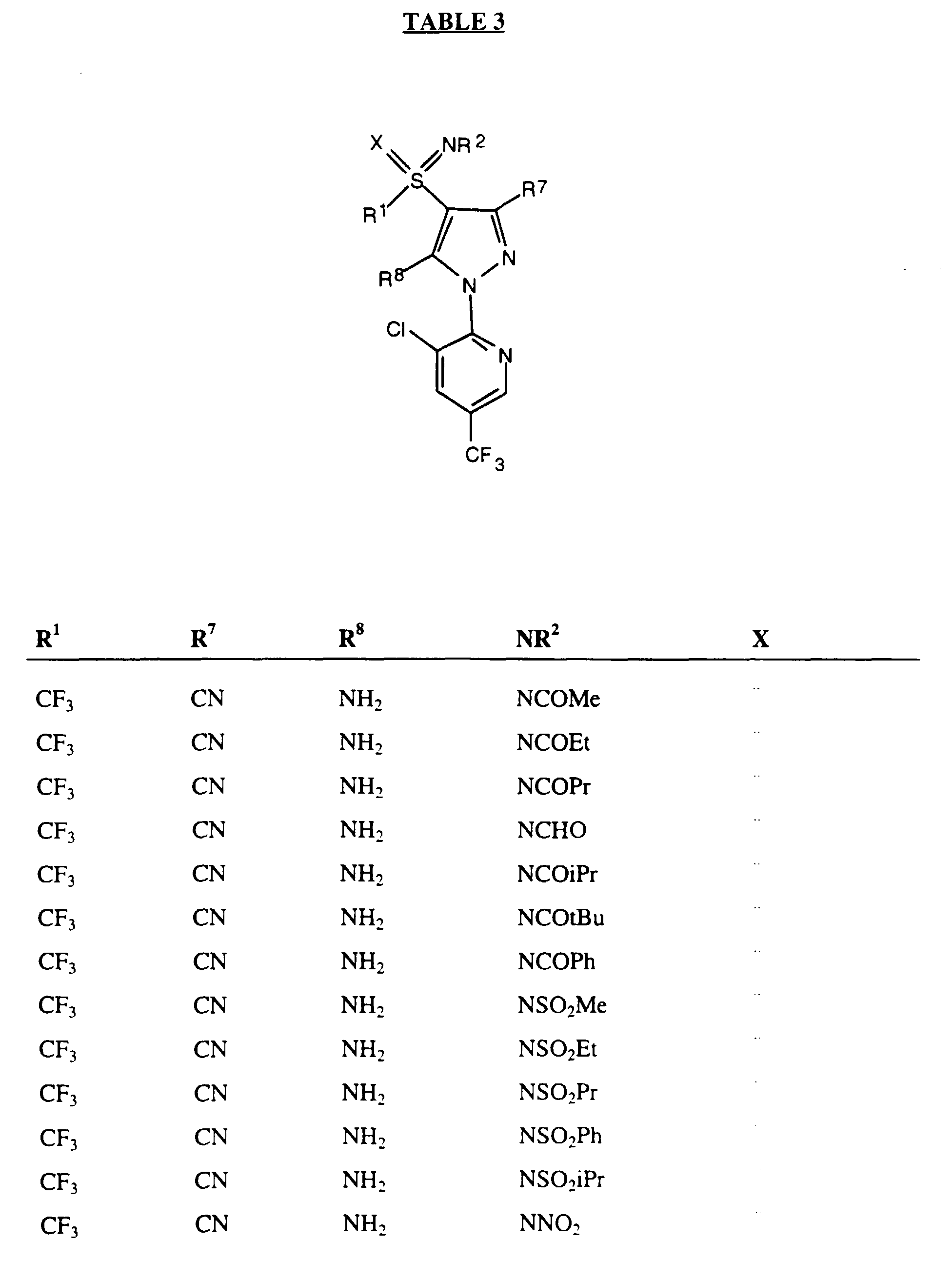

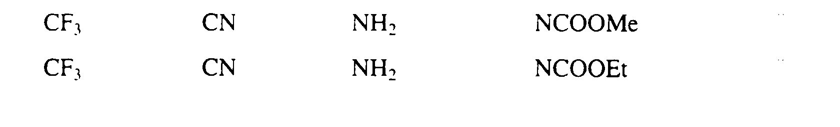

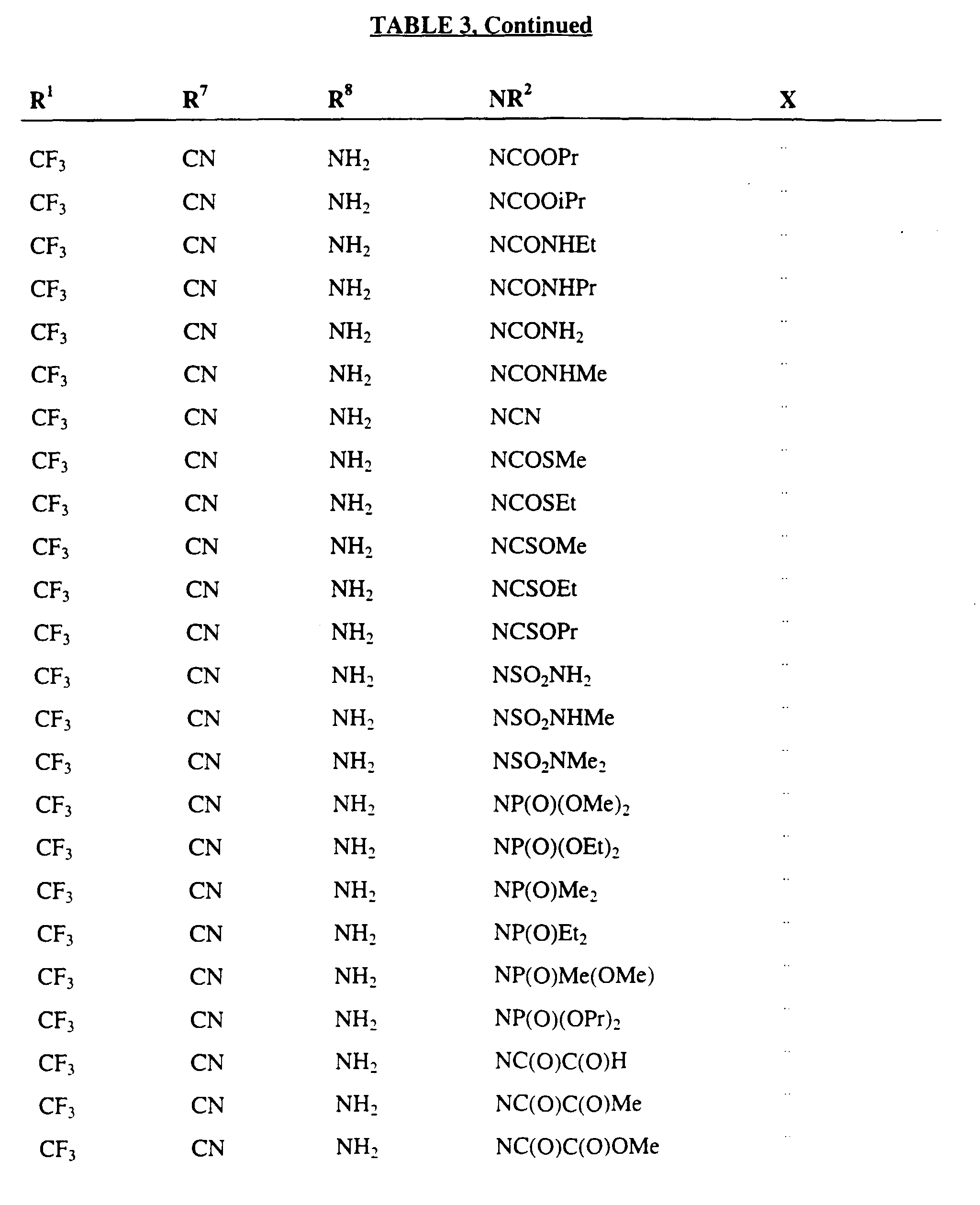

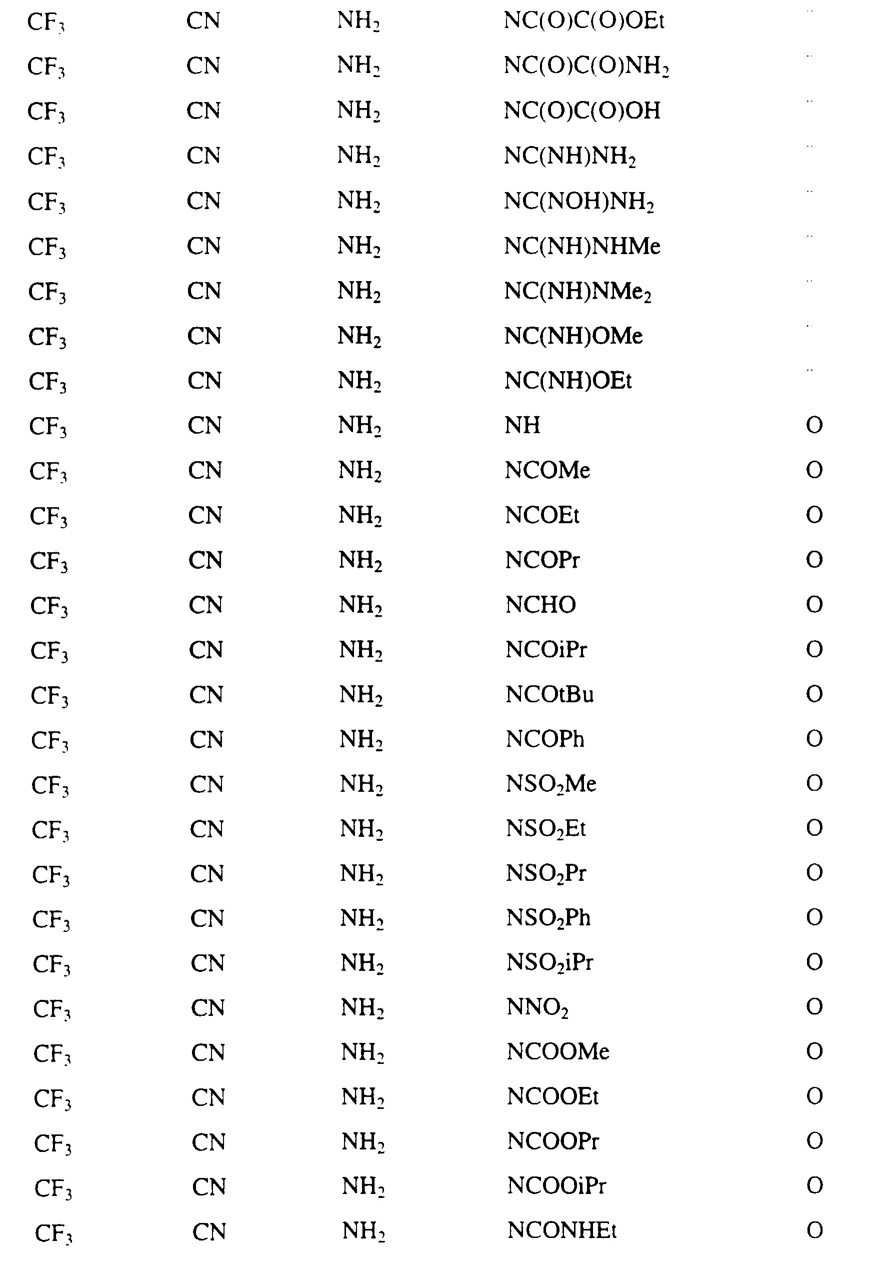

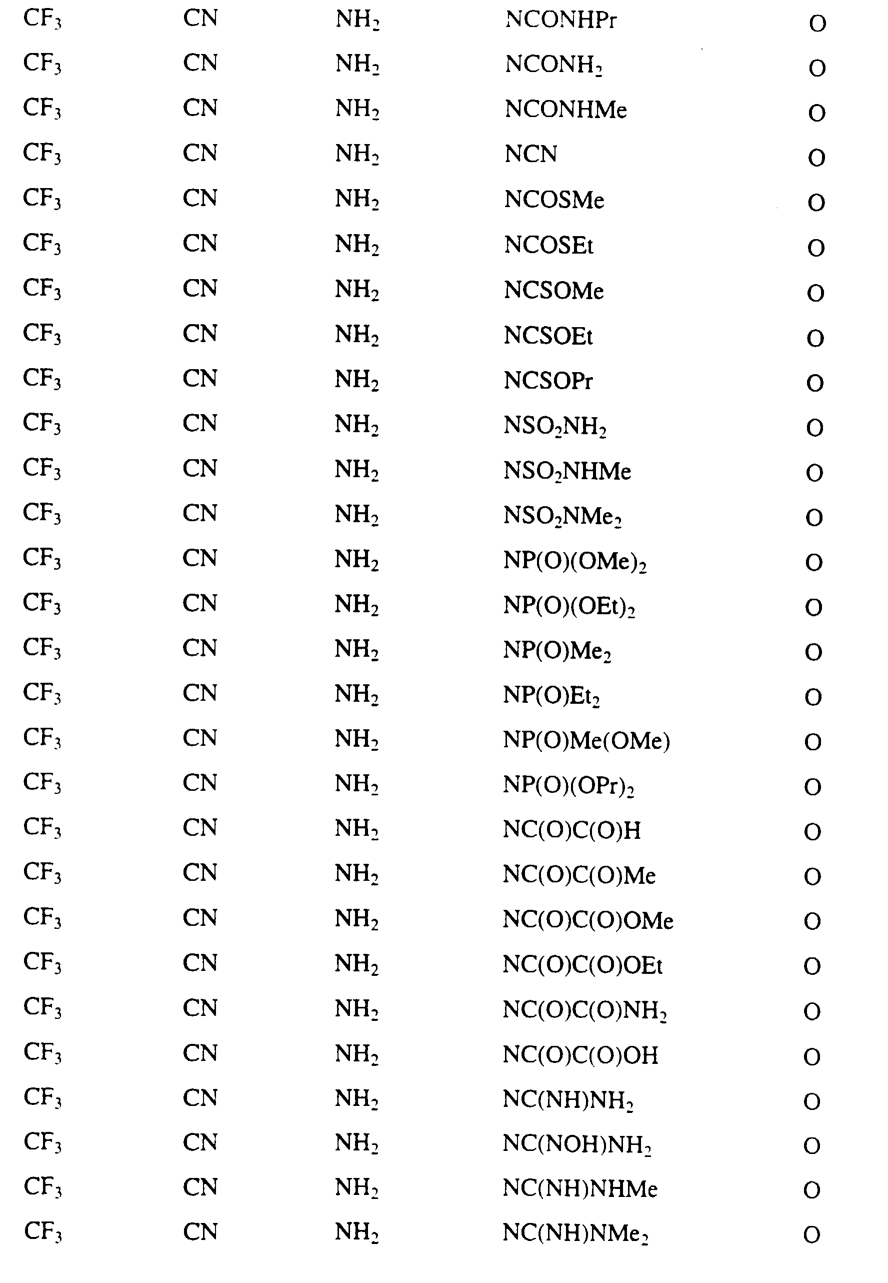

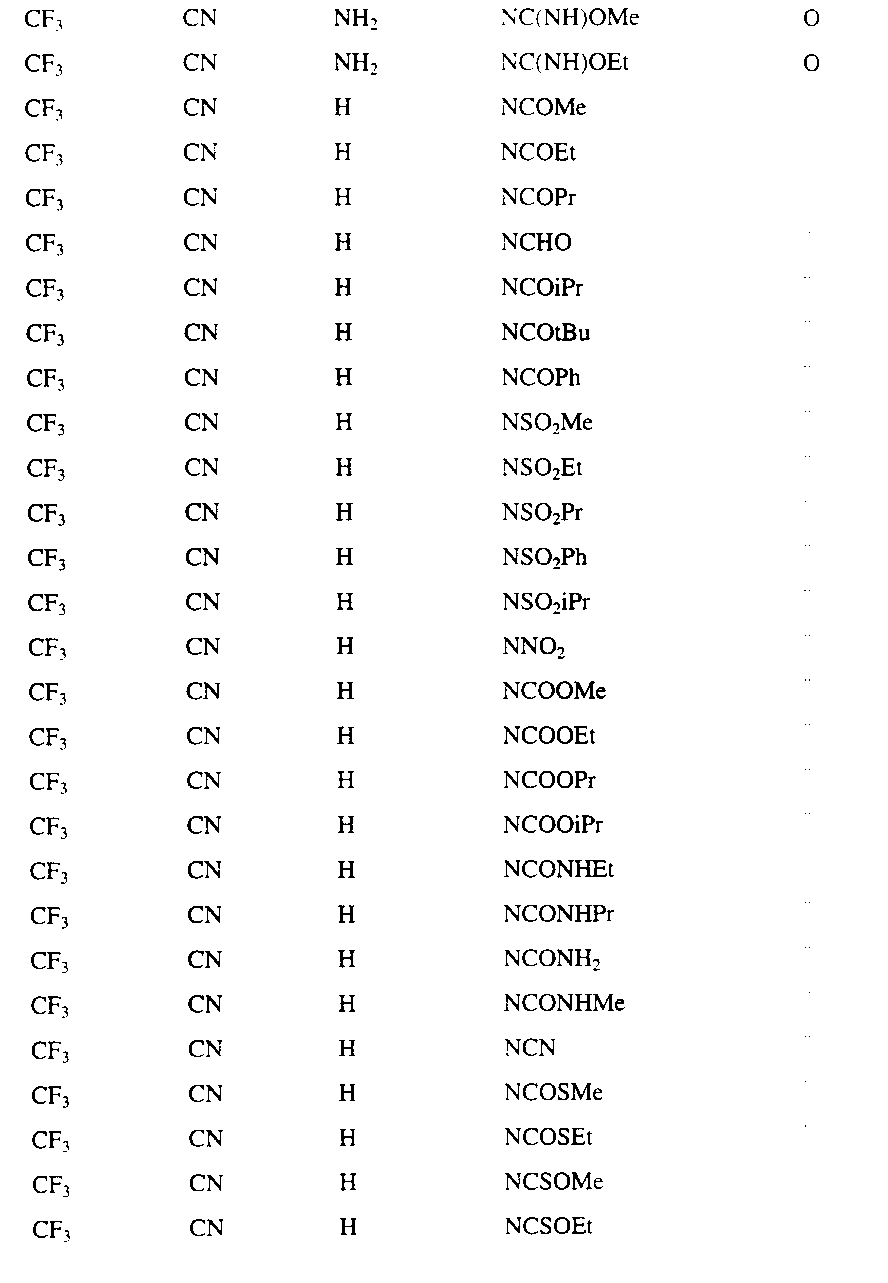

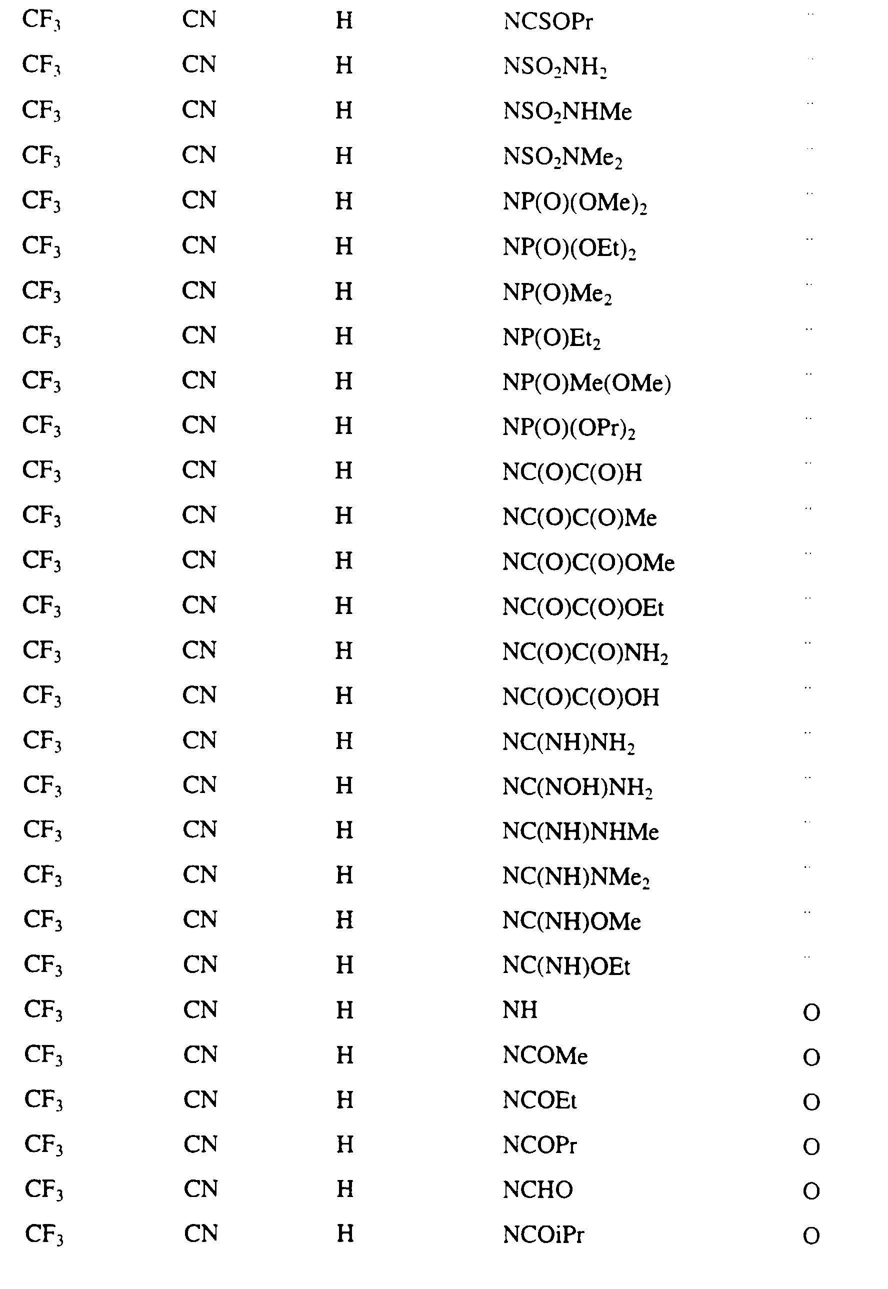

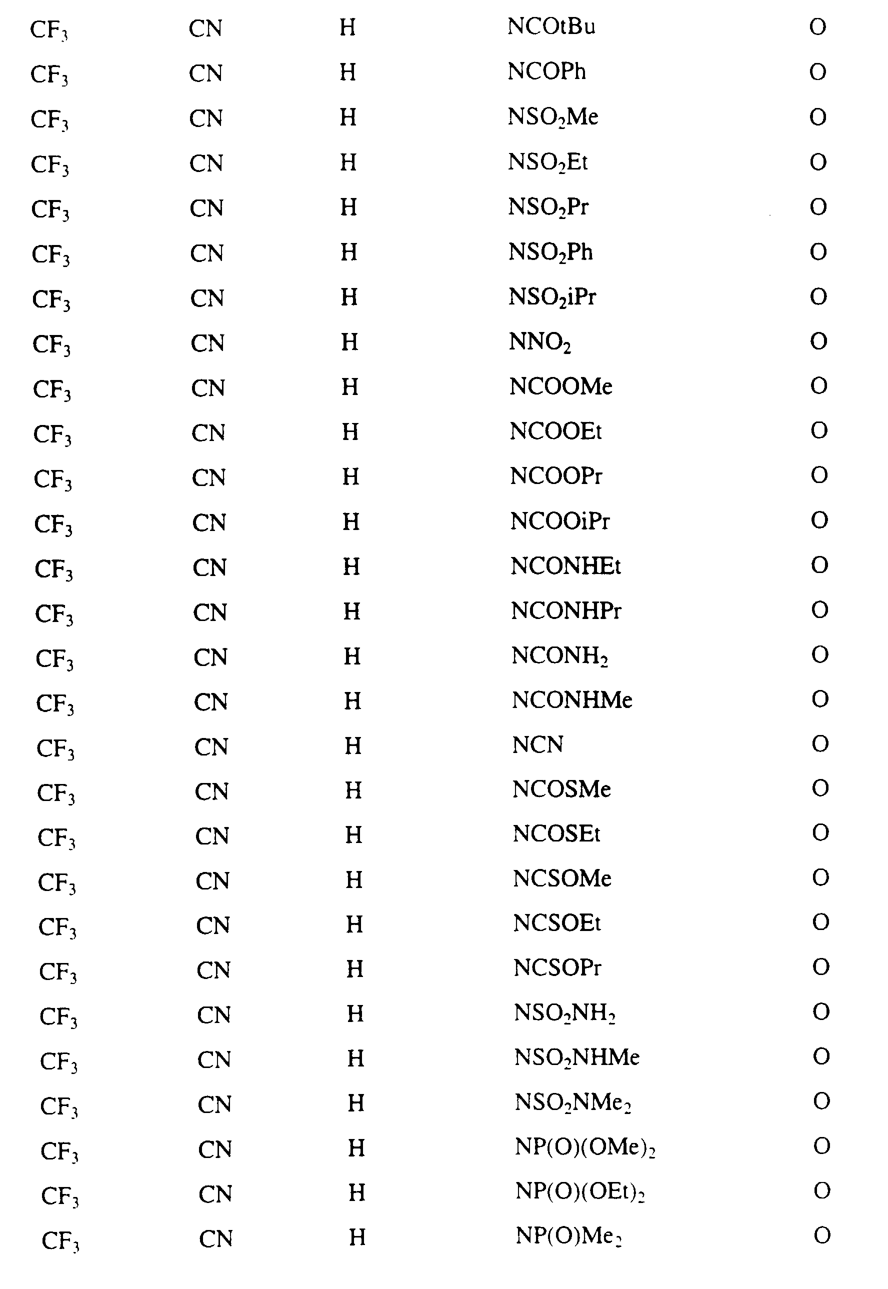

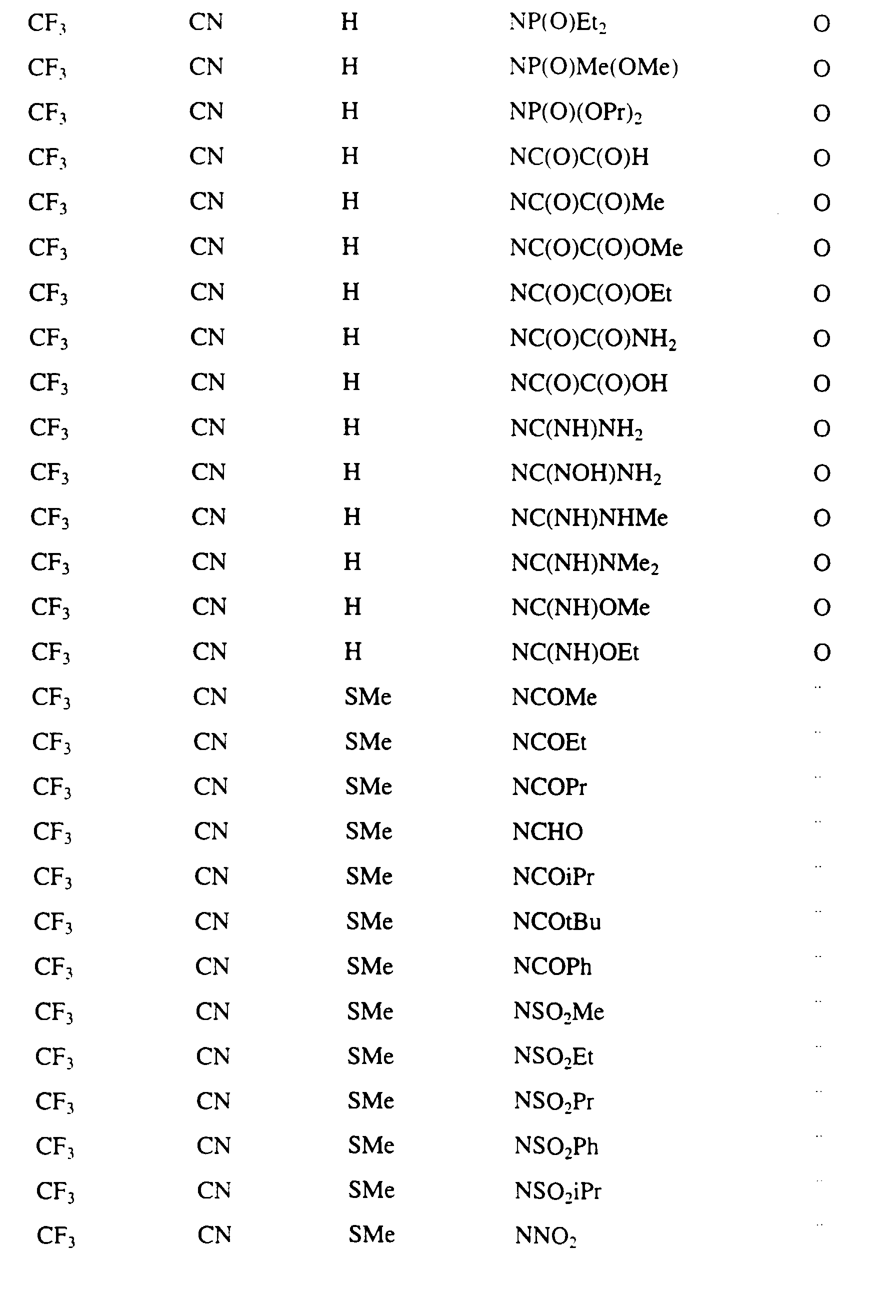

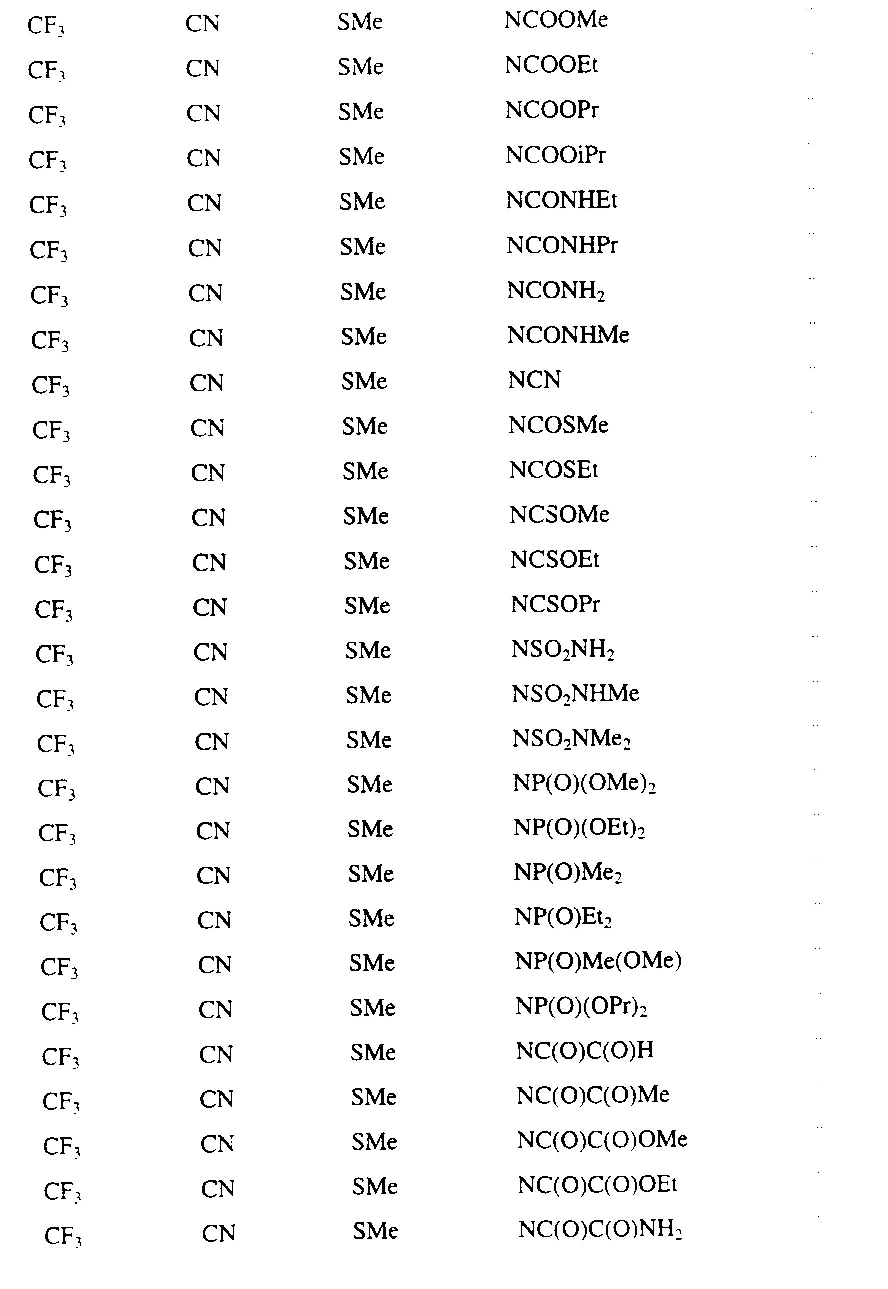

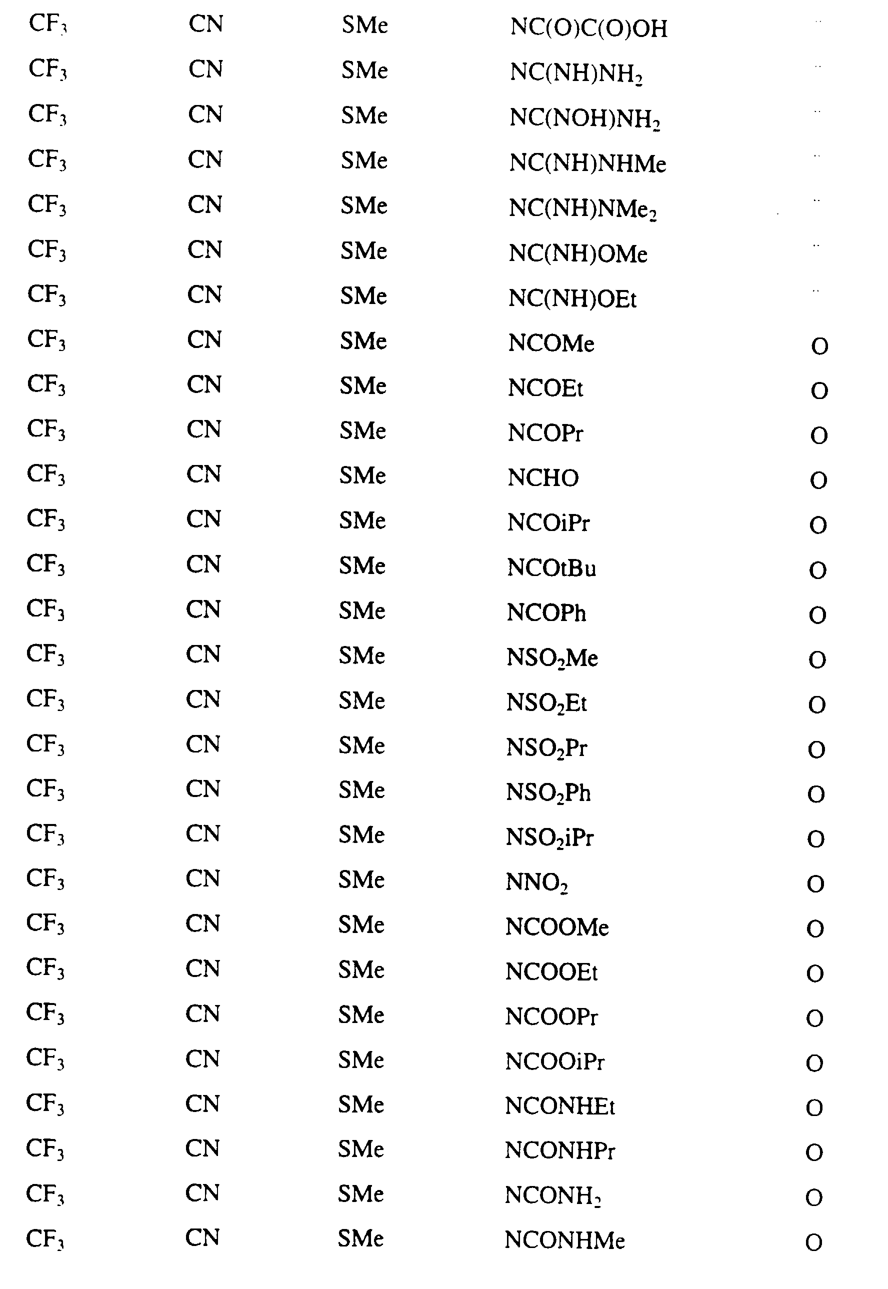

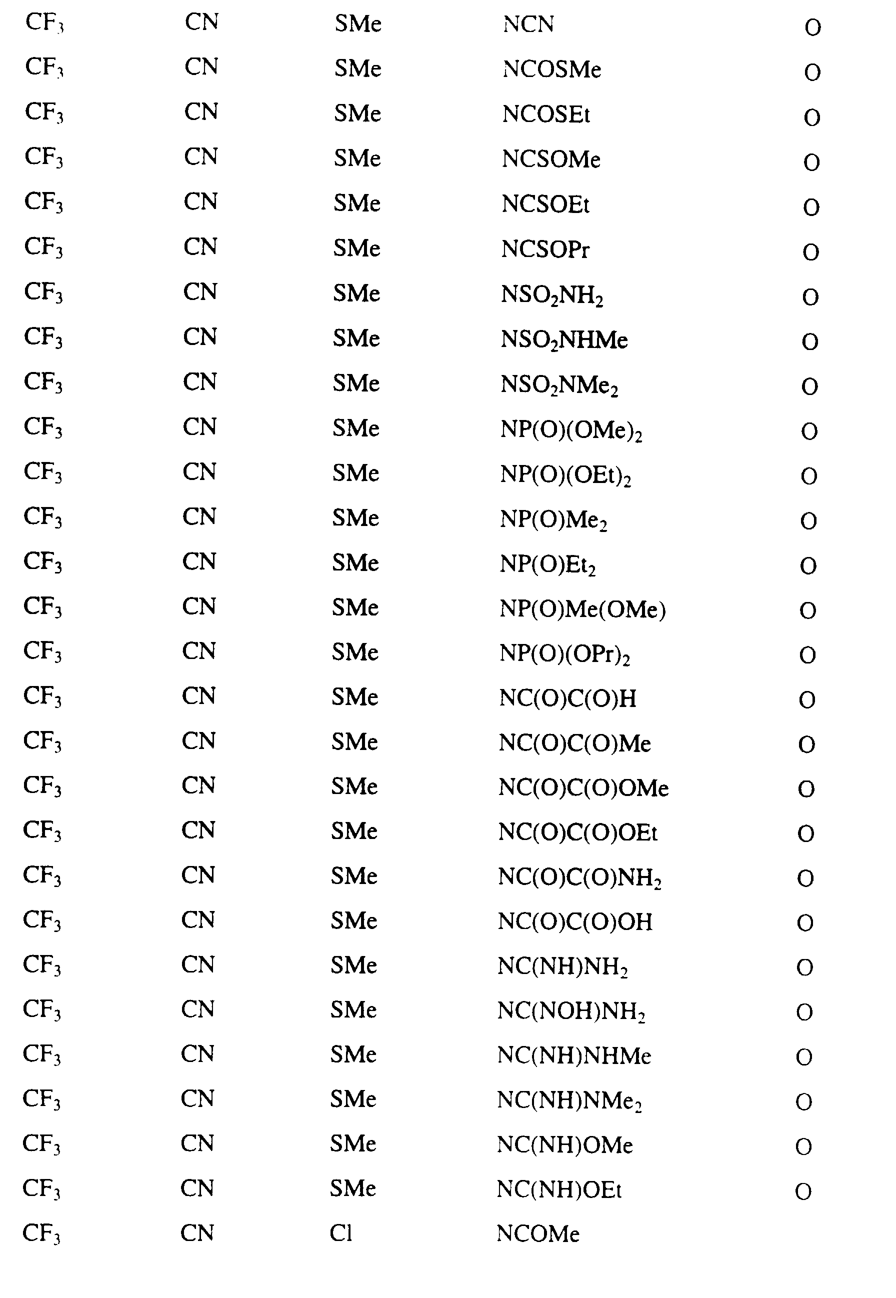

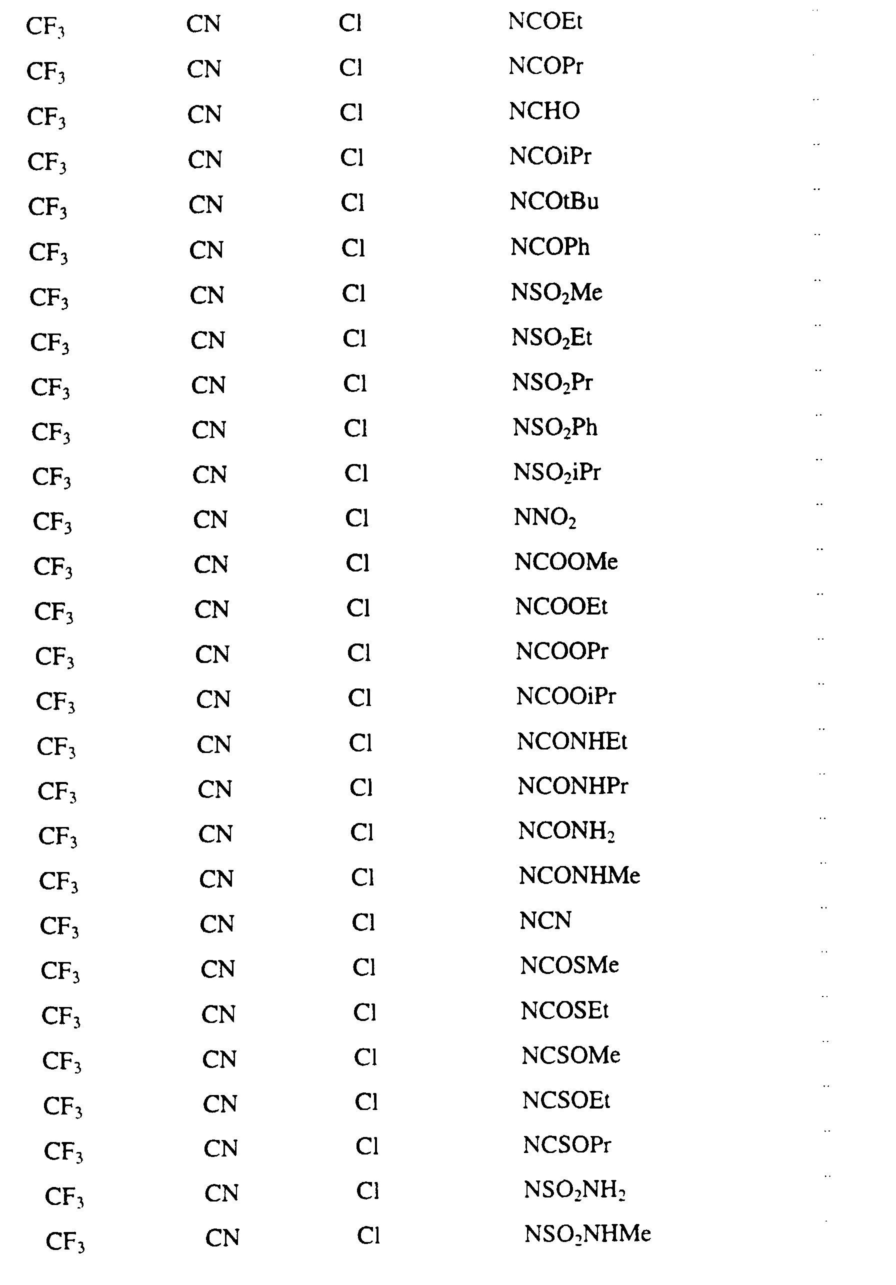

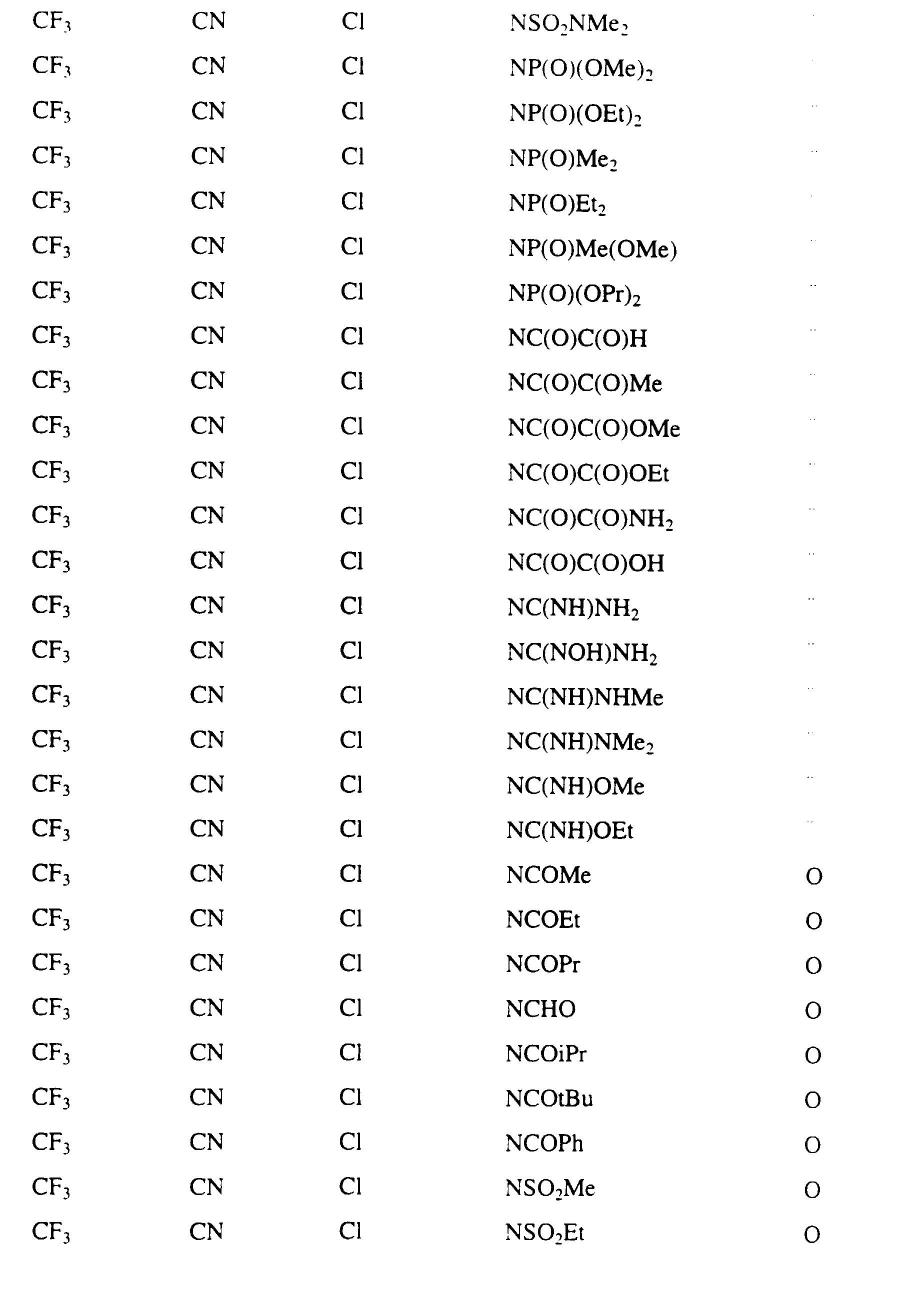

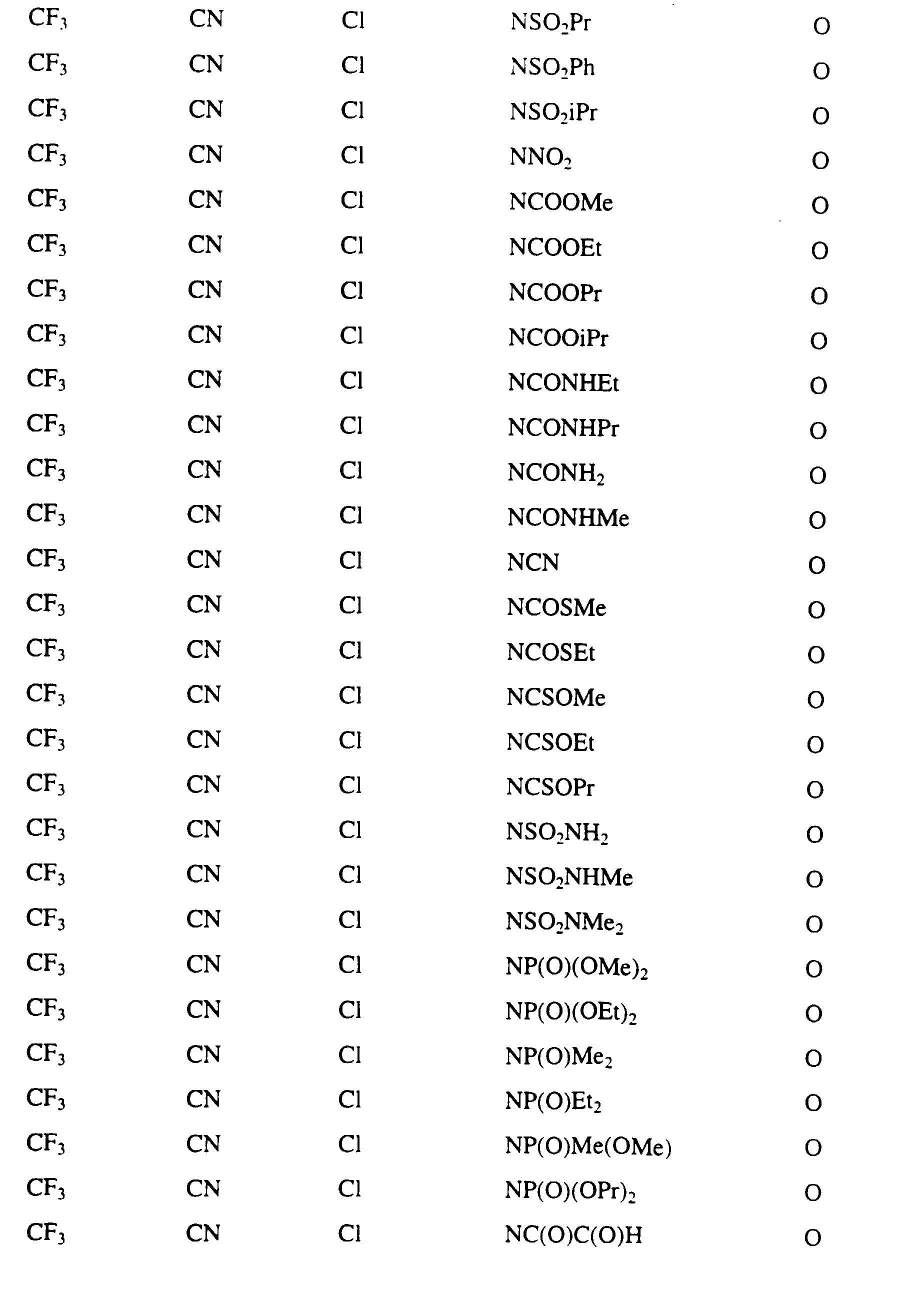

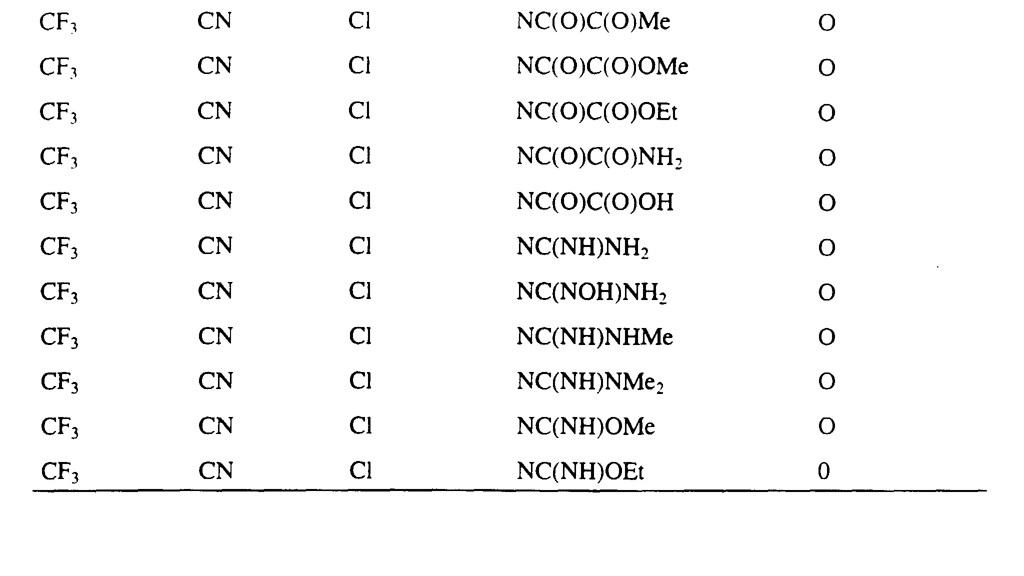

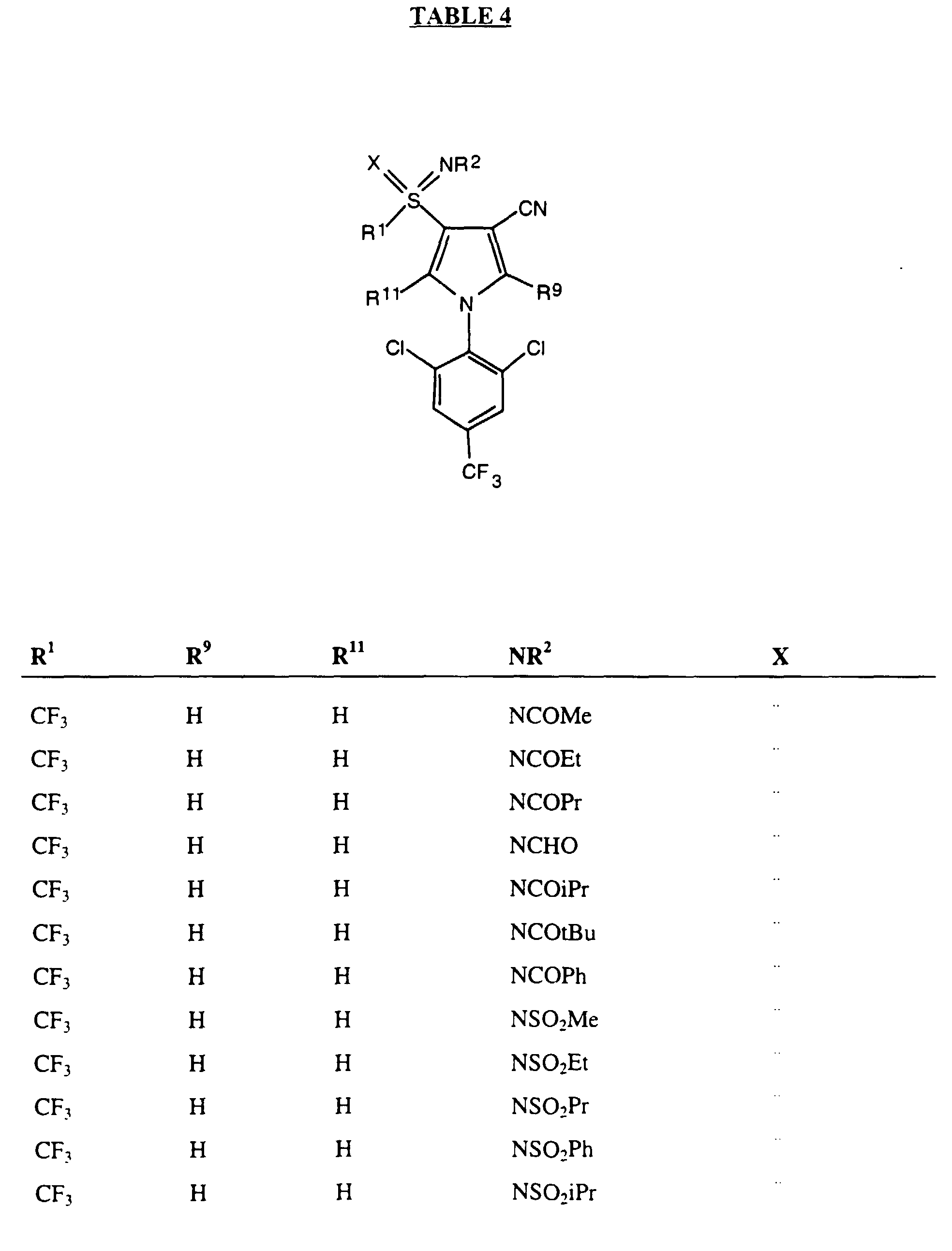

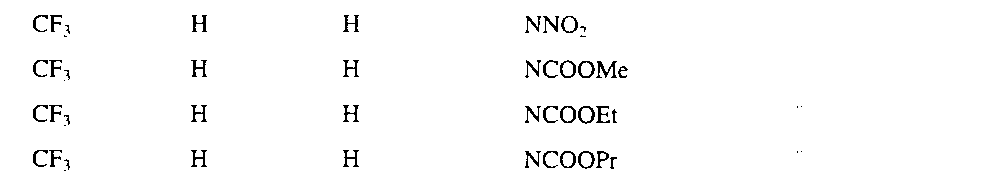

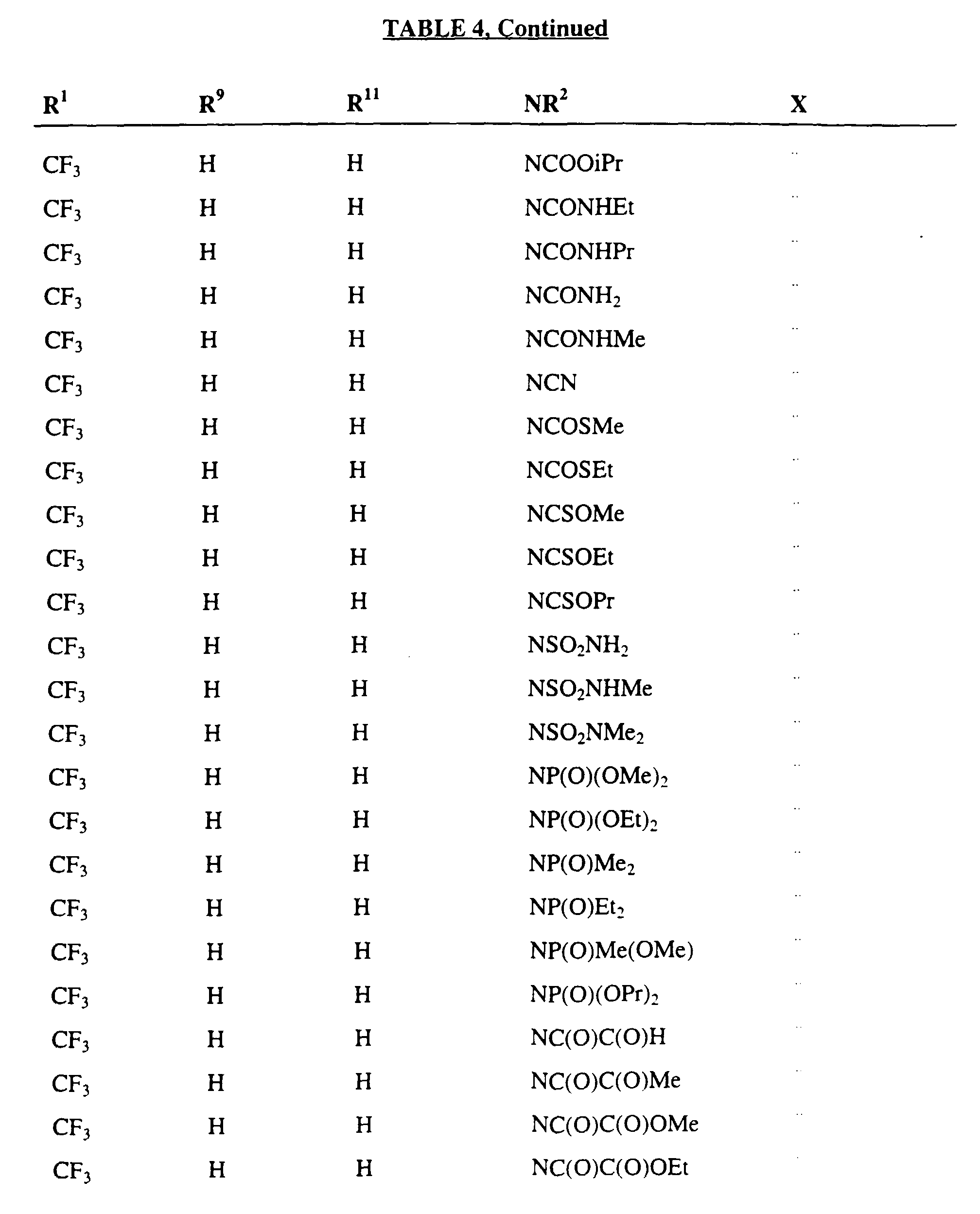

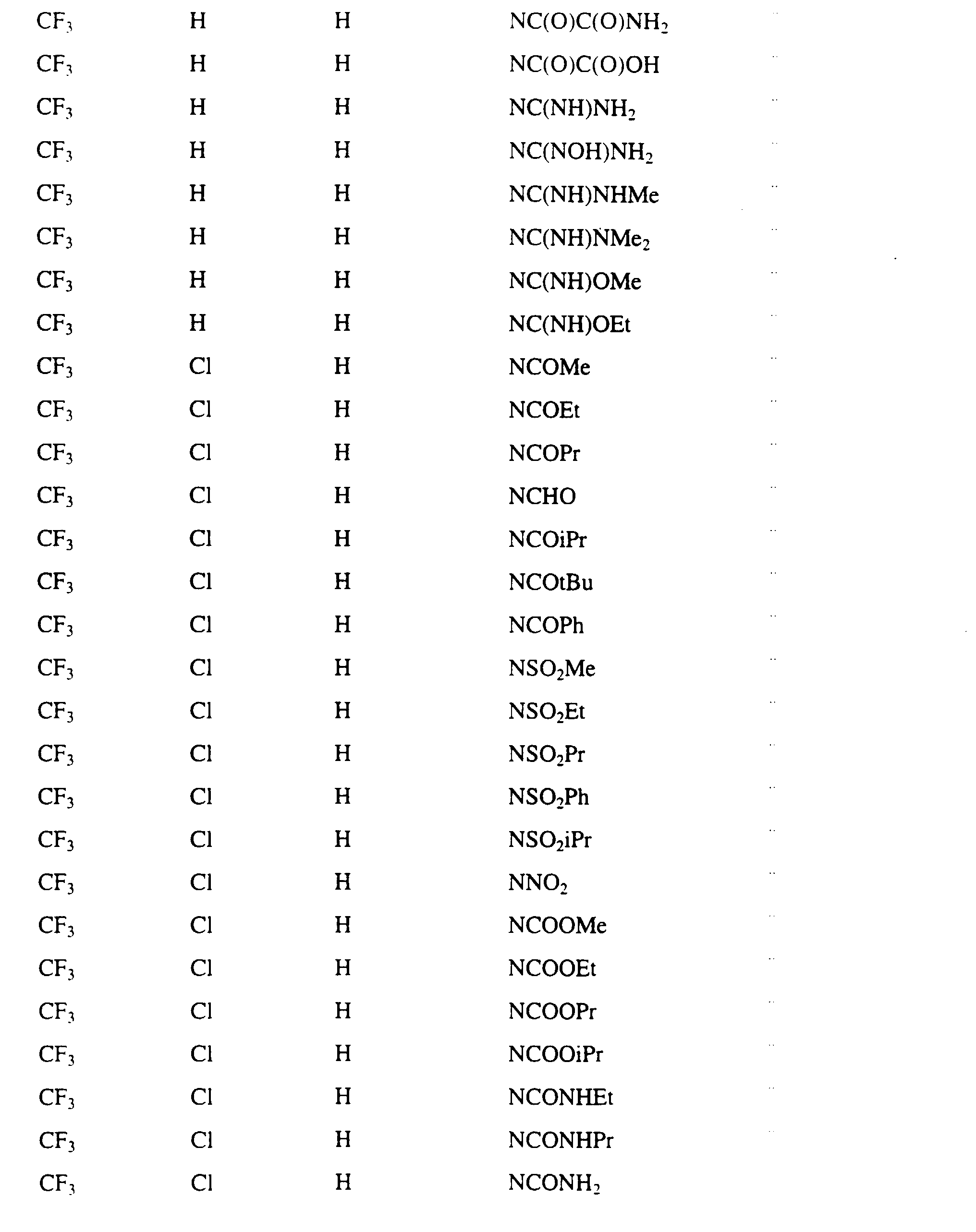

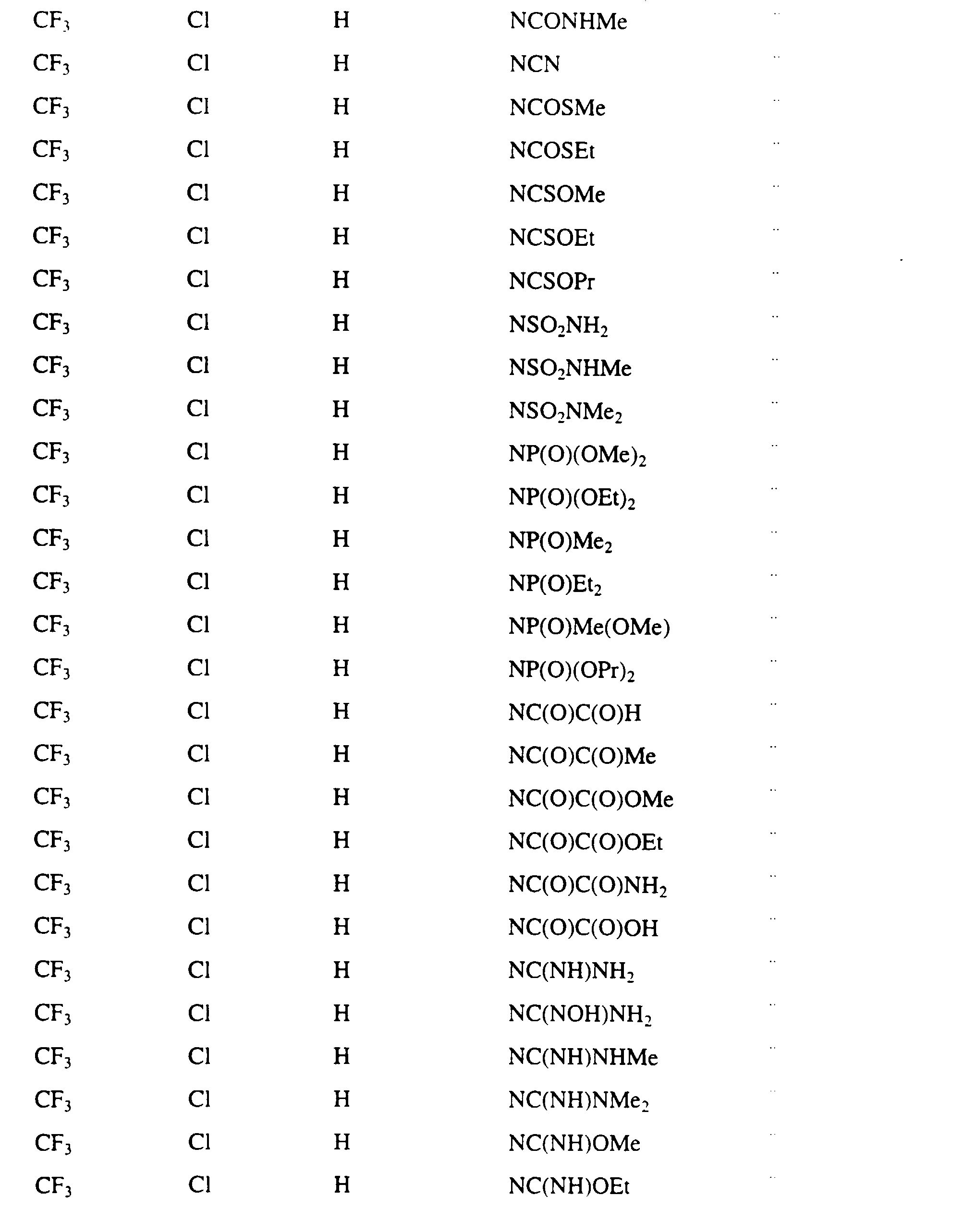

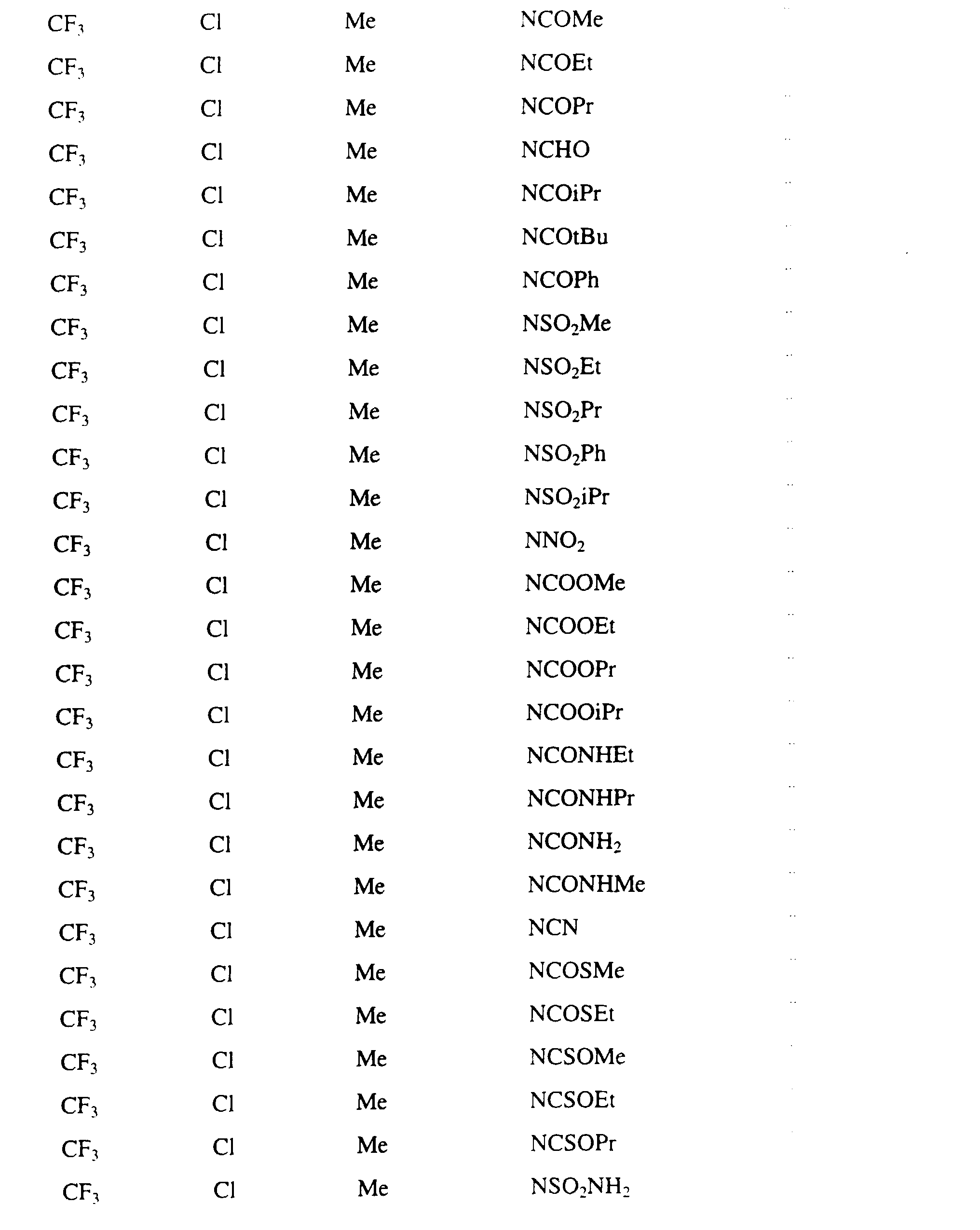

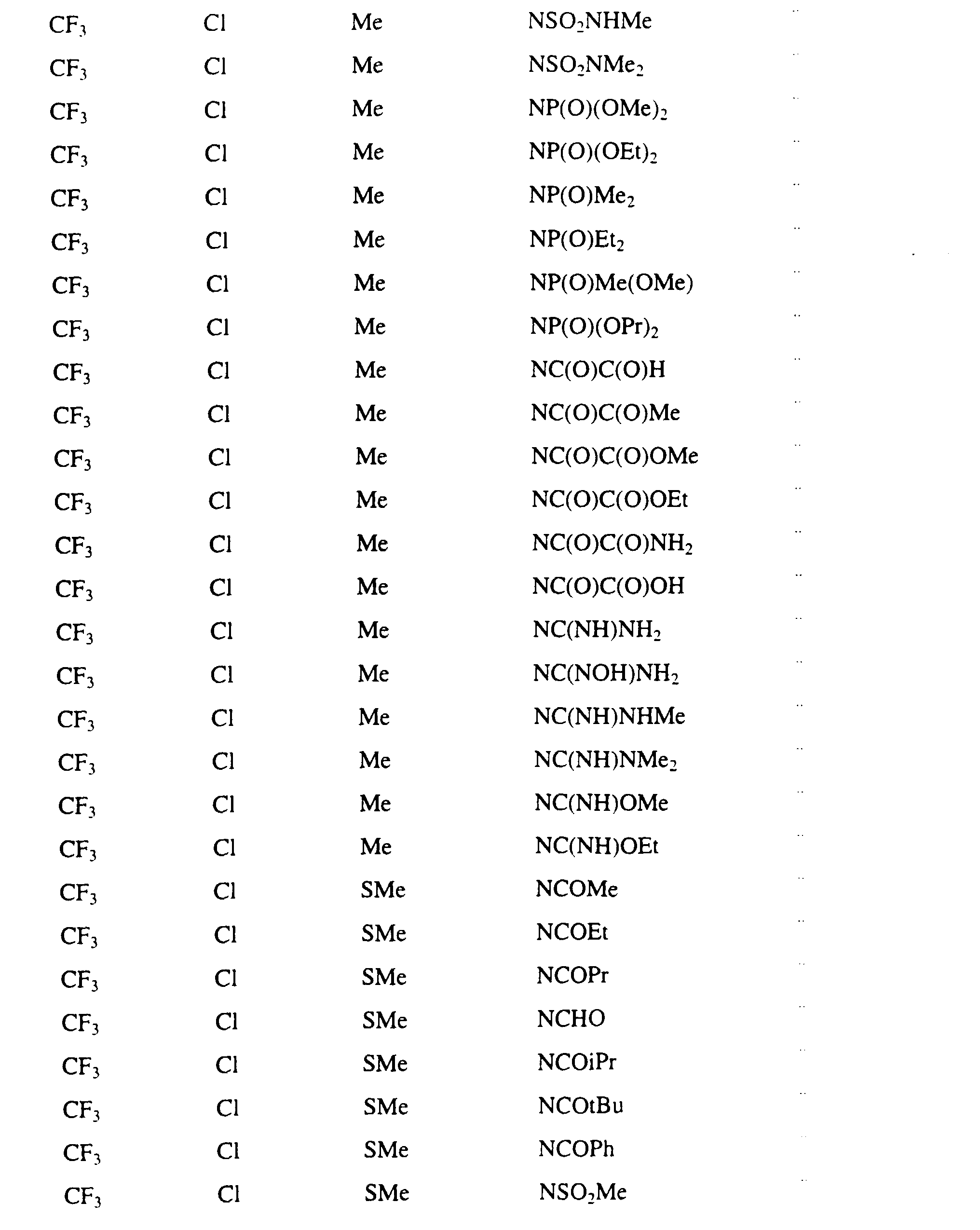

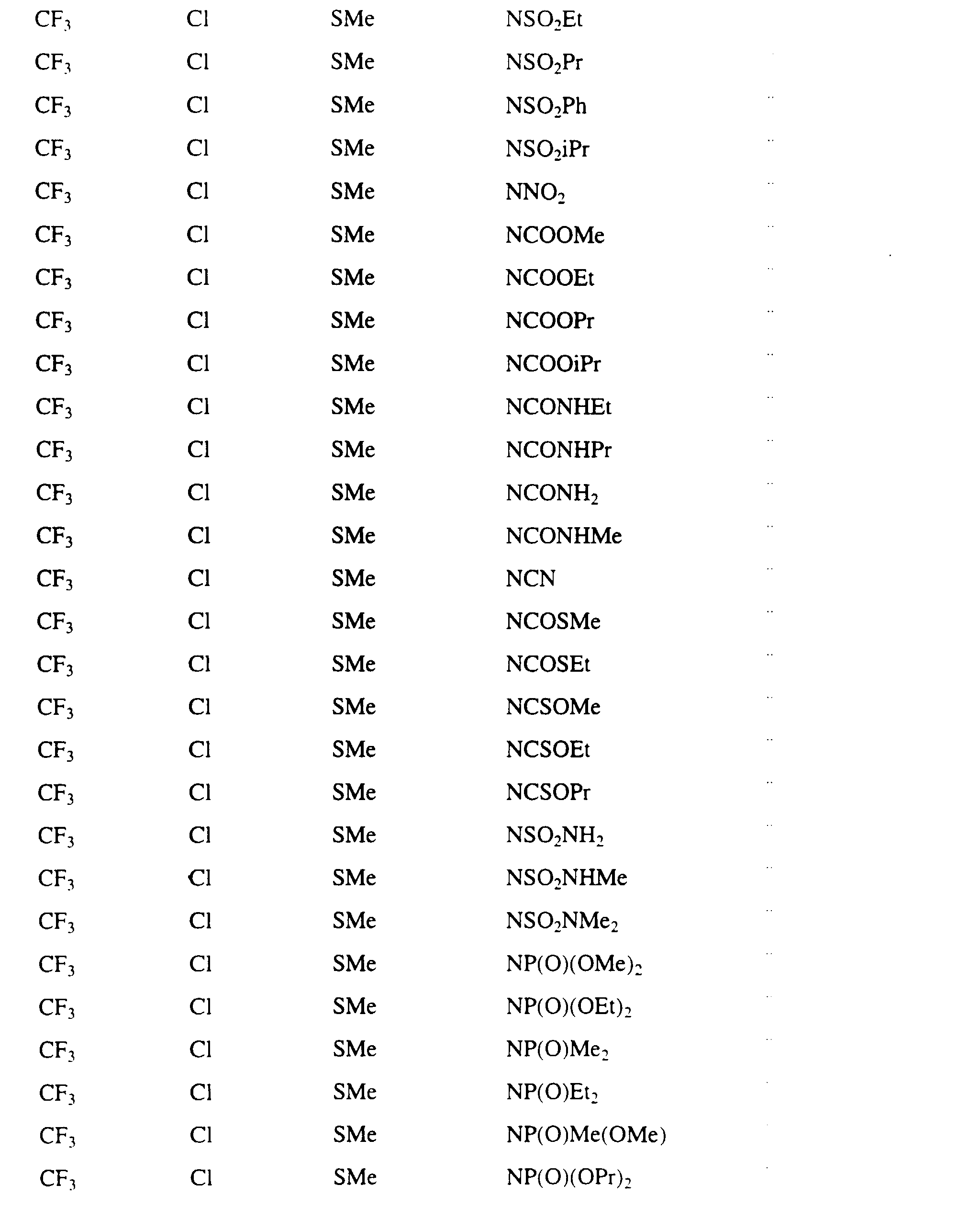

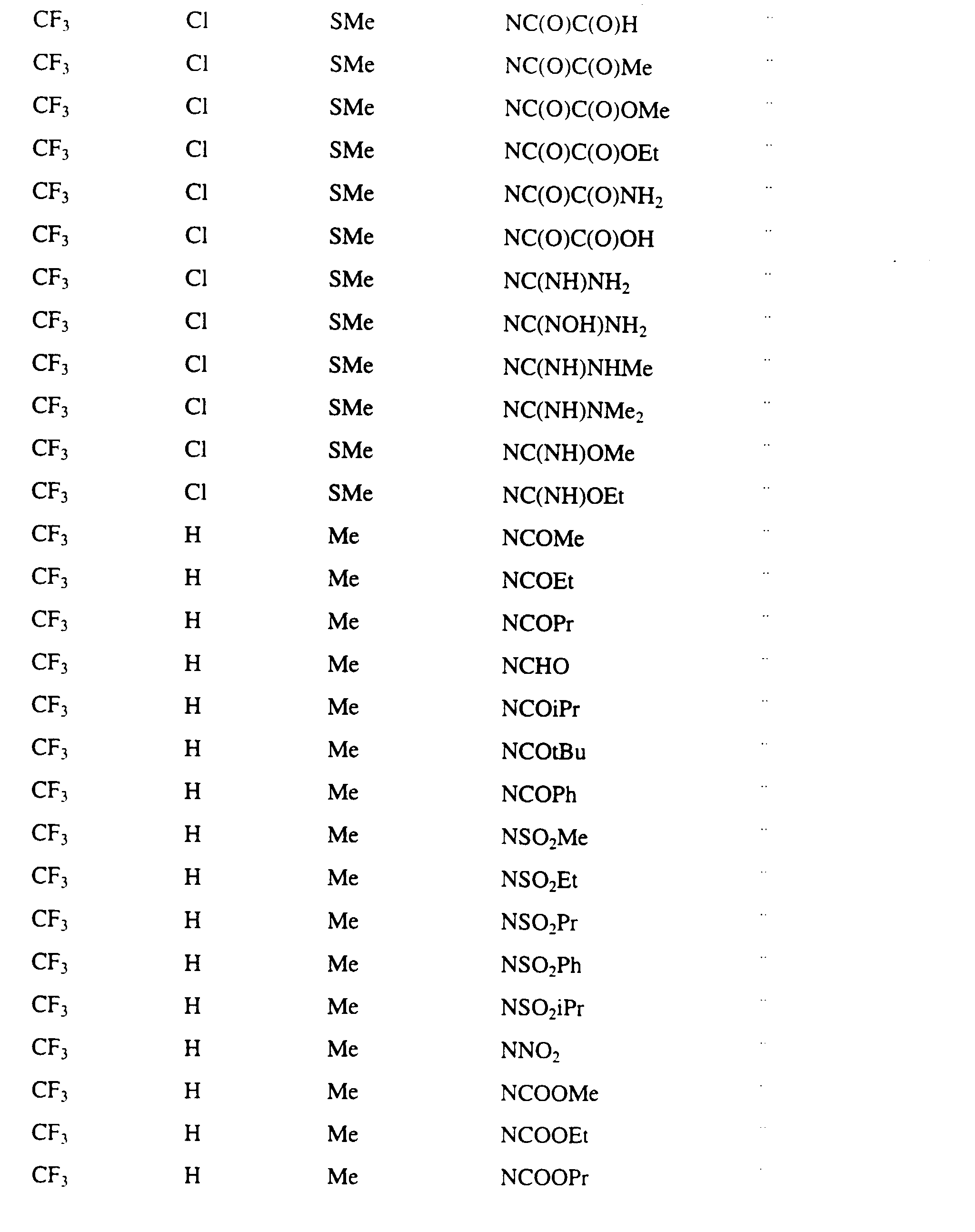

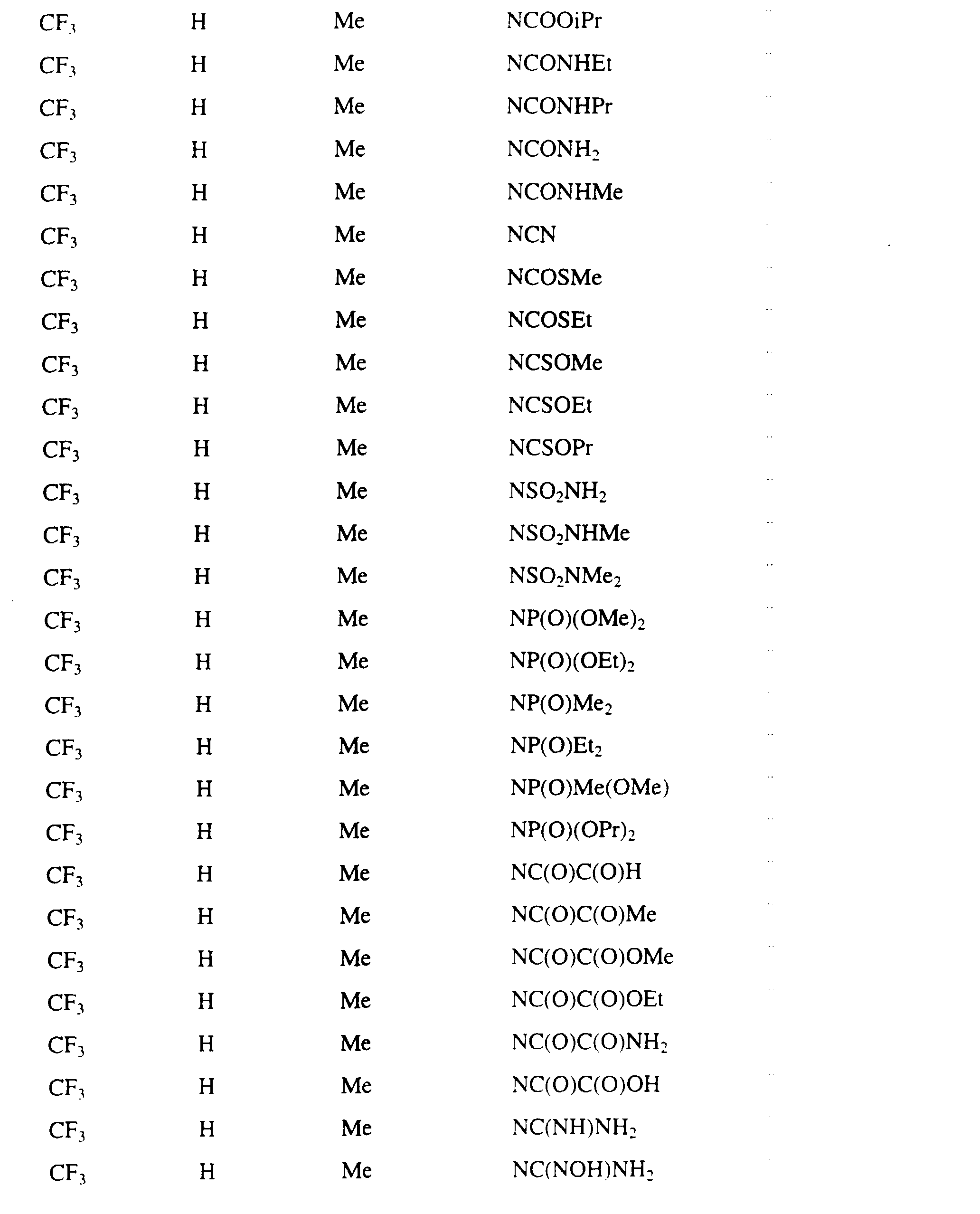

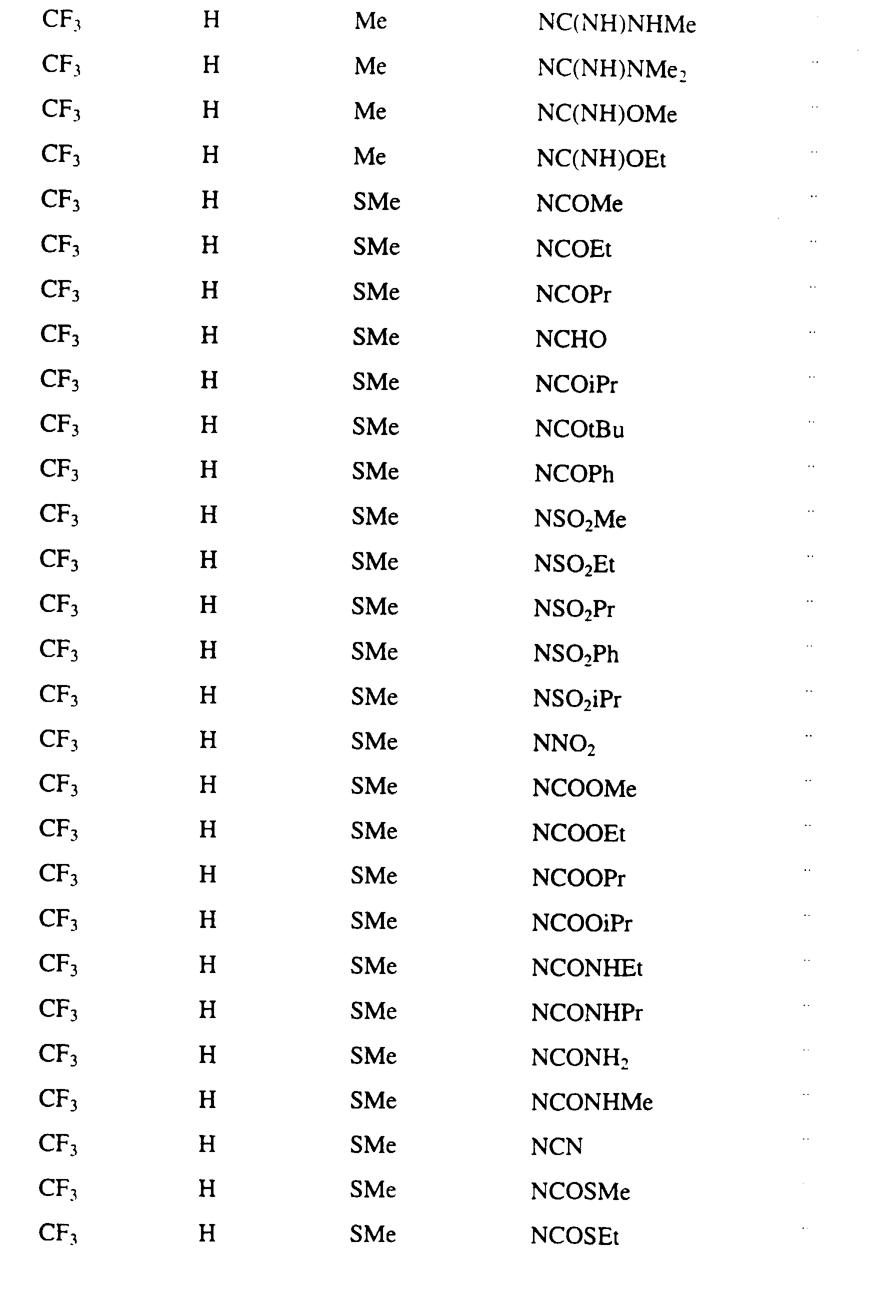

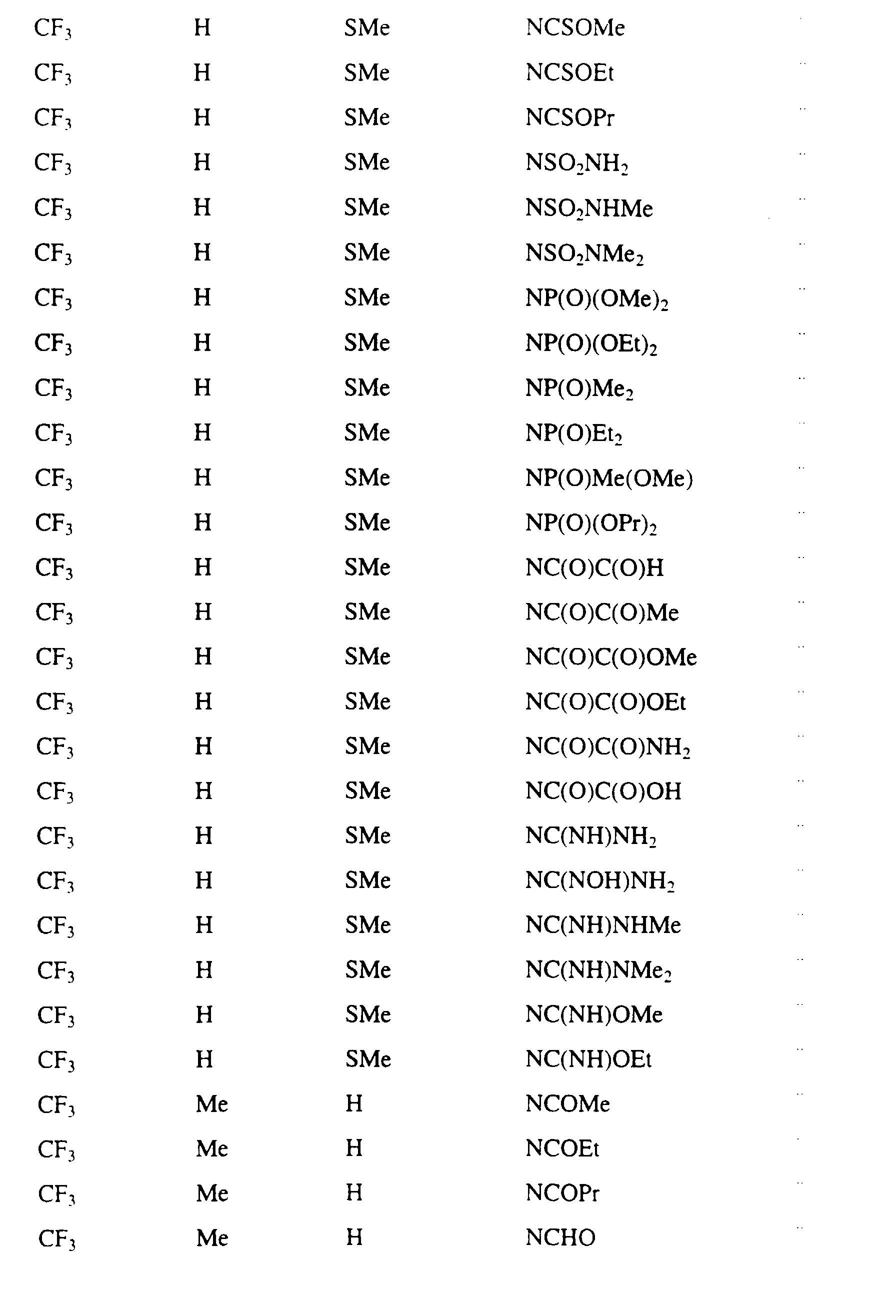

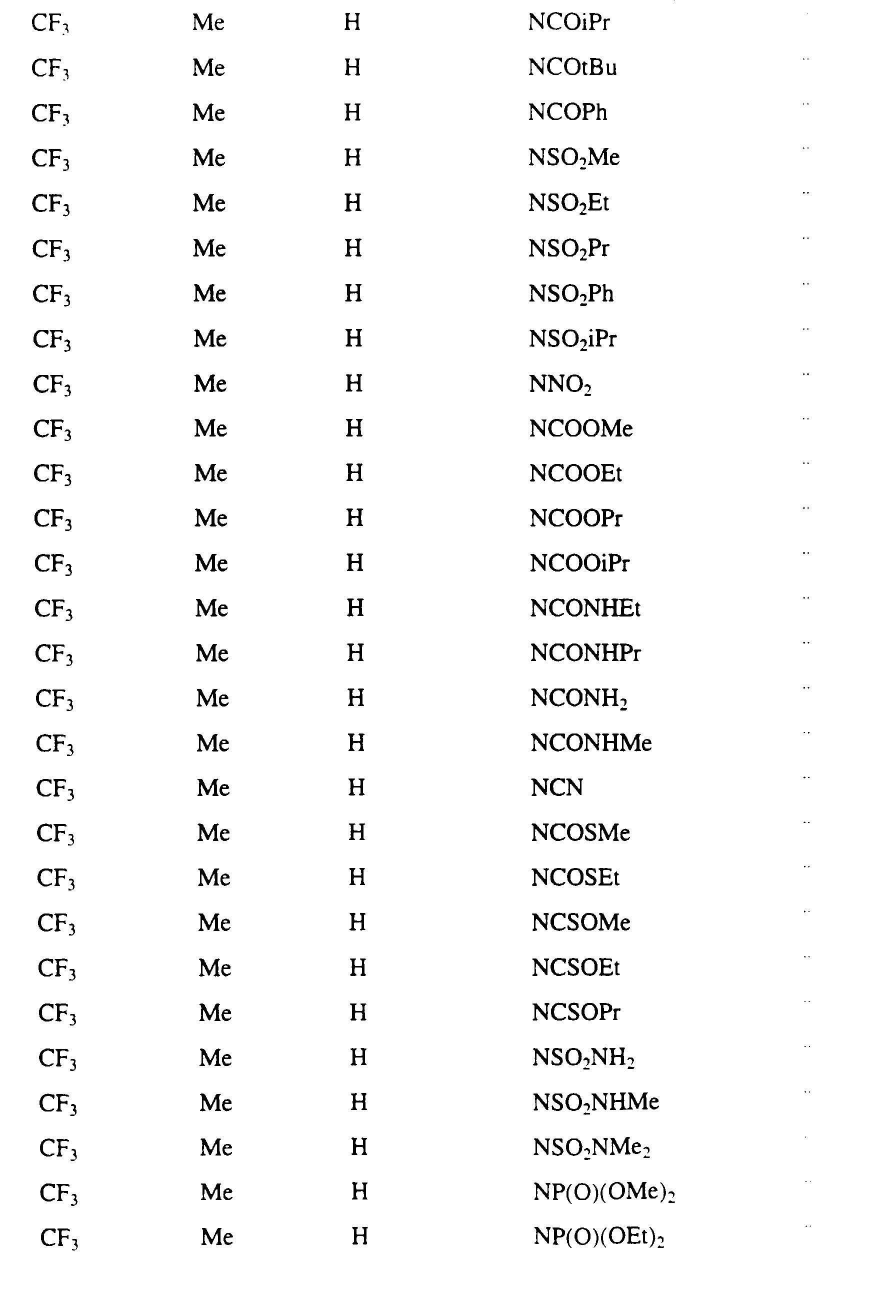

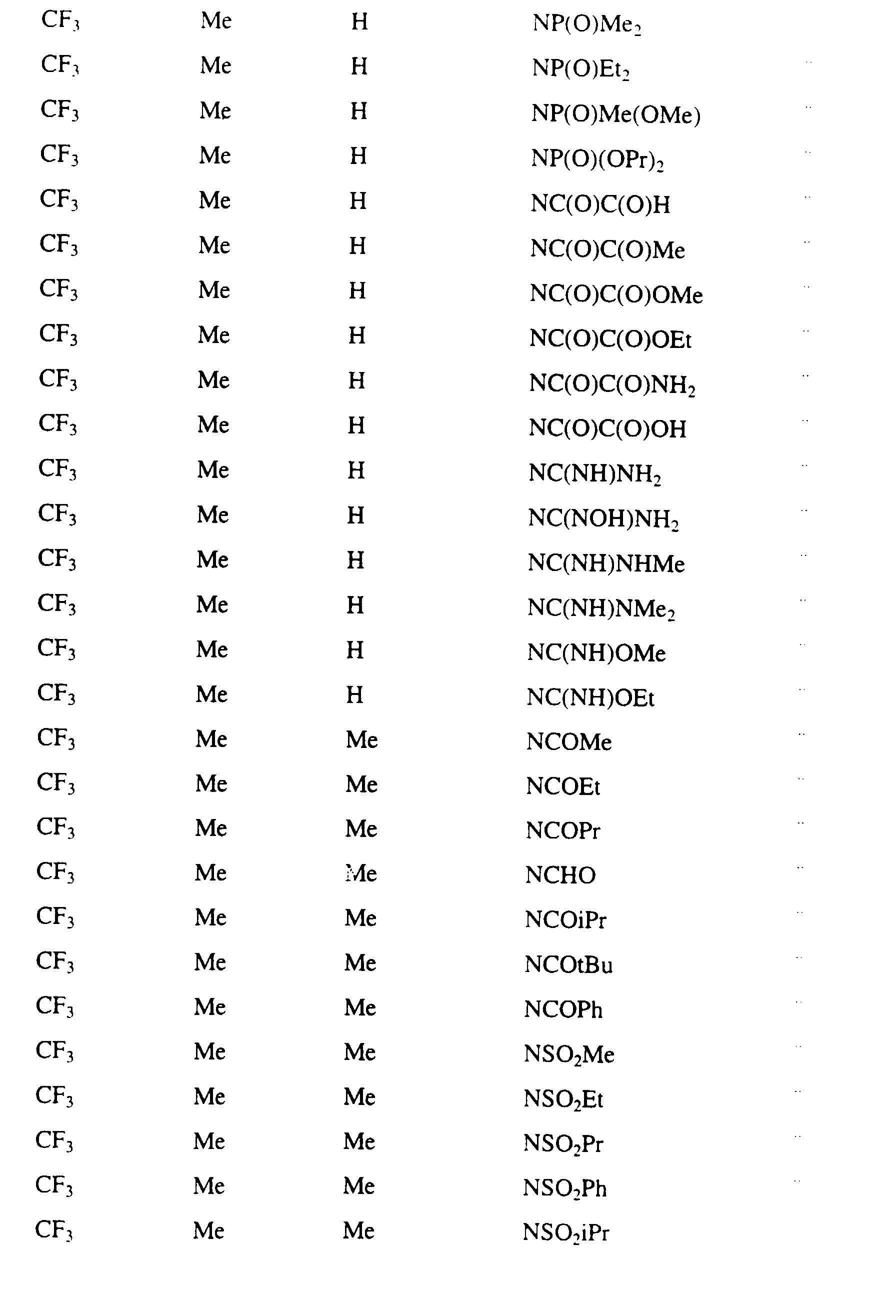

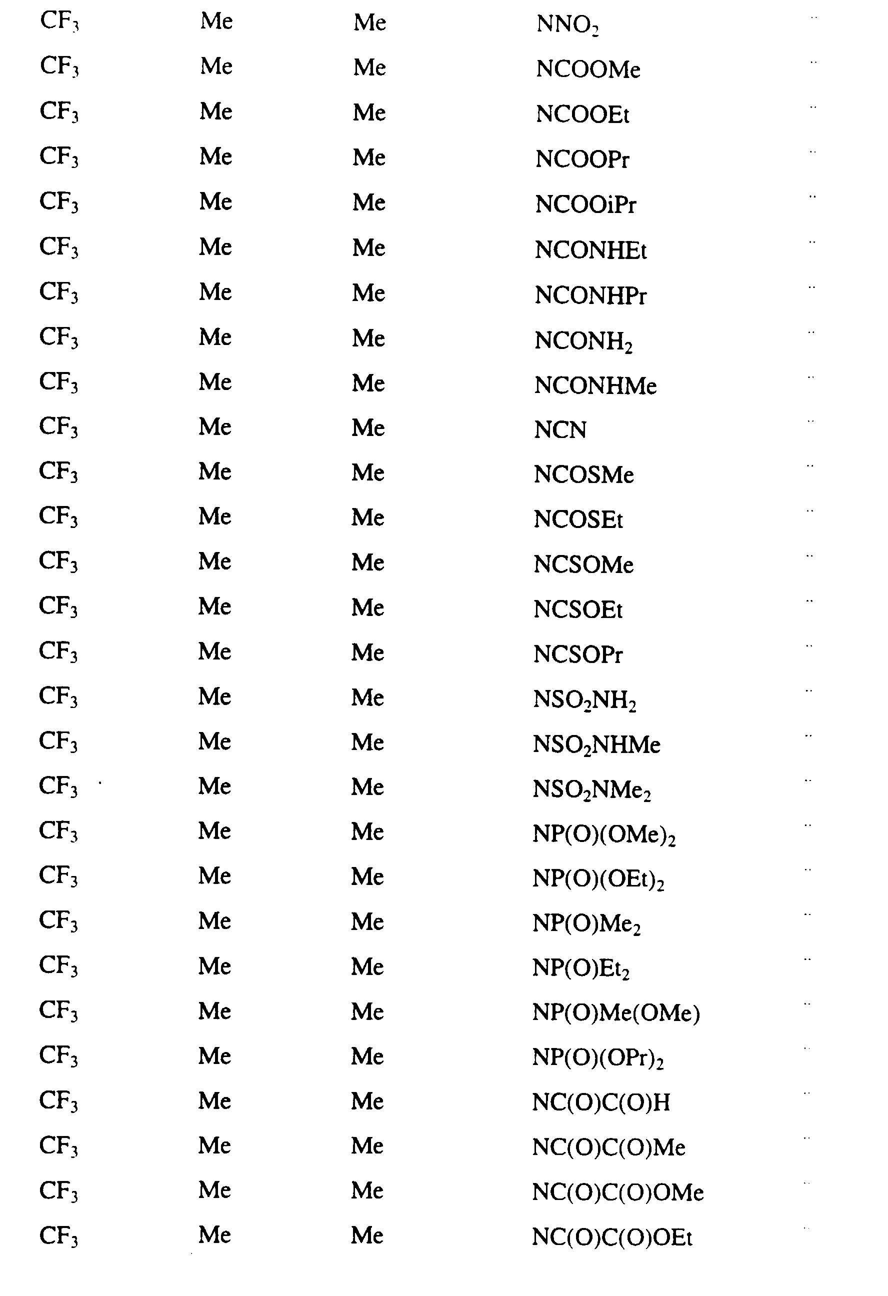

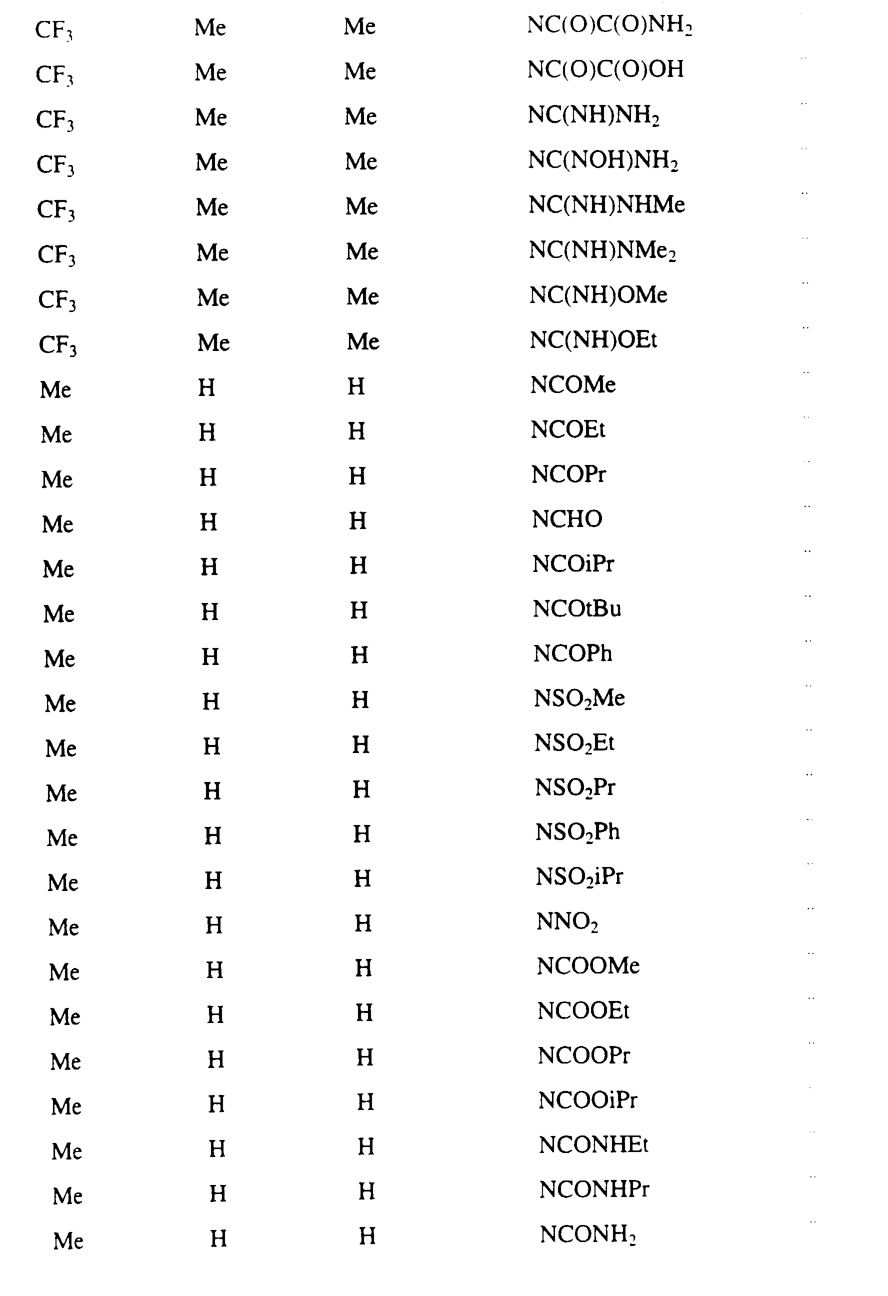

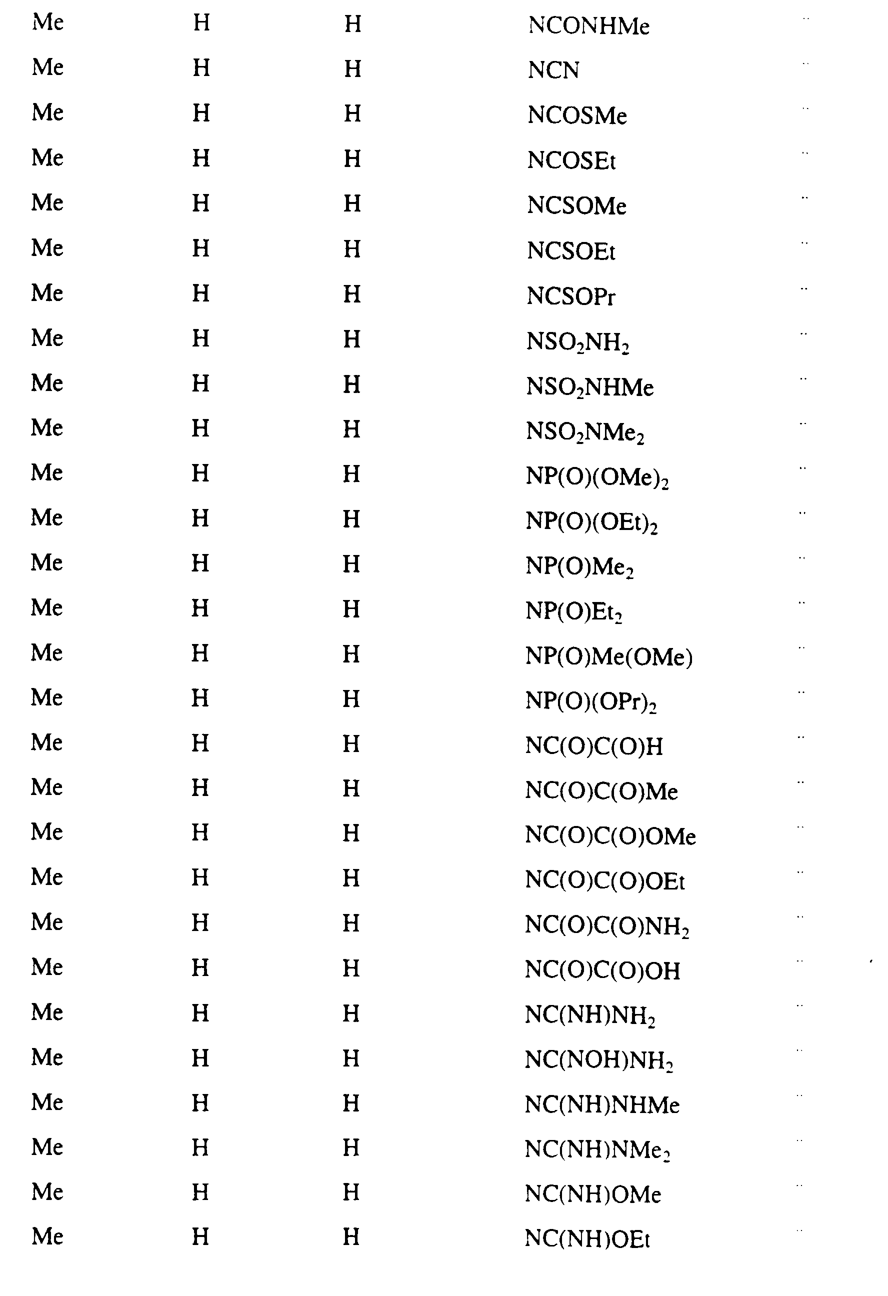

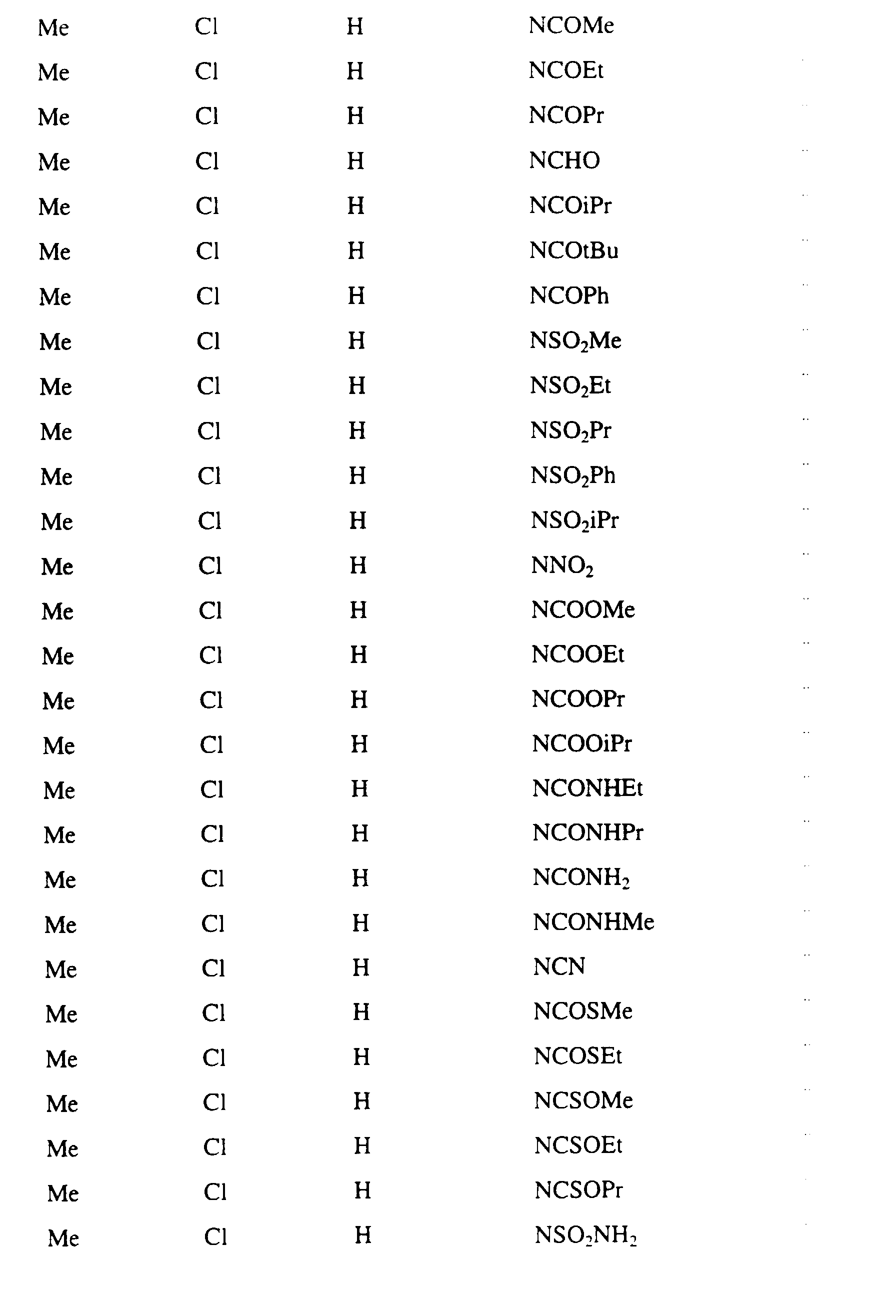

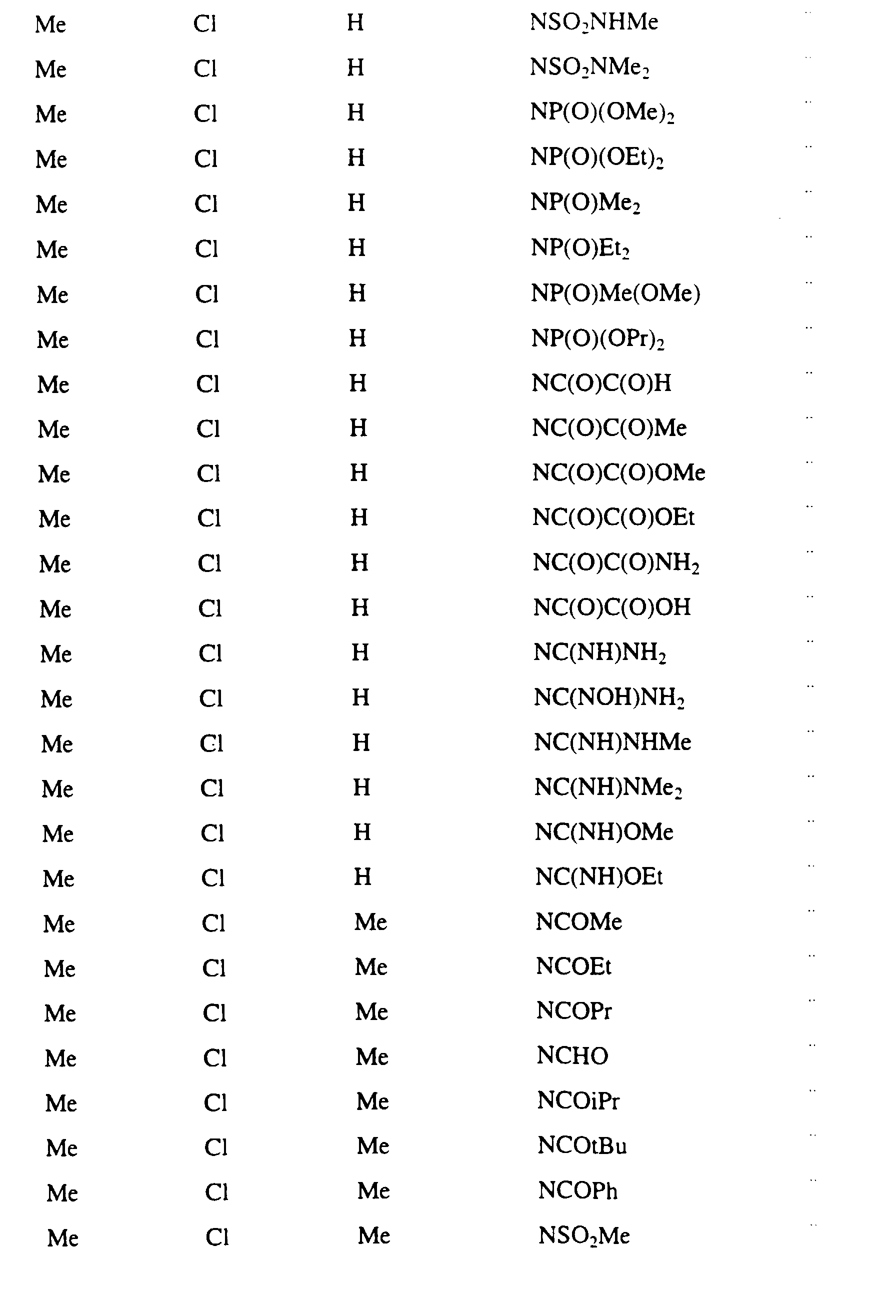

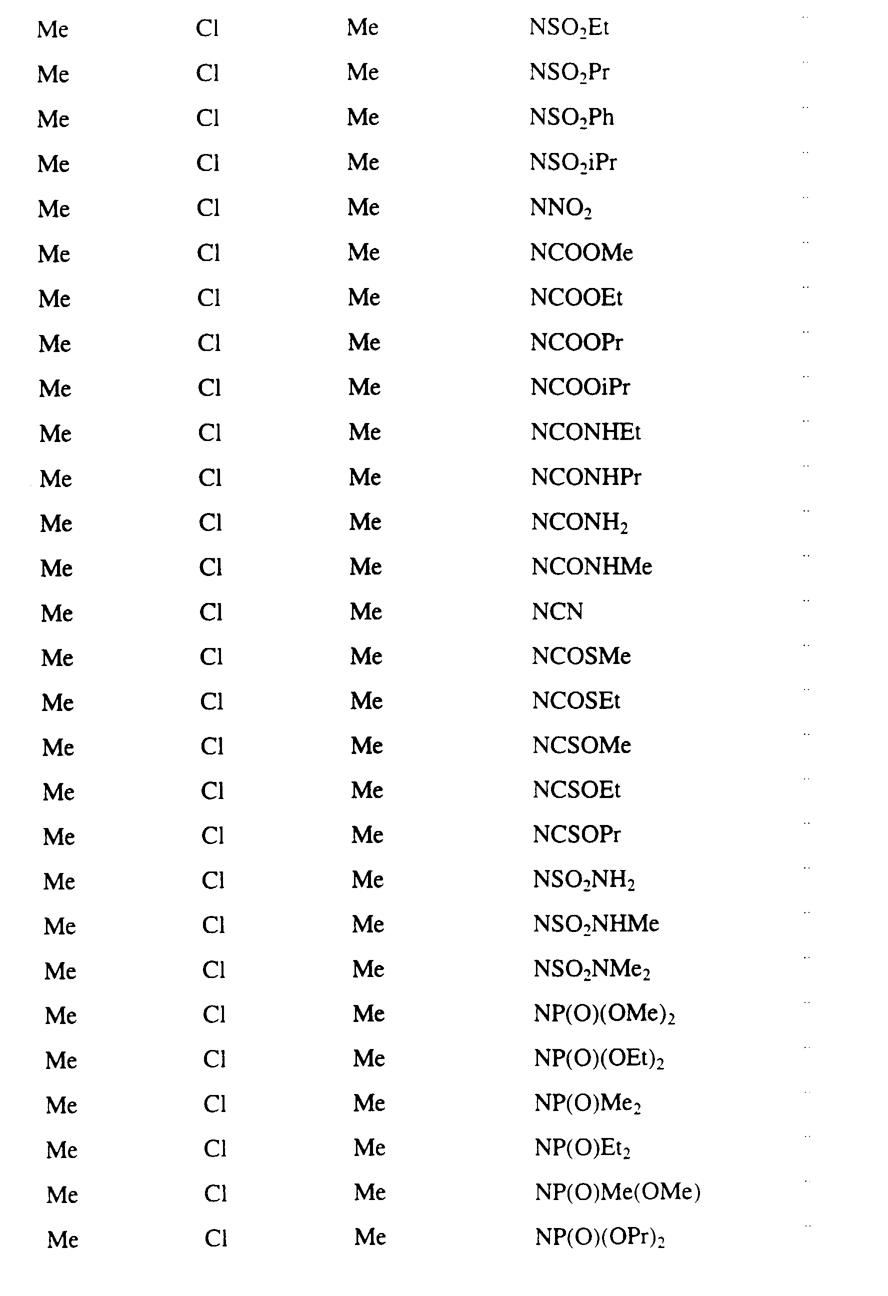

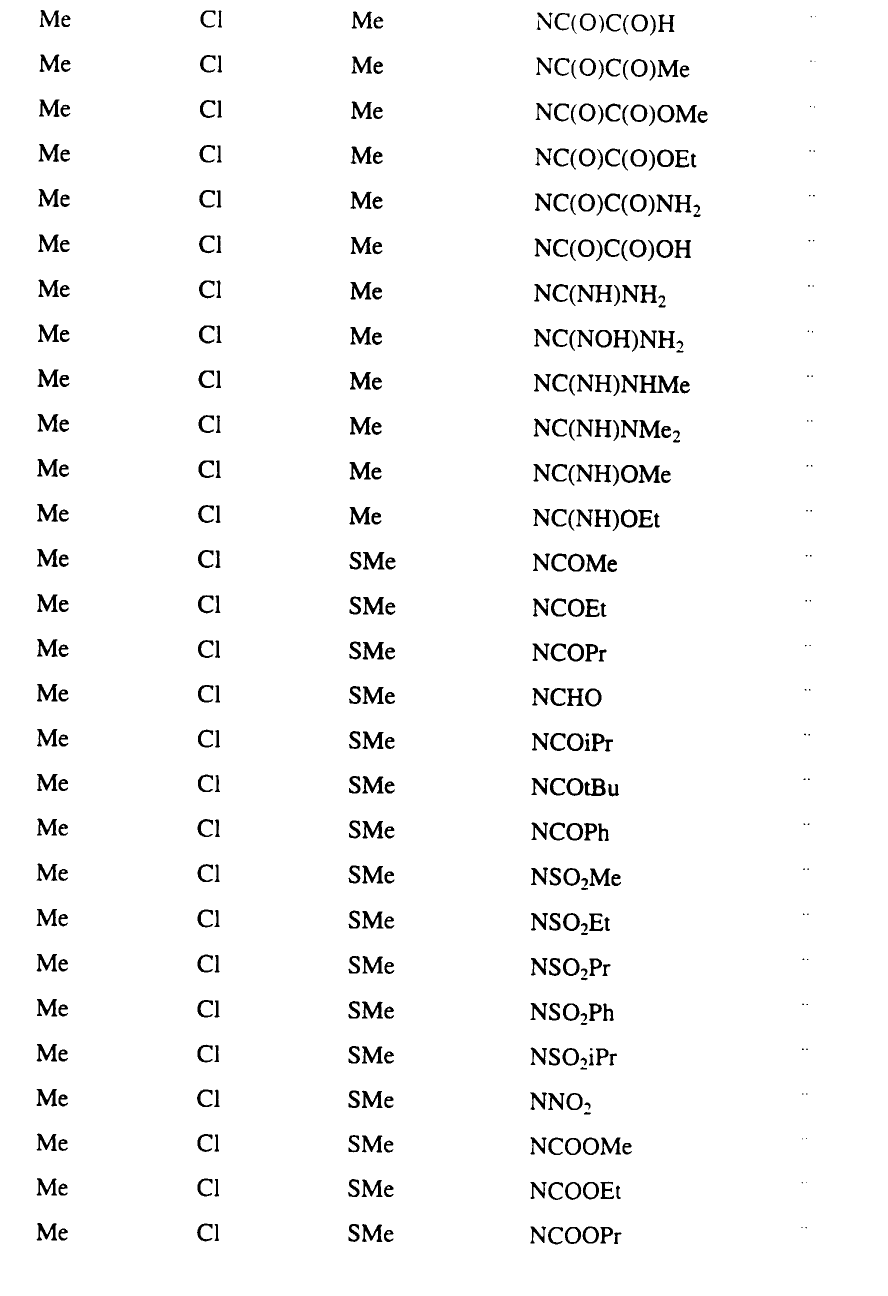

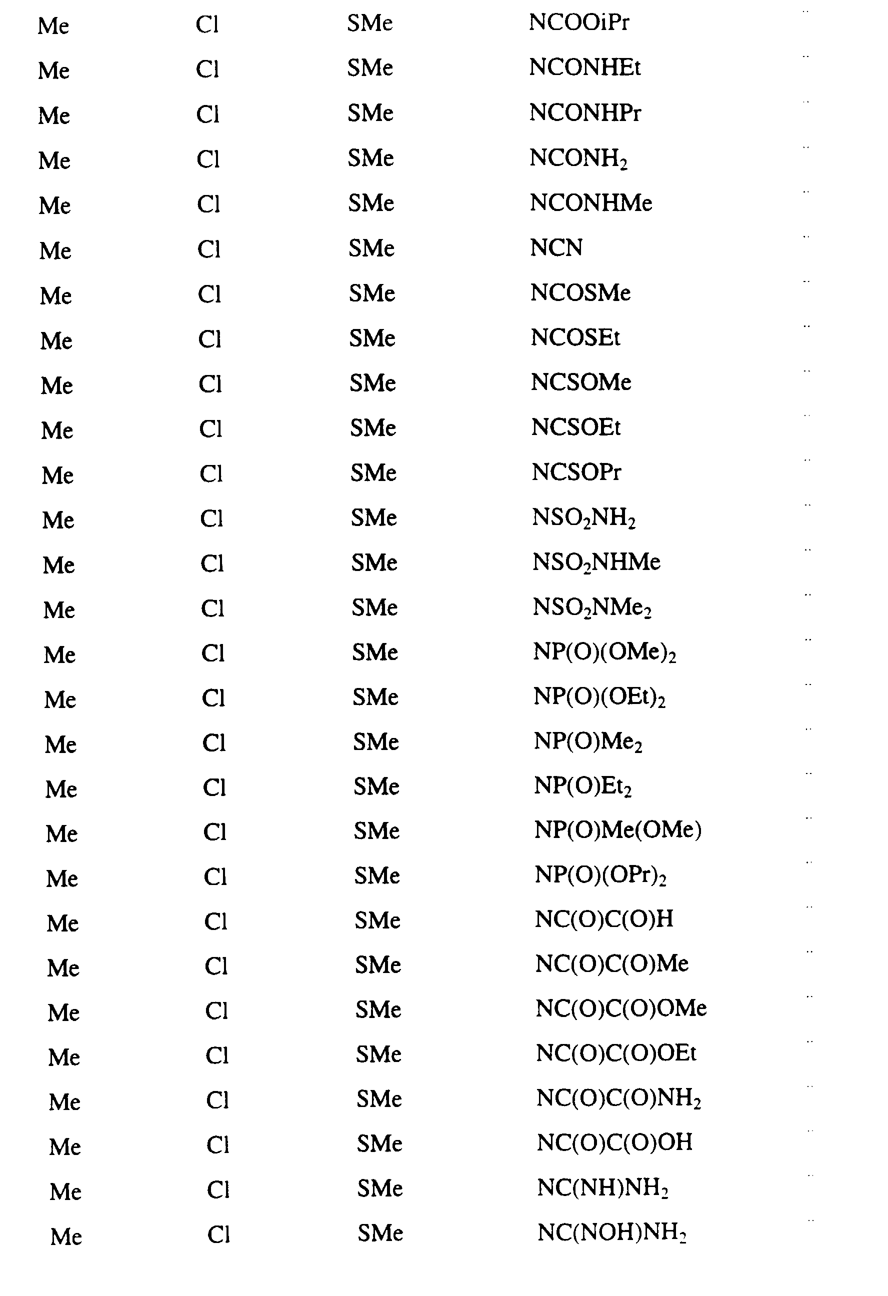

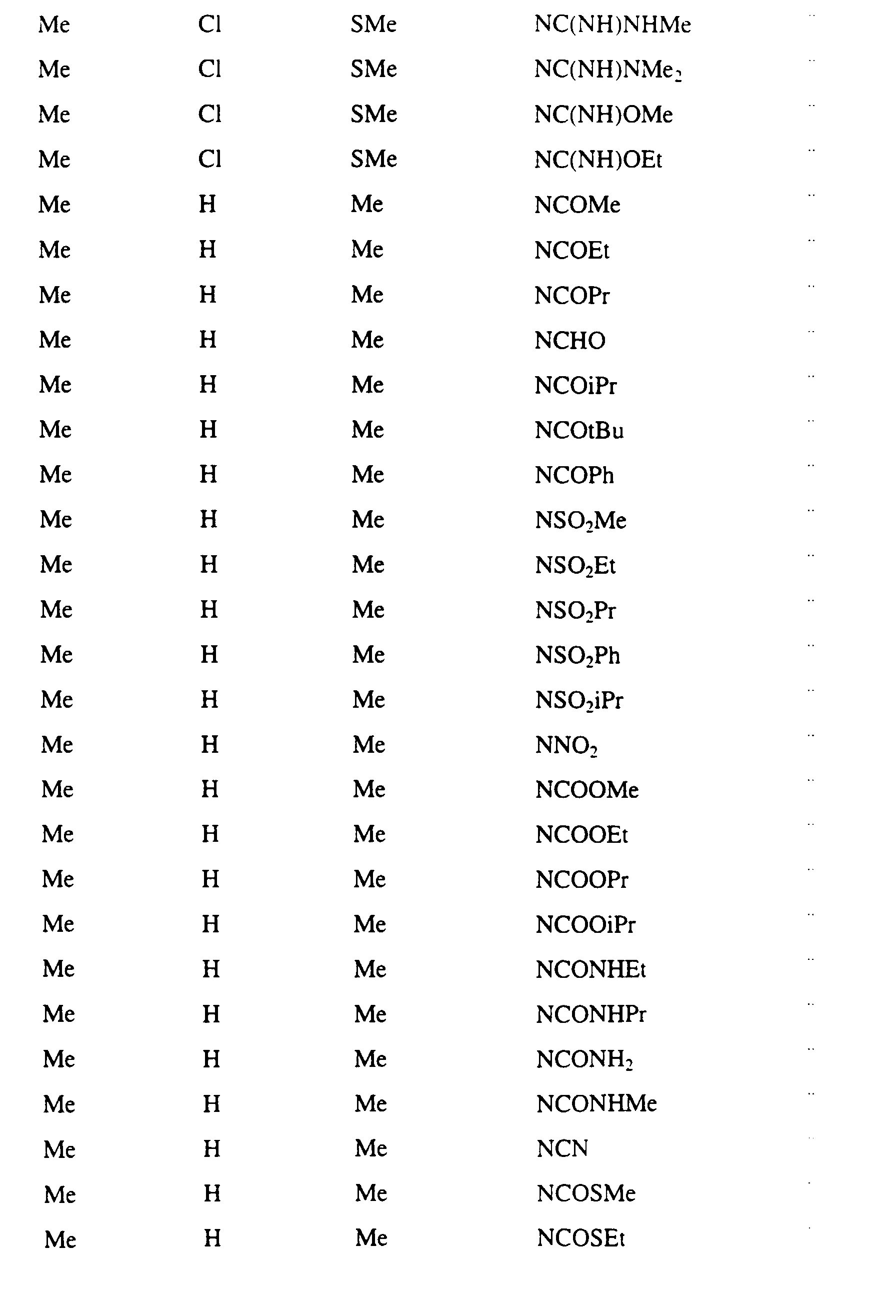

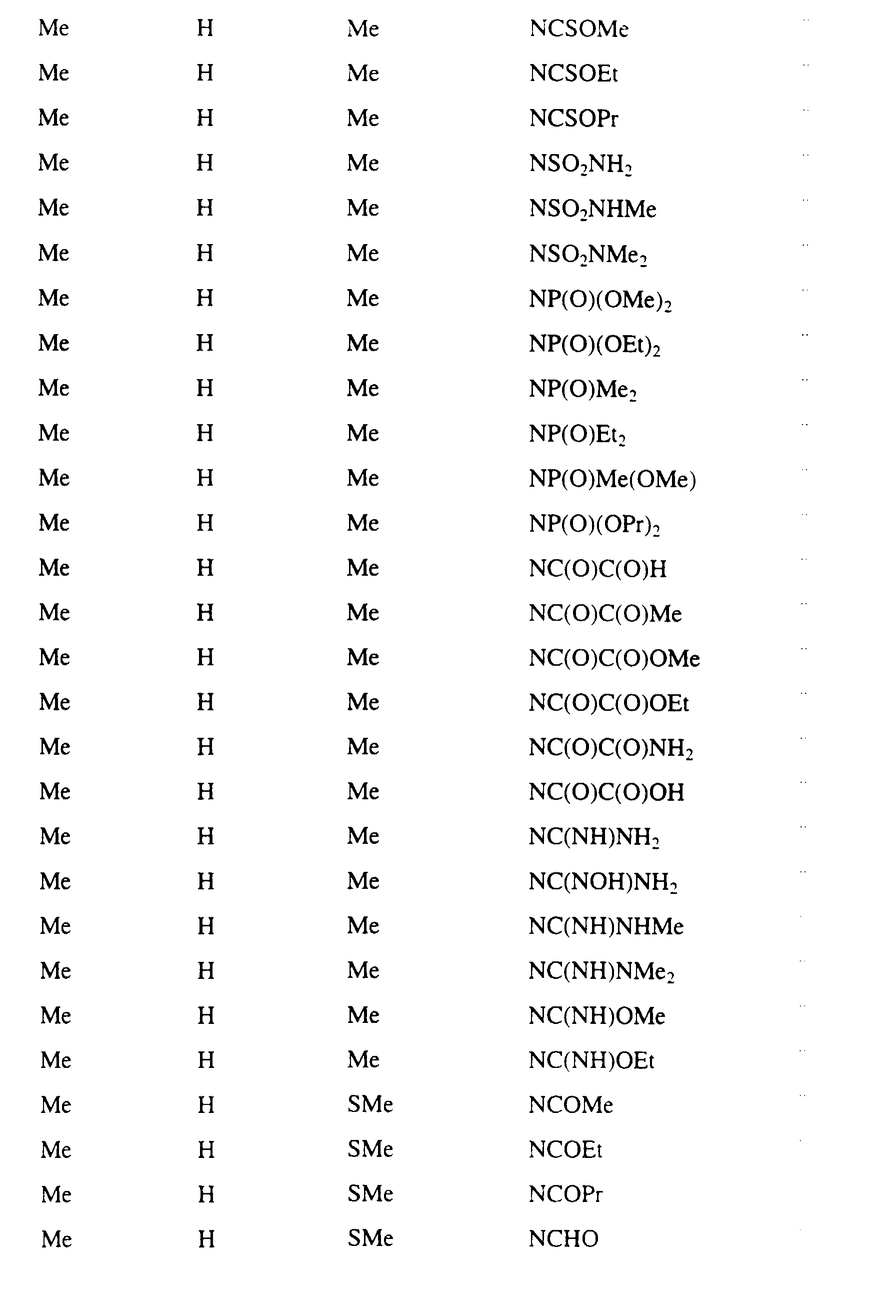

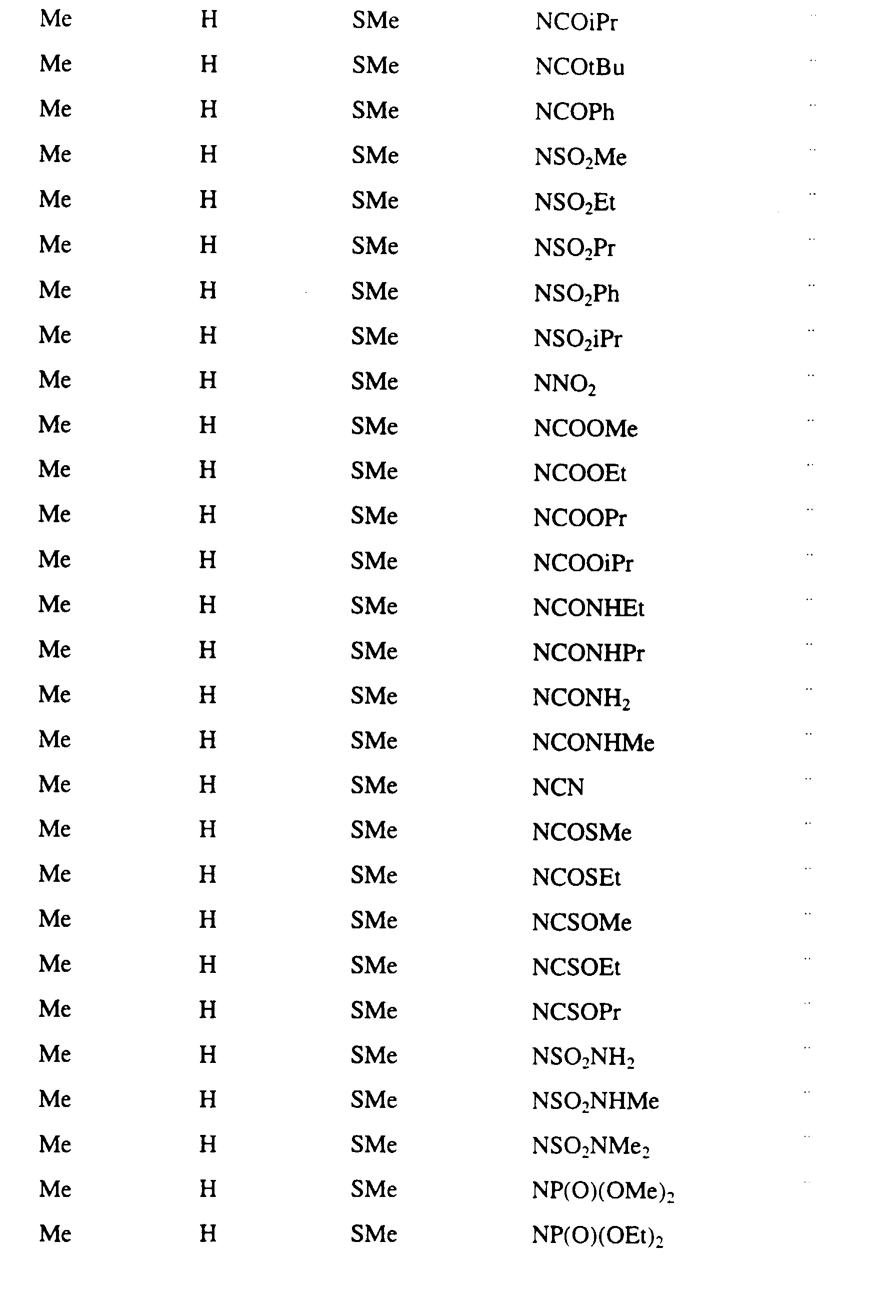

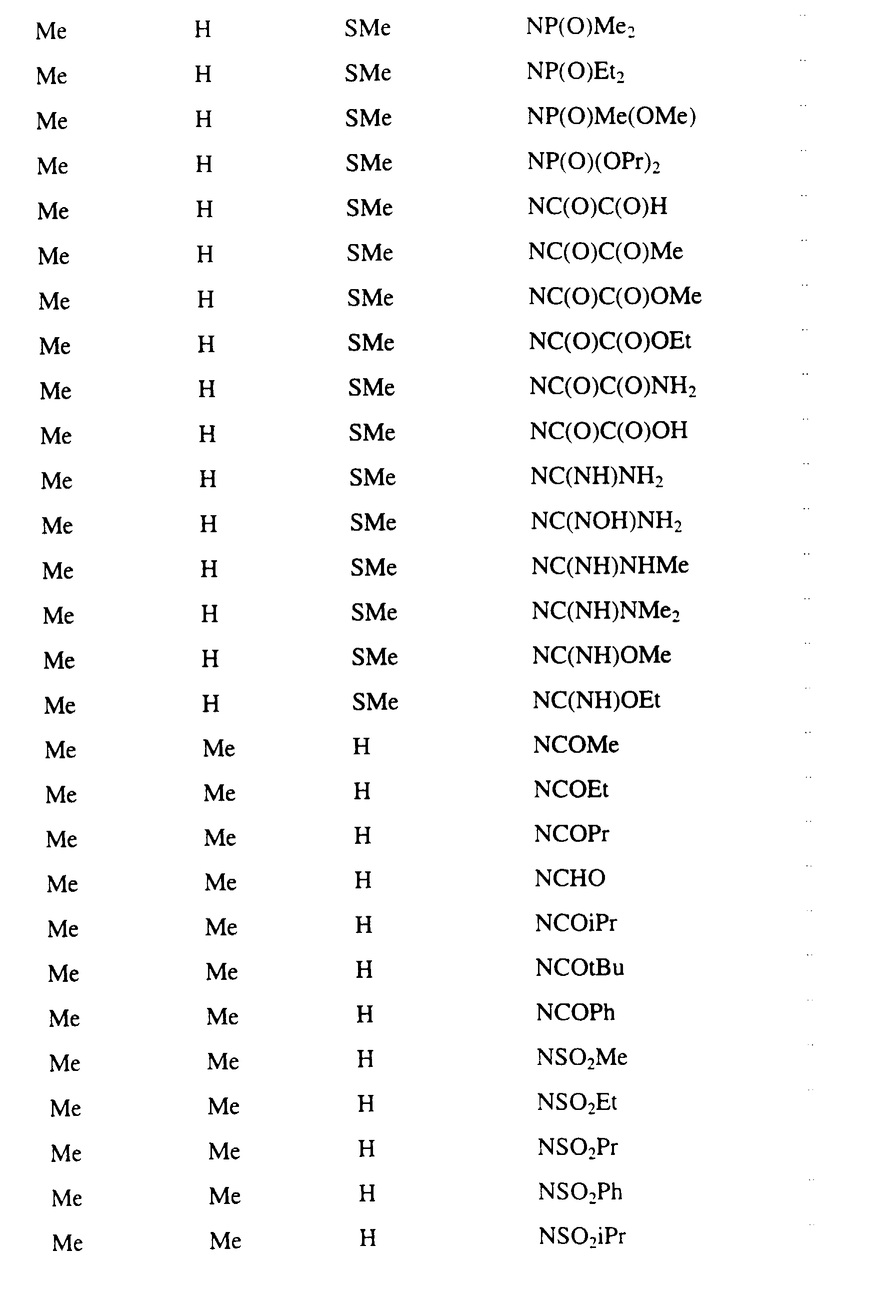

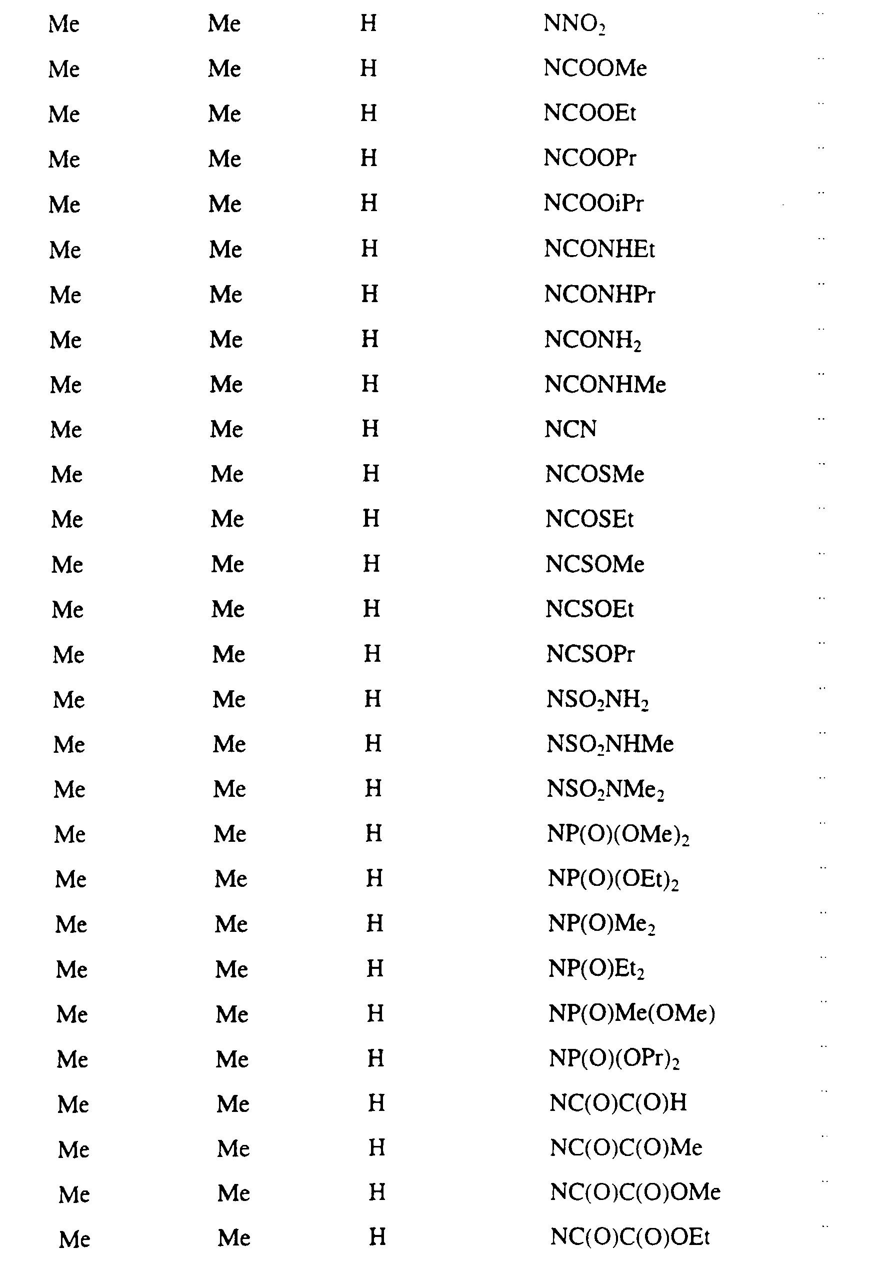

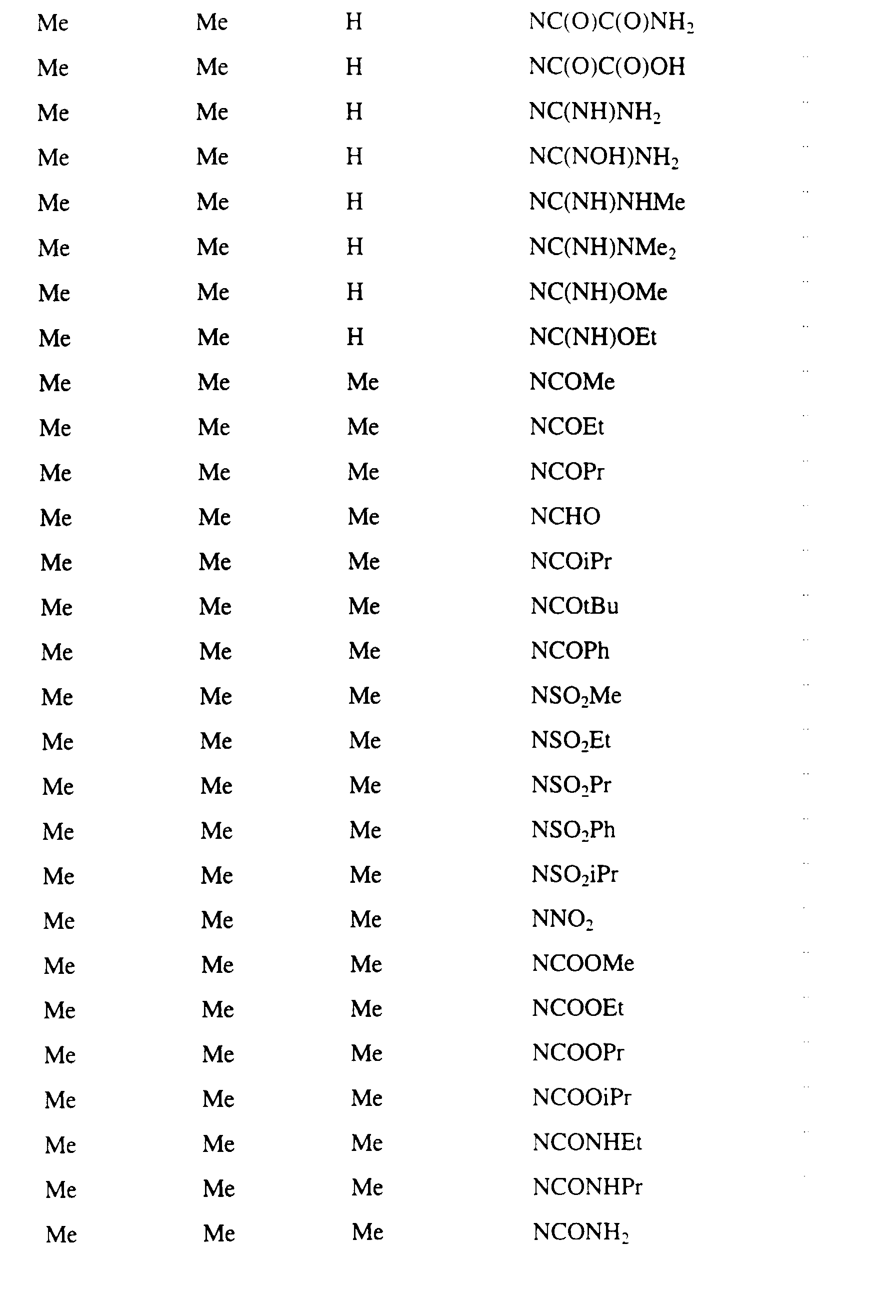

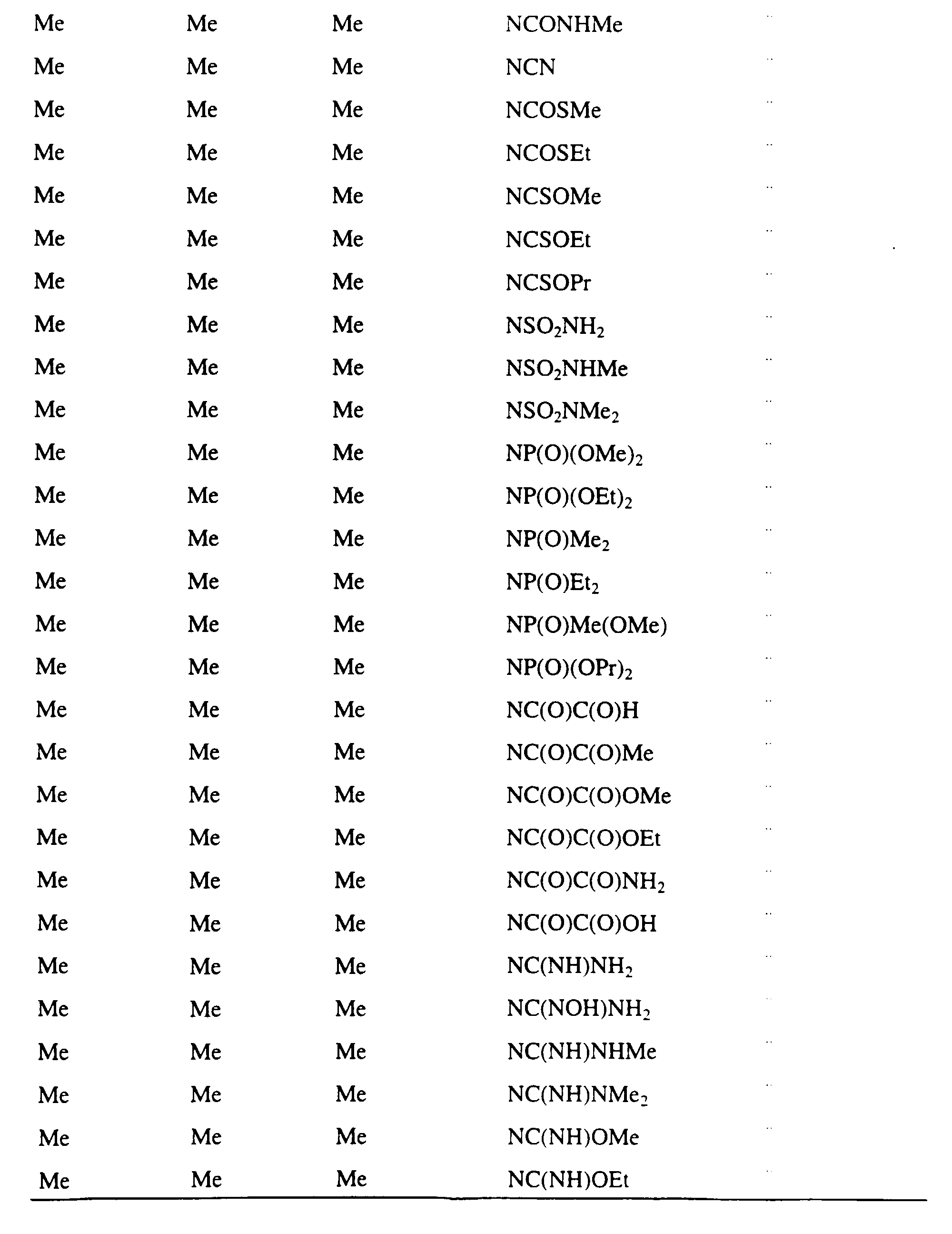

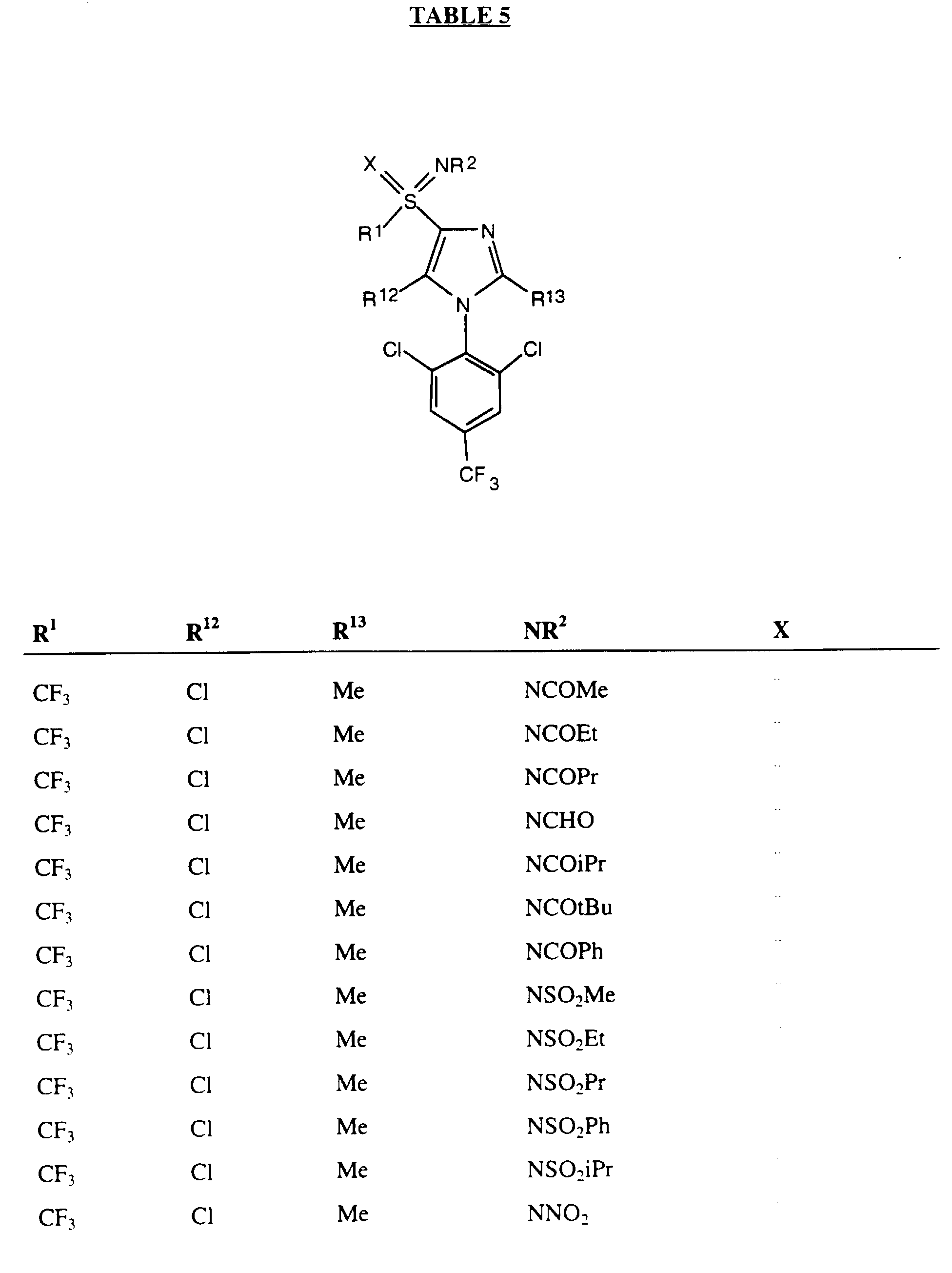

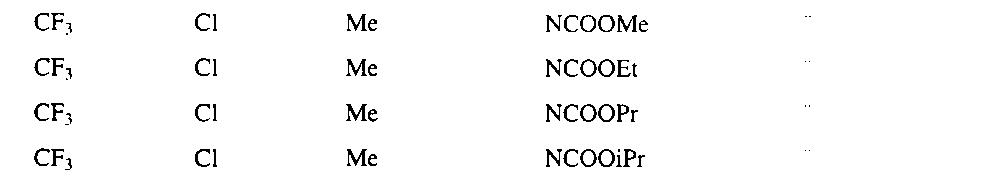

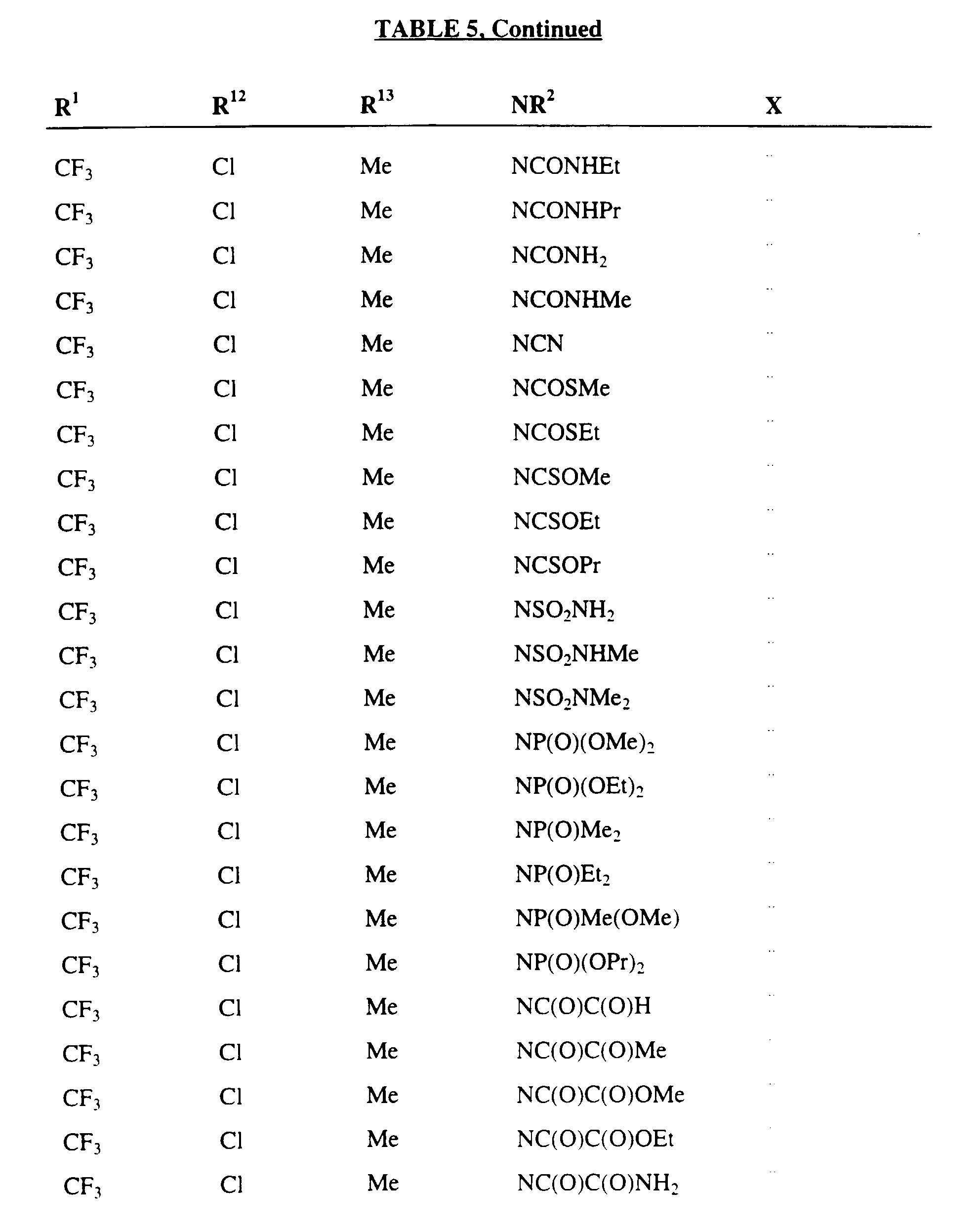

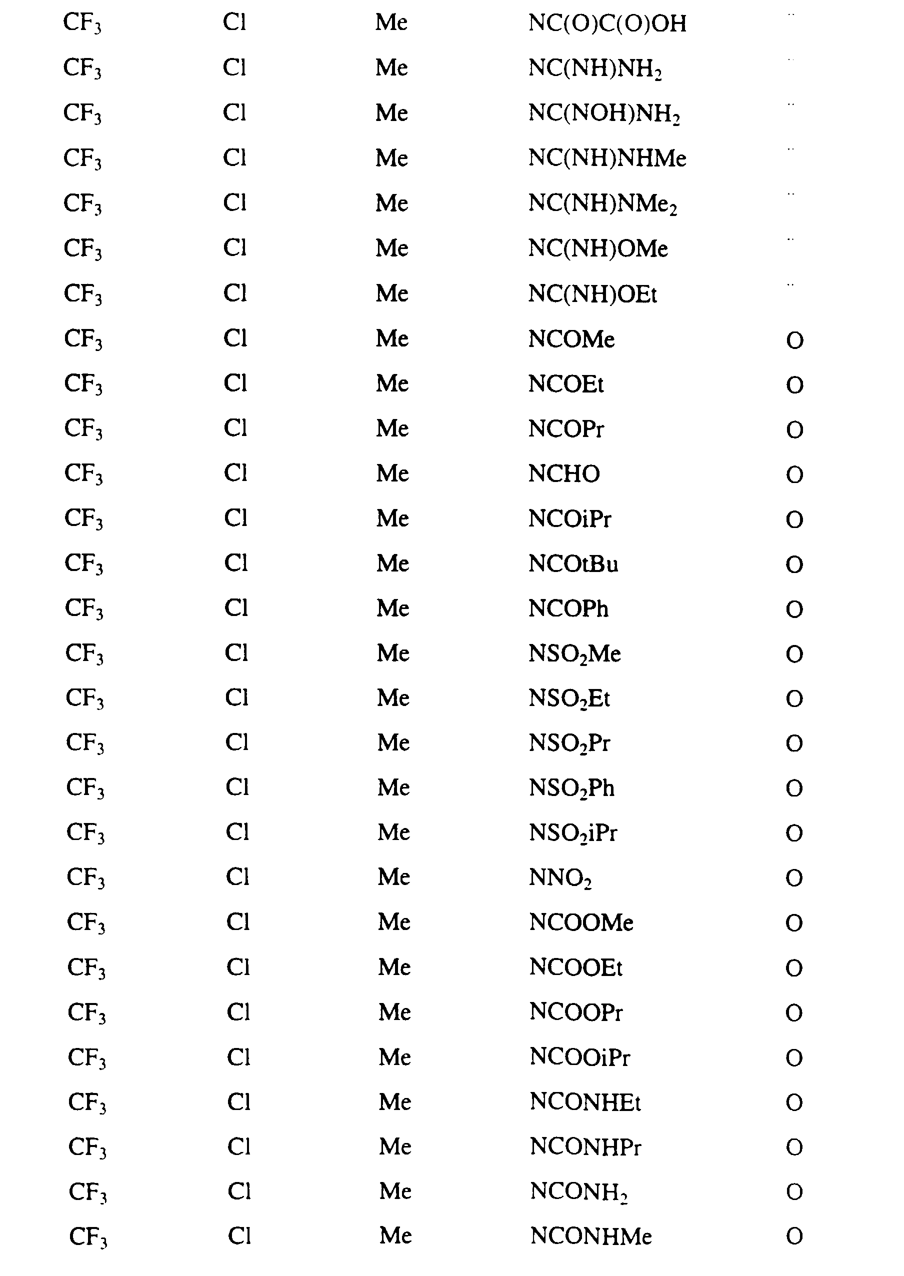

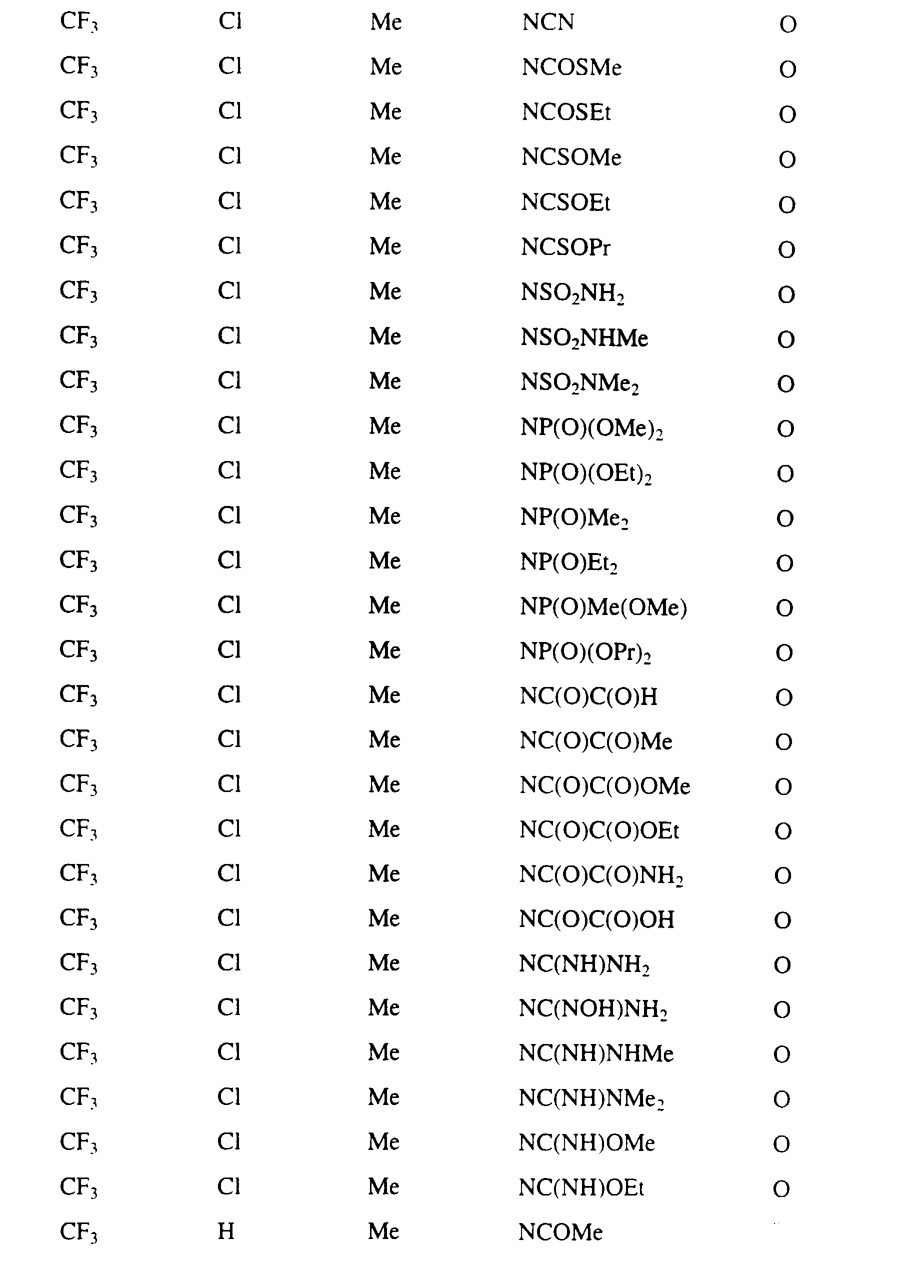

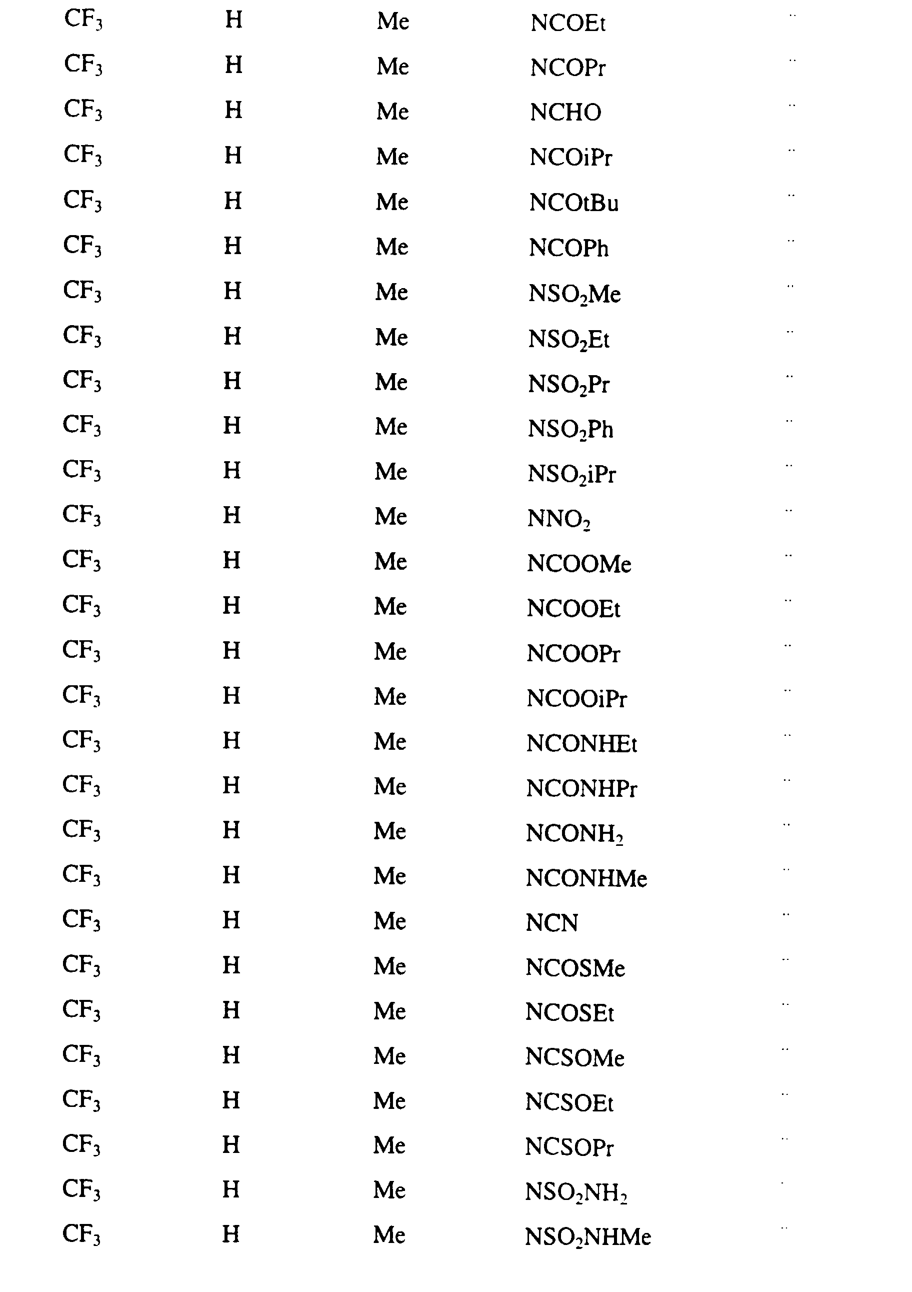

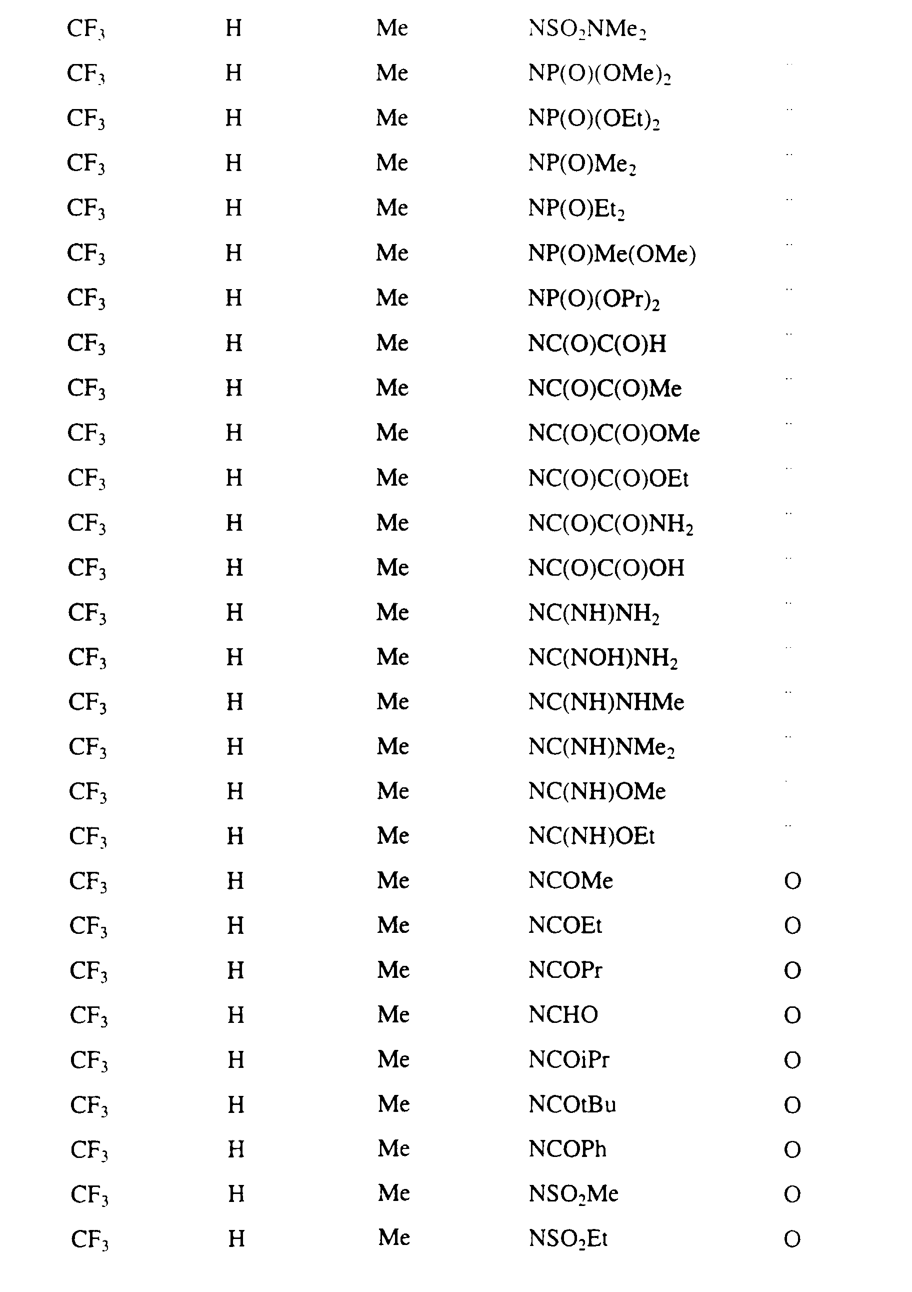

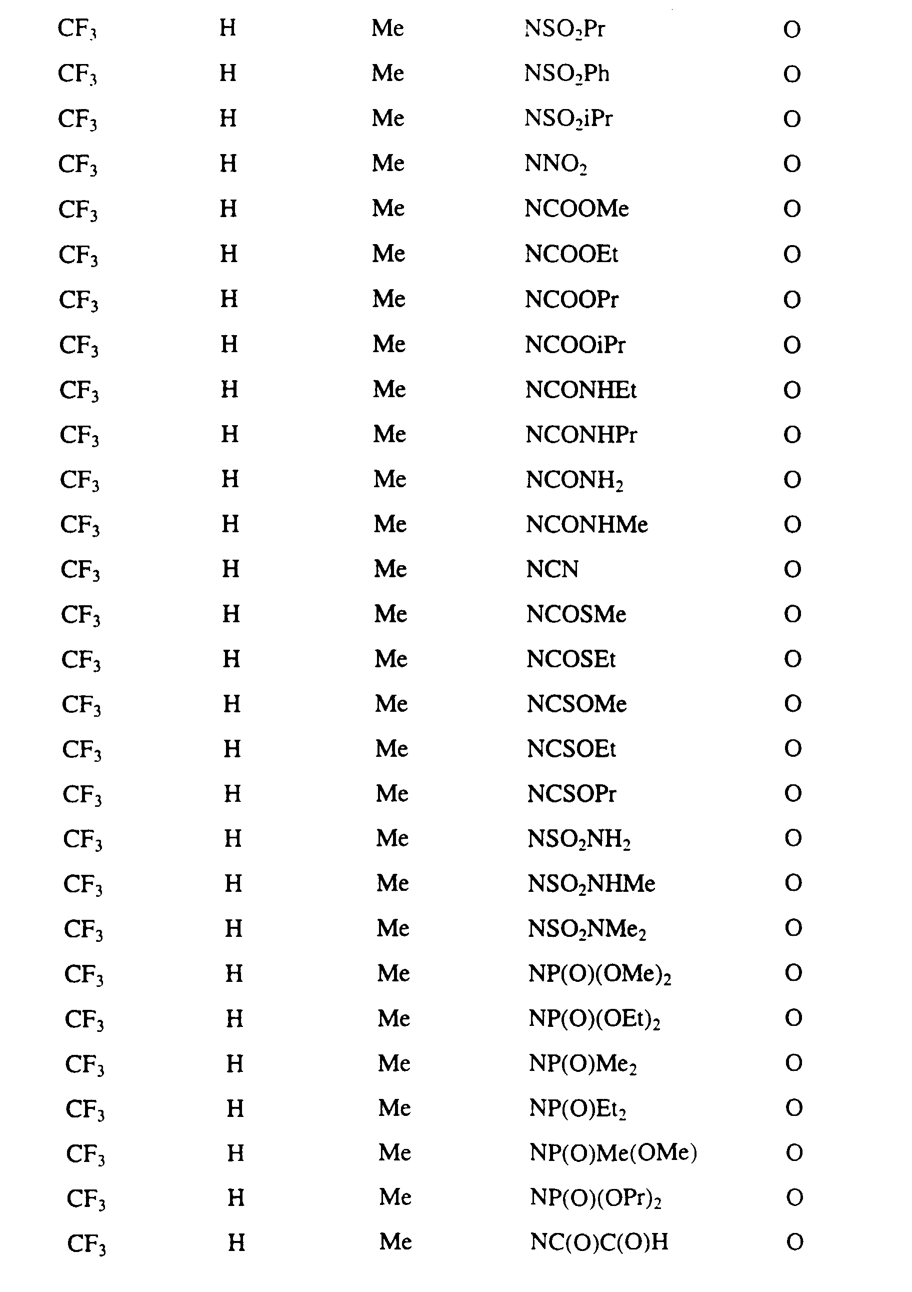

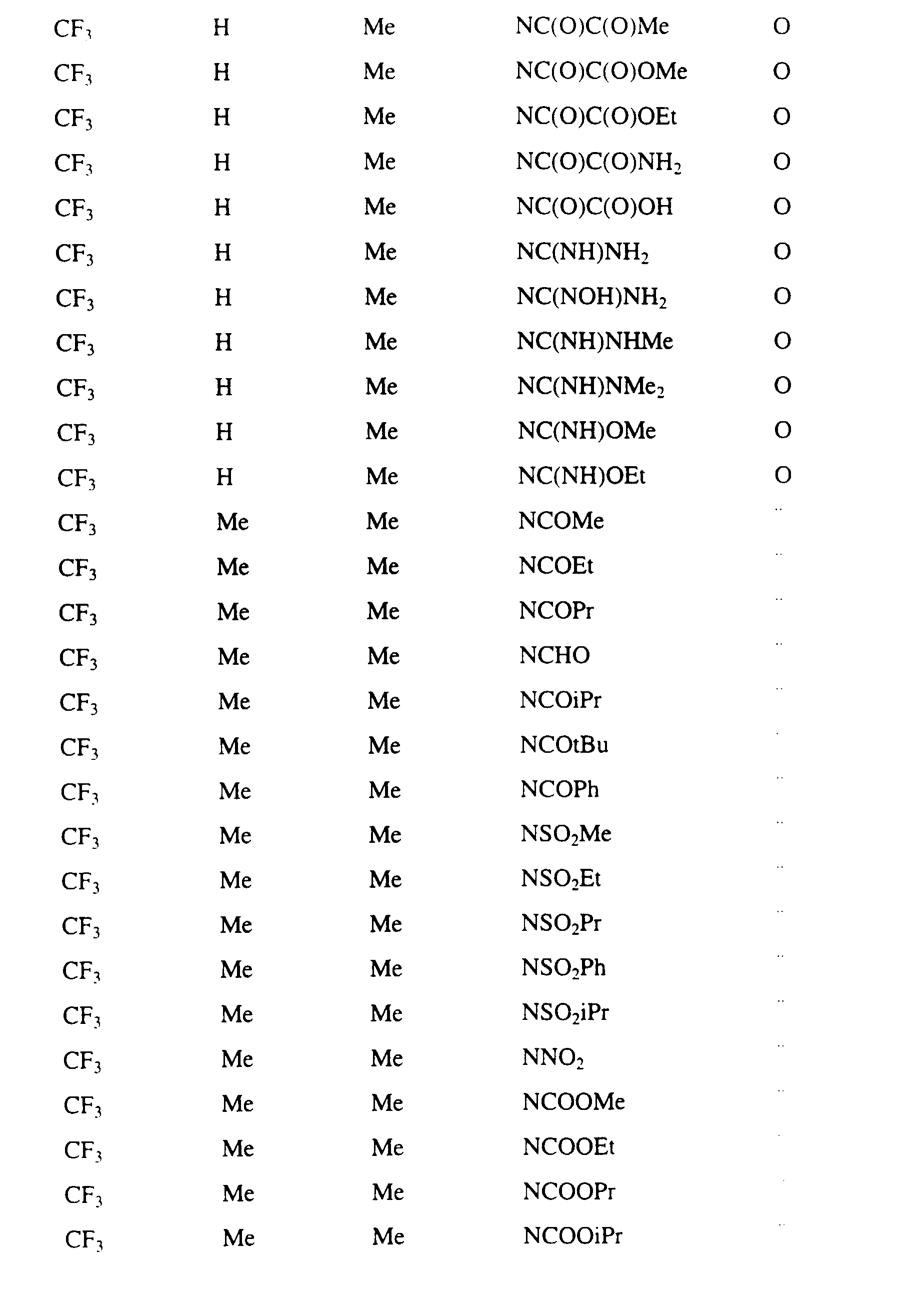

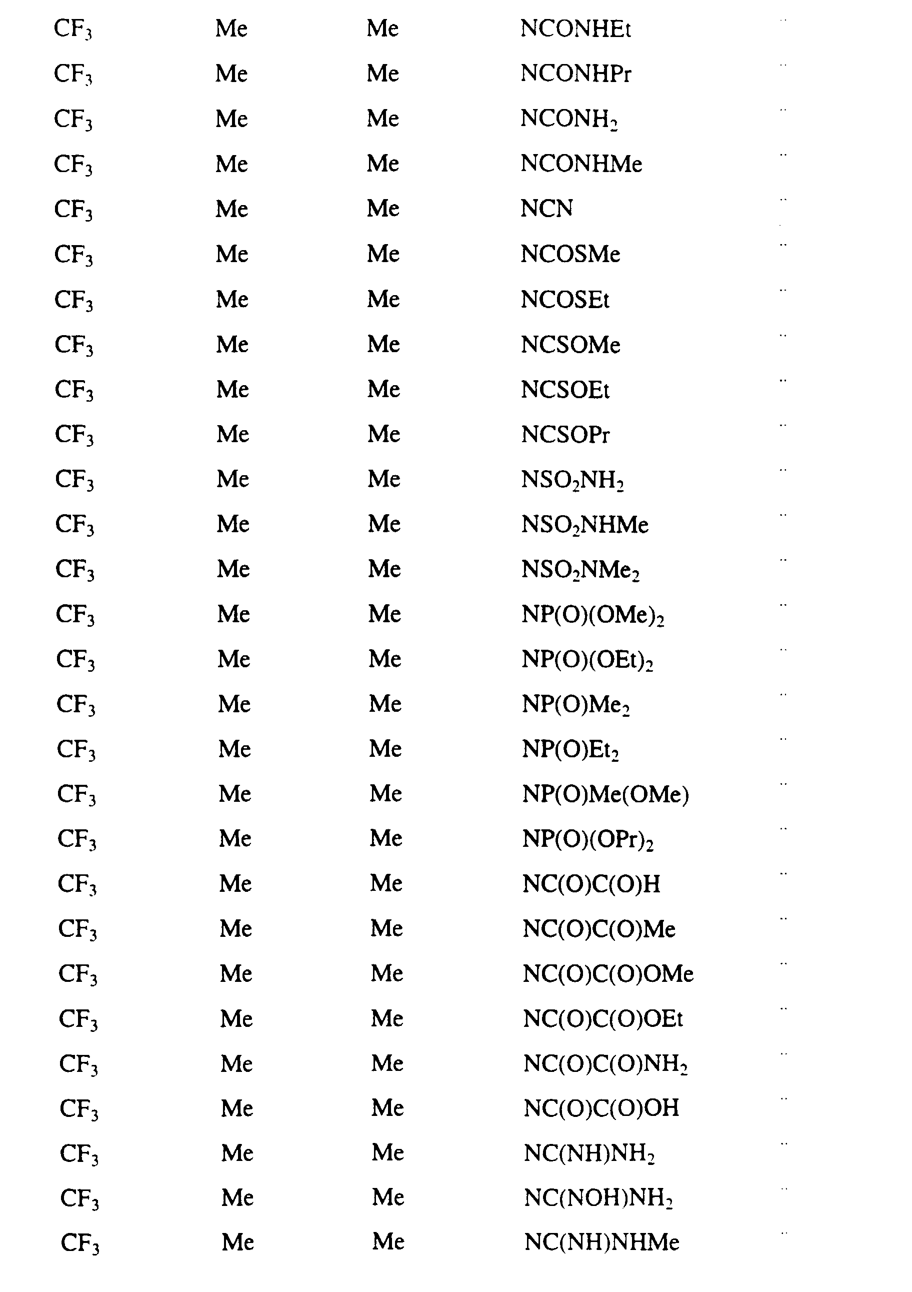

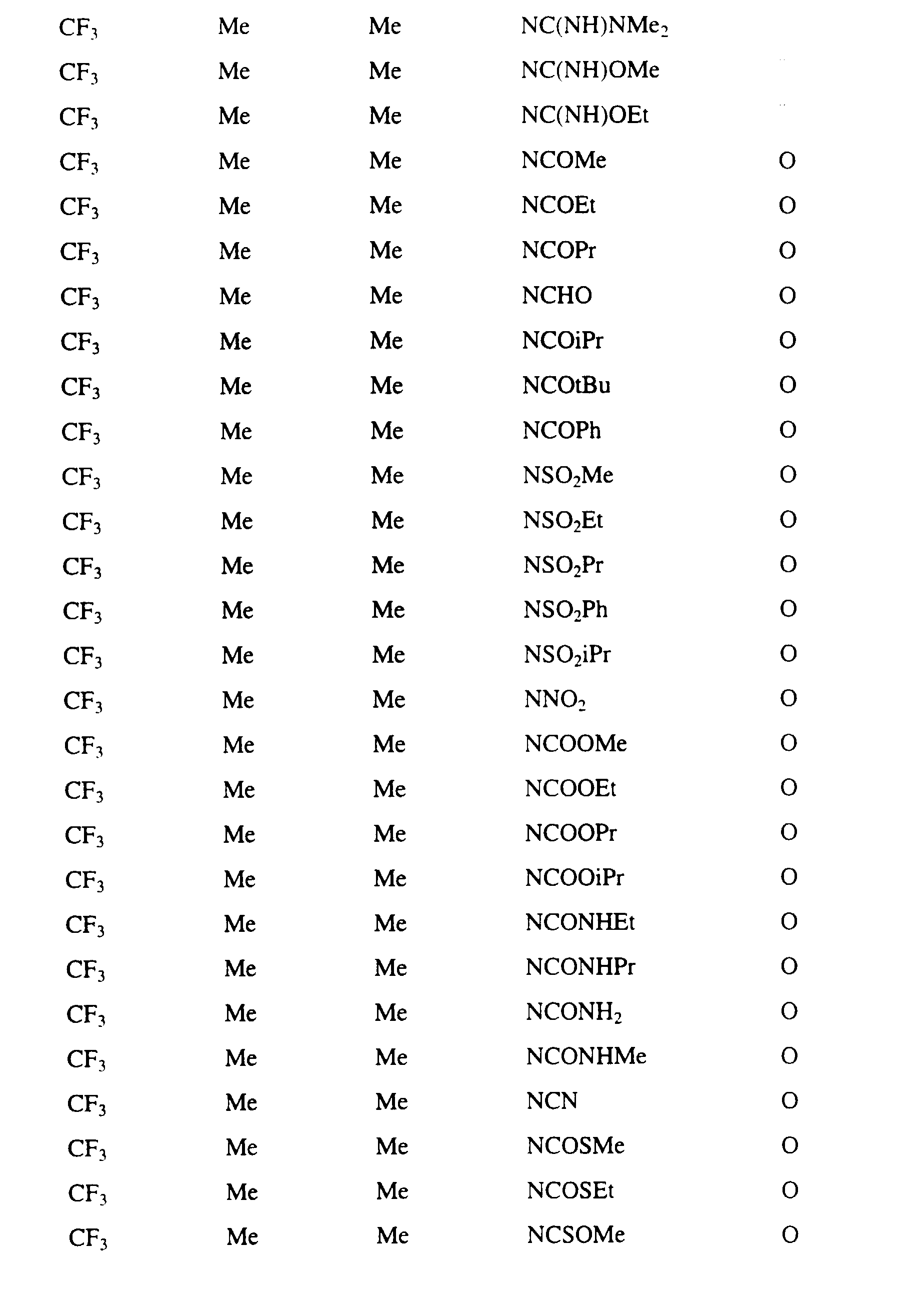

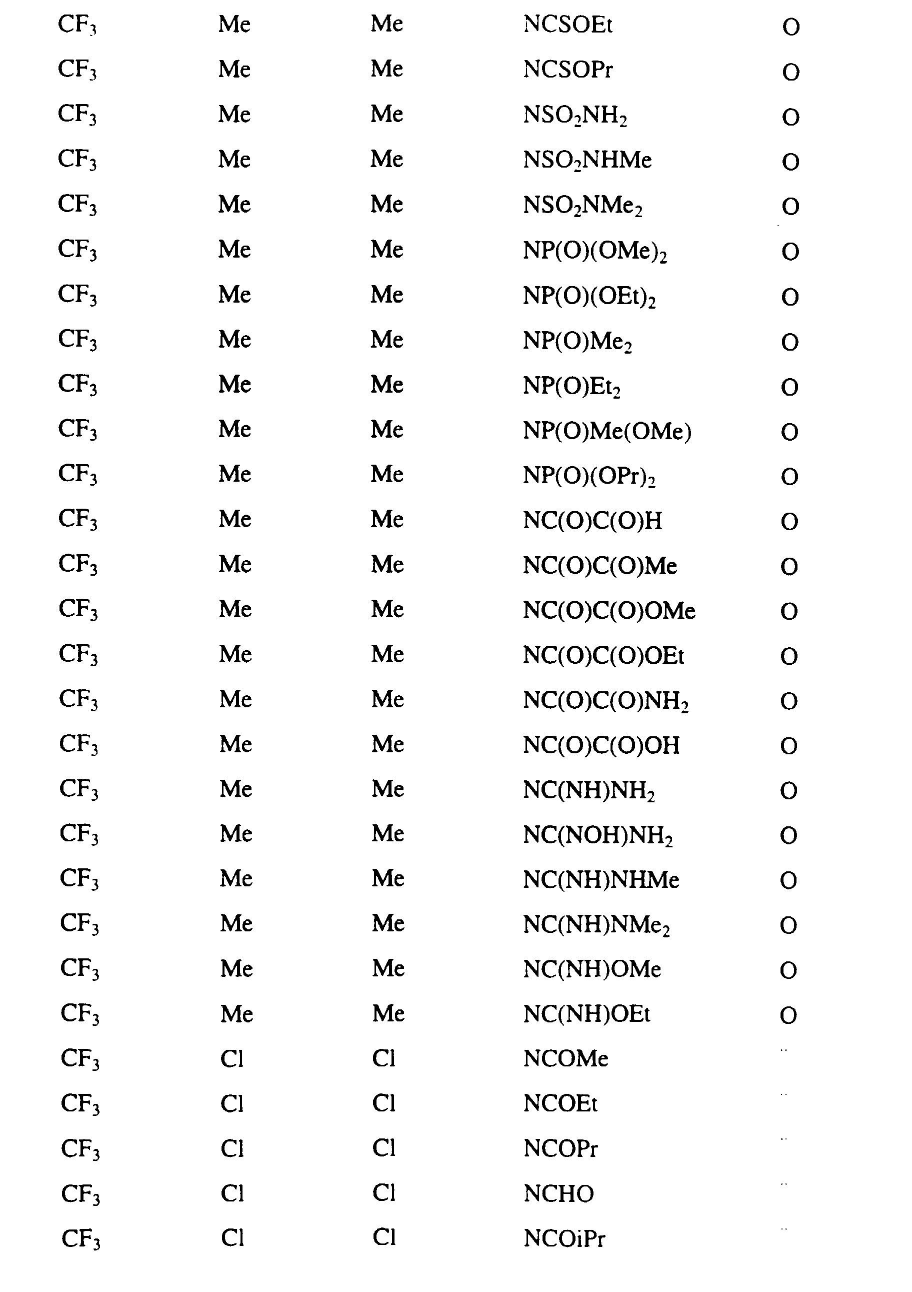

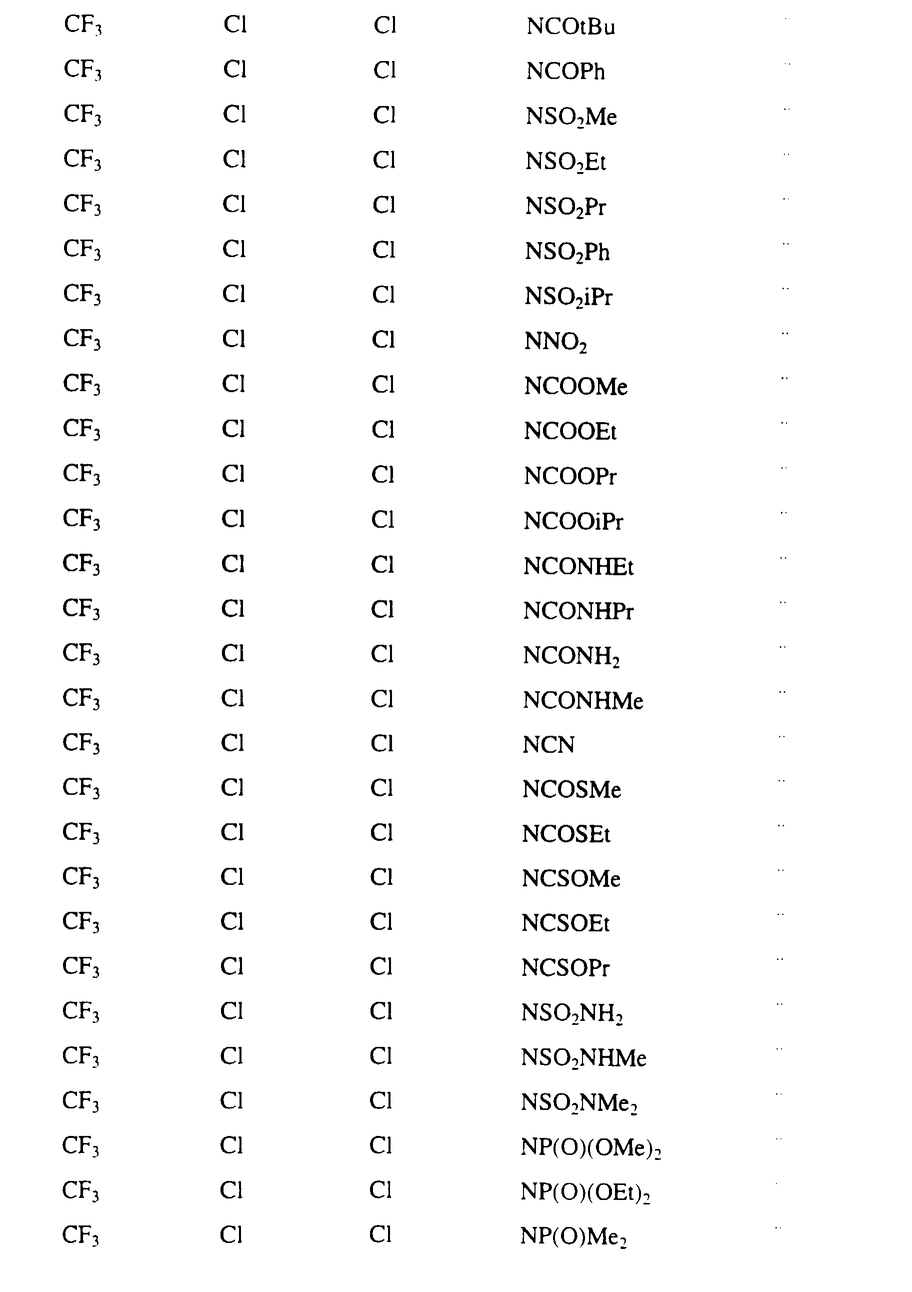

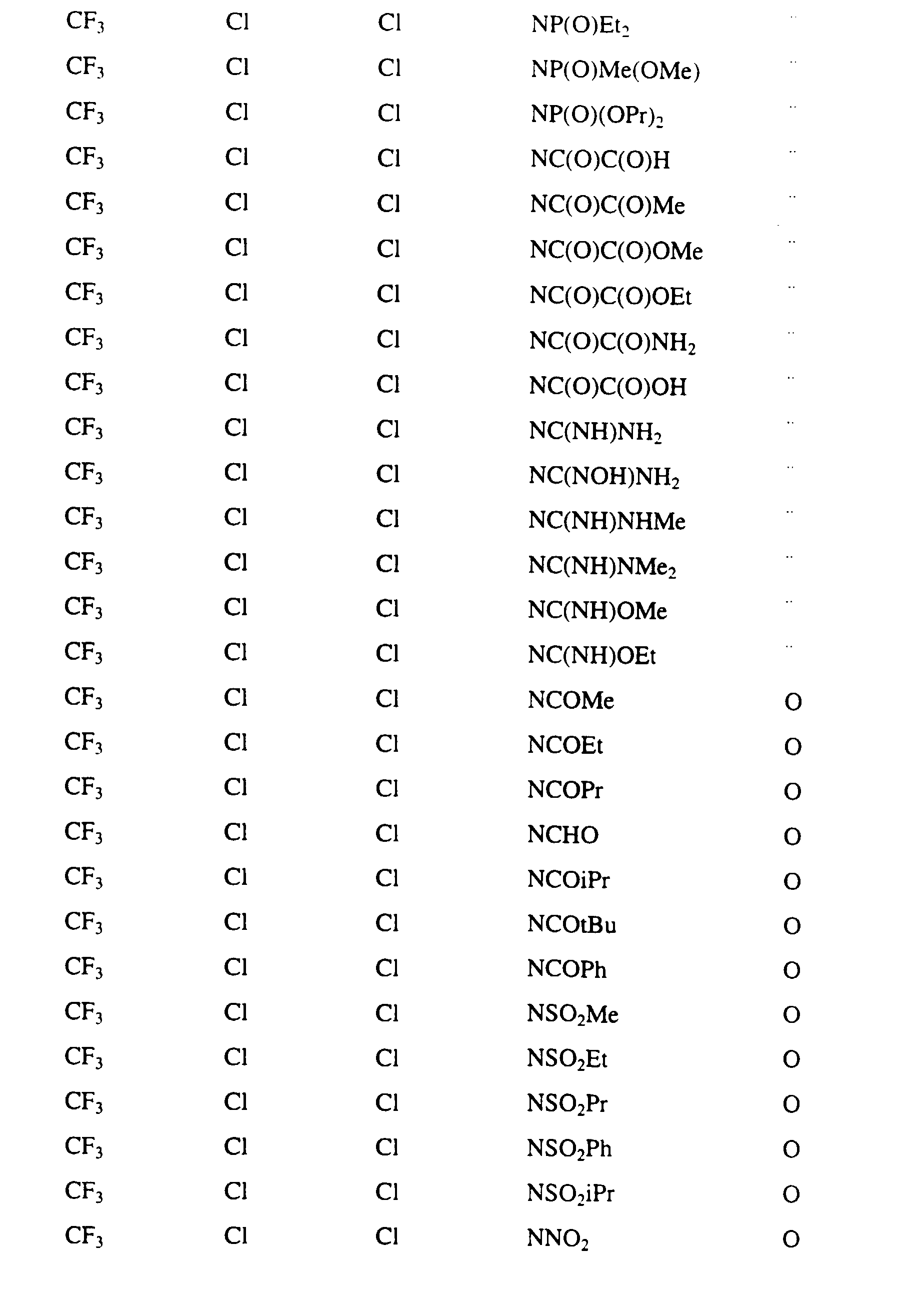

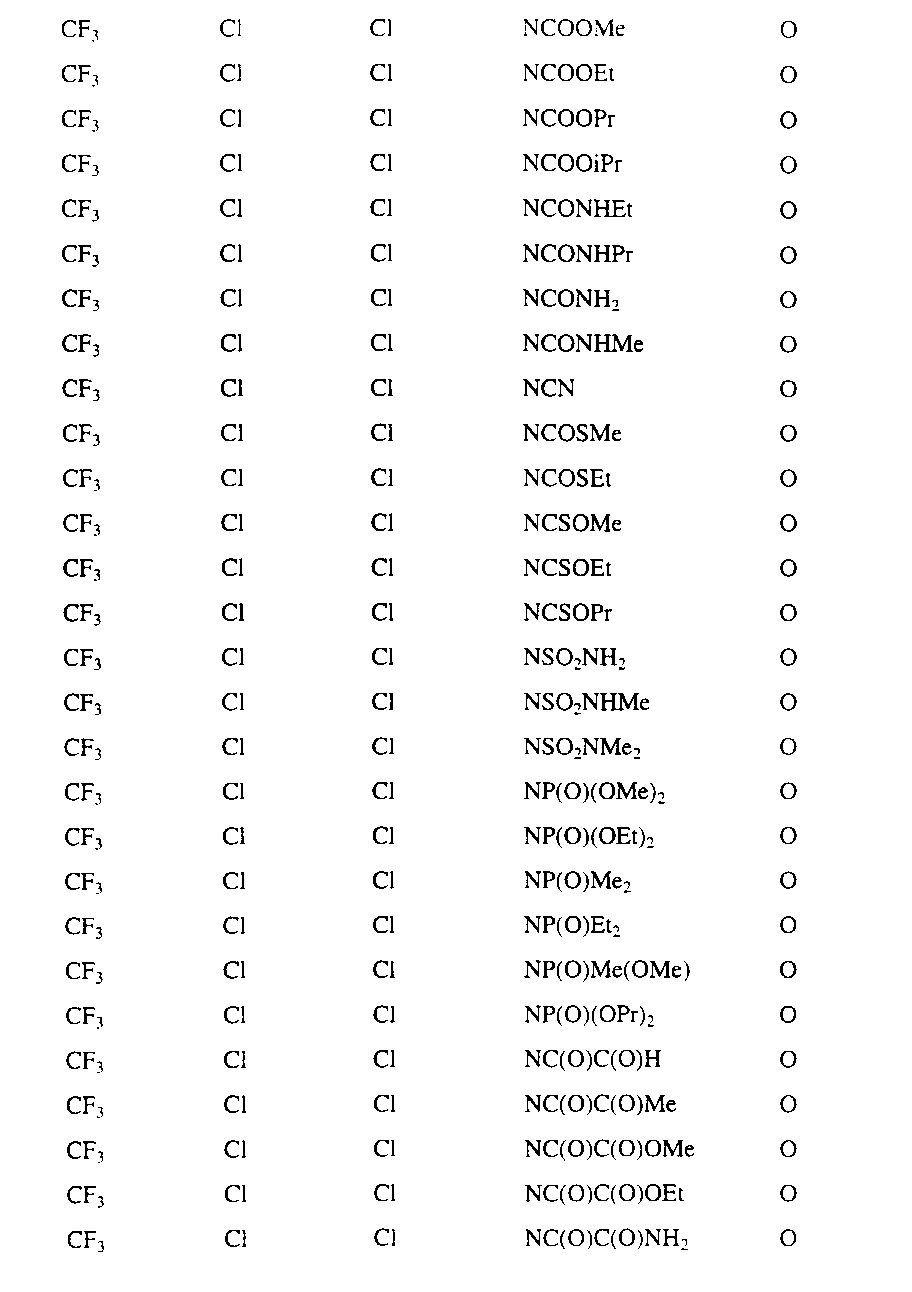

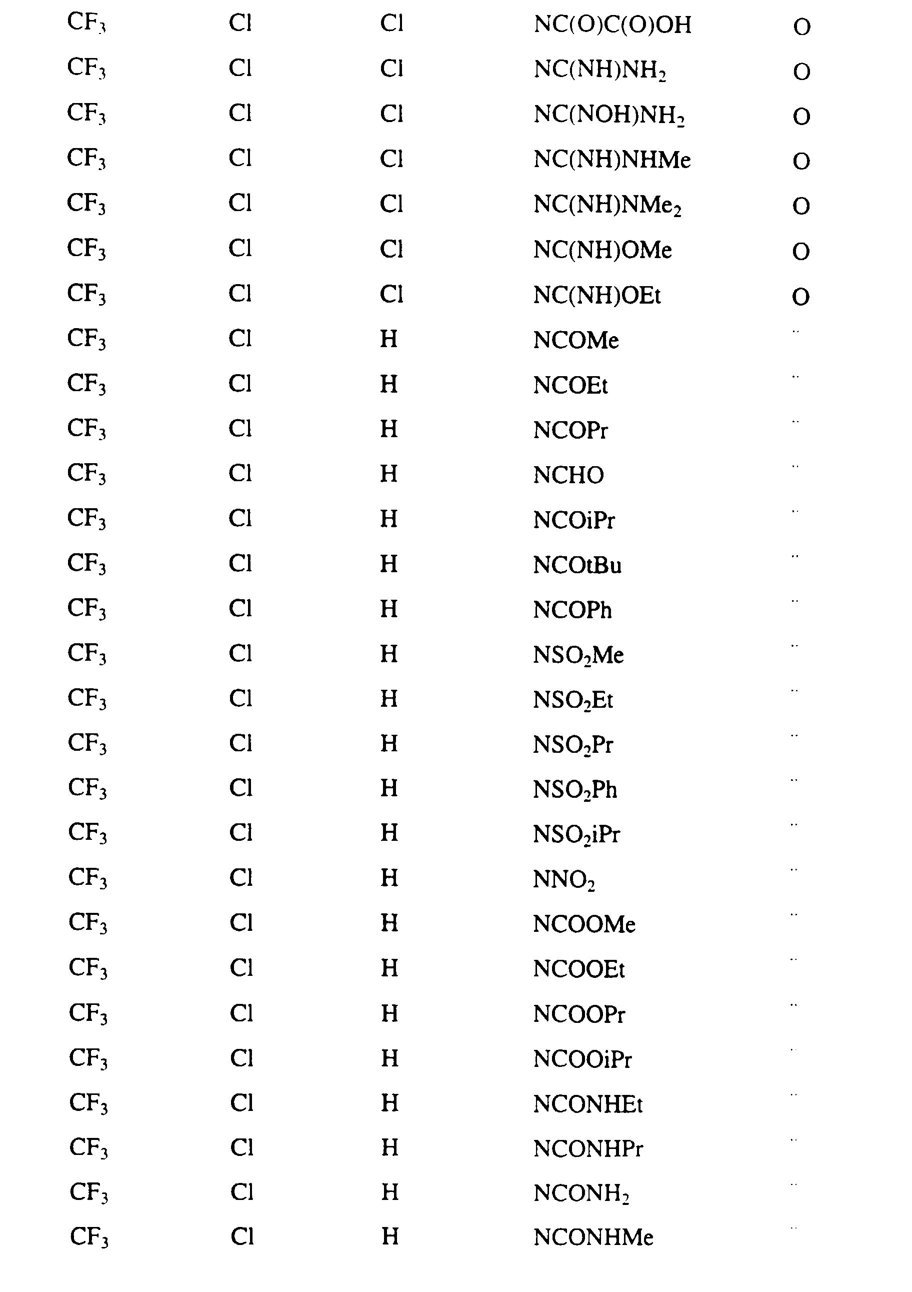

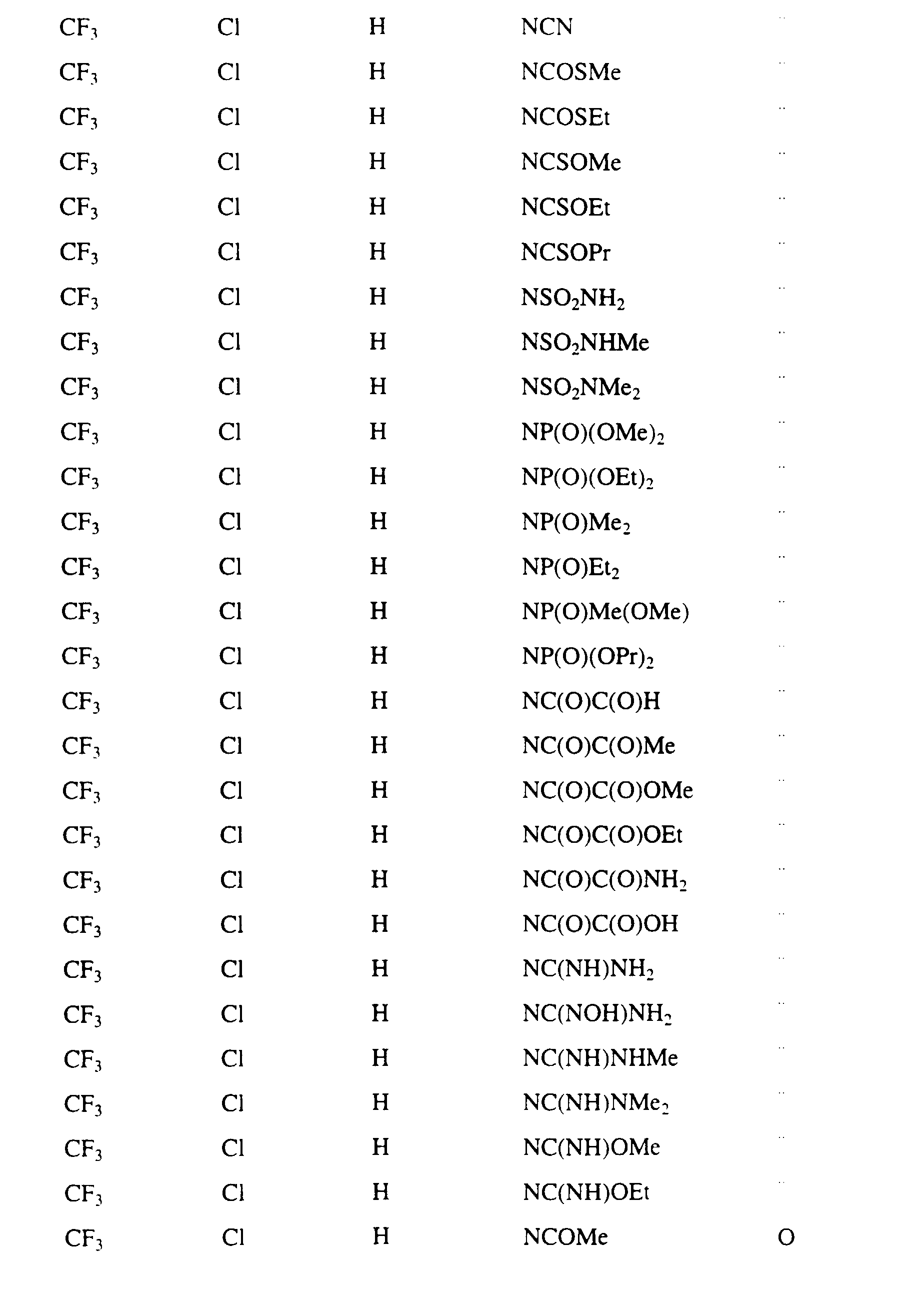

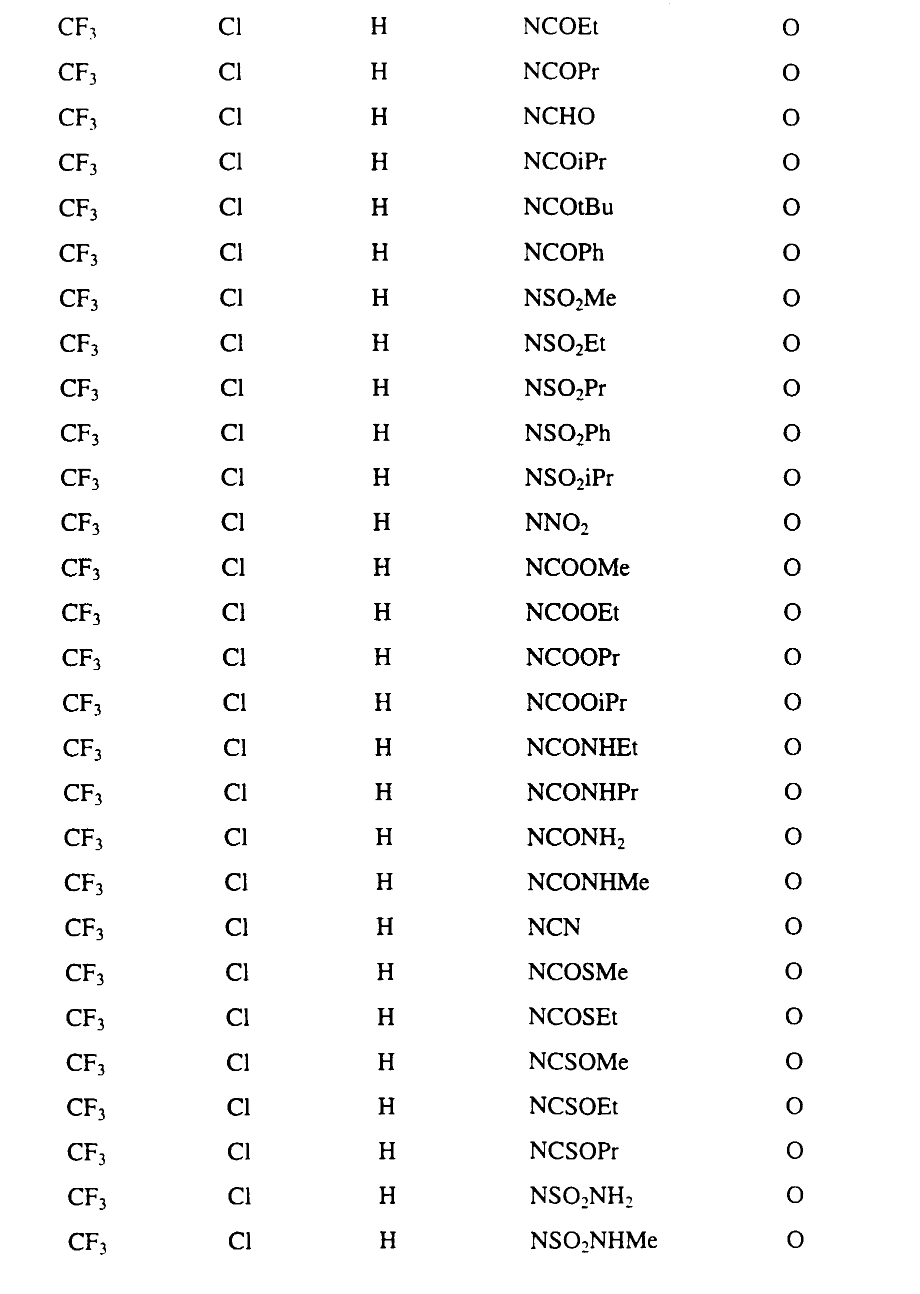

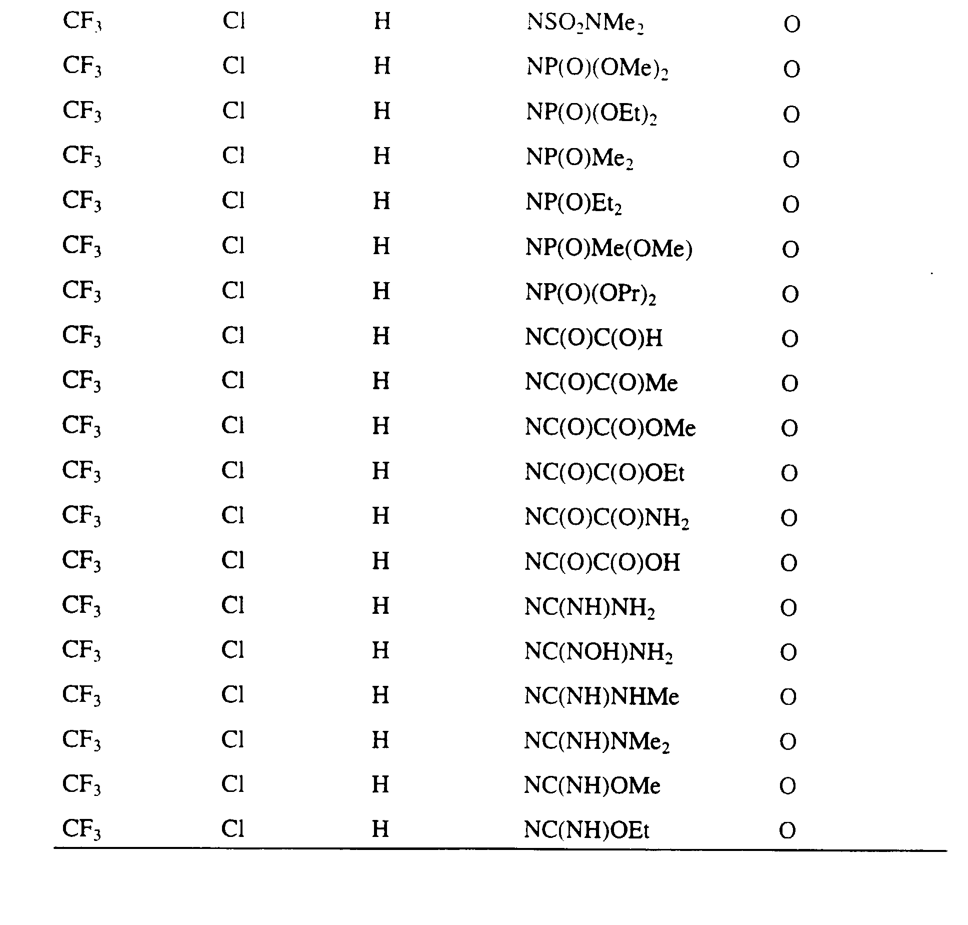

### COMPOSITIONS AND METHODS OF USE

The present invention provides pesticidal compositions and methods of controlling arthropod, nematode, helminth and protozoan pests, using the compounds of formulas (I) and (II), as set forth in the Summary of the Invention hereinabove. In a preferred aspect, the present invention provides a method for controlling arthropods or nematodes at a locus comprising applying to said locus an arthropodicidally or nematocidally effective amount of a compound of either formula (I) or formula (II) or a composition comprising said compound and an agriculturally acceptable inert carrier therefor. In a more preferred embodiment, the arthropods whose control is desired are insects, and to that end the invention provides a method for controlling insects at a locus comprising applying to said locus an insecticidally effective amount of a compound of either formula (I) or (II), or an insecticidally effective amount of an insecticidal composition comprising an insecticidally effective amount of a compound of either formula (I) or (II) and an agriculturally acceptable inert carrier therefor. In another preferred embodiment, control of nematodes is desired, and to that end the invention provides a method of controlling nematodes at a locus comprising applying to said locus a nematocidally effective amount of a compound of either formula (I) or (II), or a nematocidally effective amount of a nematocidal composition comprising a nematocidally effective amount of a compound of either formula (I) or (II) and an agriculturally acceptable inert carrier therefor. Preferably, the locus to which the arthropodicidally (especially insecticidally) or nematocidally effective amount is applied is a crop-growing area, that is, an area in which a crop is growing or in which a crop has been planted, or an area in which a crop will be planted/grown.

The compositions which can be used in the invention for the pesticidal, particularly the arthropodicidal (especially insecticidal) or nematocidal, treatment of the invention can comprise from about 0.001 to about 95% of the active ingredient of either formula (I) or (II). The term "active ingredient of either formula (I) or (II)" or "active ingredient" as used herein refers to a compound of either formula (I) or (II) or salt thereof. The expression "compound of either formula (I) or (II)" is also used herein to mean the compound or its salt.

The diluted liquid formulations, as applied to the locus to be treated or crop, generally comprise from about 0.001 to about 3% of active ingredient of either formula (I) or (II), preferably from about 0.1 to about 0.5%.

The solid formulations as applied to the locus or crop generally comprise from about 0.1 to about 8% of active ingredient of either formula (I) or (II), preferably from about 0.5 to about 1.5%.

The concentrated compositions are compositions which are commercialized or transported or stored. For application to plants, they are normally diluted in water and applied in such diluted form. The diluted forms are part of the invention as well as the concentrated forms.

The concentrated formulations generally comprise from about 5 to about 95% of active ingredient of either formula (I) or (II), preferably from about 10 to about 50%.

The insecticidal compositions of the invention can be applied once, or more than once, throughout the whole insect season. Insecticidal compositions according to the invention are usually applied to the locus to be treated or crop area at a rate of from about 0.04 to about 2 kg/ha of active ingredient, preferably from about 0.1 to about 1 kg/ha.

The concentrated insecticidal compositions according to the invention can be in the form of a solid, *e.g.,* dusts or granules or wettable powders, or, preferably, in the form of a liquid, such as an emulsifiable concentrate or a true solution.

The compositions according to the instant invention generally comprise from about 0.5 to about 95% of active ingredient. The remainder of the composition up to 100% comprises a carrier as well as various additives such as those hereafter indicated.

By "carrier", there is meant herein an organic or inorganic material, which can be natural or synthetic, and which is associated with the active ingredient and which facilitates its application to the locus to be treated or crop. This carrier is thus generally inert and should be agriculturally acceptable, especially on the contemplated or treated locus or crop. The carrier can be solid (clay, silicates, silica, resins, wax, fertilizers, *etc.)* or liquid (water, alcohols, ketones, oil solvents, saturated or unsaturated hydrocarbons, chlorinated hydrocarbons, liquified petroleum gas, *etc.*)*.*

Among the many additives, the compositions of the invention can comprise surfactants as well as other ingredients such as dispersants, stickers, antifoam agents, antifreezing agents, dyestuffs, thickeners, adhesives, protective colloids, penetrating agents, stabilizing agents, sequestering agents, antiflocculating agents, corrosion inhibitors, pigments and polymers.

More generally, the compositions of the invention can comprise all kinds of solid or liquid additives which are known in the art of insecticides and insecticidal treatments.

The surfactants can be of the emulsifying or wetting type, ionic or non-ionic. Possible surfactants are salts of polyacrylic or lignosulfonic acids; salts of phenolsulfonic or naphthalenesulfonic acids; polycondensates of ethylene oxide with fatty alcohols or fatty acids or fatty amines or substituted phenols (particularly alkylphenols or arylphenols); ester-salts of sulfosuccinic acids; taurine derivatives such as alkyl taurates; phosphoric esters; or esters of alcohols or polyoxyethylated phenols. When the spraying vehicle is water, the use of at least one surfactant is generally required because the active ingredients are not water-soluble.

The method of application of the compositions of the invention is generally the spraying of a mixture which has been previously made by dilution of more concentrated formulations according to the invention.

Solid compositions can be powders for dusting or for dispersion (wherein the content of active ingredient can be up to 100%) and granules, especially extruded or compacted granules, or granules which have been made by impregnation of a powder (the content of active ingredient in such powders can then be between about 1 and about 80%).

Liquid compositions or compositions which have to be liquid when applied include solutions, water-soluble concentrates, emulsifiable concentrates, emulsions, wettable powders or pastes or water-dispersible granules.

Emulsifiable concentrates generally comprise from about 10 to about 80% of active ingredient; the emulsions when applied generally comprise from about 0.01 to about 20% of active ingredient.

For example, the emulsifiable concentrates can comprise the solvent and, to the extent needed, from about 2 to about 20% of suitable additives as stabilizers, surfactants, penetrating agents, corrosion inhibitors or other additives already recited.

These concentrates are usually diluted in tank water so as to obtain the dilution appropriate for spraying.

The concentrated suspensions can also be applied by spraying and have to be fluid without allowing any solid to separate and fall to the bottom. Generally they comprise from about 1 to about 75% of active ingredient (preferably from about 2 to about 50%), from about 0.5 to about 15% of surfactants, from about 0.1 to about 10% of thickener, and from 0 to about 10% of other suitable additives as already indicated, the remainder being water or an organic liquid wherein the active ingredient is insoluble or has a low solubility.

The wettable powders generally comprise the active ingredient (from about 1 to about 95%, preferably from about 2 to about 80%), the solid carrier, a wetting agent (from 0 to about 5%), a dispersing agent (from about 3 to about 10%) and, to the extent needed, from 0 to about 10% of other additives such as stabilizers and others as already listed.

In order to obtain these wettable powders or dusting powders, it is appropriate to intimately mix the active ingredients and the additives, as by grinding in a mill or similar device.

Dispersible granules are generally made by agglomeration of a powder followed by an appropriate granulation process.

The emulsions herein described can be of the oil-in-water or water-in-oil types. Fluidity of the emulsions can range from low viscosities up to high viscosities approaching those of gels.

Among these many compositions or formulations, one skilled in the art can choose the one most appropriate, according to the specific conditions of the treatment problem.

The compounds and compositions of the invention can also be used in admixtures with another pesticide *e.g*., an insecticide, acaricide or herbicide.

The invention is further illustrated by the following examples which are not considered as limiting the invention but are given to better enable use of it.

### BIOLOGICAL EFFICACY

The following representative methods were used to apply the compounds of the invention and to observe the biological activity obtained therefrom: a soil drench on aphid-infested plants; a bait application on flies.

The species used were as follows:

| **GENUS, SPECIES** | **COMMON NAME** |
|---|---|
| *Aphis gossypii* | cotton leaf aphid |
| *Schizaphis graminum* | greenbug |
| *Musca domestica* | housefly |

### The Soil Drench Test (systemic activity: aphids)

Cotton and sorghum plants were established in pots. One day prior to treatment, each pot was infested with about 25 aphids of a mixed population. Cotton plants were infested with aphids and sorghum plants were infested with the greenbug. The selected compound of formula (I) or formula (II) was applied to the soil surface in a dilution that delivered the equivalent of 10.0 ppm soil concentration by weight. Aphid counts were obtained at 5 DAT (*i.e.* days after treatment). The number of aphids on the treated plants was compared to the number of those on the untreated control plants. Compounds that showed greater than 30% mortality on either aphid or greenbug were the following: 1a, 1b, 3-29, 30a, 31-32

### The Housefly Bait/Contact Test

About 25 four to six-day-old adult houseflies *(Musca domestica)* were anesthetized and placed in a cage with a sugar water bait solution containing the compound. The compound concentration in the bait solution was 100 ppm. After 24 hours, flies which showed no movement on stimulation were considered dead. Compounds that showed greater than 30% mortality on housefly were the following: 1, 1a, 1b, 2, 3-10, 12-14, 16, 18-20, 22, 25, 26, 28-30, 30a, 31, 32

While the invention has been described in terms of various preferred embodiments, the skilled artisan will appreciate that various modifications, substitutions, omissions and changes can be made without departing from the spirit thereof. Accordingly, it is intended that the scope of the present invention be limited solely by the scope of the following claims, including equivalents thereof.

## Claims

1. A compound of Formula (I) or Formula (II): 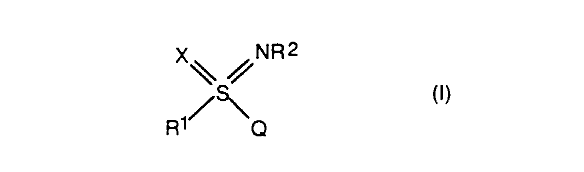 or 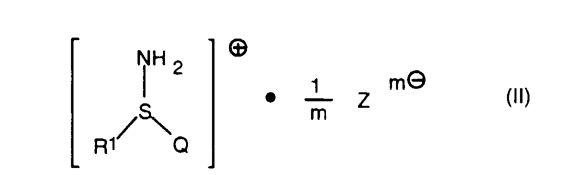 wherein:
=X is =NR³, =O or an electron pair;
R¹ is C₁-C₆ alkyl, C₃-C₆ cycloalkyl, or C₄-C₈ (cycloalkyl)alkyl, each of which is optionally substituted by one or more halogen;
R² and R³ are independently selected from H; C₁-C₆ alkyl; C₁-C₆ haloalkyl; COR⁴; S(O)ₚR⁴; CN; NO₂; COOR⁴; CONR⁴R⁵; C(O)SR⁴; C(S)OR⁴; SO₂NR⁴R⁵; P(O)_{q}(R⁴)(R⁵); P(O)_{q}(OR⁴)(R⁵); P(O)_{q}(OR⁴)(OR⁵); C=(NR⁴)NR⁵R⁶; CH=NR⁴; C=(NR⁴)(OR⁵); C(S)N(R⁴)(R⁵); C(O)C(O)R⁴; C(O)C(O)OR⁴; C(O)C(O)NR⁴R⁵; and CONR⁴SO₂R⁵;
m is 1 or 2;
p is 0, 1 or 2;
q is 0 or 1;
R⁴, R⁵ and R⁶ are independently selected from H; NO₂; CN; CHO; R¹⁴; phenyl optionally substituted by one or more of R¹⁴, halogen, CN, NO₂, OR¹⁴, SR¹⁴, COR¹⁴, COOR¹⁴, or OR¹⁴; halogen; COR¹⁴; COOR¹⁴; CHO; and OH;
Q is Q-1, Q-2 or Q-3 as designated below: 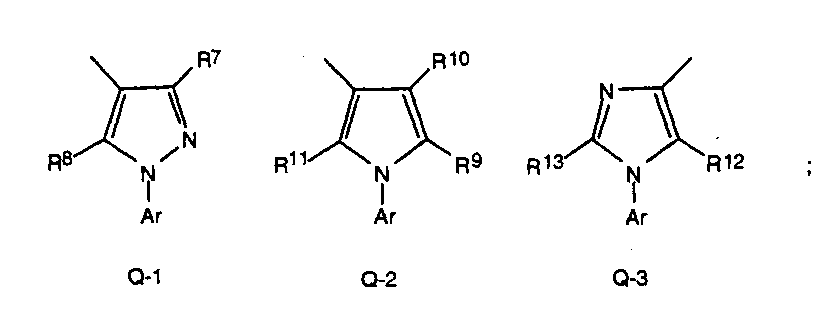
R⁷ is CN, NO₂, H, C₁-C₄ alkyl, C₁-C₄ haloalkyl, halogen, CHO, or COR²⁰;
R⁸ is H, halogen, CN, S(O)ₚR¹⁴, OR⁴, NR¹⁶R¹⁷, N(R¹⁶)CON(R¹⁷)(R¹⁸), N₃ or NH(C₁-C₅ alkyl) substituted by one or more OH, OR¹⁴, S(O)ₚR¹⁴, CN, NO₂, COOR¹⁴ or CON(R¹⁶)(R¹⁷);
R⁹ and R¹¹ are independently selected from H, halogen, OR¹⁴, SR¹⁴ and R¹⁴;
R¹⁰ is CN;
R¹² is H, halogen, R¹⁴, OR¹⁴, S(O)ₚR¹⁴ or 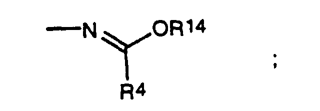
R¹³ is H, halogen or R¹⁴;
Ar is phenyl, optionally bearing one or more substituents selected from the group consisting of halogen, R¹⁵, OR¹⁵, SF₅ and S(O)ₚR¹⁵; or Ar is 2-pyridyl, optionally bearing one or more substituents selected from the group consisting of halogen, R¹⁵, OR¹⁵, SF₅ and S(O)ₚR¹⁴;
R¹⁴ and R¹⁹ are independently selected from C₁-C₆ alkyl, C₃-C₆ cycloalkyl, and C₄-C₈ (cycloalkyl)alkyl, each of which is optionally substituted by one or more halogen;
R¹⁵ is C₁-C₆ alkyl, optionally substituted by one or more halogen;
R¹⁶, R¹⁷ and R¹⁸ are independently selected from H, NO₂, CN, CHO, R¹⁹, COR¹⁹, and COOR¹⁹;
R²⁰ is selected from C₁-C₆ alkyl, optionally substituted by one or more halogen; and
Z is an anionic counter ion, such as Cl⁻, Br⁻, I⁻, F⁻, OSO₂R⁴⁻, ClO₄⁻, OCOR⁴⁻, BF₄⁻, SbF₆⁻, SO₃⁻⁻ or HSO₄⁻, a phosphate anion or a hydrogenophosphate anion or other agriculturally acceptable anion.

2. A compound according to Claim 1 wherein R¹ is optionally halogenated C₁-C₆ alkyl, preferably methyl or ethyl.

3. A compound according to Claim 1 wherein R² is H, COOR⁴, S(O)ₚR⁴, CONR⁴R⁵, CN, COR⁴, P(O)_{q}(OR⁴)(OR⁵) or P(O)_{q}(R⁴)(R⁵); more preferably R² is H; or CN; or COOR⁴ (in which R⁴ is C₁-C₆ alkyl); or R² is S(O)ₚR⁴ (in which p is two and R⁴ is C₁-C₆ alkyl or optionally substituted phenyl); or R² is CONR⁴R⁵ (in which R⁴ is H or C₁-C₆ alkyl and R⁵ is C₁-C₆ alkyl, or in which R⁴ is H and R⁵ is COR¹⁴ in which R¹⁴ is optionally halogenated C₁-C₆ alkyl); or R² is COR⁴ (in which R⁴ is H or C₁-C₆ alkyl); or R² is P(O)_{q}(OR⁴)(OR⁵) or P(O)_{q}(R⁴)(R⁵) (in which q is one and R⁴ and R⁵ are each C₁-C₆ alkyl).

4. A compound according to Claim 1 wherein Q is Q-1, more preferably wherein R⁷ is CN or H, or C₁-C₄ alkyl and/or when R⁸ is N(R¹⁶)(R¹⁷).

5. A compound according to Claim 4 wherein R⁸ is NH₂

6. A compound according to Claim 1 wherein Ar is phenyl substituted in the 2 and 6 positions by halogen and in the 4 position by halogenated C₁-C₆ alkyl or SF₅ or OR¹⁵, preferably when Ar is 2,6-dichloro-4-trifluoromethylphenyl; or Ar is 2-pyridyl substituted in the 3 position by halogen and in the 5 position by halogenated C₁-C₆ alkyl or SF₅ or OR¹⁵, preferably when Ar is 3-chloro-5-trifluoromethylpyrid-2-yl.

7. The compound according to claim 1 which is:
2,4,6-Trimethylbenzenesulfonic acid, compound with S-4-[5-amino-3-cyano-1-(2,6-dichloro-4-trifluoromethyl)phenyl]pyrazolyl-S-ethylsulfilimine ;
2,4,6-Trimethylbenzene-sulfonic acid, compound with S-4-[5-amino-3-cyano-1-(2,6-dichloro-4-trifluoromethyl)phenyl]pyrazolyl-S-methylsulfilimine;
2,4,6-Trimethylbenzenesulfonic acid, compound with S-4-[5-amino-1-(2,6-dichloro-4-trifluoromethyl)phenyl]pyrazolyl-S-methylsulfilimine;
2,4,6-Trimethylbenzenesulfonic acid, compound with S-4-[5-amino-1-(2,6-dichloro-4-trifluoromethyl)phenyl-3-methyl]pyrazolyl-S-ethylsulfilimine;
2,4,6-Trimethylbenzenesulfonic acid, compound with S-4-[5-amino-1-(2,6-dichloro-4-trifluoromethyl)phenyl-3-ethyl]pyrazolyl-S-ethylsulfilimine;
S-{[5-Amino-3-cyano-1-(2,6-dichloro-4-tfifluoromethyl)phenyl]-4-(1H-pyrazolyl)-S-ethyl-N-(tert-butoxycarbonyl)sulfilimine;
S-{[5-Amino-3-cyano-1-(2,6-dichloro-4-trifluoromemyl)phenyl]-4-(1H-pyrazolyl)}-S-ethyl-N-acetylsulfilimine;
S-{[5-Amino-3-cyano-1-(2,6-dichloro-4-trifluoromethyl)phenyl]-4-(1H-pyrazolyl)}-S-ethyl-N-(methoxycarbonyl)sulfilimine;
S-{[5-Amino-3-cyano-1-(2,6-dichloro-4-trifluoromethyl)phenyl]-4-(1H-pyrazolyl)}-S-ethyl-N-(methylsulfonyl)sulfilimine;
S-{[5-Amino-3-cyano-1-(2,6-dichloro-4-trifluoromethyl)phenyl]-4-(1H-pyrazolyl)}-S-ethyl-N-(methylcarbamoyl)sulfilimine;
S-{[5-Amino-3-cyano-1-(2,6-dichloro-4-trifluoromethyl)phenyl]-4-(1H-pyrazolyl)}-S-ethyl-N-(cyano)sulfilimine;
S-{[5-Amino-3-cyano-1-(2,6-dichloro-4-trifluoromethyl)phenyl]-4-(1H-pyrazolyl)}-S-ethyl-N-(formyl)sulfilimine;
S-{[5-Amino-3-cyano-1-(2,6-dichloro-4-trifluoromethyl)phenyl]-4-(1H-pyrazolyl)}-S-methyl-N-acetylsulfilimine;
S-{[5-Amino-3-cyano-1-(2,6-dichloro-4-trifluoromethyl)phenyl]-4-(1H-pyrazolyl)}-S-ethyl-N-(diethylphosphoryl)sulfilimine;
S-{[5-Amino-3-cyano-1-(2,6-dichloro-4-trifluoromethyl)phenyl]-4-(1H-pyrazolyl)}-S-ethyl-N-(dimethylcarbamoyl)sulfilimine;
S-{[5-Amino-3-cyano-1-(2,6-dichloro-4-trifluoromethyl)phenyl]-4-(1H-pyrazolyl)}-S-ethyl-N-(dimethylphosphinyl)sulfilimine;
S-{[5-Amino-3-cyano-1-(2,6-dichloro-4-trifluoromethyl)phenyl]-4-(1H-pyrazolyl)}-S-ethyl-N-((trichloroacetylamino)carbonyl)sulfilimine;
S-{[5-Amino-3-cyano-1-(2,6-dichloro-4-trifluoromethyl)phenyl]-4-(1H-pyrazolyl)}-S-ethyl-N-((chloroacetylamino)carbonyl)sulfilimine;
S-{[5-Amino-3-cyano-1-(2,6-dichloro-4-trifluoromethyl)phenyl]-4-(1H-pyrazolyl)}-S-ethyl-N-(methylthiocarbamoyl)sulfilimine;
S-{[5-Amino-3-cyano-1-(2,6-dichloro-4-trifluoromethyl)phenyl]-4-(1H-pyrazolyl)}-S-ethyl-N-((ethoxycarbonylmethyl)aminocarbonyl)sulfilimine;
S-{[5-Amino-3-cyano-1-(2,6-dichloro-4-trifluoromethyl)phenyl]-4-(1H-pyrazolyl)}-S-methyl-N-(methylthiocarbamoyl)sulfilimine;
S-{[5-Amino-3-cyano-1-(2,6-dichloro-4-trifluoromethyl)phenyl]-4-(1H-pyrazolyl)}-S-ethyl-N-(ethoxycarbonylaminocarbonyl)sulfilimine;
S-{[5-Amino-3-cyano-1-(2,6-dichloro-4-trifluoromethyl)phenyl]-4-(1H-pyrazolyl)}-S-methyl-N-(formyl)sulfilimine;
S-{[5-Amino-3-cyano-1-(2,6-dichloro-4-trifluoromethyl)phenyl]-4-(1H-pyrazolyl)}-S-methyl-N-(cyano)sulfilimine;
S-{[5-Amino-3-cyano-1-(2,6-dichloro-4-trifluoromethyl)phenyl]-4-(1H-pyrazolyl)}-S-methyl-N-(1,2-dioxopropyl)sulfilimine;
S-{[5-Amino-3-cyano-1-(2,6-dichloro-4-trifluoromethyl)phenyl]-4-(1H-pyrazolyl)}-S-methyl-N-(tert-butoxycarbonyl)sulfilimine;
S-{[5-Amino-1-(2,6-dichloro-4-trifluoromethyl)phenyl]-4-(1H-pyrazolyl)}-S-methyl-N-(formyl)sulfilimine;
S-{[5-Amino-1-(2,6-dichloro-4-trifluoromethyl)phenyl]-4-(1H-pyrazolyl)}-S-methyl-N-(acetyl)sulfilimine;
S-{[5-Amino-1-(2,6-dichloro-4-trifluoromethyl)phenyl]-4-(1H-pyrazolyl)}-S-methyl-N-(cyano)sulfilimine;
S-{[5-Amino-1-(2,6-dichloro-4-trifluoromethyl)phenyl-3-methyl]-4-(1H-pyrazolyl)-S-ethyl-N-(formyl)sulfilimine;
S-{[5-Amino-1-(2,6-dichloro-4-trifluoromethyl)phenyl-3-methyl]-4-(1H-pyrazolyl)}-S-ethyl-N-(cyano)sulfilimine;
S-{[5-Amino-1-(2,6-dichloro-4-trifluoromethyl)phenyl-3-ethyl]-4-(1H-pyrazolyl)}-S-ethyl-N-(formyl)sulfilimine;
S-{[5-Amino-1-(2,6-dichloro-4-trifluoromethyl)phenyl-3-ethyl]-4-(1H-pyrazolyl)}-S-ethyl-N-(cyano)sulfilimine;
N-tert-Butoxycarbonyl)-S-4-[5-amino-3-cyano-1-(2,6-dichloro-4-trifluoromethyl)phenylpyrazolyl]-S-ethylsulfoximine ;N-Acetyl-S-4-[5-amino-3-cyano-1-(2,6-dichloro-4-trifluoromethyl)phenylpyrazolyl]-S-ethylsulfoximine ;S-4-[5-Amino-3-cyano-1-(2,6-dichloro-4-trifluoromethyl)phenylpyrazolyl]-S-ethylsulfoximine ;S-[5-Amino-3-cyano-1-(2,6-dichloro-4-trifluoromethyl)phenyl-4-( 1 H-pyrazolyl)]-S-methyl-N-[(4-methylphenyl)sulfonyl]sulfilimine;
S-4-[5-Amino-3-cyano-1-(2-(3-chloro-5-trifluoromethyl)pyridyl)pyrazolyl]-S-ethylsulfoxide

8. A pesticidal composition comprising a pesticidally effective amount of a compound according to any one of the foregoing claims and an agriculturally acceptable inert carrier.

9. A method for controlling arthropod, nematode, helminths or protozoan pests at a locus, said method comprising applying to said locus a pesticidically effective amount of a compound or a composition according to any one of the foregoing claims.

## Patentansprüche

1. Verbindung der Formel (I) oder Formel (II): 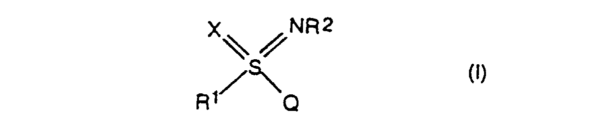 oder 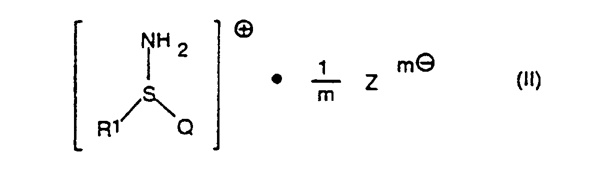 in welchen:
=X =NR³, =O oder ein Elektronenpaar ist;
R¹ C₁-C₆-Alkyl, C₃-C₆-cycloalkyl oder C₄-C₈-(cycloalkyl)alkyl, von denen jedes gegebenenfalls durch ein oder mehrere Halogenatome substituiert ist, ist;
R² und R³ unabhängig voneinander ausgewählt sind aus H; C₁-C₆-Alkyl; C₁-C₆-Halogenalkyl; COR⁴; S(O)ₚR⁴; CN, NO₂, COOR⁴; CONR⁴R⁵; C(O)SR⁴; C(S)OR⁴; SO₂NR⁴R⁵ ; P(O)_{q}(R⁴) (R⁵); P(O)_{q}(OR⁴) (R⁵); P(O)_{q}(OR⁴) (OR⁵); C=(NR⁴)NR⁵R⁶; CH=NR₄; C=(NR⁴) (OR⁵); C(S)N(R⁴) (R⁵); C(O)C(O)R⁴; C(O)C(O)OR⁴; C(O)C(O)NR⁴R⁵; und CONR⁴SO₂R⁵ ;
m 1 oder 2 ist;
p 0, 1 oder 2 ist;
q 0 oder 1 ist;
R⁴, R⁵ und R⁶ unabhängig voneinander ausgewählt sind aus H; NO₂, CN; CHO; R¹⁴, Phenyl, gegebenenfalls substituiert durch ein oder mehrere von R¹⁴, Halogen, CN, NO₂, OR¹⁴, SR¹⁴ , COR¹⁴, COOR¹⁴ oder OR¹⁴ ; Halogen; COR¹⁴; COOR¹⁴; CHO; und OH;
Q Q-1, Q-2 oder Q-3 ist, wie nachfolgend angegeben: 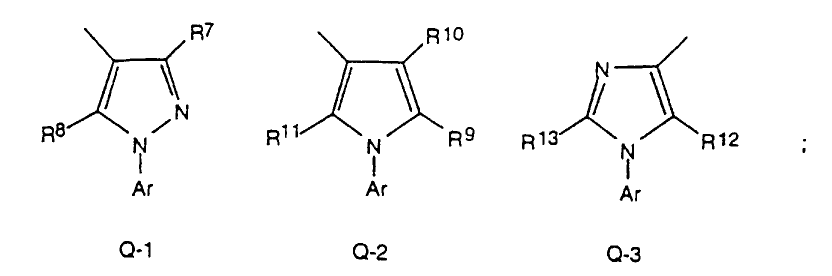
R⁷ CN, NO₂, H, C₁-C₄-Alkyl, C₁-C₄-Halogenalkyl, Halogen, CHO oder COR²⁰ ist;
R⁸ H, Halogen, CN, S(O)ₚR¹⁴, OR⁴, NR¹⁶R¹⁷, N(R¹⁶)CON(R¹⁷) (R¹⁸), N₃ oder NH(C₁-C₅-alkyl), substituiert durch ein oder mehrere OH, OR¹⁴, S(O)ₚR¹⁴, CN, NO₂, COOR¹⁴ oder CON(R¹⁶) (R¹⁷), ist;
R⁹ und R¹¹ unabhängig voneinander ausgewählt sind aus H, Halogen, OR¹⁴, SR¹⁴ und R¹⁴;
R¹⁰ CN ist,
R¹² H, Halogen, R¹⁴, OR¹⁴, S(O)ₚR¹⁴ oder 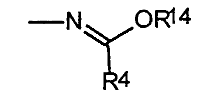 ist;
R¹³ H, Halogen oder R¹⁴ ist;
Ar Phenyl, das gegebenenfalls einen oder mehrere Substituenten, ausgewählt aus der Gruppe, bestehend aus Halogen, R¹⁵, OR¹⁵, SF₅ und S(O)ₚR¹⁵, trägt, ist; oder Ar 2-Pyridyl, das gegebenenfalls einen oder mehrere Substituenten, ausgewählt aus der Gruppe, bestehend aus Halogen, R¹⁵, OR¹⁵, SF₅ und S(O)ₚR¹⁴, trägt, ist;
R¹⁴ und R¹⁹ unabhängig voneinander ausgewählt sind aus C₁-C₆-Alkyl, C₃-C₆-cycloalkyl und C₄-C₈-(Cycloalkyl)alkyl, von welchen jedes gegebenenfalls durch ein oder mehrere Halogenatome substituiert ist;
R¹⁵ C₁-C₆-Alkyl, gegebenenfalls substituiert durch ein oder mehrere Halogenatome, ist;
R¹⁶, R¹⁷ und R¹⁸ unabhängig voneinander ausgewählt sind aus H, NO₂, CN, CHO, R¹⁹, COR¹⁹ und COOR¹⁹;
R²⁰ ausgewählt ist aus C₁-C₆-Alkyl, gegebenenfalls substituiert durch ein oder mehrere Halogenatome; und
Z ein anionisches Gegenion, wie Cl⁻, Br⁻, I⁻, F⁻, OSO₂R^{4'}, ClO₄⁻, OCOR₄⁻, BF₄⁻, SbF₆⁻, SO₃⁻⁻ oder HSO₄⁻, ein Phosphatanion oder ein Hydrogenphosphatanion oder ein anderes landwirtschaftlich annehmbares Anion ist.

2. Verbindung nach Anspruch 1, wobei R¹ gegebenenfalls halogeniertes C₁-C₆-Alkyl, vorzugsweise Methyl oder Ethyl ist.

3. Verbindung nach Anspruch 1, wobei R² H, COOR⁴, S(O)ₚR⁴, CONR⁴R⁵, CN, COR⁴, P(O)_{q}(OR⁴) (OR⁵) oder P(O)_{q}(R⁴) (R⁵) ist; R² mehr bevorzugt H oder CN oder COOR⁴ (worin R⁴ C₁-C₆-Alkyl ist) ist; oder R² S(O)ₚR⁴ (worin p zwei ist und R4 C₁-C₆-Alkyl oder gegebenenfalls substituiertes Phenyl ist) ist; oder R² CONR⁴R⁵ (worin R⁴ H oder C₁-C₆-Alkyl ist und R⁵ C₁-C₆-Alkyl ist oder worin R⁴ H ist und R⁵ COR¹⁴, worin R¹⁴ gegebenenfalls halogeniertes C₁-C₆-Alkyl ist,) ist; oder R² COR⁴ (worin R⁴ H oder C₁-C₆-Alkyl ist) ist; oder R² P(O)_{q}(OR⁴) (OR⁵) oder P(O)_{q}(R⁴)(R⁵) (worin q eins ist und R⁴ und R⁵ jeweils C₁-C₆-Alkyl sind) ist.

4. Verbindung nach Anspruch 1, worin Q Q-1 ist, mehr bevorzugt worin R⁷ CN oder H oder C₁-C₄-Alkyl ist und/oder wobei R⁸ N(R¹⁶)(R¹⁷) ist.

5. Verbindung nach Anspruch 4, worin R⁸ NH₂ ist.

6. Verbindung nach Anspruch 1, worin Ar Phenyl, das in den Positionen 2 und 6 durch Halogen und in der Position 4 durch halogeniertes C₁-C₆-Alkyl oder SF₅ oder OR¹⁵ substituiert ist, ist, vorzugsweise wobei Ar 2,6-Dichlor-4-trifluormethylphenyl ist; oder Ar 2-Pyridyl, das in der Position 3 durch Halogen und in der Position 5 durch halogeniertes C₁-C₆-Alkyl oder SF₅ oder OR¹⁵ substituiert ist, ist, vorzugsweise wobei Ar 3-Chlor-5-trifluormethylpyrid-2-yl ist.

7. Verbindung nach Anspruch 1, die:
2,4,6-Trimethylbenzolsulfonsäure, kombiniert mit S-4-[5-Amino-3-cyan-1-(2,6-dichlor-4-trifluormethyl)phenyl]pyrazolyl-S-ethylsulfilimin;
2,4,6-Trimethylbenzolsulfonsäure, kombiniert mit S-4-[5-Amino-3-cyan-1-(2,6-dichlor-4-trifluormethyl)phenyl]pyrazolyl-S-methylsulfilimin;
2,4,6-Trimethylbenzolsulfonsãure, kombiniert mit S-4-[5-Amino-1-(2,6-dichlor-4-trifluormethyl)phenyl]pyrazolyl-S-methylsulfilimin;
2,4,6-Trimethylbenzolsulfonsäure, kombiniert mit S-4-[5-Amino-1-(2,6-dichlor-4-trifluormethyl)phenyl-3-methyl]pyrazolyl-S-ethylsulfilimin;
2,4,6-Trimethylbenzolsulfonsäure, kombiniert mit S-4-[5-Amino-1-(2,6-dichlor-4-trifluormethyl)phenyl-3-ethyl]pyrazolyl-S-ethylsulfilimin;
S-{[5-Amino-3-cyan-1-(2,6-dichlor-4-trifluormethyl)phenyl]-4-(1H-pyrazolyl)}-S-ethyl-N-(tert.-butoxycarbonyl)sulfilimin;
S-{[5-Amino-3-cyan-1-(2,6-dichlor-4-trifluormethyl)phenyl]-4-(1H-pyrazolyl)}-S-ethyl-N-acetylsulfilimin;
S-{[5-Amino-3-cyan-1-(2,6-dichlor-4-trifluormethyl)phenyl]-4-(1H-pyrazolyl)}-S-ethyl-N-(methoxycarbonyl)sulfilimin;
S-{[5-Amino-3-cyan-1-(2,6-dichlor-4-trifluormethyl)phenyl]-4-(1H-pyrazolyl)}-S-ethyl-N-(methylsulfonyl)sulfilimin;
S-{[5-Amino-3-cyan-1-(2,6-dichlor-4-trifluormethyl)phenyl]-4-(1H-pyrazolyl)}-S-ethyl-N-(methylcarbamoyl)sulfilimin;
S-{[5-Amino-3-cyan-1-(2,6-dichlor-4-trifluormethyl)phenyl]-4-(1H-pyrazolyl)}-S-ethyl-N-(cyan)sulfilimin;
S-{[5-Amino-3-cyan-1-(2,6-dichlor-4-trifluormethyl)phenyl]-4-(1H-pyrazolyl)}-S-ethyl-N-(formyl)sulfilimin;
S-{[5-Amino-3-cyan-1-(2,6-dichlor-4-trifluormethyl)phenyl]-4-(1H-pyrazolyl)}-S-methyl-N-acetylsulfilimin;
S-{[5-Amino-3-cyan-1-(2,6-dichlor-4-trifluormethyl)phenyl]-4-(1H-pyrazolyl)}-S-ethyl-N-(diethylphosphoryl)sulfilimin;
S-{[5-Amino-3-cyan-1-(2,6-dichlor-4-trifluormethyl)phenyl]-4-(1H-pyrazolyl)}-S-ethyl-N-(dimethylcarbamoyl)sulfilimin;
S-{[5-Amino-3-cyan-1-(2,6-dichlor-4-trifluormethyl)phenyl]-4-(1H-pyrazolyl)}-S-ethyl-N-(dimethylphosphinyl)sulfilimin;
S-{[5-Amino-3-cyan-1-(2,6-dichlor-4-trifluormethyl)phenyl]-4-(1H-pyrazolyl)}-S-ethyl-N-((trichloracetylamino)carbonyl)sulfilimin;
S-{[5-Amino-3-cyan-1-(2,6-dichlor-4-trifluormethyl)phenyl]-4-(1H-pyrazolyl)}-S-ethyl-N-((chloracetylamino)carbonyl)sulfilimin;
S-{[5-Amino-3-cyan-1-(2,6-dichlor-4-trifluormethyl)phenyl]-4-(1H-pyrazolyl)}-S-ethyl-N-(methylthiocarbamoyl)sulfilimin;
S-{[5-Amino-3-cyan-1-(2,6-dichlor-4-trifluormethyl)phenyl]-4-(1H-pyrazolyl)}-S-ethyl-N-((ethoxycarbonylmethyl)aminocarbonyl)-sulfilimin;
S-{[5-Amino-3-cyan-1-(2,6-dichlor-4-trifluormethyl)phenyl]-4-(1H-pyrazolyl)}-S-methyl-N-(methylthiocarbamoyl)sulfilimin;
S-{[5-Amino-3-cyan-1-(2,6-dichlor-4-trifluormethyl)phenyl]-4-(1H-pyrazolyl)}-S-ethyl-N-(ethoxycarbonylaminocarbonyl)sulfilimin;
S-{[5-Amino-3-cyan-1-(2,6-dichlor-4-trifluormethyl)phenyl]-4-(1H-pyrazolyl)}-S-methyl-N-(formyl)sulfilimin;
S-{[5-Amino-3-cyan-1-(2,6-dichlor-4-trifluormethyl)phenyl]-4-(1H-pyrazolyl)}-S-methyl-N-(cyan)sulfilimin;
S-{[5-Amino-3-cyan-1-(2,6-dichlor-4-trifluormethyl)phenyl]-4-(1H-pyrazolyl)}-S-methyl-N-(1,2-dioxopropyl)sulfilimin;
S-{[5-Amino-3-cyan-1-(2,6-dichlor-4-trifluormethyl)phenyl]-4-(1H-pyrazolyl)}-S-methyl-N- (tert.-butoxycarbonyl)sulfilimin;
S-{[5-Amino-1-(2,6-dichlor-4-trifluormethyl)phenyl]-4-(1H-pyrazolyl)}-S-methyl-N-(formyl) sulfilimin;
S-{[5-Amino-1-(2,6-dichlor-4-trifluormethyl)phenyl] -4-(1H-pyrazolyl)}-S-methyl-N-(acetyl)sulfilimin;
S-{[5-Amino-1-(2,6-dichlor-4-trifluormethyl)phenyl]-4-(1H-pyrazolyl)}-S-methyl-N- (cyan) sulfilimin;
S-{[5-Amino-1-(2,6-dichlor-4-trifluormethyl)phenyl-3-methyl]-4-(1H-pyrazolyl)}-S-ethyl-N-(formyl)sulfilimin;
S-{[5-Amino-1-(2,6-dichlor-4-trifluormethyl)phenyl-3-methyl]-4-(1H-pyrazolyl)}-S-ethyl-N-(cyan)sulfilimin;
S-{[5-Amino-1-(2,6-dichlor-4-trifluormethyl)phenyl-3-ethyl]-4-(1H-pyrazolyl)}-s-ethyl-N-(formyl)sulfilimin;
S-{[5-Amino-1-(2,6-dichlor-4-trifluormethyl)phenyl-3-ethyl]-4-(1H-pyrazolyl)}-S-ethyl-N-(cyan)sulfilimin;
N-(tert.-Butoxycarbonyl)-S-4-[5-amino-3-cyan-1-(2,6-dichlor-4-trifluormethyl)phenylpyrazolyl]-S-ethylsulfoximin;
N-Acetyl-S-4-[5-amino-3-cyan-1-(2,6-dichlor-4-trifluormethyl)phenylpyrazolyl]-S-ethylsulfoximin;
S-4-[5-Amino-3-cyan-1-(2,6-dichlor-4-trifluormethyl)phenylpyrazolyl]-S-ethylsulfoximin;
S-[5-Amino-3-cyan-1-(2,6-dichlor-4-trifluormethyl)phenyl-4-(1H-pyrazolyl)1-s-methyl-N-[(4-methylphenyl)sulfonyl]sulfilimin;
S-4-[5-Amino-3-cyan-1-(2-(3-chlor-5-trifluormethyl)pyridyl)pyrazolyl]-S-ethylsulfoxid ist.

8. Schädlingsvernichtende Zusammensetzung, die eine pestizid wirksame Menge einer Verbindung nach einem der vorangegangenen Ansprüche und einen landwirtschaftlich annehmbaren inerten Träger umfaßt.

9. Verfahren zum Kontrollieren von Arthropoden-, Nematoden-, Helminthen- oder Protozoenschädlingen an einem Ort, wobei das Verfahren umfaßt, auf den Ort eine pestizid wirksame Menge einer Verbindung oder einer Zusammensetzung nach einem der vorangegangenen Ansprüche auszubringen.

## Revendications

1. Composé de formule (I) ou de formule (II): 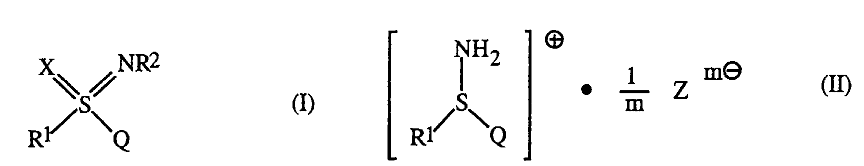 ou
dans laquelle :
=X représente =NR³, =O ou un doublet électronique ;
R¹ représente un radical alkyle en C₁-C₆, un radical cycloalkyle en C₃-C₆, ou un radical (cycloalkyl)alkyle en C₄-C₈, chacun d'entre eux étant éventuellement substitué par un ou plusieurs atomes d'halogènes ;
R² et R³ sont indépendamment choisis parmi H ; un radical alkyle en C₁-C₆ ; un radical haloalkyle en C₁-C₆ ; COR⁴ ; S(O)ₚR⁴ ; CN ; NO₂ ; COOR⁴ ; CONR⁴R⁵ ; C(O)SR⁴ ; C(S)OR⁴ ; SO₂NR⁴R⁵ ; P(O)_{q}(R⁴)(R⁵) ; P(O)_{q}(OR⁴)(R⁵) ; P(O)_{q}(OR⁴)(OR⁵) ; C=(NR⁴)NR⁵R⁶ ; CH=NR⁴ ; C=(NR⁴)(OR⁵) ; C(S)N(R⁴)(R⁵) ; C(O)C(O)R⁴ ; C(O)C(O)OR⁴ ; C(O)C(O)NR⁴R⁵ ; et CONR⁴SO₂R⁵ ;
m représente 1 ou 2 ;
p représente 0, 1 ou 2 ;
q représente 0 ou 1 ;
R⁴, R⁵ et R⁶ sont indépendamment choisis parmi H ; NO₂ ; CN ; CHO ; R¹⁴ ; phényle éventuellement substitué par un ou plusieurs radicaux R¹⁴, atomes d'halogènes, CN, NO₂, OR¹⁴, SR¹⁴, COR¹⁴, COOR¹⁴, ou OR¹⁴ ; atomes d'halogènes ; COR¹⁴ ; COOR¹⁴ ; CHO ; et OH ;
Q représente Q-1, Q-2 ou Q-3 tels que désignés ci-dessous : 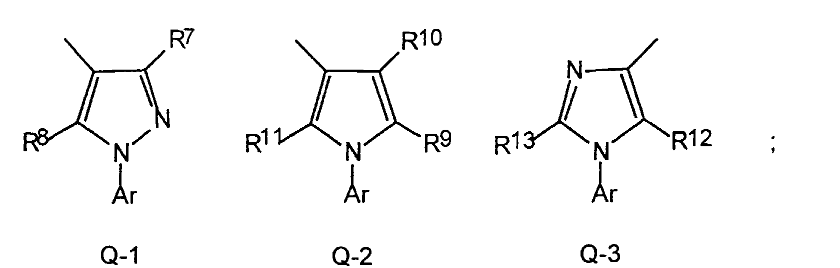
R⁷ représente CN, NO₂, H, alkyle en C₁-C₄, haloalkyle en C₁-C₄, atomes d'halogènes, CHO, ou COR²⁰ ;
R⁸ représente H, atomes d'halogènes, CN, S(O)ₚR¹⁴, OR⁴, NR¹⁶R¹⁷, N(R¹⁶)CON(R¹⁷)(R¹⁸), N₃ ou NH(alkyle en C₁-C₅) substitué par un ou plusieurs OH, OR¹⁴, S(O)ₚR¹⁴, CN, NO₂, COOR¹⁴⁻ ou CON(R¹⁶)(R¹⁷) ;
R⁹ et R¹¹ sont indépendamment choisis parmi H, atomes d'halogènes, OR¹⁴, SR¹⁴ et R¹⁴ ;
R¹⁰ représente CN ;
R¹² représente H, atomes d'halogènes, R¹⁴, OR¹⁴, S(O)ₚR¹⁴ ou 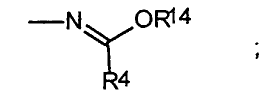
R¹³ représente H, atomes d'halogènes ou R¹⁴ ;
Ar représente un radical phényle, portant éventuellement un ou plusieurs substituants choisis parmi le groupe consistant en atomes d'halogènes, R¹⁵, OR¹⁵, SF₅ et S(O)ₚR¹⁵ ; ou bien Ar représente un radical 2-pyridyle, portant éventuellement un ou plusieurs substituants choisis parmi le groupe consistant en atomes d'halogènes, R¹⁵, OR¹⁵, SF₅ et S(O)ₚR¹⁴ ;
R¹⁴ et R¹⁹ sont indépendamment choisis parmi un radical alkyle en C₁-C₆, un radical cycloalkyle en C₃-C₆, et un radical (cycloalkyl)alkyle en C₄-C₈, chacun d'entre eux étant éventuellement substitué par un ou plusieurs atomes d'halogènes ;
R¹⁵ représente un radical alkyle en C₁-C₆, éventuellement substitué par un ou plusieurs atomes d'halogènes ;
R¹⁶, R¹⁷ et R¹⁸ sont indépendamment choisis parmi H, NO₂, CN, CHO, R¹⁹, COR¹⁹, et COOR¹⁹;
R²⁰ est choisi parmi un radical alkyle en C₁-C₆, éventuellement substitué par un ou plusieurs atomes d'halogènes ; et
Z représente un contre ion anionique, tel que Cl⁻, Br⁻, I⁻, F⁻, OSO₂R⁴⁻, ClO₄⁻, OCOR⁴⁻, BF₄⁻, SbF₆⁻, SO₃⁻⁻ ou HSO₄⁻, un anion phosphate ou un anion hydrogénophosphate ou un autre anion acceptable en agriculture.

2. Composé selon la revendication 1 dans laquelle R¹ représente un radical alkyle en C₁-C₆ éventuellement halogéné, de préférence méthyle ou éthyle.

3. Composé selon la revendication 1 dans laquelle R² représente H, COOR⁴, S(O)ₚR⁴, CONR⁴R⁵, CN, COR⁴, P(O)_{q}(OR⁴)(OR⁵) ou P(O)_{q}(R⁴)(R⁵) ; de préférence R² représente H ; ou CN ; ou COOR⁴ (dans lequel R⁴ représente un radical alkyle en C₁-C₆) ; ou R² représente S(O)ₚR⁴ (dans lequel p représente deux et R⁴ représente un radical alkyle en C₁-C₆ ou un radical phényle éventuellement substitué) ; ou R² représente CONR⁴R⁵ (dans lequel R⁴ représente H ou un radical alkyle en C₁-C₆ et R⁵ représente un radical alkyle en C₁-C₆, ou dans lequel R⁴ représente H et R⁵ représente COR¹⁴ dans lequel R¹⁴ représente un radical alkyle en C₁-C₆ éventuellement halogéné) ; ou R² représente COR⁴ (dans lequel R⁴ représente H ou un radical alkyle en C₁-C₆) ; ou R² représente P(O)_{q}(OR⁴)(OR⁵) ou P(O)_{q}(R⁴)(R⁵) (dans lesquels q représente un et R⁴ et R⁵ représentent chacun un radical alkyle en C₁-C₆).

4. Composé selon la revendication 1 dans laquelle Q représente Q-1, de préférence dans lequel R⁷ représente CN ou H, ou un radical alkyle en C₁-C₄ et/ou R⁸ représente N(R¹⁶)(R¹⁷).

5. Composé selon la revendication 4 dans laquelle R⁸ représente NH₂.

6. Composé selon la revendication 1 dans laquelle Ar représente un radical phényle substitué en positions 2 et 6 par des atomes d'halogènes et en position 4 par un radical alkyle en C₁-C₆ halogéné ou SF₅ ou OR¹⁵, de préférence Ar représente 2,6-dichloro-4-trifluorométhylphényle ; ou Ar représente un radical 2-pyridyle substitué en position 3 par un atome d'halogènes et en position 5 par un radical alkyle en C₁-C₆ halogéné ou SF₅ ou OR¹⁵, de préférence Ar représente 3-chloro-5-trifluorométhylpyrid-2-yle.

7. Composé selon la revendication 1 qui est :
Acide 2,4,6-triméthylbenzène sulfonique, composé avec S-4-[5-amino-3-cyano-1-(2,6-dichloro-4-trifluorométhyl)phényl]pyrazolyl-S-éthylsulfylimine ;
Acide 2,4,6-triméthylbenzène sulfonique, composé avec S-4-[5-amino-3-cyano-1-(2,6-dichloro-4-trifluorométhyl)phényl]pyrazolyl-S-méthylsulfylimine;
Acide 2,4,6-triméthylbenzène sulfonique, composé avec S-4-[5-amino-1-(2,6-dichloro-4-trifluorométhyl)phényl]pyrazolyl-S-méthylsulfylimine;
Acide 2,4,6-triméthylbenzène sulfonique, composé avec S-4-[5-amino-1-(2,6-dichloro-4-trifluorométhyl)phényl-3-méthyl]pyrazolyl-S-éthylsulfylimine ;
Acide 2,4,6-triméthylbenzène sulfonique, composé avec S-4-[5-amino-1-(2,6-dichloro-4-trifluorométhyl)phényl-3-éthyl]pyrazolyl-S-éthylsulfylimine ;
S-{[5-Amino-3-cyano-1-(2,6-dichloro-4-trifluorométhyl)phényl]-4-(1H-pyrazolyl)}-S-éthyl-N-(tert-butoxycarbonyl)sulfylimine;
S-{[5-Amino-3-cyano-1-(2,6-dichloro-4-trifluorométhyl)phényl]-4-(1H-pyrazolyl)}-S-éthyl-N-acétylsulfylimine;
S-{[5-Amino-3-cyano-1-(2,6-dichloro-4-trifluorométhyl)phényl]-4-(1H-pyrazolyl)}-S-éthyl-N-(méthoxycarbonyl)sulfylimine;
S-{[5-Amino-3-cyano-1-(2,6-dichloro-4-trifluorométhyl)phényl]-4-(1H-pyrazolyl)}-S-éthyl-N-(méthylsulfonyl)sulfylimine;
S-{[5-Amino-3-cyano-1-(2,6-dichloro-4-trifluorométhyl)phényl]-4-(1H-pyrazolyl)}-S-éthyl-N-(méthylcarbamoyl)sulfylimine;
S-{[5-Amino-3-cyano-1-(2,6-dichloro-4-trifluorométhyl)phényl]-4-(1H-pyrazolyl)}-S-éthyl-N-(cyano)sulfylimine;
S-{[5-Amino-3-cyano-1-(2,6-dichloro-4-trifluorométhyl)phényl]-4-(1H-pyrazolyl)}-S-éthyl-N-(formyl)sulfylimine;
S-{[5-Amino-3-cyano-1-(2,6-dichloro-4-trifluorométhyl)phényl]-4-(1H-pyrazolyl)}-S-méthyl-N-acétylsulfylimine;
S-{[5-Amino-3-cyano-1-(2,6-dichloro-4-trifluorométhyl)phényl]-4-(1H-pyrazolyl)}-S-éthyl-N-(diéthylphosphoryl)sulfylimine;
S-{[5-Amino-3-cyano-1-(2,6-dichloro-4-trifluorométhyl)phényl]-4-(1H-pyrazolyl)}-S-éthyl-N-(diméthylcarbamoyl)sulfylimine;
S-{[5-Amino-3-cyano-1-(2,6-dichloro-4-trifluorométhyl)phényl]-4-(1H-pyrazolyl)}-S-éthyl-N-(diméthylphosphinyl)sulfylimine;
S-{[5-Amino-3-cyano-1-(2,6-dichloro-4-trifluorométhyl)phényl]-4-(1H-pyrazolyl)}-S-éthyl-N-((trichloroacétylamino)carbonyl)sulfylimine;
S-{[5-Amino-3-cyano-1-(2,6-dichloro-4-trifluorométhyl)phényl]-4-(1H-pyrazolyl)}-S-éthyl-N-((chloroacétylamino)carbonyl)sulfylimine;
S-{[5-Amino-3-cyano-1-(2,6-dichloro-4-trifluorométhyl)phényl]-4-(1H-pyrazolyl)}-S-éthyl-N-(méthylthiocarbamoyl)sulfylimine;
S-{[5-Amino-3-cyano-1-(2,6-dichloro-4-trifluorométhyl)phényl]-4-(1H-pyrazolyl)}-S-éthyl-N-((éthoxycarbonylméthyl)aminocarbonyl)sulfylimine;
S-{[5-Amino-3-cyano-1-(2,6-dichloro-4-trifluorométhyl)phényl]-4-(1H-pyrazolyl)}-S-méthyl-N-(méthylthiocarbamoyl)sulfylimine;
S-{[5-Amino-3-cyano-1-(2,6-dichloro-4-trifluorométhyl)phényl]-4-(1H-pyrazolyl)}-S-éthyl-N-(éthoxycarbonylaminocarbonyl)sulfylimine;
S-{[5-Amino-3-cyano-1-(2,6-dichloro-4-trifluorométhyl)phényl]-4-(1H-pyrazolyl)}-S-méthyl-N-(formyl)sulfylimine;
S-{[5-Amino-3-cyano-1-(2,6-dichloro-4-trifluorométhyl)phényl]-4-(1H-pyrazolyl)}-S-méthyl-N-(cyano)sulfylimine;
S-{[5-Amino-3-cyano-1-(2,6-dichloro-4-trifluorométhyl)phényl]-4-(1H-pyrazolyl)}-S-méthyl-N-(1,2-dioxopropyl)sulfylimine;
S-{[5-Amino-3-cyano-1-(2,6-dichloro-4-trifluorométhyl)phényl]-4-(1H-pyrazolyl)}-S-méthyl-N-(tert-butoxycarbonyl)sulfylimine;
S-{[5-Amino-1-(2,6-dichloro-4-trifluorométhyl)phényl]-4-(1H-pyrazolyl)}-S-méthyl-N-(formyl)sulfylimine;
S-{[5-Amino-1-(2,6-dichloro-4-trifluorométhyl)phényl]-4-(1H-pyrazolyl)}-S-méthyl-N-(acétyl)sulfylimine;
S-{[5-Amino-1-(2,6-dichloro-4-trifluorométhyl)phényl]-4-(1H-pyrazolyl)}-S-méthyl-N-(cyano)sulfylimine;
S-{[5-Amino-1-(2,6-dichloro-4-trifluorométhyl)phényl-3-méthyl]-4-(1H-pyrazolyl)}-S-éthyl-N-(formyl)sulfylimine;
S-{[5-Amino-1-(2,6-dichloro-4-trifluorométhyl)phényl-3-méthyl]-4-(1H-pyrazolyl)}-S-éthyl-N-(cyano)sulfylimine;
S-{[5-Amino-1-(2,6-dichloro-4-trifluorométhyl)phényl-3-éthyl]-4-(1H-pyrazolyl)}-S-éthyl-N-(formyl)sulfylimine;
S-{[5-Amino-1-(2,6-dichloro-4-trifluorométhyl)phényl-3-éthyl]-4-(1H-pyrazolyl)}-S-éthyl-N-(cyano)sulfylimine;
N-(tert-Butoxycarbonyl)-S-4-[5-amino-3-cyano-1-(2,6-dichloro-4-trifluorométhyl)phénylpyrazolyl]-S-éthylsulfoximine ;N-Acétyl-S-4-[5-amino-3-cyano-1-(2,6-dichloro-4-trifluorométhyl)phénylpyrazolyl]-S-éthylsulfoximine ;S-4-[5-Amino-3-cyano-1-(2,6-dichloro-4-trifluorométhyl)phénylpyrazolyl]-S-éthylsulfoximine ;S-[5-Amino-3-cyano-1-(2,6-dichloro-4-trifluorométhyl)phényl-4-(1H-pyrazolyl)]-S-méthyl-N-[(4-méthylphényl)sulfonyl]sulfylimine;
S-4-[5-Amino-3-cyano-1-(2-(3-chloro-5-trifluorométhyl)pyridyl)pyrazolyl]-S-éthylsulfoxyde.

8. Composition pesticide comprenant une quantité efficace du point de vue pesticide d'un composé selon l'une quelconque des revendications précédentes et un support inerte acceptable en agriculture.

9. Méthode pour la lutte contre les arthropodes, nématodes, helminthes ou protozoaires en un lieu, la dite méthode comprenant l'application au dit lieu d'une quantité efficace du point de vue pesticide d'un composé ou d'une composition selon l'une quelconque des revendications précédentes.
